# EUROPEAN PATENT APPLICATION

(11) **EP 2 511 269 A1**
(43) Date of publication of application: **17.10.2012**
(21) Application number: 10836029.8
(22) Date of filing: 09.12.2010
(51) Int. Cl.: C07D 265/18, A61K 31/5365, A61K 31/5415, A61K 31/542, A61P 25/28, A61P 43/00, C07D 279/08, C07D 417/12, C07D 498/04, C07D 513/04

(54) **FUSED HETEROCYCLIC COMPOUND HAVING AMINO GROUP**

(30) Priority: 11.12.2009 JP 2009282193
(71) Applicant: Shionogi & Co., Ltd., Osaka 541-0045 (JP)
(72) Inventor: YOSHIDA, Syuhei, Toyonaka-shi, Osaka 561-0825 (JP); KATO, Issei, Toyonaka-shi, Osaka 561-0825 (JP); FUCHINO, Kouki, Toyonaka-shi, Osaka 561-0825 (JP); KOORIYAMA, Yuuji, Chuo-ku, Osaka-shi Osaka 541-0045 (JP); BABA, Yoshiyasu, Toyonaka-shi, Osaka 561-0825 (JP); TANIMOTO, Norihiko, Amagasaki-shi Hyogo 660-0813 (JP)
(74) Representative: Harmsen, Dirk
(86) International application number: PCT/JP2010/072121
(87) International publication number: WO 2011/071109

(57) **Abstract**

The present invention provides, for example, a compound mentioned below as a medicament for treating or preventing the diseases induced by production, secretion or deposition of amyloid-β proteins.
A compound of the formula (I): 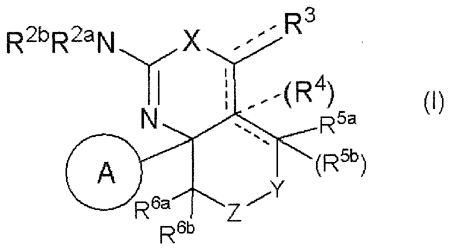 wherein R¹, R^{2a}, R^{2b}, R³, R⁴, R^{5a}, R^{5b}, R^{6a}, R^{6b}, X, Y, Z, ring A and the dashed lines are defined in the specification,
its pharmaceutically acceptable salt or a solvate thereof.

## Description

### [Technical field]

The present invention relates to a compound which has amyloid β production inhibitory activity, and is useful as an agent for treating or preventing disease induced by production, secretion and/or deposition of amyloid β protein.

### [Background Art]

In the brain ofAlzheimer's patient, the peptide composed of about 40 amino acids residue as is called amyloid β protein, that accumulates to form insoluble specks (senile specks) outside nerve cells is widely observed. It is concerned that this senile specks kill nerve cells to cause Alzheimer's disease, so the therapeutic agents for Alzheimer's disease, such as decomposition agents of amyloid β protein and amyloid vaccine, are under investigation.

Secretase is an enzyme which cleaves a protein called amyloid β precursor protein (APP) in cell and produces amyloid β protein. The enzyme which controls the production of N terminus of amyloid β protein is called as β -secretase (beta-site APP-cleaving enzyme 1, BACE1). It is thought that inhibition of this enzyme leads to reduction of producing amyloid β protein and that the therapeutic agent for Alzheimer's disease will be created due to the inhibition.

Patent Literature 1 to 15 are known as β secretase inhibitor, however, all compounds in these literatures have different structures from the present invention.

### [Prior Art Literatures]

### [Patent Literatures]

[Patent Literature 1] International Patent Application Publication WO 2007/049532
[Patent Literature 2] International Patent Application Publication WO 2008/133274
[Patent Literature 3] International Patent Application Publication WO 2008/133273
[Patent Literature 4] International Patent Application Publication WO 2009/091016
[Patent Literature 5] International Patent Application Publication WO 2006/138264
[Patent Literature 6] International Patent Application Publication WO 2005/058311
[Patent Literature 7] International Patent Application Publication WO 2009/131974
[Patent Literature 8] International Patent Application Publication WO 2009/131975
[Patent Literature 9] International Patent Application Publication WO 2009/151098
[Patent Literature 10] International Patent Application Publication WO 2010/038686
[Patent Literature 11] International Patent Application Publication WO 2010/013794
[Patent Literature 12] International Patent Application Publication WO 2010/013302
[Patent Literature 13] International Patent Application Publication WO 2008/073365
[Patent Literature 14] International Patent Application Publication WO 2008/073370

### [Summary of the invention]

### [Problems to be solved by the Invention]

The present invention provides compounds which have reducing effects to produce amyloid β protein, especially BACE1 inhibitory activity, and are useful as an agent for treating or preventing diseases induced by production, secretion and/or deposition of amyloid β protein.

### [Means to Solve the Problems]

For example, the present invention provides the following items:

### (1) a compound of formula (I):

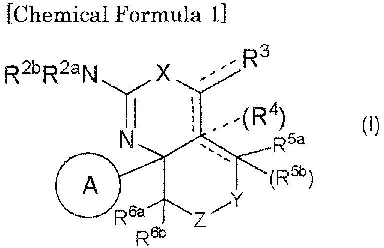

wherein -X- is -O-, -S- or -N(R¹)-,
R¹ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
ring A is a substituted or unsubstituted carbocycle or a substituted or unsubstituted heterocycle,

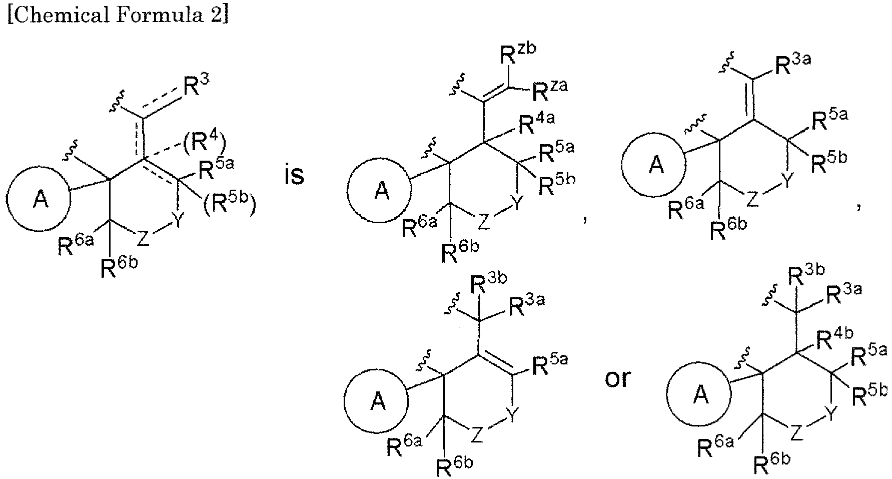

wherein -Y- is a bond, substituted or unsubstituted C1-C3 alkylene, substituted or unsubstituted C2-C3 alkenylene, -N(R⁷)-, -O-, -S-, -SO- or -SO₂-, -Z- is a bond, substituted or unsubstituted C1-C3 alkylene, substituted or unsubstituted C2-C3 alkenylene, -N(R⁷)Ak-, -OAk-, -SAk-, -SOAk- or -SO₂Ak-,
Ak is a bond, substituted or unsubstituted C1-C3 alkylene or substituted or unsubstituted C2-C3 alkenylene,
provided that when -Y- is -N(R⁷)-, -O-, -S-, -SO-or -SO₂ -, then Ak is not a bond,
R⁷ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbamoyl, substituted or unsubstituted thiocarbamoyl, substituted or unsubstituted sulfamoyl, substituted or unsubstituted amino, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R^{z a} and R^{z b} are each independently hydrogen, halogen, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl, or R^{z a} and R^{zb} together with the carbon atom to which they are attached may form a substituted or unsubstituted non-aromatic carbocycle or a substituted or unsubstituted non-aromatic heterocycle,
R^{3a} and R^{3b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl, or R^{3a} and R^{3b} together with the carbon atom to which they are attached may form a substituted or unsubstituted non-aromatic carbocycle or a substituted or unsubstituted non-aromatic heterocycle,
R^{4a} and R^{4b} are each independently hydrogen, halogen, hydroxy, cyano, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl,
provided that when -X- is -S-, then R^{4b} is not hydrogen, and
R^{5a}, R^{5b}, R^{6a} and R^{6b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl, or
R^{5a} and R^{5b} together with the carbon atom to which they are attached may form a substituted or unsubstituted carbocycle or a substituted or unsubstituted heterocycle,
R^{6a} and R^{6b} together with the carbon atom to which they are attached may form a substituted or unsubstituted carbocycle or a substituted or unsubstituted heterocycle,
its pharmaceutically acceptable salt or a solvate thereof,

### (1-1) a compound of formula (I'):

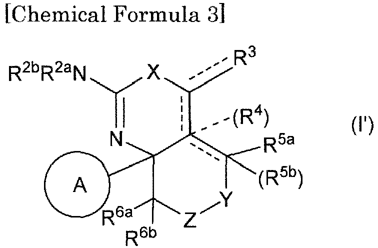

wherein -X- is -O-, -S- or -N(R¹)-,
R¹ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl,
R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
ring A is a substituted or unsubstituted carbocycle or a substituted or unsubstituted heterocycle,

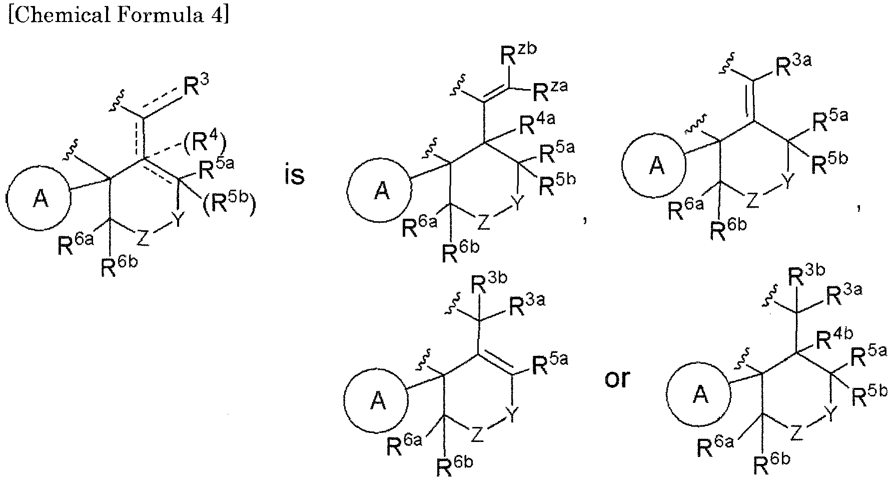

wherein -Y- is a bond, substituted or unsubstituted C1-C3 alkylene, substituted or unsubstituted C2-C3 alkenylene, -N(R⁷)-, -O-, -S-, -SO- or -SO₂ -,
-Z- is a bond, substituted or unsubstituted C1-C3 alkylene, substituted or unsubstituted C2-C3 alkenylene, -N(R⁷)Ak-, -OAk-, -SAk-, -SOAk- or -SO₂Ak-,
Ak is a bond, substituted or unsubstituted C1-C3 alkylene or substituted or unsubstituted C2-C3 alkenylene,
provided that when -Y- is -N(R⁷)-, -O-, -S-, -SO- or -SO₂-, then Ak is not a bond,
R⁷ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyl oxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbamoyl, substituted or unsubstituted thiocarbamoyl, substituted or unsubstituted sulfamoyl, substituted or unsubstituted amino, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl,
R^{za} and R^{zb} are each independently hydrogen, halogen, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted heterocyclic group, substituted or unsubstituted heterocyclyloxy, substituted or unsubstituted heterocyclylthio, or substituted or unsubstituted heterocyclyloxycarbonyl, or R^{z a} and R^{z b} together with the carbon atom to which they are attached may form a substituted or unsubstituted carbocycle or a substituted or unsubstituted heterocycle,
R^{3a} and R^{3b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfanyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl, or R^{3a} and R^{3b} together with the carbon atom to which they are attached may form a substituted or unsubstituted carbocycle or a substituted or unsubstituted heterocycle,
R^{4a} and R^{4b} are each independently hydrogen, halogen, hydroxy, cyano, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, a substituted or unsubstituted heterocyclic group, substituted or unsubstituted heterocyclyloxy,
provided that when -X- is -S-, then R^{4b} is not hydrogen, and
R^{5a}, R^{5b}, R^{6a} and R^{6b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl, or
R^{5a} and R^{5b} together with the carbon atom to which they are attached may form a substituted or unsubstituted carbocycle or a substituted or unsubstituted heterocycle,
R^{6a} and R^{6b} together with the carbon atom to which they are attached may form a substituted or unsubstituted carbocycle or a substituted or unsubstituted heterocycle,
its pharmaceutically acceptable salt or a solvate thereof, (2) the compound according to the item (1) or (1-1) wherein -X- is -O- or -S-, its pharmaceutically acceptable salt or a solvate thereof,

(3) the compound according to the item (1), (1-1) or (2) wherein

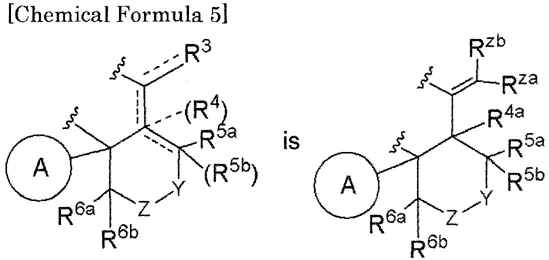

R^{za} and R^{zb} are each independently hydrogen, halogen or substituted or unsubstituted alkyl, and
R^{4a}, R^{5a}, R^{5b}, R^{6a} and R^{6b} are each independently hydrogen or substituted or unsubstituted alkyl, its pharmaceutically acceptable salt or a solvate thereof,

(4) the compound according to any one of the item (1), (1-1), (2) and (3) wherein -Y- is substituted or unsubstituted C1-C3 alkylene or -O-, and -Z- is a bond, its pharmaceutically acceptable salt or a solvate thereof,

(5) the compound according to any one of the item (1), (1-1) and (2) to (4) wherein ring A is

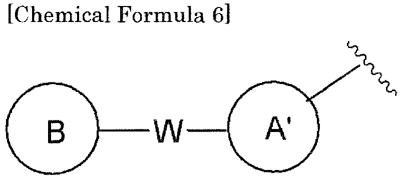

wherein ring A' and ring B are each independently a substituted or unsubstituted carbocycle or a substituted or unsubstituted heterocycle, -W- is a bond, -L¹-C(=O)N(R⁸)-L²-, -L¹-N(R⁸)C(=O)-L²- or -L¹-N(R⁸)-L²-,
L¹ and L² are each independently a bond, substituted or unsubstituted alkylene, substituted or unsubstituted alkenylene or substituted or unsubstituted alkynylene,
R⁸ is hydrogen, substituted or unsubstituted alkyl, substituted or unsubstituted alkenyl, substituted or unsubstituted alkynyl or substituted or unsubstituted acyl,
its pharmaceutically acceptable salt or a solvate thereof,

(6) the compound according to item (5) wherein -W- is -C(=O)N(R⁸)-, its pharmaceutically acceptable salt or a solvate thereof,

(7) the compound according to the item (5) or (6) wherein ring A' is substituted or unsubstituted benzene, ring B is substituted or unsubstituted pyridine, substituted or unsubstituted pyrimidine or substituted or unsubstituted pyrazine, its pharmaceutically acceptable salt or a solvate thereof,

(8) a pharmaceutical composition comprising the compound according to any one of the item (1), (1-1) and (2) to (7), its pharmaceutically acceptable salt or a solvate thereof as an active ingredient,

(9) a pharmaceutical composition having BACE1 inhibitory activity comprising the compound according to any one of the item (1), (1-1) and (2) to (7), its pharmaceutically acceptable salt or a solvate thereof as an active ingredient,
(10) the pharmaceutical composition according to the item (8) or (9), which is a medicament for treating or preventing the diseases induced by production, secretion or deposition of amyloid β proteins,
(11) the pharmaceutical composition according to the item (8) or (9), which is a medicament for treating or preventing Alzheimer's disease,
(12) a method for inhibiting BACE1 activity comprising administering the compound according to any one of the item (1), (1-1) and (2) to (7), its pharmaceutically acceptable salt or a solvate thereof,
(13) the compound according to any one of the item (1), (1-1) and (2) to (7), its pharmaceutically acceptable salt or a solvate thereof for use in a method for inhibiting BACE1 activity,
(14) a method for treating or preventing diseases induced by production, secretion or deposition of amyloid β proteins comprising administering the compound according to any one of the item (1), (1-1) and (2) to (7), its pharmaceutically acceptable salt or a solvate thereof,
(15) the compound according to any one of the item (1), (1-1) and (2) to (7), its pharmaceutically acceptable salt or a solvate thereof for use in a method for treating or preventing diseases induced by production, secretion or deposition of amyloid β proteins,
(16) a method for treating or preventing Alzheimer's disease comprising administering the compound according to any one of the item (1), (1-1) and (2) to (7), its pharmaceutically acceptable salt or a solvate thereof,
(17) the compound according to any one of the item (1), (1-1) and (2) to (7), its pharmaceutically acceptable salt or a solvate thereof for use in a method for treating or preventing Alzheimer's disease,

(18) a method, a system, an apparatus, a kit or the like for preparing the compound according to any one of the item (1), (1-1) and (2) to (7), its pharmaceutically acceptable salt or a solvate thereof,

(19) a method, a system, an apparatus, a kit or the like for preparing the pharmaceutical composition comprising the compound according to any one of the item (1), (1-1) and (2) to (7), its pharmaceutically acceptable salt or a solvate thereof,

(20) a method, a system, an apparatus, a kit or the like for use the compound according to any one of the item (1), (1-1) and (2) to (7), its pharmaceutically acceptable salt or a solvate thereof,

### [Effect of the Invention]

The compounds of the present invention are useful as an agent for treating or preventing diseases induced by production, secretion or deposition of amyloid β protein such as Alzheimer's disease.

### [Mode for Carrying Out the Invention]

As used herein, the "halogen" includes fluorine, chlorine, bromine, and iodine.

As used herein, the "alkyl" includes linear or branched alkyl of a carbon number of 1 to 15, for example, a carbon number of 1 to 10, a carbon number of 1 to 6, or a carbon number of 1 to 3. Examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, and n-decyl.

An alkyl part of the " alkoxy", the "halogenoalkyl", the "hydroxyalkyl", the "halogenoalkoxy", the "hydroxyalkoxy", the " alkoxycarbonyl", the "halogenoalkoxycarbonyl", , the " alkylamino", the "aminoalkyl", the " alkoxyalkoxy", the " alkoxyalkenyloxy", the " alkoxyalkynyl", the "alkoxyalkynyloxy", the " alkylcarbamoyl", the "hydroxyalkylcarbamoyl", the " alkoxyimino", the "alkylcarbonyl", the " alkylthio", the " alkylsulfonyl", the " alkylsulfonyloxy", the " alkylsulfonylamino", the " alkylsulfonyl alkylamino", the " alkylsulfonylimino", the " alkylsulfinylamino", the " alkylsulfinylalkylamino", the " alkylsulfinylimino", the " alkylsulfamoyl", the " alkylsulfinyl", the "carbocyclylalkyl", the "carbocyclylalkoxy", the "carbocyclylalkoxycarbonyl", the "carbocyclylalkylamino", the "carbocyclylalkylcarbamoyl", the "cycloalkylalkyl", the "cycloalkylalkoxy", the "cycloalkylalkylamino", the "cycloalkylalkoxycarbonyl", the "cycloalkylalkylcarbamoyl", the "arylalkyl", the "arylalkoxy", the "arylalkylamino", the "arylalkoxycarbonyl", the "arylalkylcarbamoyl", the "heterocyclylalkyl", the "heterocyclylalkoxy", the "heterocyclylalkylamino", the "heterocyclylalkoxycarbonyl", and the "heterocyclylalkylcarbamoyl" is the same as the above " alkyl".

The "substituted or unsubstituted alkyl" may be substituted with one or more substituents selected from a substituent group α.

As used herein, the substituent group α is a group consisting of halogen, hydroxy, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein the carbocycle and the heterocycle may be each substituted with one or more substituents selected from halogen, alkyl, hydroxyl and alkoxy.

Examples of the substituent of the "substituted or unsubstituted alkoxy", the "substituted or unsubstituted alkoxycarbonyl", "substituted or unsubstituted alkylthio", "substituted or unsubstituted alkylsulfinyl", and the "substituted or unsubstituted alkylsulfonyl" include one or more groups selected from the above substituent group α.

Examples of the "halogenoalkyl" include trifluoromethyl, fluoromethyl, and trichloromethyl.

Examples of of the "halogenoalkoxy" include trifluoromethoxy, fluoromethoxy, and trichloromethoxy.

The "alkylidene" includes a divalent group of the above "alkyl", and examples include methylidene, ethylidene, propylidene, isopropylidene, butylidene, pentylidene, and hexylidene.

The "alkenyl" includes linear or branched alkenyl of a carbon number of 2 to 15, for example, a carbon number of 2 to 10, a carbon number of 2 to 6, or a carbon number of 2 to 4, having one or more double bonds at any available position. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.

An alkenyl part of the "alkenyloxy", the "alkenyloxycarbonyl", the "alkenylcarbonyl", the "alkoxyalkenyloxy", the "alkenylthio", the "alkenylamino" , the "alkenylsulfonyl", and the "alkenylsulfinyl" is the same as the above "alkenyl".

The "alkynyl" includes linear or branched alkynyl of a carbon number of 2 to 10, for example, a carbon number of 2 to 8, or a carbon number of 3 to 6, having one or more triple bonds at any available position. Examples include ethynyl, propynyl, butynyl, pentynyl, hexynyl, heptynyl, octynyl, nonynyl, and decynyl. These may further a double bond at any available position.

An alkynyl part of the "alkoxyalkynyl", the "alkynyloxy", the "alkynyloxycarbonyl", "alkynylcarbonyl", the "alkoxyalkynyloxy", the "alkynylthio", the "alkynylsulfinyl", the "alkynylsulfonyl" and the "alkynylamino" is the same as the above "alkynyl".

Examples of the substituent of the "substituted or unsubstituted alkenyl", "substituted or unsubstituted alkenyloxy", the "substituted or unsubstituted alkenyloxycarbonyl", the "substituted or unsubstituted alkenylthio", the "substituted or unsubstituted alkenylsulfinyl", the "substituted or unsubstituted alkenylsulfonyl", the "substituted or unsubstituted alkynyl", the "substituted or unsubstituted alkynyloxy", the "substituted or unsubstituted alkynylthio", the "substituted or unsubstituted alkynyloxycarbonyl", the "substituted or unsubstituted alkynylsulfinyl" and the "substituted or unsubstituted alkynylsulfonyl" include one or more substituents selected from the above substituent group α.

Examples of the substituent of the "substituted or unsubstituted amino", "substituted or unsubstituted carbamoyl", "substituted or unsubstituted thiocarbamoyl" and the "substituted or unsubstituted sulfamoyl" include 1 to 2 substituents selected from alkyl, acyl, hydroxy, alkoxy, alkoxycarbonyl, a carbocyclic group and a heterocyclic group.

The "acyl" includes formyl, alkylcarbonyl of a carbon number of 1 to 10, alkenylcarbonyl of a carbon number of 2 to 10, alkynylcarbonyl of a carbon number of 2 to 10, carbocyclylcarbonyl and heterocyclylcarbonyl. Examples include formyl, acetyl, propionyl, butyryl, isobutyryl, valeryl, pivaloyl, hexanoyl, acryloyl, propioloyl, methacryloyl, crotonoyl, benzoyl, cyclohexanecarbonyl, pyridinecarbonyl, furancarbonyl, thiophenecarbonyl, benzothiazolecarbonyl, pyrazinecarbonyl, piperidinecarbonyl, and thiomorpholino.

An acyl part of the "acyloxy" and the "acylamino " is as described above "acyl".

Examples of the substituent of the "substituted or unsubstituted acyl" and "substituted or unsubstituted acyloxy" include one or more substituents selected from the substituent group α. In addition, a ring part of the carbocyclylcarbonyl and the heterocyclylcarbonyl may be substituted with one or more substituents selected from alkyl, the substituent group α, and alkyl substituted with one or more groups selected from the substituent group α.

The "carbocyclic group" includes cycloalkyl, cycloalkenyl, aryl and a non-aromatic fused carbocyclic group.

The "cycloalkyl" is a carbocyclic group of a carbon number of 3 to 10, for example, a carbon number of 3 to 8, or a carbon number of 4 to 8, and examples include cyclopropyl, cyclobutyl, cyclopentyl, cyclohexyl, cycloheptyl, cyclooctyl, cyclononyl, and cyclodecyl.

A cycloalkyl part of the "cycloalkylalkyl", the "cycloalkoxy", the "cycloalkylalkoxy", the "cycloalkylthio", the "cycloalkylamino", the "cycloalkylalkylamino", the "cycloalkylsulfamoyl", the "cycloalkylsulfonyl", the "cycloalkylcarbamoyl", the "cycloalkylalkylcarbamoyl", the "cycloalkylalkoxycarbonyl", and the "cycloalkoxycarbonyl" is the same as the above "cycloalkyl".

The "cycloalkenyl" includes the cycloalkyl having one or more double bonds at any available position in the ring, and examples include cyclopropenyl, cyclobutenyl, cyclopentenyl, cyclohexenyl, cycloheptynyl, cyclooctynyl and cyclohexadienyl.

The "aryl" includes phenyl, naphthyl, anthryl and phenanthryl, and specific example is phenyl.

The "non-aromatic fused carbocyclic group" includes non-aromatic groups in which two or more cyclic groups selected from the above "cycloalkyl", the above "cycloalkenyl" and the above "aryl" are fused, and examples include indanyl, indenyl, tetrahydronaphthyl and fluorenyl.

A carbocyclyl part of the "non-aromatic carbocyclic group" is the same as the "cycloalkyl", "cycloalkenyl" and "non-aromatic fused carbocyclic group". Examples are cyclopropane, cyclobutane, cyclopentane, cyclohexane, cycloheptane, cyclooctane, cyclononane, cyclodecane, cyclopropene, cyclobutene, cyclopentene, cyclohexene, cycloheptene and cyclooctene.
A carbocyclyl part of the "carbocyclic", the "carbocyclyloxy", the "carbocyclylalkyl", the "carbocyelylalkoxy", the "carbocyclylalkoxycarbonyl", the "carbocyclylthio", the "carbocyclylamino", the "carbocyclylalkylamino", the "carbocyclylcarbonyl", the "carbocyclylsulfamoyl", "carbocyclylsulfinyl", the "carbocyclylsulfonyl", the "carbocyclylcarbamoyl", the "carbocyclylalkylcarbamoyl", the "carbocyclyloxycarbonyl" and the "carbocyclylsulfonyl" is the same as the "carbocyclic group".

An aryl part of the "arylalkyl", the "aryloxy", the "aryloxycarbonyl", the "aryl alkoxycarbonyl", the "arylthio", the "arylamino", the "arylalkoxy", the "arylalkylamino", the "arylsulfonyl", the "arylsulfamoyl", the "arylcarbamoyl", and the "arylalkylcarbamoyl" is the same as the "aryl".

The "heterocyclic group" includes a heterocyclic group having one or more hetero atoms optionally selected from O, S and N in a ring, and examples include 5- to 6-membered heteroaryl such as pyrrolyl, imidazolyl, pyrazolyl, pyridyl, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl, and thiadiazolyl;
non-aromatic heterocyclic groups such as dioxanyl, thiiranyl, oxyranyl, oxetanyl, oxathioranyl, azetidinyl, thianyl, thiazolidinyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydropyridyl, tetrahydrofuryl, tetrahydropyranyl, dihydrothiazolyl, tetrahydrothiazolyl, tetrahydroisothiazolyl, dihydrooxazinyl, hexahydroazepinyl, tetrahydrodiazepinyl, and tetrahydropyridazinyl;
fused bicyclic heterocyclic groups such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzotriazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, thienopyridyl, thienopyrrolyl, thienopyrazolyl, thienopyrazinyl, furopyrrolyl, thienothienyl, imidazopyridyl, imidazopyrazolyl, pyrazolopyridyl, pyrazolopyrazinyl, thiazolopyridyl, pyrazolopyrimidinyl, pyrazolotrianidyl, pyridazolopyridyl, triazolopyridyl, imidazothiazolyl, pyrazinopyridazinyl, dihydrothiazolopyrimidinyl, tetrahydroquinolyl, tetrahydroisoquinolyl, dihydrobenzofuryl, dihydrobenzoxazinyl, dihydrobenzimidazolyl, tetrahydrobenzothienyl, tetrahydrobenzofuryl, benzodioxolyl, benzodioxonyl, chromanyl, chromenyl, octahydrochromenyl, dihydrobenzodioxinyl, dihydrobenzoxazinyl, dihydrobenzodioxepinyl, and dihydrothienodioxinyl;
tricyclic fused heterocyclic groups such as carbazolyl, acridinyl, xanthenyl, phenothiazinyl, phenoxathiinyl, phenoxazinyl, dibenzofuryl, imidazoquinolyl, and tetrahydrocarbazolyl. Example is a 5- to 6-membered heteroaryl or a non-aromatic heterocyclic group.

A heterocyclyl part of the "heterocyclylalkyl", the "heterocyclyloxy", the "heterocyclylthio", the "heterocyclycarbonyl", the "heterocyclylalkoxy", the "heterocyclylamino", the "heterocyclylsulfamoyl", the "heterocyclylsulfonyl", the "heterocyclylsulfinyl", the "heterocyclylcarbamoyl", the "heterocyclyloxycarbonyl", the "heterocyclylalkylamino", the "heterocyclylalkoxycarbonyl" and the "heterocyclylalkylcarbamoyl" is the same as the "heterocyclic group".
A heterocycle portion of "non-aromatic heterocycle" is the same as the heterocycle portion of the above "non-aromatic heterocyclyl." Specific examples are dioxane, thiirane, oxirane, oxetane, oxathiolane, azetidine, thiane, thiazolidine, pyrrolidine, pyrroline, imidazolidine, imidazoline, pyrazolidine, pyrazoline, piperidine, piperazine, morpholine, thiomorpholine, dihydropyridine, tetrahydropyridine, tetrahydrofuran, tetrahydropyran, dihydrothiazole, tetrahydrothiazole, tetrahydroisothiazole, dihydrooxazine, hexahydroazepine, tetrahydrodiazepine and tetrahydropyridazine.

A bond of the "heterocyclic group" may be situated on any ring.

The "heteroaryl" includes an aromatic cyclic group among the "heterocyclic group".

In the specification, examples of ring A are as follows:

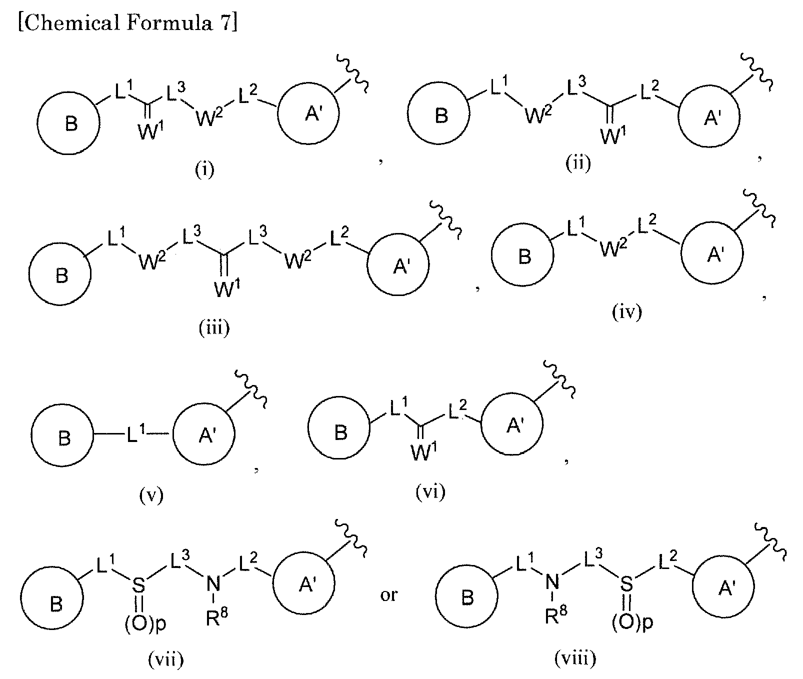

wherein ring A' and ring B are each independently substituted or unsubstituted carbocycle or substituted or unsubstituted heterocycle,
L¹, L², and L³ are each independently a bond, substituted or unsubstituted alkynylene, substituted or unsubstituted alkenylene or substituted or unsubstituted alkynylene,
= W¹ is =O, =S, or =NR⁹,
W² is O, S, or N (R⁸),
R⁸ is hydrogen, substituted or unsubstituted alkyl, substituted or unsubstituted alkenyl, substituted or unsubstituted alkynyl or substituted or unsubstituted acyl,
R⁹ is hydrogen, hydroxy, substituted or unsubstituted alkyl, substituted or unsubstituted alkenyl, substituted or unsubstituted alkynyl or substituted or unsubstituted acyl,
when ring A is (i), then the constituent carbon atom of L¹ and the constituent carbon atom of L², or th]e constituent carbon atom of L¹ and the nitrogen atom of W² may be connected with substituted or unsubstituted alkylene to form a ring,
when ring A is (ii), then the constituent carbon atom of L¹ and the constituent carbon atom of L², or the nitrogen atom of W² and the constituent atom of L² may be connected with substituted or unsubstituted alkylene to form a ring,
when ring A is (iii), then two nitrogen atoms of W² may be connected with substituted or unsubstituted alkylene to form a ring,
when ring A is (vi), then the constituent carbon atom of L¹ and the constituent carbon atom of L² may be connected with substituted or unsubstituted alkylene to form a ring,
p is 1 or 2, and
when multiple L³, multiple W², or multiple R⁹ are present, each of them may be independently different.
Specific examples are as follows:

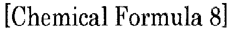

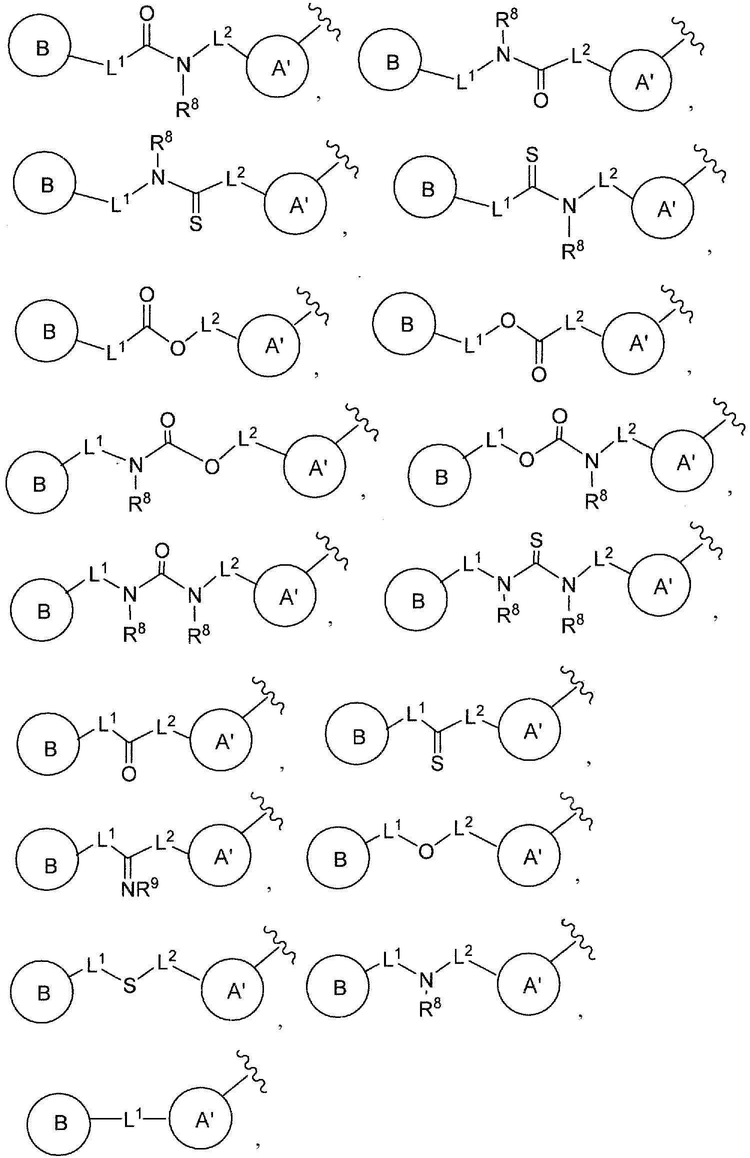

wherein each symbol is the same as defined above.

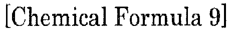

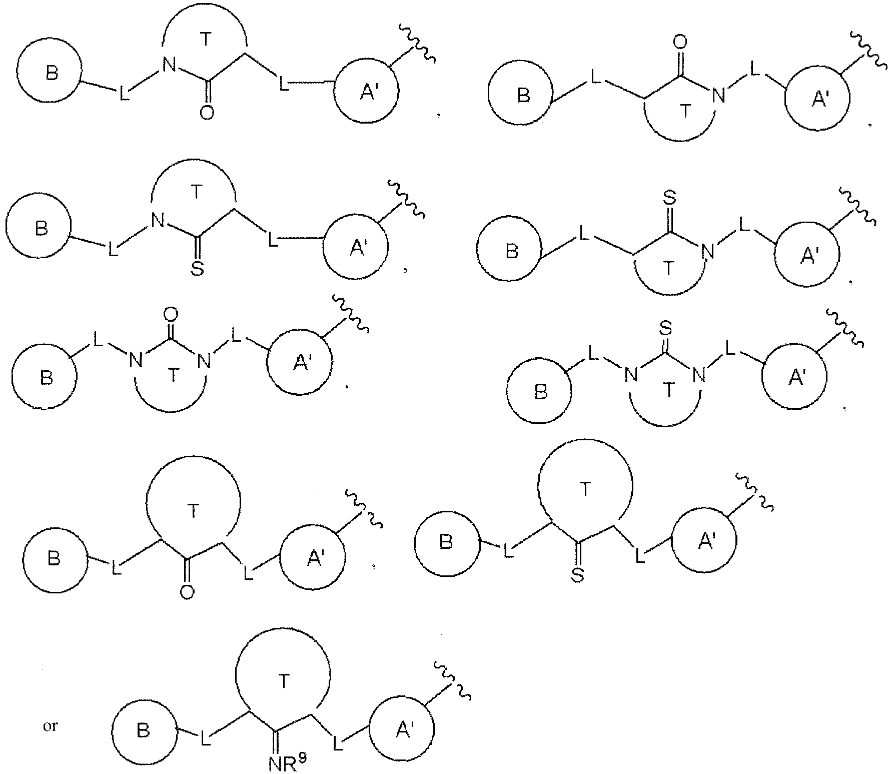

wherein L is each independently a bond, substituted or unsubstituted alkylene, substituted or unsubstituted alkenylene or substituted or unsubstituted alkynylene, ring T is a carbocycle optionally substituted with a group(s) selected from the substituent group α, or a heterocycle optionally substituted with a group(s) selected from the group α, and that and other symbols are the same as defined above.

More specific examples are as follows:

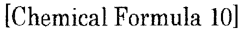

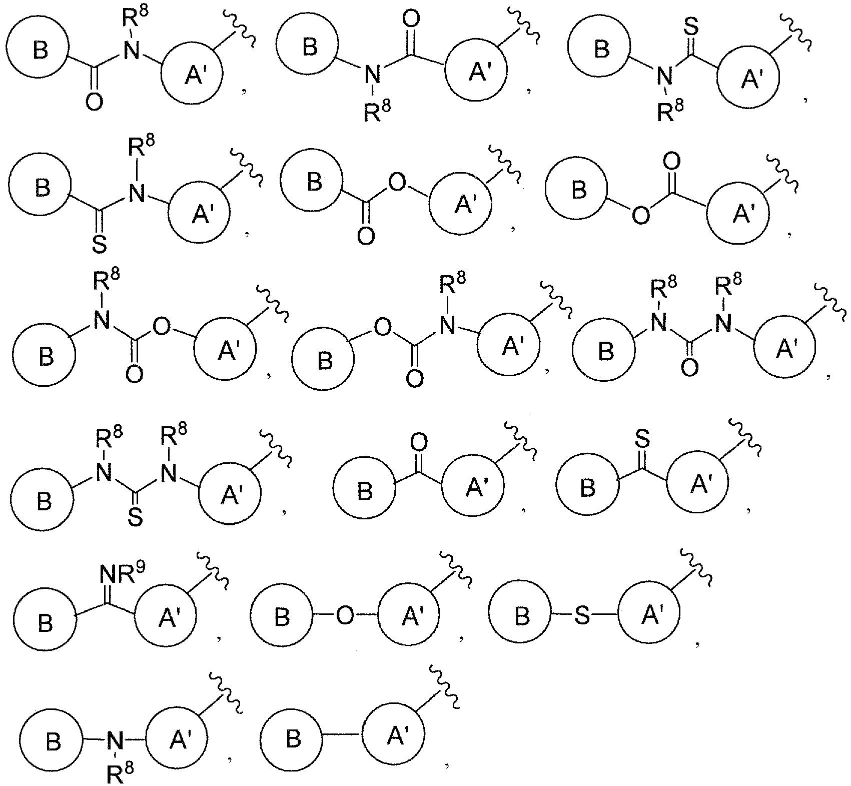

wherein each symbol is the same as defined above.

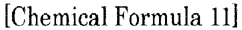

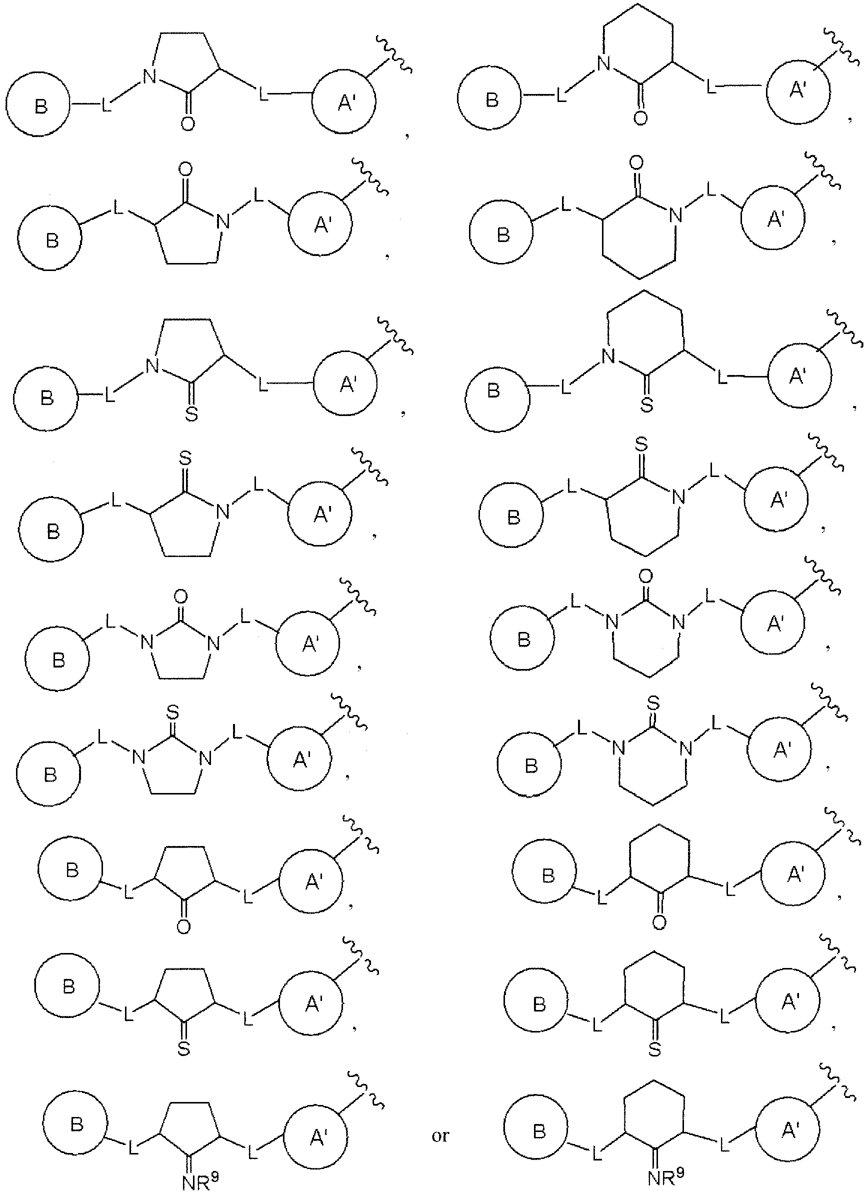

wherein each symbol is the same as defined above.

Other examples of the substituent of "substituted or unsubstituted carbocycle", "substituted or unsubstituted heterocycle" and " substituted or unsubstituted benzene" in ring A, ring A', and ring B , and " substituted or unsubstituted pyridine", "substituted or unsubstituted pyrimidine" and "substituted or unsubstituted pyrazine" in ring B include:
a group selected from the substituent group α such as halogen, hydroxy, alkoxy, acyl, acyloxy, carboxy, alkoxycarbonyl, carbamoyl, amino, cyano, alkylamino and/or alkylthio;
alkyl substituted with one or more groups selected from the substituent group α, hydroxyimino and alkoxyimino, wherein the substituent is, for example, halogen, hydroxy, alkoxy and/or alkoxycarbonyl, or unsubstituted alkyl;
aminoalkyl substituted with one or more groups selected from the substituent group a; wherein the substituent is, for example, acyl, alkyl and/or alkoxy;
alkenyl substituted with one or more substituents selected from the substituent group α, wherein the substituent is, for example, alkoxycarbonyl, halogen, and/or halogenoalkoxycarbonyl, or unsubstituted alkenyl;
alkynyl substituted with one or more substituents selected from the substituent group α, wherein the substituent is, for example, alkoxycarbonyl, or unsubstituted alkynyl;
alkoxy substituted with one or more substituents selected from the substituent group α, wherein the substituent is, for example, halogen, carbamoyl, alkylcarbamoyl and/or hydroxyalkylcarbamoyl;
alkoxyalkoxy substituted with one or more substituents selected from the substituent group α; alkenyloxy substituted with one or more substituents selected from the substituent group α, wherein the substituent is, for example, halogen, hydroxy, amino and/or alkylamino, or unsubstituted alkenyloxy;
alkoxyalkenyloxy substituted with one or more substituents selected from the substituent group α;
alkynyloxy substituted with one or more substituents selected from the substituent group α, wherein the substituent is, for example, halogen and/or hydroxy, or unsubstituted alkynyloxy;
alkoxyalkynyloxy substituted with one or more groups selected from the substituent group α; alkylthio substituted with one or more substituents selected from the substituent group α, or unsubstituted alkylthio;
alkenylthio substituted with one or more substituents selected from the substituent group α, or unsubstituted alkenylthio;
alkynylthio substituted with one or more substituents selected from the substituent group α, or unsubstituted alkynylthio;
alkylamino substituted with one or more substituents selected from the substituent group α;
alkenylamino substituted with one or more substituents selected from the substituent group α;
alkynylamino substituted with one or more substituents selected from the substituent group α; aminooxy substituted with one or more substituents selected from the substituent group α and alkylidene, or unsubstituted aminooxy;
acyl substituted with one or more substituents selected from the substituent group α;
alkylsulfonyl substituted with one or more substituents selected from the substituent group α, or unsubstituted alkylsulfonyl;
alkylsulfinyl substituted with one or more substituents selected from the substituent group α, or unsubstituted alkylsulfinyl;
alkylsulfamoyl substituted with one or more substituents selected from the substituent group α; a carbocyclic group such as cycloalkyl and aryl, substituted with one or more substituents selected from the substituent group α, azide, alkyl and halogenoalkyl;
a heterocyclic group substituted with one or more substituents selected from the substituent group α, azide, alkyl and halogenoalkyl;
carbocyclylalkyl such as cycloalkylalkyl and arylalkyl, substituted with one or more substituents selected from the substituent group α, azide, alkyl and halogenoalkyl, or unsubstituted. carbocyclylalkyl;
heterocyclylalkyl substituted with one or more substituents selected from the substituent group α, azide, alkyl and halogenoalkyl, or unsubstituted heterocyclylalkyl;
carbocyclyloxy such as cycloalkyloxy and aryloxy, substituted with one or more substituents selected from the substituent group α, azide, alkyl and halogenoalkyl, or unsubstituted carbocyclyloxy;
heterocyclyloxy substituted with one or more substituents selected from the substituent group α, azide, alkyl and halogenoalkyl, or unsubstituted heterocyclyloxy;
carbocyclylalkoxy such as cycloalkylalkoxy and arylalkoxy, substituted with one or more substituents selected from the substituent group α, azide, alkyl and halogenoalkyl, or unsubstituted carbocyclylalkoxy;
heterocyclylalkoxy substituted with one or more substituents selected from the substituent group α, azide, alkyl and halogenoalkyl, or unsubstituted heterocyclylalkoxy;
carbocyclylalkoxycarbonyl such as cycloalkylalkoxycarbonyl and arylalkoxycarbonyl, substituted with one or more substituents selected from the substituent group α, azide, alkyl and halogenoalkyl, or unsubstituted carbocyclylalkoxycarbonyl such as cycloalkylalkoxycarbonyl and arylalkoxycarbonyl;
heterocyclylalkoxycarbonyl substituted with one or more substituents selected from the substituent group α, azide, alkyl and halogenoalkyl, or unsubstituted heterocyclylalkoxycarbonyl;
carbocyclylthio such as cycloalkylthio and arylthio, substituted with one or more substituents selected from the substituent group α, azide, alkyl and halogenoalkyl, or unsubstituted carbocyclylthio such as cycloalkylthio and arylthio;
heterocyclylthio substituted with one or more substituents selected from the substituent group α, azide, alkyl and halogenoalkyl, or unsubstituted heterocyclylthio;
carbocyclylamino such as cycloalkylamino and arylamino, substituted with one or more substituents selected from the substituent group α, azide, alkyl and halogenoalkyl, or unsubstituted carbocyclylamino such as cycloalkylamino and arylamino;
heterocyclylamino substituted with one or more substituents selected from the substituent group α, azide, alkyl and halogenoalkyl, or unsubstituted heterocyclylamino;
carbocyclylalkylamino such as cycloalkylalkylamino and arylalkylamino, substituted with one or more substituents selected from the substituent group α, azide, alkyl and halogenoalkyl or unsubstituted carbocyclylalkylamino such as cycloalkylalkylamino and arylalkylamino;
heterocyclylalkylamino substituted with one or more substituents selected from the substituent group α, azide, alkyl and halogenoalkyl, or unsubstituted heterocyclylalkylamino;
carbocyclylsulfamoyl such as cycloalkylsulfamoyl and arylsulfamoyl, substituted with one or more substituents selected from the substituent group α, azide, alkyl and halogenoalkyl, or unsubstituted carbocyclylsulfamoyl;
heterocyclylsulfamoyl substituted with one or more substituents selected from the substituent group α, azide, alkyl and halogenoalkyl, or unsubstituted heterocyclylsulfamoyl;
carbocyclylsulfonyl such as cycloalkylsulfonyl and arylsulfonyl, substituted with one or more substituents selected from the substituent group α, azide, alkyl and halogenoalkyl, or unsubstituted carbocyclylsulfonyl such as cycloalkylsulfonyl and arylsulfonyl;
heterocyclylsulfonyl substituted with one or more substituents selected from the substituent group α, azide, alkyl and halogenoalkyl, or unsubstituted heterocyclylsulfonyl;
carbocyclylcarbamoyl such as cycloalkylcarbamoyl and arylcarbamoyl, substituted with one or more substituents selected from the substituent group α, azide, alkyl and halogenoalkyl, or unsubstituted carbocyclylcarbamoyl such as cycloalkylcarbamoyl and arylcarbamoyl;
heterocyclylcarbamoyl substituted with one or more substituents selected from the substituent group α, azide, alkyl and halogenoalkyl, or unsubstituted heterocyclylcarbamoyl;
carbocyclylalkylcarbamoyl such as cycloalkylalkylcarbamoyl and arylalkylcarbamoyl, substituted with one or more substituents selected from the substituent group α, azide, alkyl and halogenoalkyl, or unsubstituted carbocyclylalkylcarbamoyl such as cycloalkylalkylcarbamoyl and arylalkylcarbamoyl;
heterocyclylalkylcarbamoyl substituted with one or more substituents selected from the substituent group α, azide, alkyl and halogenoalkyl, or unsubstituted
heterocyclylalkylcarbamoyl;
carbocyclyloxycarbonyl such as cycloalkyloxycarbonyl and aryloxycarbonyl, substituted with one or more substituents selected from the substituent group α, azide, alkyl and halogenoalkyl, or unsubstituted carbocyclyloxycarbonyl such as cycloalkyloxycarbonyl and aryloxycarbonyl;
heterocyclyloxycarbonyl substituted with one or more substituents selected from the substituent group α, azide, alkyl and halogenoalkyl, or unsubstituted heterocyclyloxycarbonyl;
alkylenedioxy substituted with halogen, or unsubstituted alkylenedioxy;
oxo; and
azide.
"Substituted or unsubstituted carbocycle", "substituted or unsubstituted heterocycle", "substituted or unsubstituted benzene", "substituted or unsubstituted pyridine", "substituted or unsubstituted pyrimidine" and "substituted or unsubstituted pyrazine" may be substituted with one or more substituents selected from them.

In the specification, examples of the substituent of "substituted or unsubstituted carbocycle", "substituted or unsubstituted heterocycle "and "substituted or unsubstituted benzene" in ring A' and ring B, and "substituted or unsubstituted pyridine", "substituted or unsubstituted pyrimidine " and "substituted or unsubstituted pyrazine" in ring B include halogen, cyano, hydroxy, nitro, carboxy, alkyl substituted with one or more substituents selected from the substituent group α, unsubstituted alkyl, alkenyl substituted with one or more substituents selected from the substituent group α, unsubstituted alkenyl, alkynyl substituted with one or more substituents selected from the substituent group α, unsubstituted alkynyl, alkoxy substituted with one or more substituents selected from the substituent group α, unsubstituted alkoxy, alkenyloxy substituted with one or more substituents selected from the substituent group α, unsubstituted alkenyloxy, alkynyloxy substituted with one or more substituents selected from the substituent group α, unsubstituted alkynyloxy, amino substituted with one or more substituents selected from the substituent group α, unsubstituted amino, carbamoyl substituted with one or more substituents selected from the substituent group α, unsubstituted carbamoyl, alkylcarbamoyl substituted with one or more substituents selected from the substituent group α, unsubstituted alkylcarbamoyl, alkoxycarbonyl substituted with one or more substituents selected from the substituent group α, and unsubstituted alkoxycarbonyl.

Examples of the substituent other than "-W-ring B" of "substituted or unsubstituted carbocycle" or "substituted or unsubstituted heterocycle" in ring A include halogen, alkyl, alkenyl, alkynyl, alkoxy, alkenyloxy, alkynyloxy, or cyano.

Examples of the substituents of "substituted or unsubstituted carbocycle", "substituted or unsubstituted heterocycle" or "substituted or unsubstituted benzene" in ring A' include halogen.

Examples of the substituents of "substituted or unsubstituted pyridine", "substituted or unsubstituted pyrimidine", or "substituted or unsubstituted pyrazine" in ring B include halogen, alkyl, alkenyl, alkynyl, alkoxy, alkenyloxy, alkynyloxy, or cyano.

Examples of the substituents of "substituted or unsubstituted carbocyclic group", "substituted or unsubstituted heterocyclic group", "substituted or unsubstituted arylalkyl", "substituted or unsubstituted arylalkoxy", "substituted or unsubstituted carbocyclyloxy", "substituted or unsubstituted carbocyclylsulfonyl", "substituted or unsubstituted carbocyclylsulfinyl", "substituted or unsubstituted carbocyclyloxycarbonyl", "substituted or unsubstituted heterocyclyloxy", "substituted or unsubstituted heterocyclylthio", "substituted or unsubstituted heterocycylsulfonyl", "substituted or unsubstituted heterocyclylsulfinyl", "substituted or unsubstituted heterocyclyloxycarbonyl", "substituted or unsubstituted carbocycle" and "substituted or unsubstituted heterocycle" in other than the above ring A, ring A' and ring B include alkyl substituted with one or more substituents selected from the substituent group α, unsubstituted alkyl, and the substituent group α.

The term "alkylene" include a linear or branched divalent carbon chain of a carbon number of 1 to 10, for example, a carbon number of 1 to 6, or a carbon number of 1 to 3. Examples include methylene, dimethylene, trimethylene, tetramethylene, and methyltrimethylene.

The alkylene portion in "alkylenedioxy" is the same as the above "alkylene."

The term "alkenylene" includes a linear or branched divalent carbon chain of a carbon number of 2 to 10, for example, a carbon number of 2 to 6, or a carbon number of 2 to 4, having a double bond at any available position. Examples include vinylene, propenylene, butenylene, butadienylene, methylpropenylene, pentenylene and hexenylene.

The term "alkynylene", includes a linear or branched divalent carbon chain of a carbon number of 2 to 10, for example, a carbon number of 2 to 6, or, a carbon number of 2 to 4, having a triple bond at any available position and, further, optionally having a double bond. Examples include ethynylene, propynylene, butynylene, pentynylene and hexynylene.

Examples of the substituents of "substituted or unsubstituted alkylene", "substituted or unsubstituted C1-C3 alkylene", "substituted or unsubstituted alkenylene", "substituted or unsubstituted C2-C3 alkenylene", and "substituted or unsubstituted alkynylene " include the groups selected from the substituent group α, and specific examples are halogen and hydroxy.

The phrase "R^{za} and R^{zb} together with the carbon atom to which they are attached may form a substituted or unsubstituted non-aromatic carbocycle or a substituted or unsubstituted non-aromatic heterocycle" include the following examples.

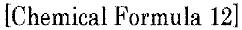

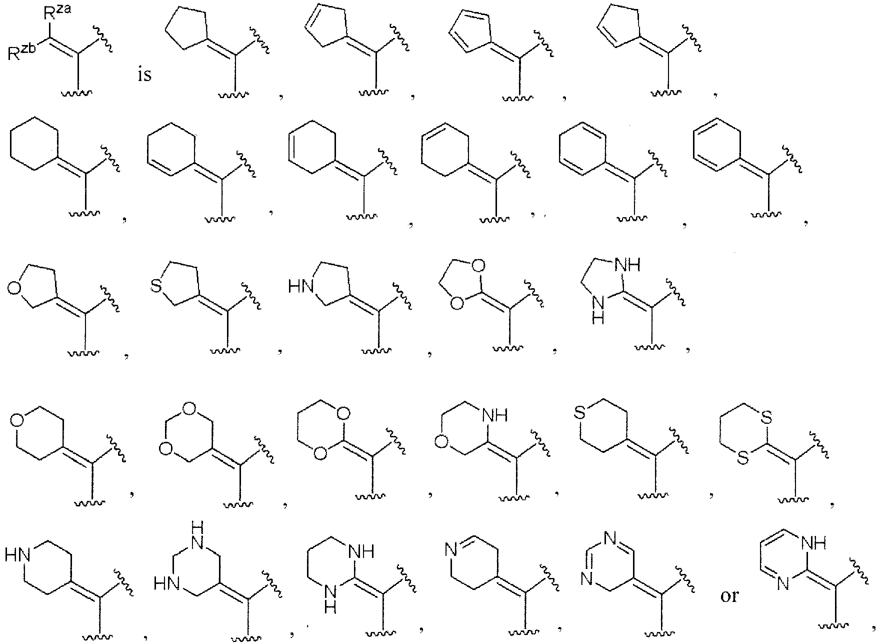

These groups may be substituted with one or more selected from alkyl substituted with one or more selected from the substituent group α, unsubstituted alkyl and the substituent group α at any available position.

The phrases "R^{3a} and R^{3b} together with the carbon atom to which they are attached may form a substituted or unsubstituted carbocycle or a substituted or unsubstituted heterocycle", "R^{5a} and R^{5b} together with the carbon atom to which they are attached may form a substituted or unsubstituted carbocycle or a substituted or unsubstituted heterocycle" and "R^{6a} and R^{6b} together with the carbon atom to which they are attached may form a substituted or unsubstituted carbocycle or a substituted or unsubstituted heterocycle"" include the following examples.

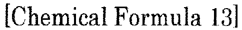

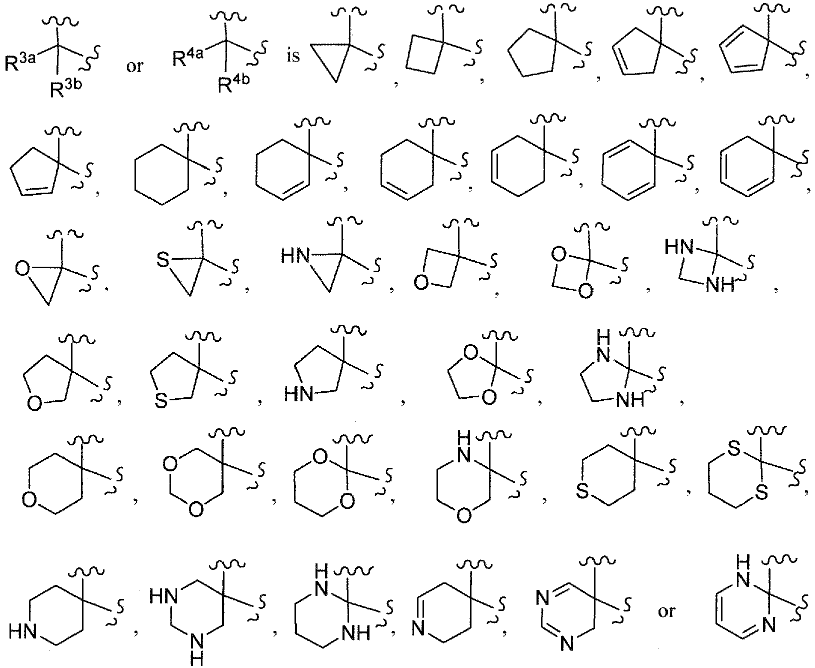

These groups may be substituted with one or more selected from alkyl substituted with one or more selected from the substituent group α, unsubstituted alkyl and the substituent group α at any available position.

In the specification, the term "solvate" includes, for example, solvates with organic solvents and hydrates. It can be prepared in accordance with the known methods. Examples of solvate include a solvate with acetone, 2-butanol, 2-propanol, ethanol, ethyl acetate, tetrahydrofuran or diethyl ether. For example, it includes a non-toxic and water-soluble hydrate or solvate such as a solvate with ethanol. In the case that a hydrate or solvate is formed, the compound or salt may be coordinated with any number of solvate molecules or water molecules.

The compound of the formula (I) includes a pharmaceutically acceptable salt. Examples include salts with alkali metals such as lithium, sodium or potassium; alkaline earth metals such as calcium; magnesium; transition metals such as zinc or iron; ammonium; organic bases; and amino acids; or salts with inorganic acids such as hydrochloric acid, sulfuric acid, nitric acid, hydrobromic acid, phosphoric acid or hydroiodic acid; and organic acids such as acetic acid, trifluoroacetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methanesulfonic acid or ethanesulfonic acid. Specific examples are hydrochloric acid, phosphoric acid, tartaric acid and methanesulfonic acid. These salts can be formed by ordinary methods.

In addition, the compound of the formula (I) is not limited to a specific isomer, but includes all possible isomers, such as keto-enol isomers, imine-enamine isomers, diastereoisomers, optical isomers and rotation isomers; and racemate. For example, the compound of the formula (I) wherein R^{2a} is hydrogen includes the following tautomers.

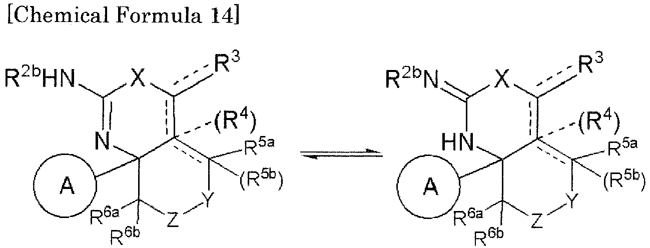

Compounds of the formulas (I-a1) to (I-a3), (I-b1) to (I-b6), (I-c1) and (I-c2) include the similar tautomers.

In addition, one or more hydrogen, carbon or other atoms of the compound of the formula (I) can be replaced by an isotope of the hydrogen, carbon or other atoms. Compounds of the formula (I) include all radiolabeled forms of compounds of the formula (I). The "radiolabeled," "radiolabeled form" and the like of the compound of the formula (I) are encompassed by the present invention and useful as a research and/or diagnostic tool in metabolism pharmacokinetic studies and in binding assays. It is also useful for a medicament.

Examples of isotopes that can be incorporated into the compound of the formula (I) of the invention include isotopes of hydrogen, carbon, nitrogen, oxygen, phosphorous, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³Iand ³⁶Cl, respectively. Radiolabeled compounds of the invention can be prepared by methods known in the art. For example, tritiated compounds of formula (I) can be prepared by introducing tritium into the particular compound of formula (I), for example, by catalytic dehalogenation with tritium. This method may include reacting a suitably halogen-substituted precursor of a compound of formula (I) with tritium gas in the presence of a suitable catalyst such as Pd/C, in the presence or absence of a base. Other suitable methods for preparing tritiated compounds can be found in Isotopes in the Physical and Biomedical Sciences, Vol. 1, Labeled Compounds (Part A) Chapter 6, (1987). ¹⁴C-labeled compounds can be prepared by employing starting materials having a ¹⁴C carbon.

The present compound of the formula (I-a1) to (I-a3), (I-b1) to (I-b6), (I-c1) and (I-c2) can be prepared, for example, according to the method described in Patent Literature 4, or by the following method.

### (General Procedures)

The present compound of the formula (I) can be prepared by the following method. In the following all steps, when a substituent which interferes with the reaction, e.g. hydroxy, mercapto, amino, formyl, carbonyl, carboxy, is possessed, the substituent is protected by the method such as those described in Protective Groups in organic Synthesis, and Theodora W Greene (John Wiley & Sons) in advance, and the protective group may be removed at a desirable stage.

In the following formulas, a crossed bond means that the compound is either E isomer or Z isomer, or a mixture thereof with respect to double bond.

### Preparation Method A-I (X=S)

### Synthesis of Compound (I-a1)

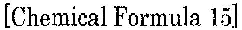

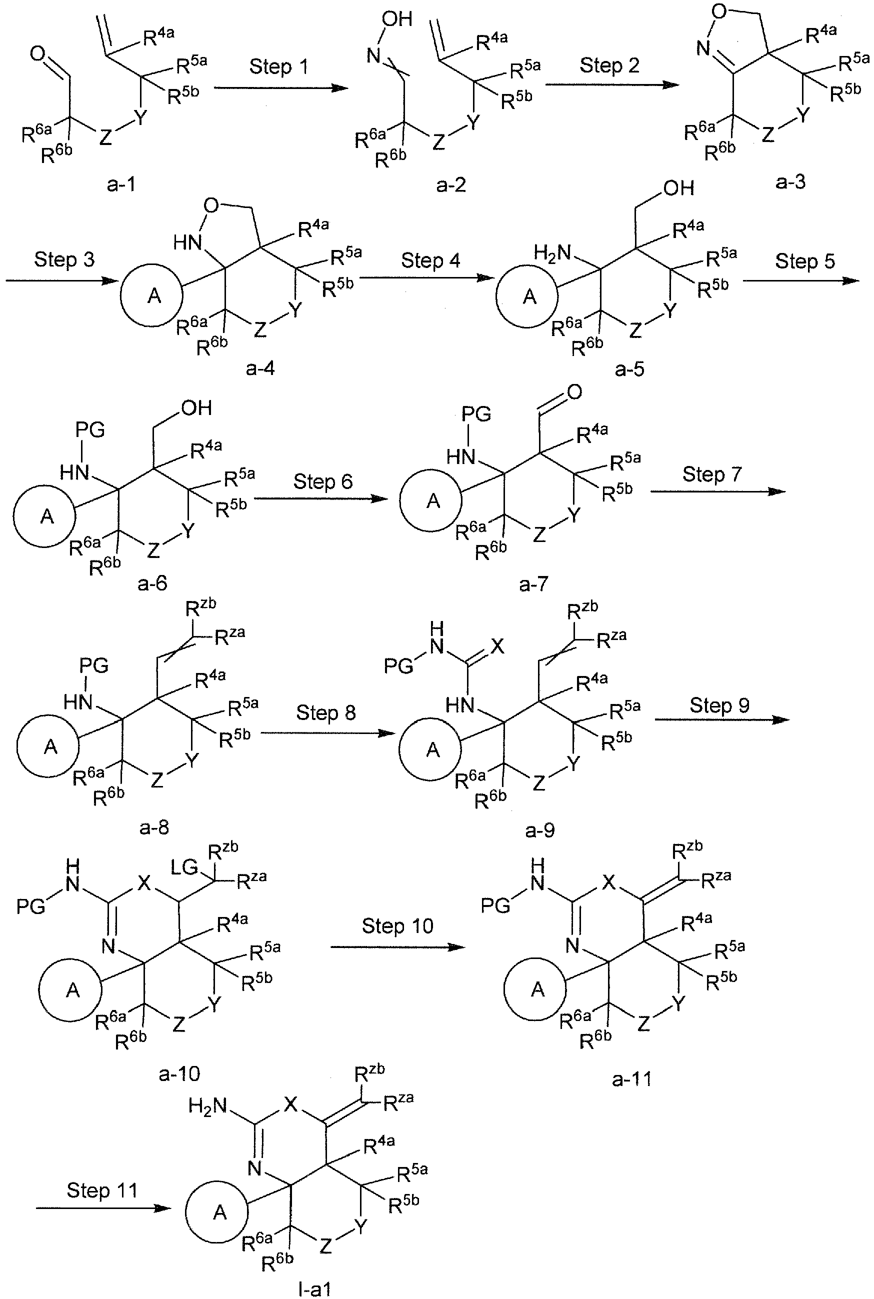

wherein PG represents an amino protective group such as acyl, alkoxycarbonyl, trialkylsilyl, and alkylsulfonyl, and the other symbols represent the same as defined above.
Compound (a-1) can be prepared by the Preparing Method D described below from a commercially available compound. It can be also prepared by the known methods from a commercially available compound, and can be also prepared by methods described in Examples.

### Step 1

Compound (a-2) can be prepared by reacting Compound (a-1) with hydroxylamine or a hydroxylamine salt such as hydroxylamine hydrochloride or hydroxylamine sulfate in the presence or absence of a base, in an organic solvent such as ethanol, methanol, tetrahydrofuran, 1,4-dioxane, 1,2-dimethoxyethane and dichloromethane, and a mixed solvent of them and water. Examples of a base include sodium acetate, pyridine, sodium hydroxide, potassium hydroxide, cesium hydroxide, barium hydroxide and 2,6-lutidine. The reaction time is not particularly limited and is usually between 5 minutes and 30 hours, and preferably between 5 minutes and 12 hours. The reaction temperature is usually between -20 °C and solvent reflux temperature, and preferably between 0°C and solvent reflux temperature.

The reaction in this step can be performed under the usual conditions for an oximation reaction of a carbonyl compound such as the conditions described in Org. Lett., 9 (2007) 5, 753-6, Tetrahedron 54 (1998) 22, 5808-82, and Tetrahedron: Asymmetry 5 (1994) 6, 1018-28.

### Step 2

Compound (a-3) can be prepared by reacting Oxime Compound (a-2) with N-chlorosuccinimide or sodium hypochlorite in a solvent such as dichloromethane, chloroform, benzene, toluene, xylene, N,N-dimethylformamide, tetrahydrofuran and 1,4-dioxane at the temperature between ice-cooling to solvent reflux temperature for 30 minutes to 48 hours, preferably 0.5 to 24 hours to convert a nitrile oxide, followed by a 1,3-dipolar cycloaddition reaction. More preferable results such as suppression of side reactions and improved yield may be achieved by carrying out this reaction in the presence of a base such as sodium carbonate, potassium carbonate, cesium carbonate, potassium phosphate, an aqueous solution thereof, triethylamine and pyridine.

This step is for preparing Compound (a-3) by converting Compound (a-2) to a nitrile oxide derivative and subjecting to a 1,3-dipolar cycloaddition reaction with the olefin moiety in the same molecule. The reaction in this step can be performed under the usual conditions for a 1,3-dipolar cycloaddition reaction, for example, the conditions described in Org. Lett. ,9 (2007) 5, 753-6, Tetrahedron 54 (1998) 22, 5868-82 and Tetrahedron: Asymmetry 5 (1994) 6, 1018-28.

### Step 3

Compound (a-4) can be prepared by reacting Compound (a-3) with an organic metal reagent such as an aryl lithium reagent (wherein aryl includes heterocyclyl), a Grignard reagent (wherein the substituent includes heterocyclyl), an aryl magnesium reagent (wherein aryl includes heterocyclyl), which can be prepared by methods known to a person skilled in the art in a solvent which does not interfere with the preparation of a reagent and this reaction such as diethyl ether, tetrahydrofuran, 1,4-dioxane, 1,2-dimethoxyethane, benzene and toluene, and a mixed solvent thereof. The reaction time is usually between 5 minutes and 48 hours, and preferably between 5 minutes and 12 hours. The reaction temperature varies according to a starting material, a reagent, solubility and the like, and in general, is preferably maintained to be the temperature for minimizing the formation of a by-product, for example, 78 °C. Favorable results such as an improved yield and a reduced reaction time may be achieved by addition of an additive such as tetramethylethylene diamine, hexamethylphosphoramide or a Lewis acid such as a boron trifluoride-diethyl ether complex.

The reaction in this step can be performed under similar conditions to those described in SYNLETT, 2004, 8, 1408-13, J. Am. Chem. Soc., 2005, 127, 5376-83 and Bull. Chem. Soc. Jpn., 1993, 66, 2730-7.

### Step 4

Compound (a-5) can be prepared by subjecting Compound (a-4) to a cleavage reaction of the N-O bond by using a zinc-acid, a hydrogen-metal catalyst, a hydride reductant and the like in a solvent which does not interfere with the reaction such as methanol, ethanol, 1,4-dioxane, THF, ether and water.

Examples of the acid include acetic acid, formic acid and hydrochloric acid. The solvent used in the reaction can be selected from the above solvent, and the above acid can be also used as a solvent. The reaction temperature is usually between -20 °C and solvent reflux temperature, and preferably between 0 °C and solvent reflux temperature. The reaction time is usually between 5 minutes and 48 hours, and preferably between 5 minutes and 24 hours. The reaction can be performed under similar conditions to those described in Org. Lett. 7 (2005) 25, 5741-5742 and J. Org. Chem. 200, 68, 1207-1215.

The reaction can be performed using a metal catalyst such as platinum oxide under similar conditions to those described in Tetrahedron: Asymmetry 5 (1994) 6, 1019-1028 and Tetrahedron, Vol. 53, No. 16, pp 5752-5746, 1997. Compound (a-5) can be prepared by hydrogenating Compound (a-4) using platinum oxide as a catalyst in a solvent such as methanol under hydrogen atmosphere.

The reaction using a hydride reductant such as a lithium aluminum hydride can be performed under similar conditions to those described in Bull. Chem. Soc. Jpn., 66, 2730-2737 (1993). Specifically, compound (a-5) can be prepared by reducing Compound (a-4) using lithium aluminum hydride in a solvent such as ether.

### Step 5

Compound (a-6) can be prepared by subjecting Compound (a-5) to a functional group protection reaction which is known to a person skilled in the art. For example, when a protective group is a t-butoxycarbonyl group (hereafter referred to as a Boc group) or a 9-fluorenemethyloxycarbonyl group (hereafter referred to as a Fmoc group), a target compound can be prepared under the usual conditions for protection of an amine compound such as the conditions described in T. W Green and P. G M. Wuts, "Protective Groups in Organic Chemistry, Third Edition", John Wiley & Sons.

### Step 6

Compound (a-7) can be prepared by adding an oxidant such as 2-iodoxybenzoic acid to a solution of Compound (a-6) in a solvent such as dimethyl sulfoxide and dichloromethane, and reacting at 0 °C to 80 °C, preferably 10 °C to 40 °C for 0.5 hours to 48 hours, preferably 1 hour to 12 hours.

Examples of oxidation conditions used in the reaction include Swern oxidation, Corey-Kim oxidation, Moffatt oxidation, pyridinium chlorochromate oxidation (PCC oxidation), pyridinium dichromate oxidation (PDC oxidation), Dess-Martin oxidation, SO₃-pyridine oxidation, and 2,2,6,6-tetramethyl-1-piperidinyloxy free radical oxidation (TEMPO oxidation), which are known to a person skilled in the art, and the conditions described in "Comprehensive Organic Transformations, Richard C. Larock; VCH".

### Step 7

To R^{3a}R^{3b}CHPPh₃ such as methyltriphenylphosphonium iodide which is commercially available or can be prepared by methods known to a person skilled in the art is added a strong base such as an alkyl metal reagent such as n-butyllithium in a solvent such as ether, tetrahydrofuran, dioxane, and a mixed solvent thereof to prepare a Wittig reagent which corresponds to a target compound. Compound (a-8) can be prepared by adding a solution of Compound (a-7) in a solvent such as ether, tetrahydrofuran, and dioxane to the Wittig reagent at -40 °C to 60 °C, preferably -20 °C to 30 °C and reacting them for 0.1 hours to 24 hours, preferably 0.3 hours to 6 hours.

### Step 8

An amine free compound can be prepared by deprotecting Compound (a-8) under conditions known to a person skilled in the art such as the usual conditions for deprotecting of a protective group of an amine compound, for example, when a protective group is a Boc group or a Fmoc group, the conditions described in T. W Green and P. G M. Wuts, "Protective Groups in Organic Chemistry, Third Edition", John Wiley & Sons. Compound (a-9) can be prepared by adding isocyanate or isothiocyanate such as benzoyl isocyanate and benzoyl isothiocyanate having a protective group, which is commercially available or can be prepared by the known methods to the obtained amine-free compound in a solvent such as dichloromethane, tetrahydrofuran and toluene and a mixed solvent thereof at -30 °C to 50 °C, preferably -10 °C to 25 °C for 0.5 hours to 24 hours, preferably 0.5 hours to 12 hours.

### Step 9

Compound (a-10) can be prepared by adding a halogenium cation source such as iodine, bromine, N-bromosuccinimide (hereafter referred to as NBS), and N-chloro succinimide (hereafter referred to as NCS) to a solution of Compound (a-9) in a solvent such as dichloromethane, and reacting at -20 °C to 40 °C, preferably 0 °C to 20 °C for 0.1 hours to 12 hours, preferably 0.1 hours to 6 hours.

### Step 10

Compound (a-11) can be prepared by adding a base such as pyrrolidine, piperidine, piperazine, morpholine and 1,8-diazabicyclo [5.4.0] undeca-7-en (hereafter referred to as DBU) to a solution of Compound (a-10) in a solvent such as dioxane, tetrahydrofuran and toluene, and the mixture thereof, and reacting at 20 °C to 100 °C, preferably 40 °C to 80 °C for 0.1 hours to 24 hours, preferably 1 hour to 12 hours.

### Step 11

An amino group of Compound (a-11) can be deprotected under conditions for deprotection known to a person skilled in the art. For example, when a protective group is a Boc group or a Fmoc group, Compound (I-a1) can be prepared under the usual conditions for deprotecting a protective group of an amine compound such as the conditions described in T. W Green and P. G M. Wuts, "Protective Groups in Organic Chemistry, Third Edition", John Wiley & Sons.

### Preparation Method A-II (X=NR¹)

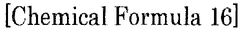

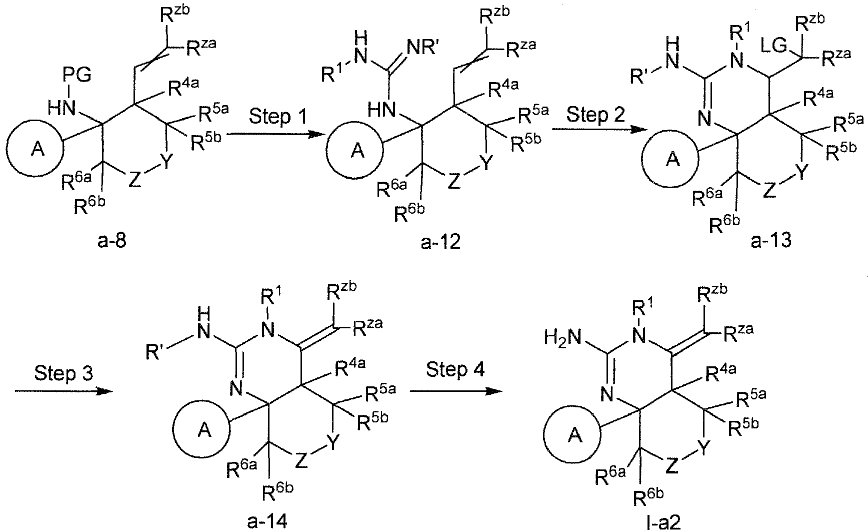

wherein R' represents a group which does not interfere with the reaction such as an amino group, LG represents a leaving group such as halogen and alkylsulfonyloxy, and the other symbols represent the same as defined above.

### Step 1

An amine free compound can be prepared by deprotecting Compound (a-8) under conditions known to a person skilled in the art such as the usual conditions for deprotecting a protective group of an amine compound, for example, when a protective group is a Boc group or a Fmoc group, the conditions described in T. W Green and P. G M. Wuts, "Protective Groups in Organic Chemistry, Third Edition", John Wiley & Sons. Compound (a-12) can be prepared by reacting the obtained amine-free compound with 3,5-dimethylpyrazole-1-carboxyamidine, S-methylthiourea or the like in a solvent such as acetonitrile, tetrahydrofuran and dimethylformamide at 0 °C to 150 °C, preferably 20 °C to 100 °C for 0.5 hours to 120 hours, preferably 1 hour to 24 hours.

### Step 2

Compound (a-13) can be prepared by adding a halogenium cation source such as iodine, bromine, NBS, and NCS to a solution of Compound (a-12) in a solvent such as dichloromethane, and reacting at -20 °C to 40 °C, preferably 0 °C to 20 °C for 0.1 hour to 12 hours, preferably 0.1 hour to 6 hours.

### Step 3

Compound (a-14) can be prepared by adding a base such as pyrrolidine, piperidine, piperazine, morpholine and DBU to a solution of Compound (a-13) in a solvent such as dioxane, tetrahydrofuran and toluene, and the mixture thereof, and reacting at 20 °C to 100 °C, preferably 40 °C to 80 °C for 0.1 hours to 24 hours, preferably 1 hour to 12 hours.

### Step 4 (the case wherein R' is a protective group)

An amine group of Compound (a-14) can be deprotected under conditions for deprotection known to a person skilled in the art. When a protective group is a Boc group or a Fmoc group, Compound (I-a2) can be prepared under the usual conditions for deprotection of a protective group of an amine compound such as the conditions described in T. W Green and P. G M. Wuts, "Protective Groups in Organic Chemistry, Third Edition", John Wiley & Sons.

### Preparation Method A-III (X=O)

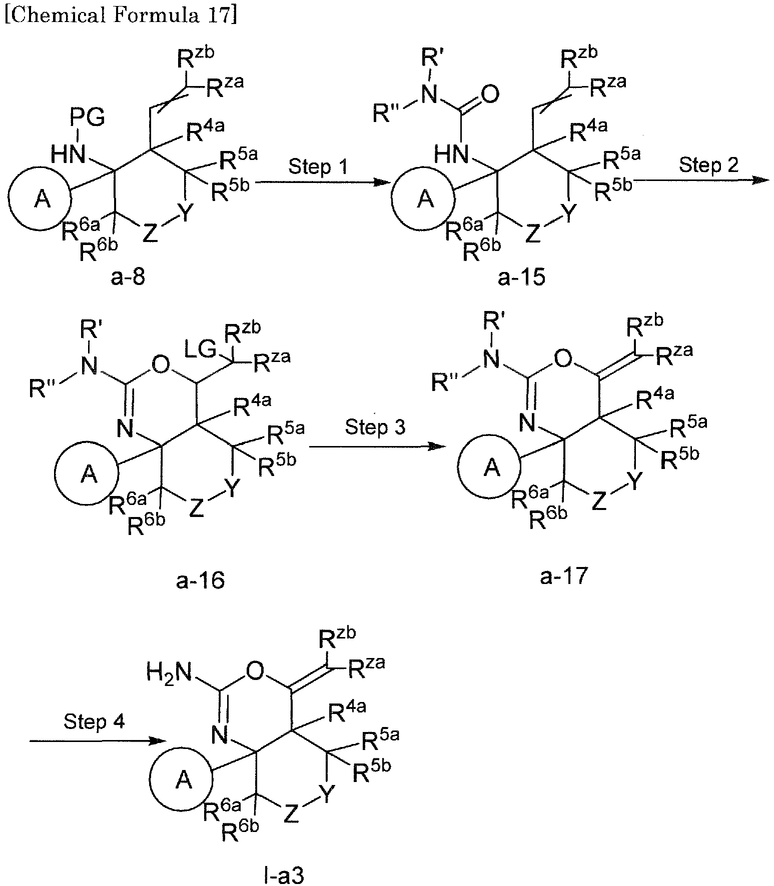

wherein R and R" represent groups which do not interfere with the reaction such as an amino protective group and the other symbols are the same as defined above.

### Step 1

An amine free compound can be prepared by deprotecting Compound (a-8) under conditions known to a person skilled in the art such as the usual conditions for deprotection of a protective group of an amine compound, for example, when a protective group is a Boc group or a Fmoc group, the conditions described in T. W Green and P. G M. Wuts, "Protective Groups in Organic Chemistry, Third Edition", John Wiley & Sons. Compound (a-15) can be prepared by adding isocyanate such as benzoyl isocyanate having a protective group, which is commercially available or can be prepared by the known methods, to the obtained amine-free compound in a solvent such as dichloromethane, dioxane, tetrahydrofuran, toluene and acetone, and a mixed solvent thereof at -30 °C to 50 °C, preferably -10 °C to 25 °C for 0.1 hours to 12 hours, preferably 0.1 hours to 3 hours.

### Step 2

Compound (a-16) can be prepared by adding a halogenium cation source such as iodine, bromine, NBS, and NCS to a solution of Compound (a-15) in a solvent such as dichloromethane, and reacting at -20 °C to 40 °C, preferably 0 °C to 20 °C for 1 hour to 12 hours, preferably 0.1 hours to 6 hours.

### Step 3

Compound (a-17) can be prepared by adding a base such as pyrrolidine, piperidine, piperazine, morpholine and DBU to a solution of Compound (a-16) in a solvent such as dioxane, tetrahydrofuran and toluene, and a mixed solvent thereof, and reacting at 20 °C to 100 °C, preferably 40 °C to 80 °C for 0.1 hours to 24 hours, preferably 1 hour to 12 hours.

### Step 4 (the case wherein the deprotection of R' and/or R" is necessary)

An amino group of Compound (a-17) can be deprotected under conditions for deprotection known to a person skilled in the art. When a protective group is a Boc group or a Fmoc group, Compound (I-a3) can be prepared under the usual conditions for deprotection of a protective group of an amine compound such as the conditions described in T. W Green and P. G M. Wuts, "Protective Groups in Organic Chemistry, Third Edition", John Wiley & Sons.

### Preparation Method B-1 (X=S)

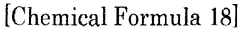

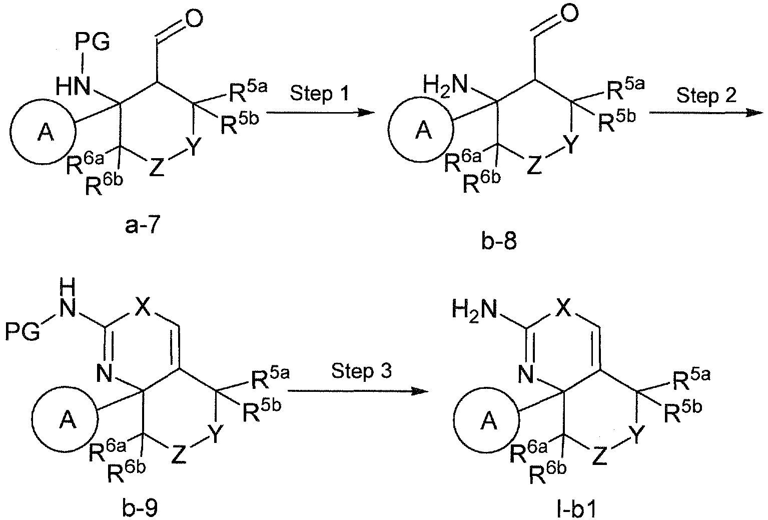

wherein the symbols are the same as defined above.

### Step 1

An amino group of Compound (a-7) which can be prepared by the method mentioned above can be deprotected under conditions for deprotection known to a person skilled in the art. When a protective group is a Boc group or a Fmoc group, Compound (b-8) can be prepared under the usual conditions for deprotection of a protective group of an amine compound such as the conditions described in T. W Green and P. G M. Wuts, "Protective Groups in Organic Chemistry, Third Edition", John Wiley & Sons.

### Step 2

To a solution of Compound (b-8) in a solvent such as dichloromethane, dioxane, tetrahydrofuran, toluene and acetone, and a mixed solvent thereof is added isocyanate such as benzoyl isocyanate having a protective group, which is commercially available or can be prepared by the known methods, and the mixture is reacted at -30 °C to 70 °C, preferably -20 °C to 50 °C for 0.1 hours to 12 hours, preferably 0.1 hours to 6 hours. Compound (b-9) can be prepared by evaporating the solvent, adding concentrated sulfuric acid, concentrated nitric acid or the like to the mixture, and reacting at -30 °C to 70 °C, preferably -20 °C to 50 °C for 1 hour to 12 hours, preferably 1 hour to 6 hours.

### Step 3

If necessary, an amino group of Compound (b-9) can be deprotected under conditions for deprotection known to a person skilled in the art. For example, when a protective group is a Boc group or a Fmoc group, Compound (I-b1) can be prepared under the usual conditions for deprotection of a protective group of an amine compound such as the conditions described in T. W Green and P. G M. Wuts, "Protective Groups in Organic Chemistry, Third Edition", John Wiley & Sons.

### Preparation Method B-II (X=NR¹)

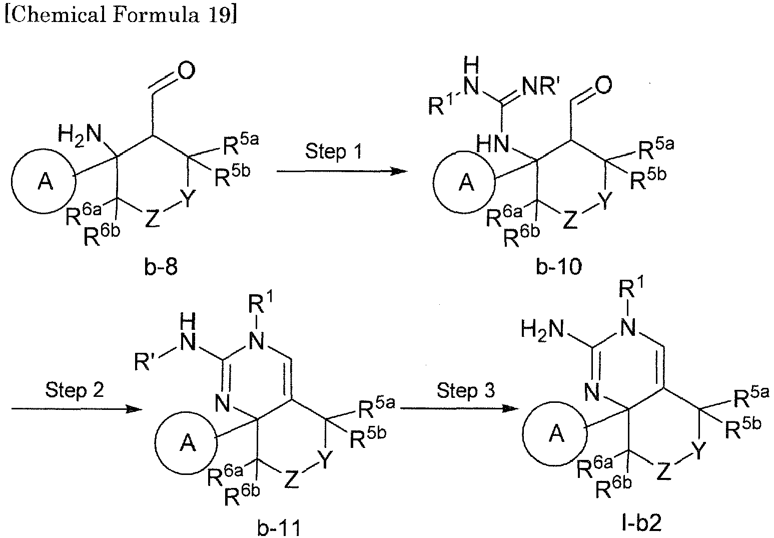

wherein the symbols are the same as defined above.

### Step 1

Compound (b-10) can be prepared by reacting Compound (b-8) with 3,5-dimethylpyrazole-1-carboxyamidine, S-methylthiourea or the like in a solvent such as acetonitrile, tetrahydrofuran and dimethylformamide at 0 °C to 150 °C, preferably 20 °C to 100 °C for 0.5 hours to 120 hours, preferably 1 hour to 24 hours.

### Step 2

Compound (b-11) can be prepared by adding concentrated sulfuric acid, concentrated nitric acid or the like to Compound (b-10), and reacting at -30 °C to 70 °C, preferably -20 °C to 50 °C for 1 hour to 12 hours, preferably 1 hour to 6 hours.

### Step 3 (the case wherein R' is a protective group)

An amino group of Compound (a-14) can be deprotected under conditions for deprotection known to a person skilled in the art. For example, when a protective group is a Boc group or a Fmoc group, Compound (I-b2) can be prepared under the usual conditions for deprotection of a protective group of an amine compound such as the conditions described in T. W Green and P. G M. Wuts, "Protective Groups in Organic Chemistry, Third Edition", John Wiley & Sons.

### Preparation Method B-III (X=O)

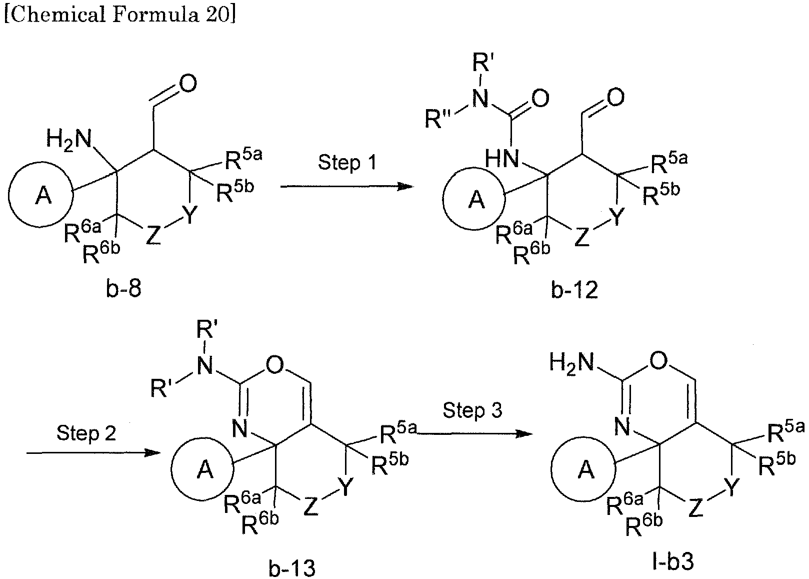

wherein the symbols are the same as defined above.

### Step 1

Compound (b-12) can be prepared by adding isocyanate such as benzoyl isocyanate having a protective group, which is commercially available or can be prepared by the known methods, to a solution of Compound (b-8) in a solvent such as dioxane, tetrahydrofuran, toluene and acetone, and a mixed solvent thereof, and reacting at -30 °C to 50 °C, preferably -10 °C to 25 °C for 0.1 hours to 12 hours, preferably 0.1 hours to 3 hours.

### Step 2

Compound (b-13) can be prepared by adding concentrated sulfuric acid, concentrated nitric acid or the like to Compound (b-12), and reacting at 0 °C to 100 °C, preferably 0 °C to 60 °C for 0.5 hours to 24 hours, preferably 1 hour to 12 hours.

### Step 3 (the case wherein deprotection is needed)

An amino group of Compound (b-13) can be deprotected under conditions for deprotection known to a person skilled in the art. For example, when a protective group is a Boc group or a Fmoc group, Compound (I-b3) can be prepared under the usual conditions for deprotection of a protective group of an amine compound such as the conditions described in a document such as T. W Green and P. G M. Wuts, "Protective Groups in Organic Chemistry, Third Edition", John Wiley & Sons.

### Preparation Method B-IV (the case wherein R³ is not hydrogen)

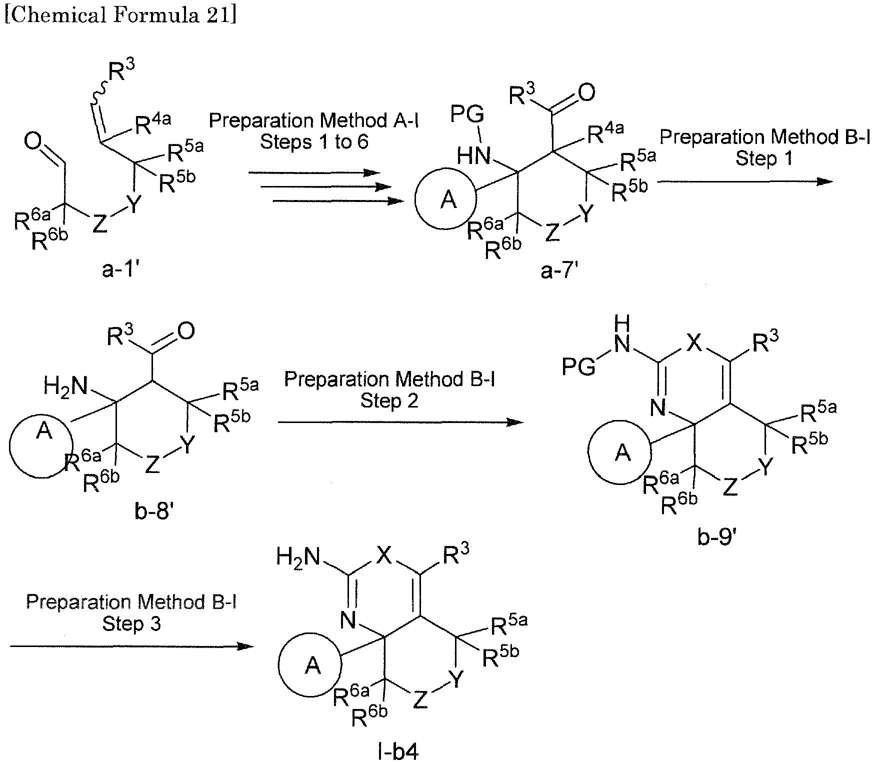

wherein the symbols are the same as defined above.
Compound (a-1') can be prepared from a commercially available compound by methods in a similar manner to Method D described below. It can also be prepared by methods known to a person skilled in the art from a commercially available compound or by methods described in Examples.

Compound (I-b4) can be prepared by subjecting Compound (a-7') which is prepared by Step 1 to Step 6 of Preparation Method A-I to a reaction of Step 1 to Step 3 of Preparation Method B.I using Compound (a-1') as a starting material. Similarly, compounds wherein X is NR¹ or O can be prepared according to the scheme described below.

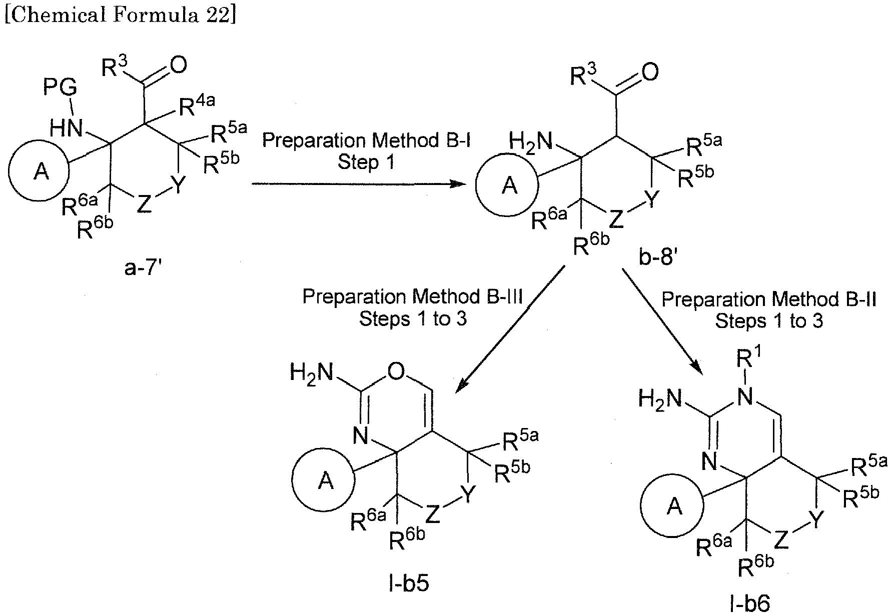

wherein the symbols are the same as defined above.

### Synthesis of Compound (a-7') (Alternative method)

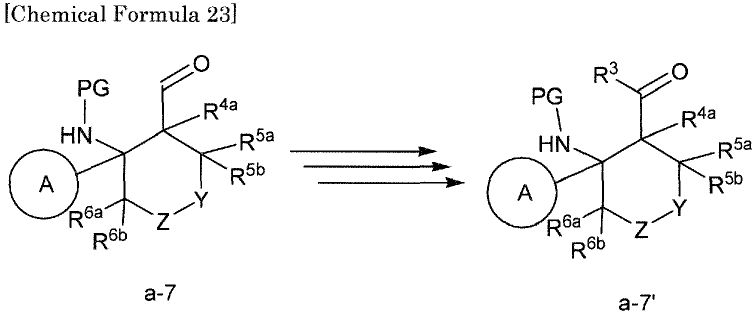

wherein the symbols are the same as defined above.
Compound (a-7') can be prepared by reacting Compound (a-'7) with a Grignard reagent according to usual methods known to a person skilled in the art, and oxidizing the obtained alcohol. Alternatively, Compound (a-7') can be prepared by oxidizing Compound (a-7) to a carboxylic acid according to the usual methods, converting into a Weinreb amide, reacting with a Grignard reagent, and oxidizing the obtained alcohol. The conditions described in "Comprehensive Organic Transformations, Richard C. Larock; VCH" can be also used for conversion of these functional groups.

### Preparation Method C-I

### Synthesis of Compounds (I-c1) and (I-c2)

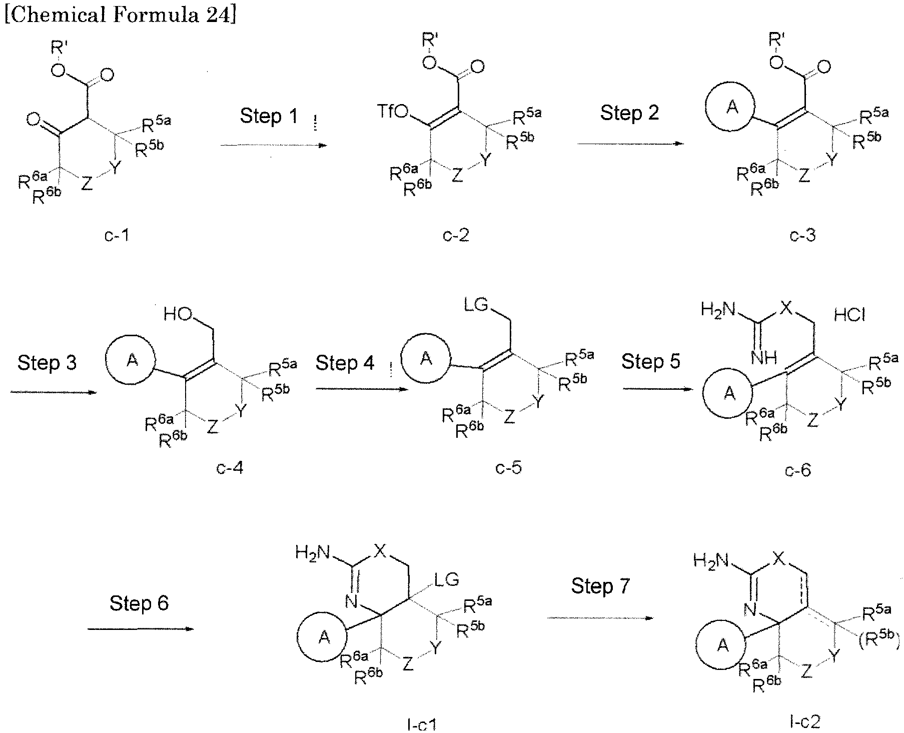

wherein LG is a leaving group such as halogen or alkylsulfonyloxy, R represents alkyl, arylalkyl or the like, Tf represents trifluoromethanesulfonyl, either one of dotted lines represent the presence of a bond, and the other symbols are the same as defined above.
Compound (c-1) can be a commercially available compound. It can be also prepared by methods known to a person skilled in the art from a commercially available compound or by methods described in Examples.

### Step 1

Compound (c-2) can be prepared by reacting a base such as sodium hydride, lithium diisopropylamide, lithium bis(trimethylsilyl)amide, diisopropylethylamine, pyridine and 2,6-lutidine with Compound (c-1), and reacting N-phenyltrifluoromethanesulfonimide or trifluoromethanesulfonic anhydride. Examples of the solvent include ether, tetrahydrofuran, 1,4-dioxane, 1,2-dimethoxyethane, dichloromethane, 1,2-dichloroethane, benzene and toluene. The reaction time is usually between 5 minutes and 24 hours, and preferably between 5 minutes and 12 hours. The reaction temperature is usually between -100 °C and 40 °C, and preferably between -78 °C and room temperature.

The reaction in this step can be performed under the usual conditions for a trifluoromethanesulfonylation reaction of a carbonyl compound such as the conditions described in J. Org. Chem., 57, 6972-6975 (1992), Tetrahedron., 61, 4129-4140 (2005) and Tetrahedron Letters., 40, 8133-8136 (1999).

### Step 2

Compound (c-3) can be prepared by a coupling reaction of Compound (c-2) using a transition metal. This coupling reaction can be performed under the usual conditions for coupling reaction such as Suzuki-Miyaura coupling reaction or Stille coupling reaction. For example, the reaction conditions described in Chem.Rev.,2007, 107, 133-73 and Tetrahedron, 58 (2002), 9633-35 can be applied.

The organometallic catalyst used in this reaction is not particularly limited. Examples of the organometallic catalyst include tetrakis(triphenylphosphine)palladium (0), dichlorobis(triphenylphosphine)palladium(II), [1,1'-bis(diphenylphosphino)ferrocene]palladium(II) dichloride, bis(tert-butylphosphine)palladium(0), palladium(II) acetate and [1,3-bis(diphenylphosphino)propane]nickel(II). The amount of the organometallic catalyst is about 0.001 to 0.1 equivalent with respect to the raw material. The organometallic compound is not particularly limited. Preferable examples of the organometallic compound include organotin reagents such as aryl tri-n-butyltin, and organoboron reagents such as aryl boronic acid. The amount of the organometallic compound is 1 equivalent to an excess amount with respect to the raw material. The solvent is not particularly limited insofar as it does not interfere with the reaction. Examples of the solvent include benzene, toluene, xylene, N,N-dimethylformamide, 1-methyl-2-pyrrolidone, 1,4-dioxane and propionitrile. The reaction temperature is usually between ice-cold temperature and solvent reflux temperature, and preferably between room temperature and solvent reflux temperature. The reaction time is usually between 10 minutes and 48 hours, and preferably between 30 minutes and 24 hours.

A more preferable result such as a suppression of side reactions and an improved yield may be achieved by carrying out this reaction in the presence of a base such as sodium carbonate, potassium carbonate, cesium carbonate, potassium phosphate, an aqueous solution thereof, and triethylamine.

### Step 3

Compound (c-4) can be prepared by subjecting Compound (c-3) to a reduction reaction by using diisobutylaluminum hydride, lithium aluminum hydride, lithium borohydride or the like in a solvent such as dioxane, tetrahydrofuran or toluene, or a mixed solvent thereof and reacting at -80 °C to 0 °C, preferably -80 °C to -20 °C for 0.1 hours to 12 hours, preferably 0.1 hours to 3 hours.

### Step 4

Compound (c-5) can be prepared by reacting Compound (c-4) with a halogenating agent such as thionyl chloride, phosphorous oxychloride, carbon tetrabromide-triphenylphosphine, and carbon tetrachloride-triphenylphosphine in the presence or absence of a solvent such as toluene or dichloromethane at -80 °C to 50 °C, preferably -20 °C to 20 °C for 0.1 hours to 24 hours, preferably 0.5 hours to 12 hours. Compound (c-5) can be also prepared by reacting Compound (c-4) with a sulfonylation agent such as methanesulfonyl chloride and p-toluenesulfonyl chloride in the presence of a base such as triethylamine in a solvent such as toluene or dichloromethane at -80 °C to 50 °C, preferably -20 °C to 20 °C for 0.1 hours to 24 hours, preferably 0.5 hours to 12 hours.

### Step 5

Compound (c-6) can be prepared from Compound (c-5) by methods known to a person skilled in the art. Specifically, for example, Compound (c-6) can be prepared by reacting Compound (c-5) with thiourea or the like in an organic solvent such as ethanol, 1-propanol, 2-propanol, 1-buthanol, tetrahydrofuran, 1,4-dioxane, and N,N-dimethylformamide. The reaction time is usually between 5 minutes and 24 hours, and preferably between 5 minutes and 12 hours. The reaction temperature is usually between 0 °C and 150 °C, and preferably between room temperature and 100 °C.

### Step 6

Compound (I-c1) can be prepared by adding a halogenium cation source such as iodine, bromine, NBS and NCS to a solution of Compound (c-6) in a solvent such as dichloromethane, and reacting the mixture at -20 °C to 40 °C, preferably 0 °C to 20 °C for 0.1 hours to 12 hours, preferably 0.1 hours to 6 hours.

The objective substituent R^{4b} can be introduced to thus-obtained compounds by methods known to a person skilled in the art.

### Step 7

Compound (1-c2) can be prepared by adding a base such as pyrrolidine, piperidine, piperazine, morpholine and DBU to a solution of Compound (I-c1) in a solvent such as dioxane, tetrahydrofuran and toluene, and a mixed solvent thereof, and reacting at 20 °C to 100 °C, preferably 40 °C to 80 °C for 0.1 hours to 24 hours, preferably 1 hour to 12 hours.

### Preparation Method C-II (the case wherein R³ is not hydrogen)

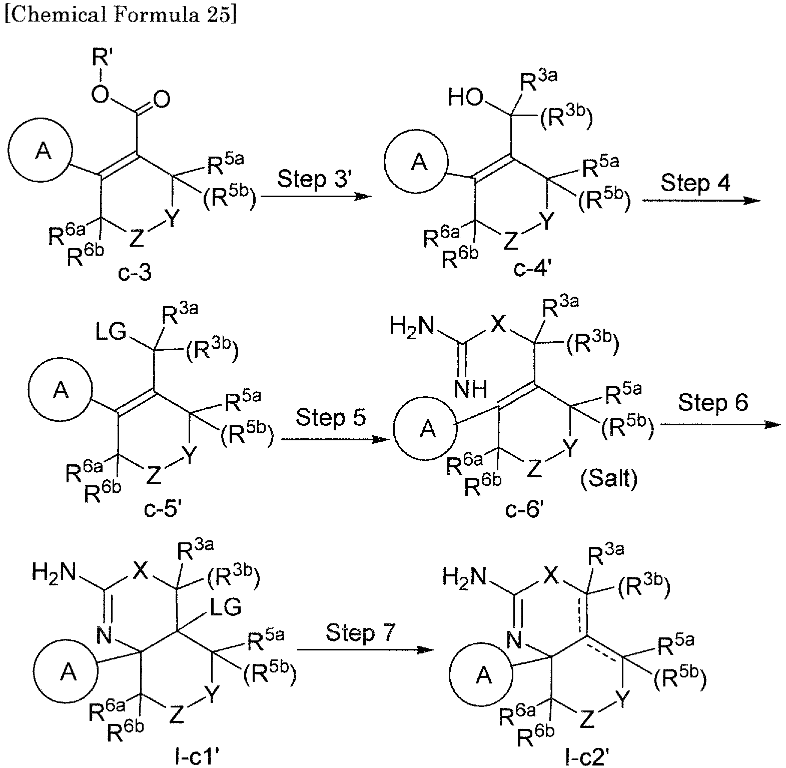

wherein the symbols are the same as defined above.

### Step 3'

To a solution of Compound (c-3) in a solvent such as ether or tetrahydrofuran, or a mixed solvent such as ether-tetrahydrofuran is added a Grignard reagent having a substituent corresponding to the objective compounds such as methylmagnesiumchloride and ethylmagnesiumbromide at -100 °C to 50 °C, preferably -80 °C to 30 °C. Alternatively, Compound (c-3) is hydrolyzed, followed by being converted into a Weinreb Amide, and reacted with a Grignard reagent having a substituent corresponding to the objective compounds such as R^{3a}MgBr and R^{3b}MgBr. Thus obtained compound is reacted for 0.2 hours to 24 hours, preferably 0.2 hours to 5 hours to give Compound (c-4').

Compound (I-c1') and Compound (I-c2') can be prepared by the subsequent steps according to Preparation Method C-I.

### Introduction of R⁴

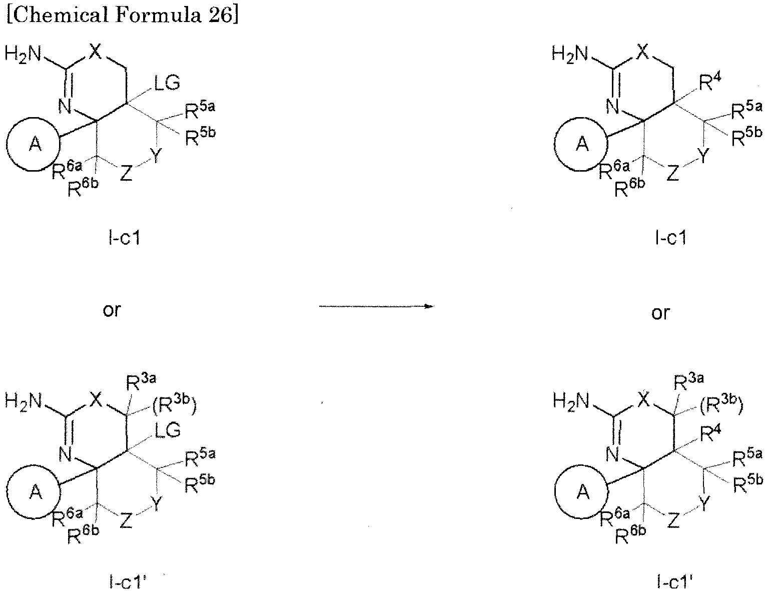

wherein the symbols are the same as defined above.

### Conversion of a leaving group

Introduction of R⁴ into Compound (I-c1) or (I-c1') can be performed under the suitably applied conditions according to methods described in the following documents which are known to a person skilled in the art.
MAHAMMED, K. A.; MURTHY, P. S. K.; RAJU, K. M.; Indian J. Chem. Sect. B: Org. Chem. Incl. Med. Chem., 2008, 47 (4), 575-578.,
KISS, L.; MANGELINCKX, S.; FUELOEP, F.; DE KIMPE, N.; Org. Lett., 2007, 9 (21), 4399-4402.,
CAO, Y.-Q.; WU,G.-Q.; LI,Y.-B.; DAI,Z.; CHEN,B.-H.; Synth. Commun., 2006, 36 (22),3353-3358., VACHAL,P.; FLETCHER, J.M.; HAGMANN, W. K.; Tetrahedron Lett., 2007, 48 (33), 5761-5765 and the like.

### Preparation Method D

### Synthesis of Compound (a-1) (Y=O)

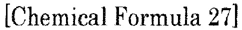

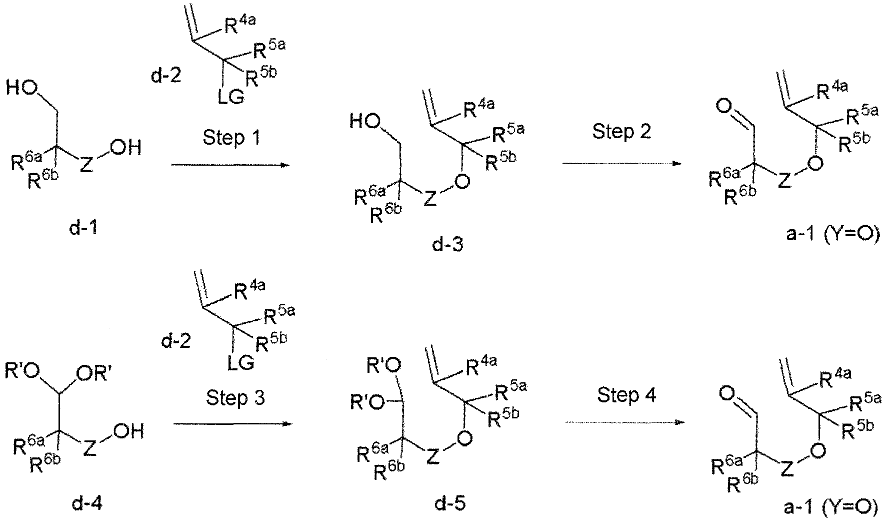

wherein the symbols are the same as defined above.
Compounds (d-1), (d-2) and (d-4) can be a commercially available compound. They can also be prepared by methods known to a person skilled in the art from a commercially available compound, or by methods described in Examples.

### Step 1

Compound (d-3) can be prepared by adding a base such as sodium hydride, potassium hydride and potassium t-butoxide to a solution of Compound (d-1) in a solvent such as THF, DMF and dimethyl sulfoxide to give an alkoxide, and reacting with Compound (d-2). The amounts of the base and Compound (d-2) are each 1 equivalent to an excess amount with respect to Compound (d-1). The reaction time is usually between 5 minutes and 90 hours, and preferably between 30 minutes and 24 hours. The reaction temperature is usually between -20 °C and 80 °C A more preferable result such as an improved yield may be achieved by performing the reaction in the presence of a salt such as tetrabutylammonium iodide.

This reaction can be performed under the usual conditions for O-alkylation of alcohol such as the conditions described in Tetrahedron Lett., 46(2005) 45, 7751-5.

### Step 2

Compound (a-1; Y=O) can be prepared by adding an oxidant such as 2-iodoxy benzoic acid to a solution of Compound (d-3) in a solvent such as dimethyl sulfoxide and dichloromethane, and reacting at 0 °C to 80 °C, preferably 10 °C to 40 °C for 0.5 hours to 48 hours, preferably 1 hour to 12 hours.

Examples of the oxidation conditions include Swern oxidation, Corey-Kim oxidation, Moffatt oxidation, pyridinium chlorochromate oxidation (PCC oxidation), pyridinium chromate oxidation (PDC oxidation), Dess-Martin oxidation, SO₃-pyridine oxidation, and 2, 2, 6, 6 -tetramethyl-1-piperizinyloxy free radical oxidation(TEMPO oxidation).

### Step 3

Compound (d-5) can be prepared from Compound (d-4) in a similar manner to the above preparation of the above Compound (d-3).

### Step 4

Compound (a-1; Y=O) can be prepared by deprotecting an acetal group of Compound (d-5) under similar conditions to those described in T. W Green and P. G M. Wuts, "Protective Groups in Organic Chemistry, Third Edition", John Wiley & Sons.

### Preparation Method E

### Synthesis of Compound (a-1, Y=N-PG)

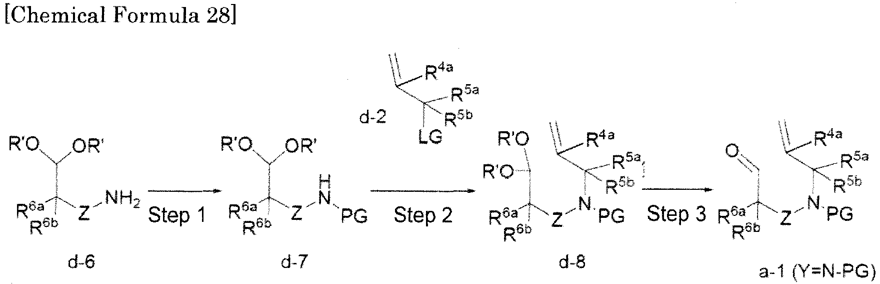

wherein the symbols are the same as defined above.
Compound (d-6) can be a commercially available compound. It can be also prepared by methods known to a person skilled in the art from a commercially available compound or by methods described in Examples.

Compound (d-7) can be prepared by subjecting Compound (d-6) to a reaction under the conditions for functional group protection known to a person skilled in the art. For example, when a protective group is a Boc group or a Fmoc group, Compound (d-7) can be prepared under the usual conditions for protection of an amine compound such as the conditions described in T. W Green and P. G M. Wuts, "Protective Groups in Organic Chemistry, Third Edition", John Wiley & Sons.

### Step 2

Compound (d-8) can be prepared by adding powder of sodium hydroxide, potassium hydroxide, sodium hydride, potassium hydride or potassium t-butoxide to a solution of Compound (d-7) in a solvent such as toluene, THF, DMF and dimethyl sulfoxide, and reacting with Compound (d-2) at -20 °C to 100 °C for 5 minutes to 72 hours, preferably 30 minutes to 24 hours. The amounts of the base and Compound (d-2) are each 1 equivalent to an excess amount with respect to Compound (d-7). A more preferable result such as an improved yield may be achieved by performing the reaction in the presence of a salt such as tetrabutylammonium iodide.

This reaction can be performed under the usual conditions for N-alkylation of Compound (d-7) such as the conditions described in J. Med. Chem., 2007,50,5493-508.

### Step 3

Compound (a-1) wherein Y=NH can be prepared by deprotecting a PG group of Compound (d-8) under similar conditions to those described in T. W Green and P. G M. Wuts, "Protective Groups in Organic Chemistry, Third Edition", John Wiley & Sons.

Compound (I-a1) wherein Y is N(R') can be prepared by subjecting thus-obtained compounds to each reaction in Preparation Method A and introducing the substituent R¹ by methods known to a person skilled in the art at an appropriate stage. Alternatively, the objective compound can be prepared by deprotecting Compound (a-1), introducing R¹ using methods known to a person skilled in the art, and subjecting to each reaction in Preparation Method A

In the all steps, an order of steps to be implemented may be appropriately changed, and each intermediate may be isolated, and used in a next step.

### Conversion of a substituent

Above-mentioned Compounds (I-a1) to (I-a3), (I-b1) to (I-b6), (I-c1), (I-c2) and these compounds wherein each ring A is substituted with various substituents such as ring B-W- group can be prepared according to the method mentioned above or the known methods such as those described in Patent Literature 1, Patent Literature 2, Patent Literature 3 and the like.

Moreover, the optically active isomer of the compound (I) can be prepared by using an optically active compound as a starting material, performing an asymmetric synthesis in the suitable step to prepare an optically active intermediate, or optical resolution of the racemate of the intermediate or the objective compound in the appropriate step. The method of optical resolution include the separation of optical isomer using an optically active column, the kinetics optical resolution using enzyme reactions or the like, the crystallization and separation of diastereomers by the salt formation using chiral acids or chiral bases, the preferential crystallization or the like.

Preferable embodiments of the present invention are illustrated below. Each symbol represents the same as described above.

(A) In the formula (I), the followings are exemplified.

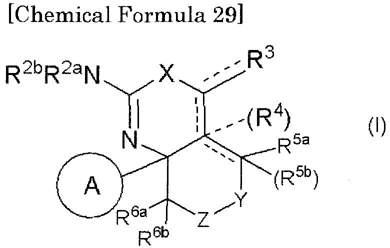

-X- is, for example, -O-, -S- or -N (R¹)-.

-X- is, for example, -O- or -S-.

-X- is, for example, -O-.
-X- is, for example, -S-.

R¹ is, for example, hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl.

R¹ is, for example, hydrogen, hydroxy, substituted or unsubstituted alkyl, substituted or unsubstituted acyl, a substituted or unsubstituted carbocyclic group or a substituted or unsubstituted heterocyclic group.

R^{2a} and R^{2b} are, for example, each independently hydrogen, substituted or unsubstituted alkyl, substituted or unsubstituted acyl, substituted or unsubstituted alkoxycarbonyl or substituted or unsubstituted carbamoyl.

R^{2a} and R^{2b} are, for example, both hydrogen.

Ring A includes substituted carbocycle or substituted or unsubstituted heterocycle.

Ring A includes

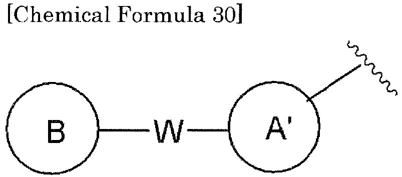

wherein

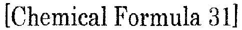

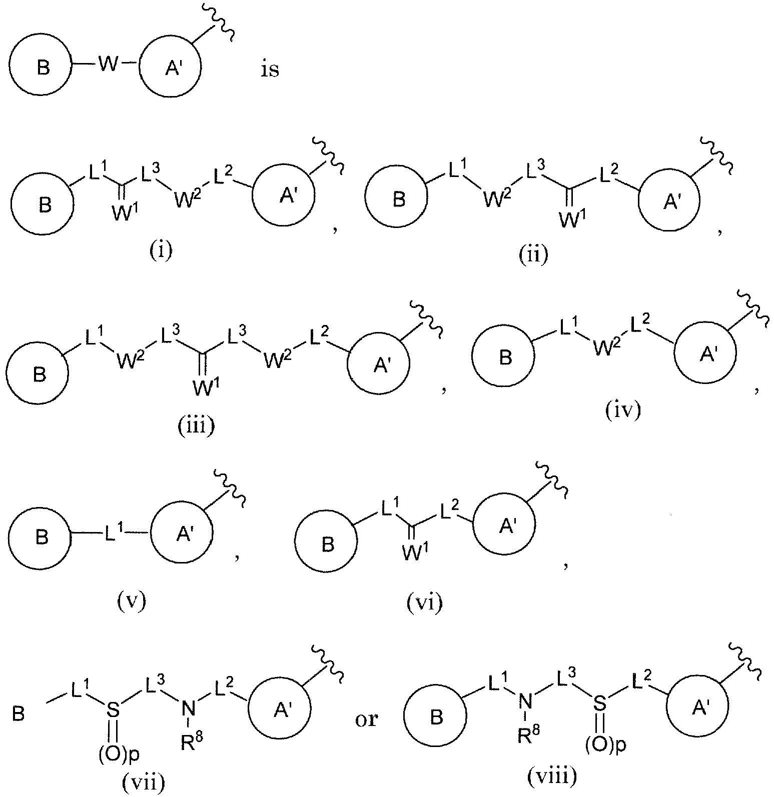

Ring A' and ring B are each independently substituted or unsubstituted carbocycle or substituted or unsubstituted heterocycle,
L¹, L², and L³ are each independently a bond, substituted or unsubstituted alkynylene, substituted or unsubstituted alkenylene or substituted or unsubstituted alkynylene,
= W¹ is =O, =S, or =NR⁹,
W² is O, S, or N(R⁸),
R⁸ is hydrogen, substituted or unsubstituted alkyl, substituted or unsubstituted alkenyl, substituted or unsubstituted alkynyl or substituted or unsubstituted acyl,
R⁹ is hydrogen, hydroxy, substituted or unsubstituted alkyl, substituted or unsubstituted alkenyl, substituted or unsubstituted alkynyl or substituted or unsubstituted acyl,
when ring A is (i), then the constituent carbon atom of L¹ and the constituent carbon atom of L², or the constituent carbon atom of L¹ and the nitrogen atom of W² may be connected with substituted or unsubstituted alkylene to form a ring,
when ring A is (ii), then the constituent carbon atom of L¹ and the constituent carbon atom of L², or the nitrogen atom of W² and the constituent carbon atom of L² may be connected with substituted or unsubstituted alkylene to form a ring
when ring A is (iii), then two nitrogen atoms of W² may be connected with substituted or unsubstituted alkylene to form a ring,
when ring A is (vi), then the constituent carbon atom of L¹ and the constituent carbon atom of L² may be connected with substituted or unsubstituted alkylene to form a ring,
p is 1 or 2, and
when multiple L³, multiple W², or multiple R⁹ are present, each of them may be independently different.

Ring A includes

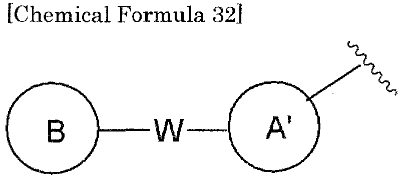

wherein

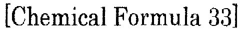

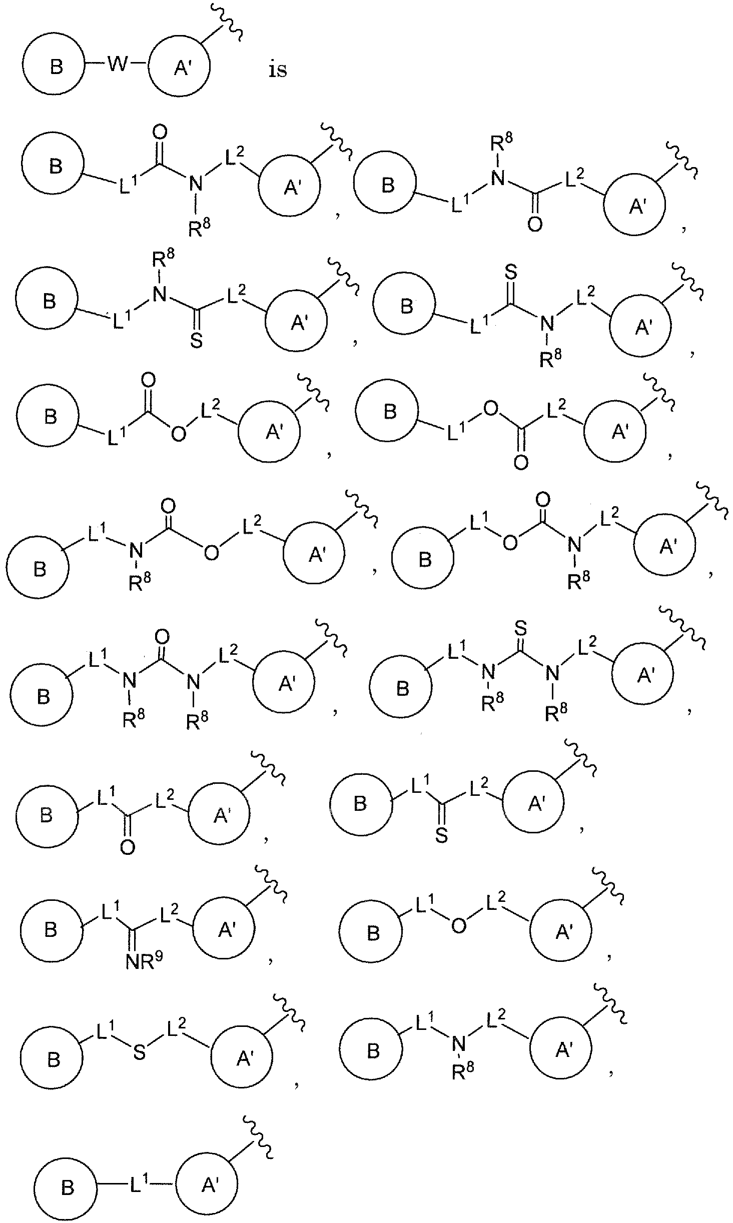

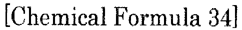

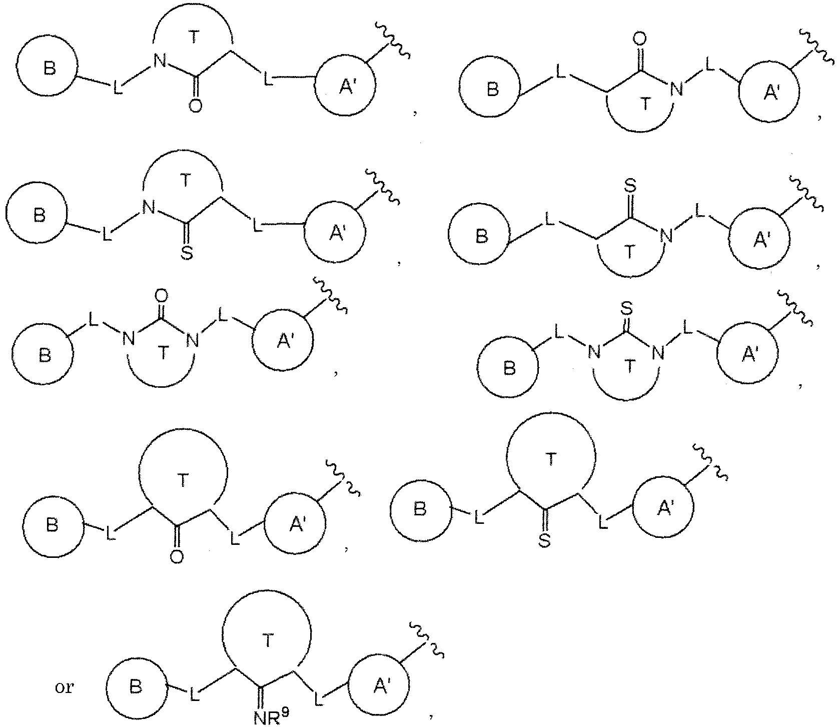

wherein L is each independently a bond, substituted or unsubstituted alkylene, substituted or unsubstituted alkenylene, substituted or unsubstituted alkynylene, ring T is a carbocycle which may be substituted with a group(s) selected from the substituent group a or a heterocycle which may be substituted with a group(s) selected from the substituent group α, and other each symbol is the same as defined above.

Ring A includes

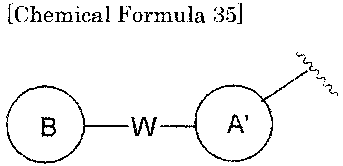

wherein

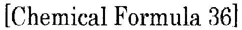

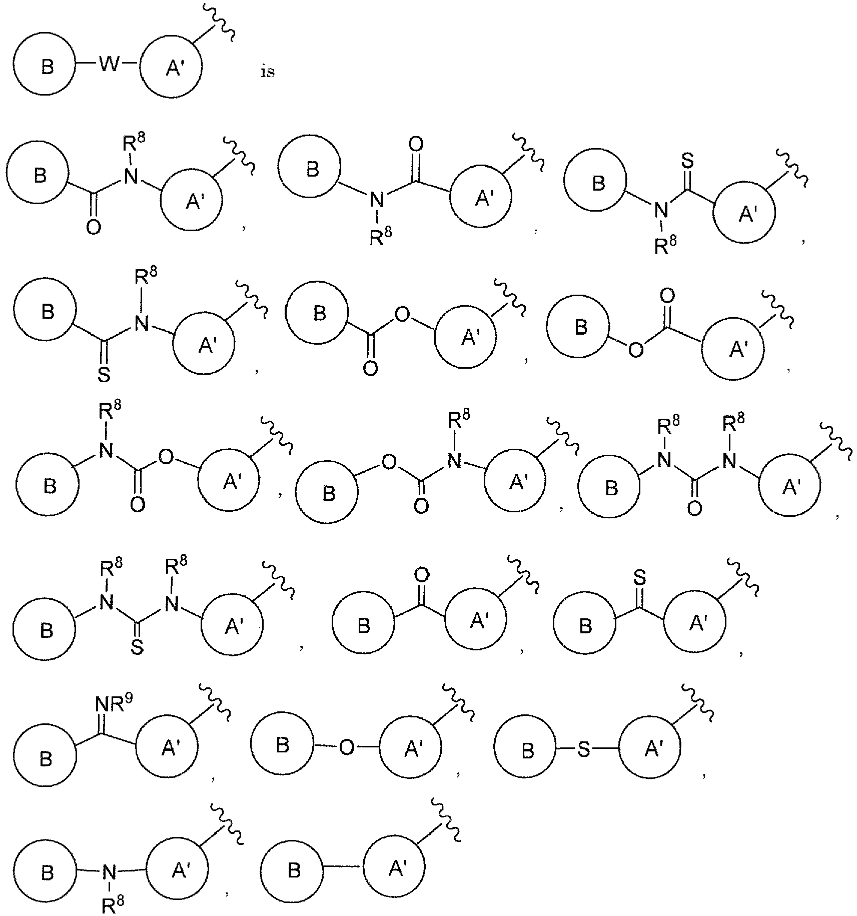

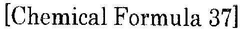

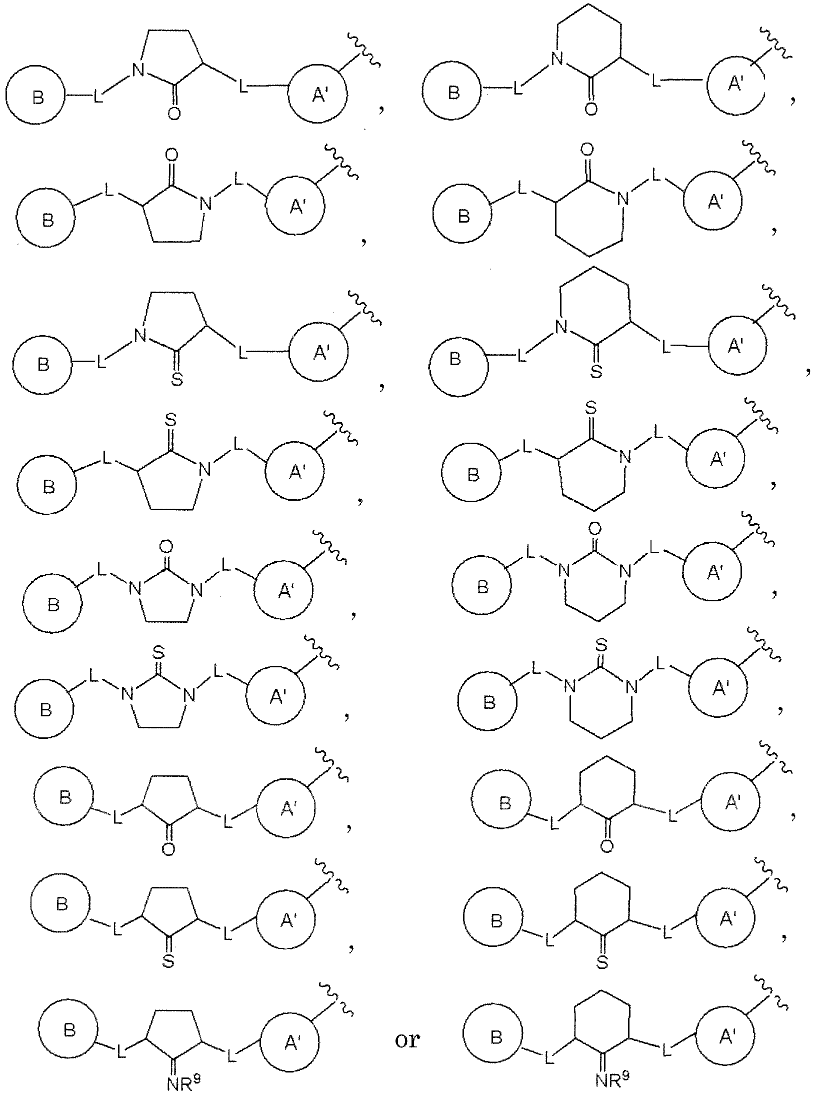

wherein each symbol is the same as defined above.

Ring A includes

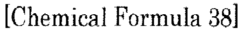

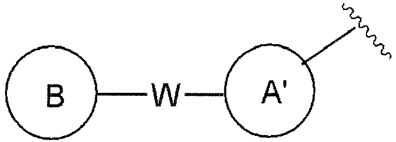

wherein

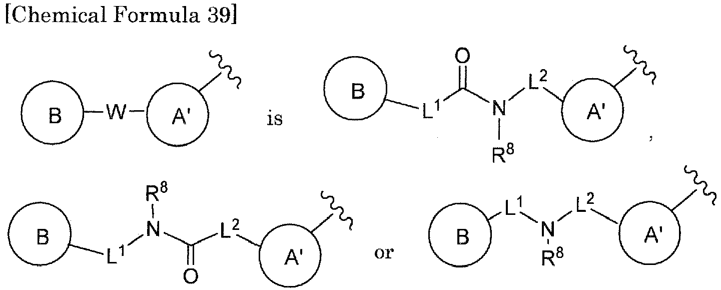

L¹ and L² include each independently a bond, substituted or unsubstituted alkylene, substituted or unsubstituted alkenylene or substituted or unsubstituted alkynylene, and
R⁸ includes hydrogen, substituted or unsubstituted alkyl, substituted or unsubstituted alkenyl, substituted or unsubstituted alkynyl or substituted or unsubstituted acyl.

Ring A includes

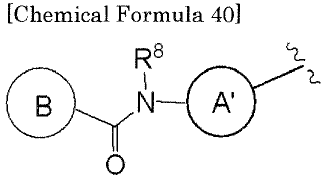

Ring A' includes each independently a substituted or unsubstituted carbocycle or a substituted or unsubstituted heterocycle.

Ring A' is, for example, substituted or unsubstituted benzene, wherein the substituent is halogen and the like.

Ring B is substituted or unsubstituted pyridine, substituted or unsubstituted pyrimidine or substituted or unsubstituted pyrazine, wherein the substituent is, for example, halogen, alkyl, alkoxy, halogenoalkoxy, alkynyloxy, alkylcarbamoyl, or cyano.

R⁸ includes hydrogen, substituted or unsubstituted alkyl, substituted or unsubstituted alkenyl, substituted or unsubstituted alkynyl or substituted or unsubstituted acyl.

R⁸ includes hydrogen.

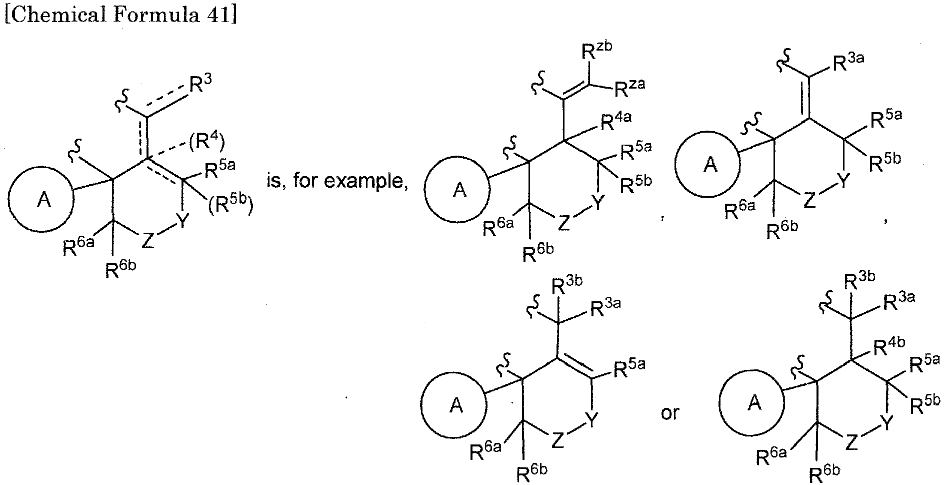

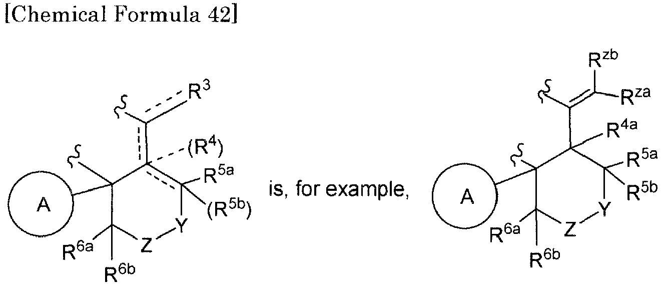

As used herein, -Y- is, for example, a bond, substituted or unsubstituted C1-C3 alkylene, substituted or unsubstituted C2-C3 alkenylene, -N(R⁷)-, -O-, -S-, -SO- or -SO₂-.

-Y- is, for example, substituted or unsubstituted C1-C3 alkylene or -O-.

-Z- is, for example, a bond, substituted or unsubstituted C1-C3 alkylene, substituted or unsubstituted C2-C3 alkenylene, -N(R⁷)Ak-, -OAk-, -SAk-, -SOAk- or -SO₂ Ak-.

-Z- is, for example, a bond.

Ak is a bond, substituted or unsubstituted C1-C3 alkylene or substituted or unsubstituted C2-C3 alkenylene.

Provided that when -Y- is -N(R⁷)-, -O-, -S-, -SO- or -SO₂-, then Ak is not a bond. -Z-Y- is, for example, substituted or unsubstituted C1-C3 alkylene, -O- or -O-(substituted or unsubstituted C1-C3 alkylene)-.

As used herein, R⁷ is, for example, hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbamoyl, substituted or unsubstituted thiocarbamoyl, substituted or unsubstituted sulfamoyl, substituted or unsubstituted amino, a substituted or unsubstituted carbocyclic group, substituted or unsubstituted carbocyclyloxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclyloxycarbonyl, substituted or unsubstituted heterocyclylsulfinyl or substituted or unsubstituted heterocyclylsulfonyl.

R⁷ is, for example, hydrogen, halogen, hydroxy, substituted or unsubstituted alkyl, substituted or unsubstituted acyl, cyano, carboxy, substituted or unsubstituted alkoxycarbonyl, or substituted or unsubstituted carbamoyl.

R^{za} and R^{zb} are, for example, each independently hydrogen, halogen, substituted or unsubstituted alkyl, or R^{za} and R^{zb} together with the carbon atom to which they are attached may form a substituted or unsubstituted non-aromatic carbocycle.

R^{za} and R^{zb} are, for example, each independently hydrogen, halogen, substituted or unsubstituted alkyl.

R^{3a} and R^{3b} are, for example, each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkylthio, substituted or unsubstituted alkenylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkylsulfonyl, substituted or unsubstituted alkenylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl.

R^{3a} and R^{3b} are, for example, both hydrogen.

R^{4a} and R^{4b} are, for example, each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, a substituted or unsubstituted carbocyclic group, substituted or unsubstituted carbocyclyloxy, a substituted or unsubstituted heterocyclic group, or substituted or unsubstituted heterocyclyloxy.

R^{4a} and R^{4b} are, for example, each independently hydrogen or substituted or unsubstituted alkyl.

However provided that when -X- is -S-, then R^{4b} is not hydrogen.

R^{5a}, R^{5b}, R^{6a} and R^{6b} are, for example, each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl, or R^{5a} and R^{5b} together with the carbon atom to which they are attached may form a substituted or unsubstituted carbocycle or a substituted or unsubstituted heterocycle,
R^{6a} and R^{6b} together with the carbon atom to which they are attached may form a substituted or unsubstituted carbocycle or a substituted or unsubstituted heterocycle.

R^{5a}, R^{5b} R^{6a} and R^{6b} are, for example, each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclyloxycarbonyl, substituted or unsubstituted heterocyclylsulfinyl or substituted or unsubstituted heterocyclylsulfonyl.

R^{5a}, R^{5b}, R^{6a} and R^{6b} are, for example, each independently hydrogen, or substituted or unsubstituted alkyl.

R^{5a}, R^{5b}, R^{6a} and R^{6b} are, for example, both hydrogen.

As specific aspect of compound (I), the followings are exemplified.

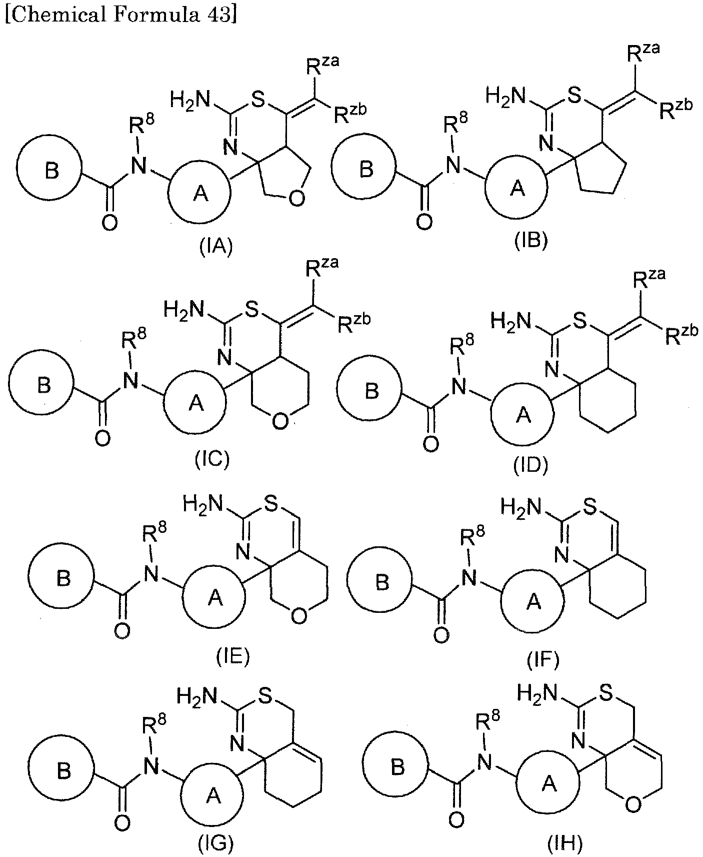

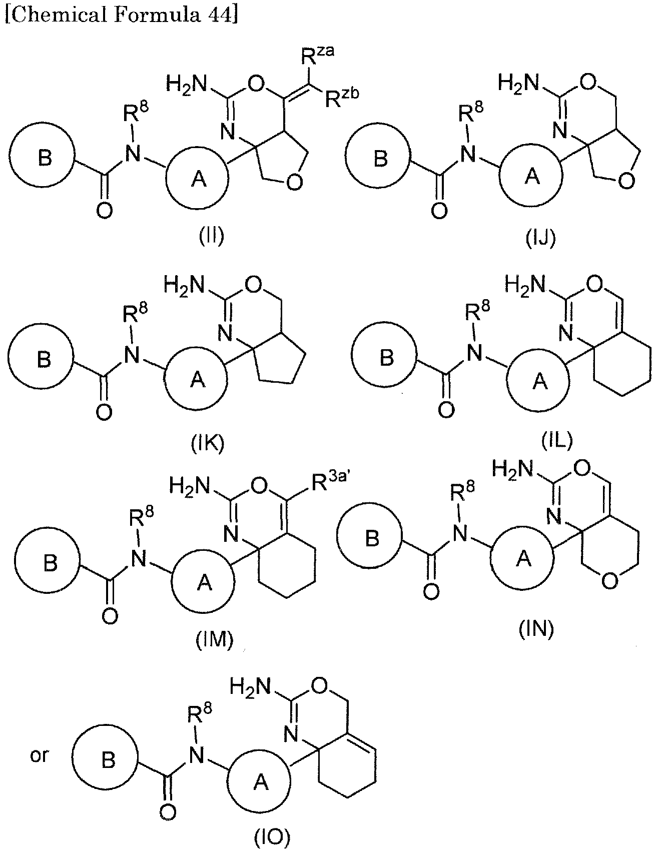

wherein ring A' is a substituted or unsubstituted carbocycle wherein the substituent is, for example, halogen,
ring B is substituted or unsubstituted pyridine, substituted or unsubstituted pyrimidine or substituted or unsubstituted pyrazine wherein the substituent is, for example, halogen, alkyl, alkoxy, halogenoalkoxy, alkynyloxy, alkylcarbamoyl or cyano,
R⁸ is hydrogen, substituted or unsubstituted alkyl, or substituted or unsubstituted acyl, more specifically hydrogen,
R^{za} and R^{zb} are each independently hydrogen, halogen or substituted or unsubstituted alkyl, and
R^{3a} is hydrogen, or substituted or unsubstituted alkyl.

As another aspect, the following compounds are exemplified.
A comopund of the formula (I) wherein

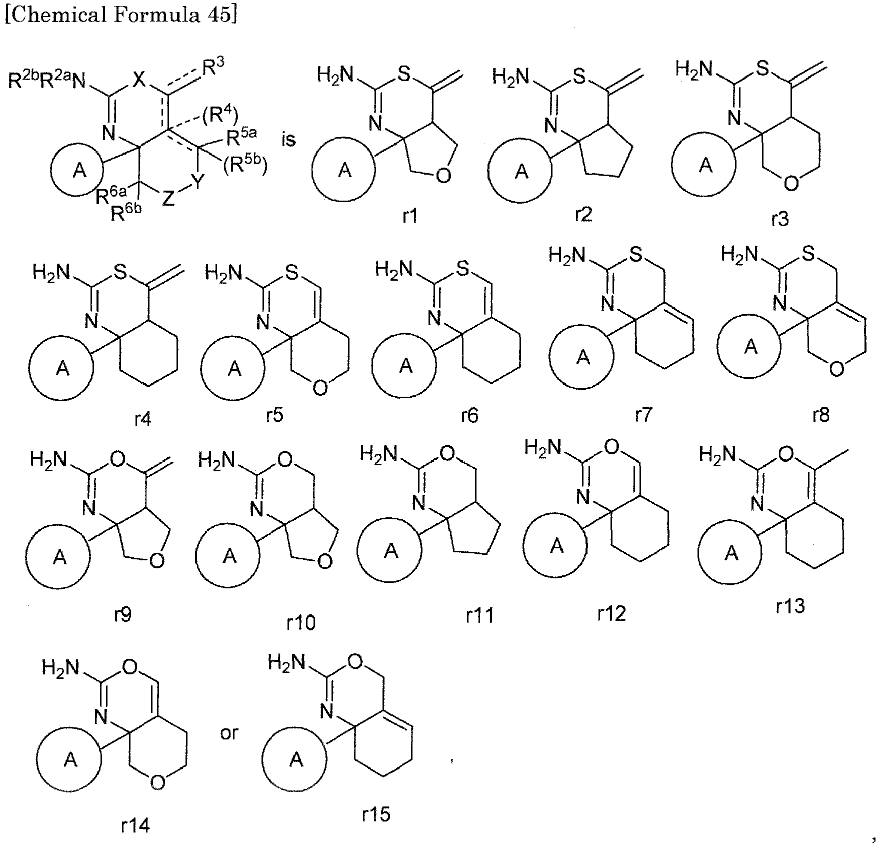

the compound wherein ring A is 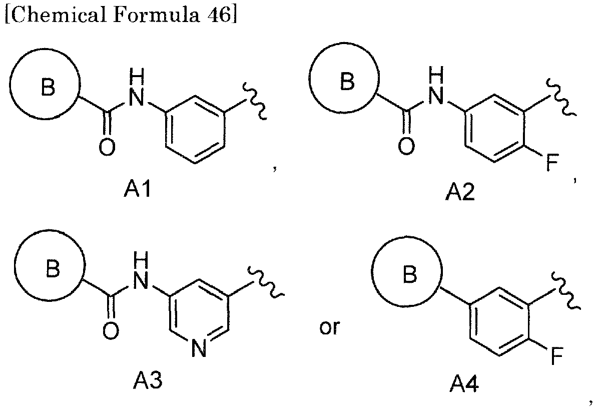 the compound wherein ring B is 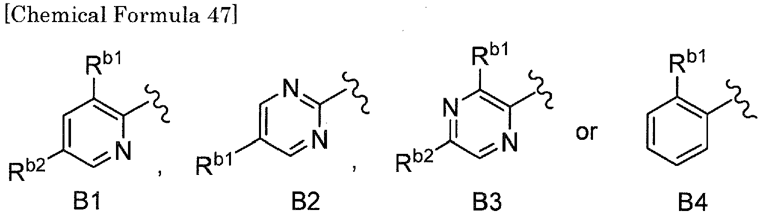 wherein R^{b1} and R^{b2} are each independently hydrogen, chloro, fluoro, methoxy, butynyloxy, cyano, amino or carbamoyl,
the compound wherein a combination of ring B, R^{b1} and R^{b2} (B, R^{b1}, R^{b2}) is as follows:
(B1, hydrogen, hydrogen)(hereinafter, referred. to as ring B is b1),
(B1, hydrogen, chloro)(hereinafter, referred to as ring B is b2),
(B1, hydrogen, fluoro)(hereinafter, referred to as ring B is b3),
(B1, hydrogen, methoxy)(hereinafter, referred to as ring B is b4),
(B1, hydrogen, butynyloxy)(hereinafter, referred to as ring B is b5),
(B1, hydrogen, cyano)(hereinafter, referred to as ring B is b6),
(B1, hydrogen, amino)(hereinafter, referred to as ring B is b7),
(B1, hydrogen, carbamoyl)(hereinafter, referred to as ring B is b8),
(B1, chloro, hydrogen)(hereinafter, referred to as ring B is b9),
(B1, chloro, chloro)(hereinafter, referred to as ring B is b10),
(B1, chloro, fluoro,)(hereinafter, referred to as ring B is b11),
(B1, chloro, methoxy)(hereinafter, referred to as ring B is b12),
(B1, chloro, butynyloxy)(hereinafter, referred to as ring B is b13),
(B1, chloro, cyano)(hereinafter; referred to as ring B is b14),
(B1, chloro, amino)(hereinafter, referred to as ring B is b15),
(B1, chloro, carbamoyl)(hereinafter, referred to as ring B is b16),
(B1, fluoro, hydrogen)(hereinafter, referred to as ring B is b17),
(B1, fluoro, chloro)(hereinafter, referred to as ring B is b18),
(B1 , fluoro, fluoro)(hereinafter, referred to as ring B is b19),
(B1, fluoro, methoxy)(hereinafter, referred to as ring B is b20),
(B1, fluoro, butynyloxy)(hereinafter, referred to as ring B is b21),
(B1, fluoro, cyano)(hereinafter, referred to as ring B is b22),
(B1, fluoro, amino)(hereinafter, referred to as ring B is b23),
(B1, fluoro, carbamoyl)(hereinafter, referred to as ring B is b24),
(B1, methoxy, hydrogen)(hereinafter, referred to as ring B is b25),
(B1, methoxy, chloro)(hereinafter, referred to as ring B is b26),
(B1, methoxy, fluoro)(hereinafter, referred to as ring B is b27),
(B1, methoxy, methoxy)(hereinafter, referred to as ring B is b28),
(B1, methoxy, butynyloxy)(hereinafter, referred to as ring B is b29),
(B1, methoxy, cyano)(hereinafter, referred to as ring B is b30),
(B1, methoxy, amino)(hereinafter, referred to as ring B is b31),
(B1, methoxy, carbamoyl)(hereinafter, referred to as ring B is b32),
(B1, butynyloxy, hydrogen)(hereinafter, referred to as ring B is b33),
(B1, butynyloxy, chloro)(hereinafter, referred to as ring B is b34),
(B1, butynyloxy, fluoro)(hereinafter, referred to as ring B is b35),
(B1, butynyloxy, methoxy)(hereinafter, referred to as ring B is b36),
(B1, butynyloxy, cyano)(hereinafter, referred to as ring B is b37),
(B1, butynyloxy, amino)(hereinafter, referred to as ring B is b38),
(B1, butynyloxy, carbamoyl)(hereinafter, referred to as ring B is b39),
(B1, cyano, hydrogen)(hereinafter, referred to as ring B is b40),
(B, cyano, chloro)(hereinafter, referred to as ring B is b41),
(B1, cyano, fluoro)(hereinafter, referred to as ring B is b42),
(B1, cyano, methoxy)(hereinafter, referred to as ring B is b43),
(B1, cyano, butynyloxy)(hereinafter, referred to as ring B is b44),
(B1, cyano, cyano)(hereinafter, referred to as ring B is b45),
(B1, cyano, amino)(hereinafter, referred to as ring B is b46),
(B1, cyano, carbamoyl)(hereinafter, referred to as ring B is b47),
(B1, amino, hydrogen)(hereinafter, referred to as ring B is b48),
(B1, amino, chloro)(hereinafter, referred to as ring B is b49),
(B1, amino, fluoro)(hereinafter, referred to as ring B is b50),
(B1, amino, methoxy)(hereinafter, referred to as ring B is b51),
(B1, amino, butynyloxy)(hereinafter, referred to as ring B is b52),
(B1, amino, cyano)(hereinafter, referred to as ring B is b53),
(B1, carbamoyl, hydrogen)(hereinafter, referred to as ring B is b54),
(B1, carbamoyl, chloro)(hereinafter, referred to as ring B is b55),
(B1, carbamoyl, fluoro)(hereinafter, referred to as ring B is b56),
(B1, carbamoyl, methoxy)(hereinafter, referred to as ring B is b57),
(B1, carbamoyl, butynyloxy)(hereinafter, referred to as ring B is b58),
(B1, carbamoyl, cyano)(hereinafter, referred to as ring B is b59),

(B3, hydrogen, hydrogen)(hereinafter, referred to as ring B is b60),
(B3, hydrogen, chloro)(hereinafter, referred to as ring B is b61),
(B3, hydrogen, fluoro)(hereinafter, referred to as ring B is b62),
(B3, hydrogen, methoxy)(hereinafter, referred to as ring B is b63),
(B3, hydrogen, butynyloxy)(hereinafter, referred to as ring B is b64),
(B3, hydrogen, cyano)(hereinafter, referred to as ring B is b65),
(B3, hydrogen, amino)(hereinafter, referred to as ring B is b66),
(B3, hydrogen, carbamoyl)(hereinafter, referred to as ring B is b67),
(B3, chloro, hydrogen)(hereinafter, referred to as ring B is b68),
(B3, chloro, chloro)(hereinafter, referred to as ring B is b69),
(B3, chloro, fluoro)(hereinafter, referred to as ring B is b70),
(B3, chloro, methoxy)(hereinafter, referred to as ring B is b71),
(B3, chloro, butynyloxy)(hereinafter, referred to as ring B is b72),
(B3, chloro, cyano)(hereinafter, referred to as ring B is b73),
(B3, chloro, amino)(hereinafter, referred to as ring B is b74),
(B3, chloro, carbamoyl)(hereinafter, referred to as ring B is b75),
(B3, fluoro, hydrogen)(hereinafter, referred to as ring B is b76),
(B3, fluoro, chloro)(hereinafter, referred to as ring B is b77),
(B3, fluoro, fluoro)(hereinafter, referred to as ring B is b78),
(B3, fluoro, methoxy)(hereinafter, referred to as ring B is b79),
(B3, fluoro, butynyloxy)(hereinafter, referred to as ring B is b80),
(B3, fluoro, cyano)(hereinafter, referred to as ring B is b81),
(B3, fluoro, amino)(hereinafter, referred to as ring B is b82),
(B3, fluoro, carbamoyl)(hereinafter, referred to as ring B is b83),
(B3, methoxy, hydrogen)(hereinafter, referred to as ring B is b84),
(B3, methoxy, chloro)(hereinafter, referred to as ring B is b85),
(B3, methoxy, fluoro)(hereinafter, referred to as ring B is b86),
(B3, methoxy, methoxy)(hereinafter, referred to as ring B is b87),
(B3, methoxy, butynyloxy)(hereinafter, referred to as ring B is b88),
(B3, methoxy, cyano)(hereinafter, referred to as ring B is b89),
(B3, methoxy, amino)(hereinafter, referred to as ring B is b90),
(B3, methoxy, carbamoyl)(hereinafter, referred to as ring B is b91),
(B3, butynyloxy, hydrogen)(hereinafter, referred to as ring B is b92),
(B3, butynyloxy, chloro)(hereinafter, referred to as ring B is b93),
(B3, butynyloxy, fluoro)(hereinafter, referred to as ring B is b94),
(B3, butynyloxy, methoxy)(hereinafter, referred to as ring B is b95),
(B3, butynyloxy, cyano)(hereinafter, referred to as ring B is b96),
(B3, butynyloxy, amino)(hereinafter, referred to as ring B is b97),
(B3, butynyloxy, carbamoyl)(hereinafter, referred to as ring B is b98),
(B3, cyano, hydrogen)(hereinafter, referred to as ring B is b99),
(B3, cyano, chloro,)(hereinafter, referred to as ring B is b100),
(B3, cyano, fluoro)(hereinafter, referred to as ring B is b101),
(B3, cyano, methoxy)(hereinafter, referred to as ring B is b102),
(B3, cyano, butynyloxy)(hereinafter, referred to as ring B is b103),
(B3, cyano, cyano)(hereinafter, referred to as ring B is b104),
(B3, cyano, amino)(hereinafter, referred to as ring B is b105),
(B3, cyano, carbamoyl)(hereinafter, referred to as ring B is b106),
(B3, amino, hydrogen)(hereinafter, referred to as ring B is b107),
(B3, amino, chloro)(hereinafter, referred to as ring B is b108),
(B3, amino, fluoro)(hereinafter, referred to as ring B is b109),
(B3, amino, methoxy)(hereinafter, referred to as ring B is b110),
(B3, amino, butynyloxy)(hereinafter, referred to as ring B is b111),
(B3, amino, cyano)(hereinafter, referred to as ring B is b112),
(B3, carbamoyl, hydrogen)(hereinafter, referred to as ring B is b113),
(B3, carbamoyl, chloro)(hereinafter, referred to as ring B is b114),
(B3, carbamoyl, fluoro)(hereinafter, referred to as ring B is b115),
(B3, carbamoyl, methoxy)(hereinafter, referred to as ring B is b116),
(B3, carbamoyl, butynyloxy)(hereinafter, referred to as ring B is b117),
(B3, carbamoyl, cyano)(hereinafter, referred to as ring B is b118),

compounds wherein a combination of ring B and R^{b1} is as follows.
(B2, hydrogen)(hereinafter, referred to as ring B is b119),
(B2, chloro)(hereinafter, referred to as ring B is b120),
(B2, fluoro)(hereinafter, referred to as ring B is b121),
(B2, methoxy)(hereinafter, referred to as ring B is b122),
(B2, butynyloxy)(hereinafter, referred to as ring B is b123),
(B2, cyano)(hereinafter, referred to as ring B is b124),
(B2, amino)(hereinafter, referred to as ring B is b125),
(B2, carbamoyl)(hereinafter, referred to as ring B is b126),
(B4, hydrogen)(hereinafter, referred to as ring B is b127),
(B4, chloro)(hereinafter, referred to as ring B is b128),
(B4, fluoro)(hereinafter, referred to as ring B is b129),
(B4, methoxy)(hereinafter, referred to as ring B is b130),
(B4, butynyloxy)(hereinafter, referred to as ring B is b131),
(B4, cyano)(hereinafter, referred to as ring B is b132),
(B4, amino)(hereinafter, referred to as ring B is b133), or
(B4, carbamoyl)(hereinafter, referred to as ring B is b134).

Compounds of the formula (I) wherein a combination of 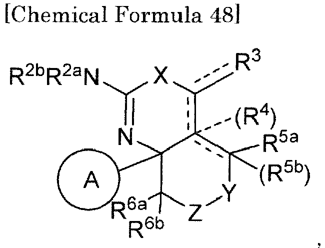 ring A and ring B (r,A,b) is as follows.
(r1,A1,b1),(r1,A1,b2),(r1,A1,b3),(r1,A1,b4),(r1,A1,b5),(r1,A1,b6),(r1,A1,b7),(r1,A1,b8),(r1,A1,b9),( r1,A1,b10),(r1,A1,b11),(r1,A1,b12),(r1,A1,b13),(r1,A1,b14),(r1,A1,b15),(r1,A1,b16),(r1,A1,b17),(r1 ,A1,b18),(r1,A1,b19),(r1,A1,b20),(r1,A1,b21),(r1,A1,b22),(r1,A1,b23),(r1,A1,b24),(r1,A1,b25),(r1,A 1,b26),(r1,A1,b27),(r1,A1,b28),(r1,A1,b29),(r1,A1,b30),(r1,A,b31),(r1,A1,b32),(r1,A1,b33),(r1,A1, b34),(r1,A1,b35),(r1,A1,b36),(r1,A1,b37),(r1,A1,b38),(r1,A1,b39),(r1,A1,b40),(r1,A1,b41),(r1,A1,b 42),(r1,A1,b43),(r1,A1,b44),(r1,A1,b45),(r1,A1,b46),(r1,A1,b47),(r1,A1,b48),(r1,A1,b49),(r1,A1,b5 0),(r1,A1,b51),(r1,A1,b52),(r1,A1,b53),(r1,A1,b54),(r1,A1,b55),(r1,A1,b56),(r1,A1,b57),(r1,A1,b58) ,(r1,A1,b59),(r1,A1,b60),(r1,A1,b61),(r1,A1,b62),(r1,A1,b63),(r1,A1,b64),(r1,A1,b65),(r1,A1,b66),( r1,A1,b67),(r1,A1,b68),(r1,A1,b69),(r1,A1,b70),(r1,A1,b71),(r1,A1,b72),(r1,A1,b73),(r1,A1,b74),(r1 ,A1,b75),(r1,A1,b76),(r1,A1,b77),(r1,A1,b78),(r1,A1,b79),r1,A1,b80),(r1,A1,b81),(r1,A1,b82),(r1,A 1,b83),(r1,A1,b84),(r1,A1,b85),(r1,A1,b86),(r1,A1,b87),(r1,A1,b88),(r1,A1,b89),(r1,A1,b90),(r1,A1, b91),(r1,A1,b92),(r1,A1,b93),(r1,A1,b94),(r1,A1,b95),(r1,A1,b96),(r1,A1,b97),(r1,A1,b98),(r1,A1,b 99),(r1,A1,b100),(r1,A1,b101),(r1,A1,b102),(r1,A1,b103),(r1,A1,b104),(r1,A1,b105),(r1,A1,b106),(r 1,A1,b107),(r1,A1,b108),(r1,A1,b109),(r1,A1,b110),(r1,A1,b111),(r1,A,b112),(r1,A1,b113),(r1,A1, b114),(r1,A1,b115),(r1,A1,b116),(r1,A1,b117),(r1,A1,b118),(r1,A1,b119),(r1,A1,b120),(r1,A1,b121), (r1,A1,b122),(r1,A1,b123),(r1,A1,b124),(r1,A1,b125),(r1,A1,b126),(r1,A1,b127),(r1,A1,b128),(r1,A 1,b129),(r1,A1,b130),(r1,A1,b131),(r1,A1,b132),(r1,A1,b133),(r1,A1,b134),(r1,A2,b1),(r1,A2,b2),(r 1,A2,b3),(r1,A2,b4),(r1,A2,b5),(r1,A2,b6),(r1,A2,b7),(r1,A2,b8),(r1,A2,b9),(r1,A2,b10),(r1,A2,b1), (r1,A2,b12),(r1,A2,b13),(r1,A2,b14),(r1,A2,b15),(r1,A2,b16),(r1,A2,b17),(r1,A2,b18),(r1,A2,b19),(r 1,A2,b20),(r1,A2,b21),(r1,A2,b22),(r1,A2,b23),(r1,A2,b24),(r1,A2,b25),(r1,A2,b26),(r1,A2,b27),(r1, A2,b28),(r1,A2,b29),(r1,A2,b30),(r1,A2,b31),(r1,A2,b32),(r1,A2,b33),(r1,A2,b34),(r1,A2,b35),(r1,A 2,b36),(r1,A2,b37),(r1,A2,b38),(r1,A2,b39),(r1,A2,b40),(r1,A2,b41),(r1,A2,b42),(r1,A2,b43),(r1,A2, b44),(r1,A2,b45),(r1,A2,b46),(r1,A2,b47),(r1,A2,b48),(r1,A2,b49),(r1,A2,b50),(r1,A2,b51),(r1,A2,b 52),(r1,A2,b53),(r1,A2,b54),(r1,A2,b55),(r1,A2,b56),(r1,A2,b577),(r1,A2,b58),(r1,A2,b59),(r1,A2,b6 0),(r1,A2,b61),(r1,A2,b62),(r1,A2,b63),(r1,A2,b64),(r1,A2,b65),(r1,A2,b66),(r1,A2,b67),(r1,A2,b68) ,(r1,A2,b69),(r1,A2,b70),(r1,A2,b71),(r1,A2,b72),(r1,A2,b73),(r1,A2,b74),(r1,A2,b75),(r1,A2,b76),( r1,A2,b77),(r1,A2,b78),(r1,A2,b79),(r1,A2,b80),(r1,A2,b81),(r1,A2,b82),(r,A2,b83),(r1,A2,b84),(r1 ,A2,b85),(r1,A2,b86),(r1,A2,b87),(r1,A2,b88),(r1,A2,b89),(r1,A2,b90),(r1,A2,b91),(r1,A2,b92),(r1,A 2,b93),(r1,A2,b94),(r1,A2,b95),(r1,A2,b96),(r1,A2,b97),(r1,A2,b98),(r1,A2,b99),(r1,A2,b 100),(r1,A 2,b101),(r1,A2,b 102),(r1,A2,b103),(r1,A2,b104),(r1,A2,b105),(r1,A2,b106),(r1,A2,b107),(r1,A2,b 10 8),(r1,A2,b109),(r1,A2,b110),(r1,A2,b111),(r1,A2,b112),(r1,A2,b113),(r1,A2,b114),(r1,A2,b115),(r1, A2,b116),(r1,A2,b117),(r1,A2,b118),(r1,A2,b119),(r1,A2,b120),(r1,A2,b121),(r1,A2,b122),(r1,A2,b1 23),(r1,A2,b124),(r1,A2,b125),(r1,A2,b126),(r1,A2,b127),(r1,A2,b128),(r1,A2,b129),(r1,A2,b 130),(r 1,A2,b131),(r1,A2,b132),(r1,A2,b133),(r1,A2,b134),(r1,A3,b1),(r1,A3,b2),(r1,A3,b3),(r1,A3,b4),(r1, A3,b5),(r1,A3,b6),(r1,A3,b7),(r1,A3,b8),(r1,A3,b9),(r1,A3,b10),(r1,A3,b11),(r1,A3,b12),(r1,A3,b13), (r1,A3,b14),(r1,A3,b15),(r1,A3,b16),(r1,A3,b17),(r1,A3,b18),(r1,A3,b19),(r1,A3,b20),(r1,A3,b21),(r 1,A3,b22),(r1,A3,b23),(r1,A3,b24),(r1,A3,b25),(r1,A3,b26),(r1,A3,b27),(r1,A3,b28),(r1,A3,b29),(r1, A3,b30),(r1,A3,b31),(r1,A3,b32),(r1,A3,b33),(r1,A3,b34),(r1,A3,b35),(r1,A3,b36),(r1,A3,b37),(r1,A 3,b38),(r1,A3,b39),(r1,A3,b40),(r1,A3,b41),(r1,A3,b42),(r1,A3,b43),(r1,A3,b44),(r1,A3,b45),(r1,A3, b46),(r1,A3,b47),(r1,A3,b48),(r1,A3,b49),(r1,A3,b50),(r1,A3,b51),(r1,A3,b52),(1,A3,b53),(r1,A3,b 54),(r1,A3,b55),(r1,A3,b56),(r1,A3,b57),(r1,A3,b58),(r1,A3,b59),(r1,A3,b60),(r1,A3,b61),(r1,A3,b6 2),(r1,A3,b63),(r1,A3,b64),(r1,A3,b65),(r1,A3,b66),(r1,A3,b67),(r1,A3,b68),(r1,A3,b69),(r1,A3,b70) ,(r1,A3,b71),(r1,A3,b72),(r1,A3,b73),(r1,A3,b74),(r1,A3,b75),(r1,A3,b76),(r1A3,b77),(r1,A3,b78),( r1,A3,b79),(r1,A3,b80),(r1,A3,b81),(r1,A3,b82),(r1,A3,b83),(r1,A3,b84),(r1,A3,b85),(r1,A3,b86),(r1 ,A3,b87),(r1,A3,b88),(r1,A3,b89),(r1,A3,b90),(r1,A3,b91),(r1,A3,b92),(r1,A3,b93),(r1,A3,b94),(r1,A 3,b95),(r1,A3,b96),(r1,A3,b97),(r1,A3,b98),(r1,A3,b99),(r1,A3,b100),(r1,A3,b101),(r1,A3,b102),(r1, A3,b1.03),(r1,A3,b104),(r1,A3,b105),(r1,A3,b106),(r1,A3,b107),(r1,A3,b108),(r1,A3,b109),(r1,A3,b 110),(r1,A3,b111),(r1,A,3,b112),(r1,A3,b113),(r1,A3,b114),(r1,A3,b115),(r1,A3,b116),(r1,A3,b117),( r1,A3,b118),(r1,A3,b11.9),(r1,A3,b120),(r1,A3,b121),(r1,A3,b122),(r1,A3,b123),(r1,A3,b124),(r1,A3 ,b125),(r1,A3,b126),(r1,A3,b127),(r1,A3,b128),(r1,A3,b129),(r1,A3,b130),(r1,A3,b131),(r1,A3,b132 ),(r1,A3,b133),(r1,A3,b134),(r1,A4,b1),(r1,A4,b2),(r1,A4,b3),(r1,A4,b4),(r1,A4,b5),(r1,A4,b6),(r1,A 4,b7),(r1,A4,b8),(r1,A4,b9),(r1,A4,b10),(r1,A4,b11),(r1,A4,b12),(r1,A4,b13),(r1,A4,b14),(r1,A4,b15 ),(r1,A4,b16),(r1,A4,b17),(r1,A4,b18),(r1,A4,b19),(r1,A4,b20),(r1,-A4,b21),(r1,A4,b22),(r1,A4,b23),( r1,A4,b24),(r1,A4,b25),(r1,A4,b26),(r1,A4,b27),(r1,A4,b28),(r1,A4,b29),(r1,A4,b30),(r1,A4,b31),(r1 ,A4,b32),(r1,A4,b33),(r1,A4,b34),(r1,A4,b35),(r1,A4,b36),(r1,A4,b37),(r1,A4,b38),(r1,A4,b39),(r1,A 4,b40),(r1,A4,b41),(r1,A4,b42),(r1,A4,b43),(r1,A4,b44),(r1,A4,b45),(r1,A4,b46),(r1,A4,b47),(r1,A4, b48),(r1,A4,b49),(r1,A4,b50),(r1,A4,b51),(r1,A4,b52),(r1,A4,b53),(r1,A4,b54),(r1,A4,b55),(r1,A4,b 56),(r1,A4,b57),(r1,A4,b58),(r1,A4,b59),(r1,A4,b60),(r1,A4,b61),(r1,A4,b62),(r1,A4,b63),(r1,A4,b6 4),(r1,A4,b65),(r1,A4,b66),(r1,A4,b67),(r1,A4,b68),(r1,A4,b69),(r1,A4,b70),(r1,A4,b71),(r1,A4,b72) ,(r1,A4,b73),(r1,A4,b74),(r1,A4,b75),(r1,A4,b76),(r1,A4,b77),(r1,A4,b78),(r1,A4,b79),(r1,A4,b80),( r1,A4,b81),(r1,A4,b82),(r1,A4,b83),(r1,A4,b84),(r1,A4,b85),(r1,A4,b86),(r1,A4,b87),(r1,A4,b88),(r1 ,A4,b89),(r1,A4,b90),(r1,A4,b91),(r1,A4,b92),(r1,A4,b93),(r1,A4,b94),(r1,A4,b95),(r1,A4,b96),(r1,A 4,b97),(r1,A4,b98),(r1,A4,b99),(r1,A4,b100),(r1,A4,b101),(r1,A4,b102),(r1,A4,b103),(r1,A4,b104),( r1,A4,b105),(r1,A4,b106),(r1,A4,b107),(r1,A4,b108),(r1,A4,b109),(r1,A4,b110),(r1,A4,b111),(r1,A4 ,b112),(r1,A4,b113),(r1,A4,b114),(r1,A4,b115),(r1,A4,b116),(r1,A4,b117),(r1,A4,b118),(r1,A4,b119) ,(r1,A4,b120),(r1,A4,b121),(r1,A4,b122),(r1,A4,b123),(r1,A4,b124),(r1,A4,b125),(r1,A4,b126),(r1, A4,b127),(r1,A4,b128),(r1,A4,b129),(r1,A4,b130),(r1,A4,b131),(r1,A4,b132),(r1,A4,b133),(r1,A4,b 134),

(r2,A1,b1),(r2,A1,b2),(r2,A1,b3),(r2,A1,b4),(r2,A1,b5),(r2,A1,b6),(r2,A1,b7),(r2,A1,b8),(r2,A1,b9),( r2,A1,b10),(r2,A1,b11),(r2,A1,b12),(r2,A1,b13),(r2,A1,b14),(r2,A1,b15),(r2,Al,b16),(r2,A1,b17),(r2 ,A1,b18),(r2,A1,b19),(r2,A1,b20),(r2,A1,b21),(r2,A1,b22),(r2,A1,b23),(r2,A1,b24),(r2,A1,b25),(r2,A 1,b26),(r2,A1,b27),(r2,A1,b28),(r2,A1,b29),(r2,A1,b30),(r2,A1,b31),(r2,A1,b32),(r2,A1,b33),(r2,A1, b34),(r2,A1,b35),(r2,A1,b36),(r2,A1,b37),(r2,A1,b38),(r2,A1,b39),(r2,A1,b40),(r2,A1,b41),(r2,A1,b 42),(r2,A1,b43),(r2,A1,b44),(r2,A1,b45),(r2,A1,b46),(r2,A1,b47),(r2,A1,b48),(r2,A1,b49),(r2,A1,b5 0),(r2,A1,b51),(r2,A1,b52),(r2,A1,b53),(r2,A1,b54),(r2,A1,b55),(r2,A1,b56),(r2,A1,b57),(r2,A1,b58) ,(r2,A1,b59),(r2,A1,b60),(r2,A1,b61),(r2,A1,b62),(r2,A1,b63),(r2,A1,b64),(r2,A1,b65),(r2,A1,b66),( r2,A1,b67),(r2,A1,b68),(r2,A1,b69),(r2,A1,b70),(r2,A1,b71),(r2,A1,b72),(r2,A1,b73),(r2,A1,b74),(r2 ,A1,b75),(r2,A1,b76),(r2,A1,b77),(r2,A1,b78),(r2,A1,b79),(r2,A1,b80),(r2,A1,b81),(r2,A1,b82),(r2,A 1,b83),(r2,A1,b84),(r2,A1,b85),(r2,A1,b86),(r2,A1,b87),(r2,A1,b88),(r2,A1,b89),(r2,A1,b90),(r2,A1, b91),(r2,A1,b92),(r2,A1,b93),(r2,A1,b94),(r2,A1,b95),(r2,A1,b96),(r2,A1,b97),(r2,A1,b98),(r2,A1,b 99),(r2,A1,b100),(r2,A1,b101),(r2,A1,b102),(r2,A1,b103),(r2,A1,b104),(r2,A1,b105),(r2,A1,b106),(r 2,A1,b107),(r2,A1,b108),(r2,A1,b109),(r2,Al,b110),(r2,A1,b111),(r2,A1,b112),(r2,A1,b113),(r2,A1, b114),(r2,A1,b115),(r2,A1,b116),(r2,A1,b117),(r2,A1,b118),(r2,A1,b119),(r2,A1,b120),(r2,A1,b121), (r2,A1,b122),(r2,A1,b123),(r2,A1,b124),(r2,A1,b125),(r2,A1,b126),(r2,A1,b127),(r2,A1,b128),(r2,A 1,b129),(r2,A1,b130),(r2,A1,b131),(r2,A1,b132),(r2,A1,b133),(r2,A1,b134),(r2,A2,b1),(r2,A2,b2),(r 2,A2,b3),(r2,A2,b4),(r2,A2,b5),(r2,A2,b6),(r2,A2,b7),(r2,A2,b8),(r2,A2,b9),(r2,A2,b10),(r2,A2,b11), (r2,A2,b 12),(r2,A2,b 13),(r2,A2,b 14),(r2,A2,b 15),(r2,A2,b 16),(r2,A2,b 17),(r2,A2,b 18),(r2,A2,b 19),(r 2,A2,b20),(r2,A2,b21),(r2,A2,b22),(r2,A2,b23),(r2,A2,b24),(r2,A2,b25),(r2,A2,b26),(r2,A2,b27),(r2, A2,b28),(r2,A2,b29),(r2,A2,b30),(r2,A2,b31),(r2,A2,b32),(r2,A2,b33),(r2,A2,b34),(r2,A2,b35),(r2,A 2,b36),(r2,A2,b37),(r2,A2,b38),(r2,A2,b39),(r2,A2,b40),(r2,A2,b41),(r2,A2,b42),(r2,A2,b43),(r2,A2, b44),(r2,A2,b45),(r2,A2,b46),(r2,A2,b47),(r2,A2,b48),(r2,A2,b49),(r2,A2,b50),(r2,A2,b51),(r2,A2,b 52),(r2,A2,b53),(r2,A2,b54),(r2,A2,b55),(r2,A2,b56),(r2,A2,b57),(r2,A2,b58),(r2,A2,b59),(r2,A2,b6 0),(r2,A2,b61),(r2,A2,b62),(r2,A2,b63),(r2,A2,b64),(r2,A2,b65),(r2,A2,b66),(r2,A2,b67),(r2,A2,b68) , (r2,A2,b69), (r2,A2,b70),(r2,A2,b71), (r2,A2,b72), (r2,A2,b73), (r2,A2,b74), (r2,A2,b75), (r2,A2,b76),( r2,A2,b77),(r2,A2,b78),(r2,A2,b79),(r2,A2,b80),(r2,A2,b81),(r2,A2,b82),(r2,A2,b83),(r2,A2,b84),(r2 ,A2,b85),(r2,A2,b86),(r2,A2,b87),(r2,A2,b88),(r2,A2,b89),(r2,A2,b90),(r2,A2,b91),(r2,A2,b92),(r2,A 2,b93),(r2,A2,b94),(r2,A2,b95),(r2,A2,b96),(r2,A2,b97),(r2,A2,b98),(r2,A2,b99),(r2,A2,b100),(r2,A 2,b101),(r2,A2,b102),(r2,A2,b103),(r2,A2,b104),(r2,A2,b105),(r2,A2,b106),(r2,A2,b107),(r2,A2,b10 8),(r2,A2,b109),(r2,A2,b110),(r2,A2,b111),(r2,A2,b112),(r2,A2,b113),(r2,A2,b114),(r2,A2,b4115), (r2, A2,b116),(r2,A2,b117),(r2,A2,b118),(r2,A2,b119),(r2,A2,b120),(r2,A2,b121),(r2,A2,b122),(r2,A2,b1 23),(r2,A2,b124),(r2,A2,b125),(r2,A2,b126),(r2,A2,b127),(r2,A2,b128),(r2,A2,b129),(r2,A2,b130),(r 2,A2,b131),(r2,A2,b132),(r2,A2,b133),(r2,A2,b134),(r2,A3,b1),(r2,A3,b2),(r2,A3,b3),(r2,A3,b4),(r2, A3,b5),(r2,A3,b6),(r2,A3,b7),(r2,A3,b8),(r2,A3,b9),(r2,A3,b10),(r2,A3,b11),(r2,A3,b12),(r2,A3,b13), (r2,A3,b14),(r2,A3,b15),(r2,A3,b16),(r2,A3,b17),(r2,A3,b18),(r2,A3,b19),(r2,A3,b20),(r2,A3,b21),(r 2,A3,b22),(r2,A3,b23),(r2,A3,b24),(r2,A3,b25),(r2,A3,b26),(r2,A3,b27),(r2,A3,b28),(r2,A3,b29),(r2, A3,b30),(r2,A3,b31),(r2,A3,b32),(r2,A3,b33),(r2,A3,b34),(r2,A3,b35),(r2,A3,b36),(r2,A3,b37),(r2,A 3,b38),(r2,A3,b39),(r2,A3,b40),(r2,A3,b41),(r2,A3,b42),(r2,A3,b43),(r2,A3,b44),(r2,A3,b45),(r2,A3, b46),(r2,A3,b47),(r2,A3,b48),(r2,A3,b49),(r2,A3,b50),(r2,A3,b51),(r2,A3,b52),(r2,A3,b53),(r2,A3,b 54),(r2,A3,b55),(r2,A3,b56),(r2,A3,b57),(r2,A3,b58),(r2,A3,b59),(r2,A3,b60),(r2,A3,b61),(r2,A3,b6 2),(r2,A3,b63),(r2,A3,b64),(r2,A3,b65),(r2,A3,b66),(r2,A3,b67),(r2,A3,b68),(r2,A3,b69),(r2,A3,b70) ,(r2,A3,b71),(r2,A3,b72),(r2,A3,b73),(r2,A3,b74),(r2,A3,b75),(r2,A3,b76),(r2,A3,b77),(r2,A3,b78),( r2,A3,b79),(r2,A3,b80),(r2,A3,b81),(r2,A3,b82),(r2,A3,b83),(r2,A3,b84),(r2,A3,b85),(r2,A3,b86),(r2 ,A3,b87),(r2,A3,b88),(r2,A3,b89),(r2,A3,b90),(r2,A3,b91),(r2,A3,b92),(r2,A3,b93),(r2,A3,b94),(r2,A 3,b95),(r2,A3,b96),(r2,A3,b97),(r2,A3,b98),(r2,A3,b99),(r2,A3,b100),(r2,A3,b101),(r2,A3,b102),(r2, A3,b103),(r2,A3,b104),(r2,A3,b105),(r2,A3,b106),(r2,A3,b107),(r2,A3,b108),(r2,A3,b109),(r2,A3,b 110),(r2,A3,b111),(r2,A3,b112),(r2,A3,b113),(r2,A3,b114),(r2,A3,b115),(r2,A3,b116),(r2,A3,b117),( r2,A3,b118),(r2,A3,b119),(r2,A3,b120),(r2,A3,b121),(r2,A3,b122),(r2,A3,b123),(r2,A3,b124),(r2,A3 ,b125),(r2,A3,b126),(r2,A3,b127),(r2,A3,b128),(r2,A3,b129),(r2,A3,b130),(r2,A3,b131),(r2,A3,b132 ),(r2,A3,b133),(r2,A3,b134),(r2,A4,b1),(r2,A4,b2),(r2,A4,b3),(r2,A4,b4),(r2,A4,b5),(r2,A4,b6),(r2,A 4,b7),(r2,A4,b8),(r2,A4,b9),(r2,A4,b10),(r2,A4,b11),(r2,A4,b12),(r2,A4,b13),(r2,A4,b14),(r2,A4,b15 ),(r2,A4,b16),(r2,A4,b17),(r2,A4,b18),(r2,A4,b19),(r2,A4,b20),(r2,A4,b21),(r2,A4,b22),(r2,A4,b23), r2,A4,b24),(r2,A4,b25),(r2,A4,b26),(r2,A4,b27),(r2,A4,b28),(r2,A4,b29),(r2,A4,b30),(r2,A4,b31),(r2 ,A4,b32),(r2,A4,b33),(r2,A4,b34),(r2,A4,b35),(r2,A4,b36),(r2,A4,b37),(r2,A4,b38),(r2,A4,b39),(r2,A 4,b40),(r2,A4,b41),(r2,A4,b42),(r2,A4,b43),(r2,A4,b44),(r2,A4,b45),(r2,A4,b46),(r2,A4,b47),(r2,A4, b48),(r2,A4,b49),(r2,A4,b50),(r2,A4,b51),(r2,A4,b52),(r2,A4,b53),(r2,A4,b54),(r2,A4,b55),(r2,A4,b 56),(r2,A4,b57),(r2,A4,b58),(r2,A4,b59),(r2,A4,b60),(r2,A4,b61),(r2,A4,b62),(r2,A4,b63),(r2,A4,b6 4),(r2,A4,b65),(r2,A4,b66),(r2,A4,b67),(r2,A4,b68),(r2,A4,b69),(r2,A4,b70),(r2,A4,b71),(r2,A4,b72) ,(r2,A4,b73),(r2,A4,b74),(r2,A4,b75),(r2,A4,b76),(r2,A4,b77),(r2,A4,b78),(r2,A4,b79),(r2,A4,b80),( r2,A4,b81),(r2,A4,b82),(r2,A4,b83),(r2,A4,b84),(r2,A4,b85),(r2,A4,b86),(r2,A4,b87),(r2,A4,b88),(r2 ,A4,b89),(r2,A4,b90),(r2,A4,b91),(r2,A4,b92),(r2,A4,b93),(r2,A4,b94),(r2,A4,b95),(r2,A4,b96),(r2,A 4,b97),(r2,A4,b98),(r2,A4,b99),(r2,A4,b100),(r2,A4,b101),(r2,A4,b102),(r2,A4,b103),(r2,A4,b104),( r2,A4,b105),(r2,A4,b106),(r2,A4,b107),(r2,A4,b108),(r2,A4,b109),(r2,A4,b110),(r2,A4,b111),(r2,A4 ,b112),(r2,A4,b113),(r2,A4,b114),(r2,A4,b115),(r2,A4,b116),(r2,A4,b117),(r2,A4,b118),(r2,A4,b119) ,(r2,A4,b120),(r2,A4,b121),(r2,A4,b122),(r2,A4,b123),(r2,A4,b124),(r2,A4,b125),(r2,A4,b126),(r2, A4,b127),(r2,A4,b128),(r2,A4,b129),(r2,A4,b130),(r2,A4,b131),(r2,A4,b132),(r2,A4,b133),(r2,A4,b 134),

(r3,A1,b1),(r3,A1,b2),(r3,A1,b3),(r3,A1,b4),(r3,A1,b5),(r3,A1,b6),(r3,A1,b7),(r3,A1,b8),(r3,A1,b9),( r3,A1,b10),(r3,A1,b11),(r3,A1,b12),(r3,A1,b13),(r3,A1,b14),(r3,A1,b15),(r3,A1,b16),(r3,A1,b17),(r3 ,A1,b18),(r3,A1,b19),(r3,A1,b20),(r3,A1,b21),(r3,A1,b22),(r3,A1,b23),(r3,A1,b24),(r3,A1,b25),(r3,A 1,b26),(r3,A1,b27),(r3,A1,b28),(r3,A1,b29),(r3,A1,b30),(r3,A1,b31),(r3,A1,b32),(r3,A1,b33),(r3,A1, b34),(r3,A1,b35),(r3,A1,b36),(r3,A1,b37),(r3,A1,b38),(r3,A1,b39),(r3,A1,b40),(r3,A1,b41),(r3,A1,b 42),(r3,A1,b43),(r3,A1,b44),(r3,A1,b45),(r3,A1,b46),(r3,A1,b47),(r3,A1,b48),(r3,A1,b49),(r3,A1,b5 0),(r3,A1,b51),(r3,A1,b52),(r3,A1,b53),(r3,A1,b54),(r3,A1,b55),(r3,A1,b56),(r3,A1,b57),(r3,A1,b58) ,(r3,A1,b59),(r3,A1,b60),(r3,A1,b61),(r3,A1,b62),(r3,A1,b63),(r3,A1,b64),(r3,A1,b65),(r3,A1,b66),( r3,A1,b67),(r3,A1,b68),(r3,A1,b69),(r3,A1,b70),(r3,A1,b71),(r3,A1,b72),(r3,A1,b73),(r3,A1,b74),(r3 ,A1,b75),(r3,A1,b76),(r3,A1,b77),(r3,A1,b78),(r3,A1,b79),(r3,A1,b80),(r3,A1,b81),(r3,A1,b82),(r3,A 1,b83),(r3,A1,b84),(r3,A1,b85),(r3,A1,b86),(r3,A1,b87),(r3,A1,b88),(r3,A1,b89),(r3,A1,b90),(r3,A1, b91),(r3,A1,b92),(r3,A1,b93),(r3,A1,b94),(r3,A1,b95),(r3,A1,b96),(r3,A1,b97),(r3,A1,b98),(r3,A1,b 99),(r3,A1,b100),(r3,A1,b101),(r3,A1,b102),(r3,A1,b103),(r3,A1,b104),(r3,A1,b105),(r3,A1,b106),(r 3,A1,b107),(r3,A1,b108),(r3,A1,b109),(r3,A1,b110),(r3,A1,b111),(r3,A1,b112),(r3,A1,b113),(r3,A1, b114),(r3,A1,b115),(r3,A1,b116),(r3,A1,b117),(r3,A1,b118),(r3,A1,b119),(r3,A1,b120),(r3,A1,b121), (r3,A1,b122),(r3,A1,b123),(r3,A1,b124),(r3,A1,b125),(r3,A1,b126),(r3,A1,b127),(r3,A1,b128),(r3,A 1,b129),(r3,A1,b130),(r3,A1,b131),(r3,A1,b132),(r3,A1,b133),(r3,A1,b134),(r3,A2,b1),(r3,A2,b2),(r 3,A2,b3),(r3,A2,b4),(r3,A2,b5),(r3,A2,b6),(r3,A2,b7),(r3,A2,b8),(r3,A2,b9),(r3,A2,b10),(r3,A2,b11), (r3,A2,b12),(r3,A2,b13),(r3,A2,b14),(r3,A2,b15),(r3,A2,b16),(r3,A2,b17),(r3,A2,b18),(r3,A2,b19),(r 3,A2,b20),(r3,A2,b21),(r3,A2,b22),(r3,A2,b23),(r3,A2,b24),(r3,A2,b25),(r3,A2,b26),(r3,A2,b27),(r3, A2,b28),(r3,A2,b29),(r3,A2,b30),(r3,A2,b31),(r3,A2,b32),(r3,A2,b33),(r3,A2,b34),(r3,A2,b35),(r3,A 2,b36),(r3,A2,b37),(r3,A2,b38),(r3,A2,b39),(r3,A2,b40),(r3,A2,b41),(r3,A2,b42),(r3,A2,b43),(r3,A2, b44),(r3,A2,b45),(r3,A2,b46),(r3,A2,b47),(r3,A2,b48),(r3,A2,b49),(r3,A2,b50),(r3,A2,b51),(r3,A2,b 52),(r3,A2,b53),(r3,A2,b54),(r3,A2,b55),(r3,A2,b56),(r3,A2,b57),(r3,A2,b58),(r3,A2,b59),(r3,A2,b6 0),(r3,A2,b61),(r3,A2,b62),(r3,A2,b63),(r3,A2,b64),(r3,A2,b65),(r3,A2,b66),(r3,A2,b67),(r3,A2,b68) ,(r3,A2,b69),(r3,A2,b70),(r3,A2,b71),(r3,A2,b72),(r3,A2,b73),(r3,A2,b74),(r3,A2,b75),(r3,A2,b76),( r3,A2,b77),(r3,A2,b78),(r3,A2,b79),(r3,A2,b80),(r3,A2,b81),(r3,A2,b82),(r3,A2,b83),(r3,A2,b84),(r3 ,A2,b85),(r3,A2,b86),(r3,A2,b87),(r3,A2,b88),(r3,A2,b89),(r3,A2,b90),(r3,A2,b91),(r3,A2,b92),(r3,A 2,b93),(r3,A2,b94),(r3,A2,b95),(r3,A2,b96),(r3,A2,b97),(r3,A2,b98),(r3,A2,b99),(r3,A2,b100)*,(r3,A 2,b101),(r3,A2,b102),(r3,A2,b103),(r3,A2,b104),(r3,A2,b105),(r3,A2,b106),(r3,A2,b107),(r3,A2,b10 8),(r3,A2,b109),(r3,A2,b110),(r3,A2,b111),(r3,A2,b112),(r3,A2,b113),(r3,A2,b114),(r3,A2,b115),(r3, A2,b116),(r3,A2,b117),(r3,A2,b118),(r3,A2,b119),(r3,A2,b120),(r3,A2,b121),(r3,A2,b122),(r3,A2,b1 23),(r3,A2,b124),(r3,A2,b125),(r3,A2,b126),(r3,A2,b127),(r3,A2,b128),(r3,A2,b129),(r3,A2,b130),(r 3,A2,b131),(r3,A2,b132),(r3,A2,b133),(r3,A2,b134),(r3,A3,b1),(r3,A3,b2),(r3,A3,b3),(r3,A3,b4),(r3, A3,b5),(r3,A3,b6),(r3,A3,b7),(r3,A3,b8),(r3,A3,b9),(r3,A3,b10),(r3,A3,b11),(r3,A3,b 12),(r3,A3,b 13), (r3,A3,b14),(r3,A3,b15),(r3,A3,b16),(r3,A3,b17),(r3,A3,b18),(r3,A3,b19),(r3,A3,b20),(r3,A3,b21),(r 3,A3,b22),(r3,A3,b23),(r3,A3,b24),(r3,A3,b25),(r3,A3,b26),(r3,A3,b27),(r3,A3,b28),(r3,A3,b29),(r3, A3,b30),(r3,A3,b31),(r3,A3,b32),(r3,A3,b33),(r3,A3,b34),(r3,A3,b35),(r3,A3,b36),(r3,A3,b37),(r3,A 3,b38),(r3,A3,b39),(r3,A3,b40),(r3,A3,b41),(r3,A3,b42),(r3,A3,b43),(r3,A3,b44),(r3,A3,b45),(r3,A3, b46),(r3,A3,b47),(r3,A3,b48),(r3,A3,b49),(r3,A3,b50),(r3,A3,b51),(r3,A3,b52),(r3,A3,b53),(r3,A3,b 54),(r3,A3,b55),(r3,A3,b56),(r3,A3,b57),(r3,A3,b58),(r3,A3,b59),(r3,A3,b60),(r3,A3,b61),(r3,A3,b6 2),(r3,A3,b63),(r3,A3,b64),(r3,A3,b65),(r3,A3,b66),(r3,A3,b67),(r3,A3,b68),(r3,A3,b69),(r3,A3,b70) ,(r3,A3,b71),(r3,A3,b72),(r3,A3,b73),(r3,A3,b74),(r3,A3,b75),(r3,A3,b76),(r3,A3,b77),(r3,A3,b78),( r3,A3,b79),(r3,A3,b80),(r3,A3,b81),(r3,A3,b82),(r3,A3,b83),(r3,A3,b84),(r3,A3,b85),(r3,A3,b86),(r3 ,A3,b87),(r3,A3,b88),(r3,A3,b89),(r3,A3,b90),(r3,A3,b91),(r3,A3,b92),(r3,A3,b93),(r3,A3,b94),(r3,A 3,b95),(r3,A3,b96),(r3,A3,b97),(r3,A3,b98),(r3,A3,b99),(r3,A3,b100),(r3,A3,b101),(r3,A3,b102),(r3, A3,b103),(r3,A3,b104),(r3,A3,b105),(r3,A3,b106),(r3,A3,b107),(r3,A3,b1.08),(r3,A3,b109),(r3,A3,b 110),(r3,A3,b 111),(r3,A3,b 112),(r3,A3,b 113),(r3,A3,b 114),(r3,A3,b 115),(r3,A3,b 116),(r3,A3,b117),( r3,A3,b118),(r3,A3,b119),(r3,A3,b120),(r3,A3,b121),(r3,A3,b122),(r3,A3,b123),(r3,A3,b124),(r3,A3 ,b125),(r,3,A3,b126),(r3,A3,b121),(r3,A3,b128),(r3,A3,b129),(r3,A3,b130),(r3,A3,b131),(r3,A3,b132 ),(r3,A3,b133),(r3,A3,b134),(r3,A4,b1),(r3,A4,b2),(r3,A4,b3),(r3,A4,b4),(r3,A4,b5),(r3,A4,b6),(r3,A 4,b7),(r3,A4,b8),(r3,A4,b9),(r13,A4,b10),(r3,A4,b11),(r3,A4,b12),(r3,A4,b13),(r3,A4,b14),(r3,A4,b15 ),(r3,A4,b16),(r3,A4,b17),(r3,A4,b18),(r3,A4,b19),(r3,A4,b20),(r3,A4,b21),(r3,A4,b22),(r3,A4,b23),( r3,A4,b24),(r3,A4,b25),(r3,A4,b26),(r3,A4,b27),(r3,A4,b28),(r3,A4,b29),(r3,A4,b30),(r3,A4,b31),(r3 ,A4,b32),(r3,A4,b33),(r3,A4,b34),(r3,A4,b35),(r3,A4,b36),(r3,A4,b37),(r3,A4,b38),(r3,A4,b39),(r3,A 4,b40),(r3,A4,b41),(r3,A4,b42),(r3,A4,b43),(r3,A4,b44),(r3,A4,b45),(r3,A4,b46),(r3,A4,b47),(r3,A4, b48),(r3,A4,b49),(r3,A4,b50),(r3,A4,b51),(r3,A4,b52),(r3,A4,b53),(r3,A4,b54),(r3,A4,b55),(r3,A4,b 56),(r3,A4,b57),(r3,A4,b58),(r3,A4,b59),(r3,A4,b60),(r3,A4,b61),(r3,A4,b62),(r3,A4,b63),(r3,A4,b6 4),(r3,A4,b65),(r3,A4,b66),(r3,A4,b67),(r3,A4,b68),(r3,A4,b69),(r3,A4,b70),(r3,A4,b71),(r3,A4,b72) ,(r3,A4,b73),(r3,A4,b74),(r3,A4,b75),(r3,A4,b76),(r3,A4,b77),(r3,A4,b78),(r3,A4,b79),(r3,A4,b80),( r3,A4,b81),(r3,A4,b82),(r3,A4,b83),(r3,A4,b84),(r3,A4,b85),(r3,A4,b86),(r3,A4,b87),(r3,A4,b88),(r3 ,A4,b89),(r3,A4,b90),(r3,A4,b91),(r3,A4,b92),(r3,A4,b93),(r3,A4,b94),(r3,A4,b95),(r3,A4,b96),(r3,A 4,b97),(r3,A4,b98),(r3,A4,b99),(r3,A4,b100),(r3,A4,b101),(r3,A4,b102),(r3,A4,b103),(r3,A4,b104),( r3,A4,b105),(r3,A4,b106),(r3,A4,b107),(r3,A4,b108),(r3,A4,b109),(r3,A4,b110),(r3,A4,b111),(r3,A4 ,b112),(r3,A4,b113),(r3,A4,b114),(r3,A4,b115),(r3,A4,b116),(r3,A4,b117),(r3,A4,b118),(r3,A4,b119) ,(r3,A4,b120),(r3,A4,b121),(r3,A4,b122),(r3,A4,b123),(r3,A4,b124),(r3,A4,b125),(r3,A4,b126),(r3, A4,b127),(r3,A4,b128),(r3,A4,b129),(r3,A4,b130),(r3,A4,b131),(r3,A4,b132),(r3,A4,b133),(r3,A4,b 134),

(r4,A1,b1),(r4,A1,b2),(r4,A1,b3),(r4,A1,b4),(r4,A1,b5),(r4,A1,b6),(r4,A1,b7),(r4,A1,b8),(r4,A1,b9),( r4,A1,b10),(r4,A1,b11),(r4,A1,b12),(r4,A1,b13),(r4,A1,b14),(r4,A1,b15),(r4,A1,b16),(r4,A1,b17),(r4 ,A1,b18),(r4,A1,b19),(r4,A1,b20),(r4,A1,b21),(r4,A1,b22),(r4,A1,b23),(r4,A1,b24),(r4,A1,b25),(r4,A 1,b26),(r4,A1,b27),(r4,A1,b28),(r4,A1,b29),(r4,A1,b30),(r4,A1,b31),(r4,A1,b32),(r4,A1,b33),(r4,A1, b34),(r4,A1,b35),(r4,A1,b36),(r4,A1,b37),(r4,A1,b38),(r4,A1,b39),(r4,A1,b40),(r4,A1,b41),(r4,A1,b 42),(r4,A1,b43),(r4,A1,b44),(r4,A1,b45),(r4,A1,b46),(r4,A1,b47),(r4,A1,b48),(r4,A1,b49),(r4,A1,b5 0),(r4,A1,b51),(r4,A1,b52),(r4,A1,b53),(r4,A1,b54),(r4,A1,b55),(r4,A1,b56),(r4,A1,b57),(r4,A1,b58) ,(r4,A1,b59),(r4,A1,b60),(r4,A1,b61),(r4,A1,b62),(r4,A1,b63),(r4,A1,b64),(r4,A1,b65),(r4,A1,b66),( r4,A1,b67),(r4,A1,b68),(r4,A1,b69),(r4,A1,b70),(r4,A1,b63),(r4,A1,b72),(r4,A1,b73),(r4,A1,b74),(r4 ,A1,b75),(r4,A1,b76),(r4,A1,b77),(r4,A1,b78),(r4,A1,b79),(r4,A1,b80),(r4,A1,b81),(r4,A1,b82),(r4,A 1,b83),(r4,A1,b84),(r4,A1,b85),(r4,A1,b86),(r4,A1,b87),(r4,A1,b88),(r4,A1,b89),(r4,A1,b90),(r4,A1, b91),(r4,A1,b92),(r4,A1,b93),(r4,A1,b94),(r4,A1,b95),(r4,A1,b96),(r4,A1,b97),(r4,A1,b98),(r4,A1,b 99),(r4,A1,b100),(r4,A1,b101),(r4,A1,b102),(r4,A1,b103),(r4,A1,b104),(r4,A1,b105),(r4,A1,b106),(r 4,A1,b107),(r4,A1,b108),(r4,A1,b109),(r4,A1,b110),(r4,A1,b111),(r4,A1,bl12),(r4,A1,b113),(r4,A1, b114),(r4,A1,b115),(r4,A1,b116),(r4,A1,b117),(r4,A1,b118),(r4,A1,b119),(r4,A1,b120),(r4,A1,b121), (r4,A1,b122),(r4,A1,b123),(r4,A1,b124),(r4,A1,b125),(r4,A1,b126),(r4,A1,b127),(r4,A1,b128),(r4,A 1,b129),(r4,A1,b130),(r4,A1,b131),(r4,A1,b132),(r4,A1,b133),(r4,A1,b134),(r4,A2,b1),(r4,A2,b2),(r 4,A2,b3),(r4,A2,b4),(r4,A2,b5),(r4,A2,b6),(r4,A2,b7),(r4,A2,b8),(r4,A2,b9),(r4,A2,b10),(r4,A2,b11), (r4,A2,b12),(r4,A2,b13),(r4,A2,b14),(r4,A2,b15),(r4,A2,b16),(r4,A2,b17),(r4,A2,b18),(r4,A2,b19),(r 4,A2,b20),(r4,A2,b21),(r4,A2,b22),(r4,A2,b23),(r4,A2,b24),(r4,A2,b25),(r4,A2,b26),(r4,A2,b27),(r4, A2,b28),(r4,A2,b29),(r4,A2,b30),(r4,A2,b31),(r4,A2,b32),(r4,A2,b33),(r4,A2,b34),(r4,A2,b35),(r4,A 2,b36),(r4,A2,b37),(r4,A2,b38),(r4,A2,b39),(r4,A2,b40),(r4,A2,b41),(r4,A2,b42),(r4,A2,b43),(r4,A2, b44),(r4,A2,b45),(r4,A2,b46),(r4,A2,b47),(r4,A2,b48),(r4,A2,b49),(r4,A2,b50),(r4,A2,b51),(r4,A2,b 52),(r4,A2,b53),(r4,A2,b54),(r4,A2,b55),(r4,A2,b56),(r4,A2,b57),(r4,A2,b58),(r4,A2,b59),(r4,A2,b6 0),(r4,A2,b61),(r4,A2,b62),(r4,A2,b63),(r4,A2,b64),(r4,A2,b65),(r4,A2,b66),(r4,A2,b67),(r4,A2,b68) ,(r4,A2,b69),(r4,A2,b70),(r4,A2,b71),(r4,A2,b72),(r4,A2,b73),(r4,A2,b74),(r4,A2,b75),(r4,A2,b76),( r4,A2,b77),(r4,A2,b78),(r4,A2,b79),(r4,A2,b80),(r4,A2,b81),(r4,A2,b82),(r4,A2,b83),(r4,A2,b84),(r4 ,A2,b85), (r4,A2,b86), (r4,A2,b87), (r4,A2,b88), (r4,A2,b89), (r4,A2,b90), (r4,A2,b91), (r4,A2,b92),(r4,A 2,b93),(r4,A2,b94),(r4,A2,b95),(r4,A2,b96),(r4,A2,b97),(r4,A2,b98),(r4,A2,b99),(r4,A2,b100),(r4,A 2,b101),(r4,A2,b102),(r4,A2,b103),(r4,A2,b104),(r4,A2,b105),(r4,A2,b106),(r4,A2,b107),(r4,A2,b10 8),(r4,A2,b109),(r4,A2,b110),(r4,A2,b111),(r4,A2,b112),(r4,A2,b113),(r4,A2,b114),(r4,A2,b115),(r4, A2,b116),(r4,A2,b117),(r4,A2,b118),(r4,A2,b119),(r4,A2,b120),(r4,A2,b121),(r4,A2,b122),(r4,A2,b1 23),(r4,A2,b124),(r4,A2,b125),(r4,A2,b126),(r4,A2,b127),(r4,A2,b128),(r4,A2,b129),(r4,A2,b130),(r 4,A2,b131),(r4,A2,b132),(r4,A2,b133),(r4,A2,b134),(r4,A3,b1),(r4,A3,b2),(r4,A3,b3),(r4,A3,b4),(r4, A3,b5),(r4,A3,b6),(r4,A3,b7),(r4,A3,b8),(r4,A3,b9),(r4,A3,b10),(r4,A3,b11),(r4,A3,b12),(r4,A3,b13), (r4,A3,b14),(r4,A3,b15),(r4,A3,b16),(r4,A3,b17),(r4,A3,b18),(r4,A3,b19),(r4,A3,b20),(r4,A3,b21),(r 4,A3,b22),(r4,A3,b23),(r4,A3,b24),(r4,A3,b25),(r4,A3,b26),(r4,A3,b27),(r4,A3,b28),(r4,A3,b29),(r4, A3,b30),(r4,A3,b31),(r4,A3,b32),(r4,A3,b33),(r4,A3,b34),(r4,A3,b35),(r4,A3,b36),(r4,A3,b37),(r4,A 3,b38),(r4,A3,b39),(r4,A3,b40),(r4,A3,b41),(r4,A3,b42),(r4,A3,b43),(r4,A3,b44),(r4,A3,b45),(r4,A3, b46),(r4,A3,b47),(r4,A3,b48),(r4,A3,b49),(r4,A3,b50),(r4,A3,b51),(r4,A3,b52),(r4,A3,b53),(r4,A3,b 54),(r4,A3,b55),(r4,A3,b56),(r4,A3,b57),(r4,A3,b58),(r4,A3,b59),(r4,A3,b60),(r4,A3,b61),(r4,A3,b6 2),(r4,A3,b63),(r4,A3,b64),(r4,A3,b65),(r4,A3,b66),(r4,A3,b67),(r4,A3,b68),(r4,A3,b69),(r4,A3,b70) ,(r4,A3,b71),(r4,A3,b72),(r4,A3,b73),(r4,A3,b74),(r4,A3,b75),(r4,A3,b76),(r4,A3,b71),(r4,A3,b78),( r4,A3,b79),(r4,A3,b80),(r4,A3,b81),(r4,A3,b82),(r4,A3,b83),(r4,A3,b84),(r4,A3,b85),(r4,A3,b86),(r4 ,A3,b87),(r4,A3,b88),(r4,A3,b89),(r4,A3,b90),(r4,A3,b91),(r4,A3,b92),(r4,A3,b93),(r4,A3,b94),(r4,A 3,b95),(r4,A3,b96),(r4,A3,b97),(r4,A3,b98),(r4,A3,b99),(r4,A3,b100),(r4,A3,b101),(r4,A3,b102),(r4, A3,b103),(r4,A3,b104),(r4,A3,b105),(r4,A3,b106),(r4,A3,b107),(r4,A3,b108),(r4,A3,b109),(r4,A3,b 110),(r4,A3,b111),(r4,A3,b112),(r4,A3,b113),(r4,A3,b114),(r4,A3,b115),(r4,A3,b116),(r4,A3,b117),( r4,A3,b118),(r4,A3,b119),(r4,A3,b120),(r4,A3,b121),(r4,A3,b122),(r4,A3,b123),(r4,A3,b124),(r4,A3 ,b125),(r4,A3,b126),(r4,A3,b127),(r4,A3,b128),(r4,A3,b129),(r4,A3,b130),(r4,A3,b131),(r4,A3,b132 ),(r4,A3,b133),(r4,A3,b134),(r4,A4,b1),(r4,A4,b2),(r4,A4,b3),(r4,A4,b4),(r4,A4,b5),(r4,A4,b6),(r4,A 4,b7),(r4,A4,b8),(r4,A4,b9),(r4,A4,b10),(r4,A4,b11),(r4,A4,b12),(r4,A4,b13),(r4,A4,b14),(r4,A4,b15 ),(r4,A4,b16),(r4,A4,b17),(r4,A4,b18),(r4,A4,b19),(r4,A4,b20),(r4,A4,b21),(r4,A4,b22),(r4,A4,b23), r4,A4,b24),(r4,A4,b25),(r4,A4,b26),(r4,A4,b27),(r4,A4,b28),(r4,A4,b29),(r4,A4,b30),(r4,A4,b31),(r4 ,A4,b32),(r4,A4,b33),(r4,A4,b34),(r4,A4,b35),(r4,A4,b36),(r4,A4,b37),(r4,A4,b38),(r4,A4,b39),(r4,A 4,b40),(r4,A4,b41),(r4,A4,b42).(r4,A4,b43),(r4,A4,b44),(r4,A4,b45),(r4,A4,b46),(r4,A4,b47),(r4,A4, b48),(r4,A4,b49),(r4,A4,b50),(r4,A4,b51),(r4,A4,b52),(r4,A4,b53),(r4,A4,b54),(r4,A4,b55),(r4,A4,b 56),(r4,A4,b57),(r4,A4,b58),(r4,A4,b59),(r4,A4,b60),(r4,A4,b61),(r4,A4,b62),(r4,A4,b63),(r4,A4,b6 4),(r4,A4,b65),(r4,A4,b66),(r4,A4,b67),(r4,A4,b68),(r4,A4,b69),(r4,A4,b70),(r4,A4,b71),(r4,A4,b72) ,(r4,A4,b73),(r4,A4,b74),(r4,A4,b75),(r4,A4,b76),(r4,A4,b77),(r4,A4,b78),(r4,A4,b79),(r4,A4,b80),( r4,A4,b81),(r4,A4,b82),(r4,A4,b83),(r4,A4,b84),(r4,A4,b85),(r4,A4,b86),(r4,A4,b87),(r4,A4,b88),(r4 ,A4,b89),(r4,A4,b90),(r4,A4,b91),(r4,A4,b92),(r4,A4,b93),(r4,A4,b94),(r4,A4,b95),(r4,A4,b96),(r4,A 4,b97),(r4,A4,b98),(r4,A4,b99),(r4,A4,b100),(r4,A4,b101),(r4,A4,b102),(r4,A4,b103),(r4,A4,b104), ( r4,A4,b105),(r4,A4,b106),(r4,A4,b107),(r4,A4,b108),(r4,A4,b109),(r4,A4,b110),(r4,A4,b111),(r4,A4 ,b112),(r4,A4,b113),(r4,A4,b114),(r4,A4,b115),(r4,A4,b116),(r4,A4,b117),(r4,A4,b118),(r4,A4,b119) ,(r4,A4,b120),(r4,A4,b121),(r4,A4,b122),(r4,A4,b123),(r4,A4,b124),(r4,A4,b125),(r4,A4,b126),(r4, A4,b127),(r4,A4,b128),(r4,A4,b129),(r4,A4,b130),(r4,A4,b131),(r4,A4,b132),(r4,A4,b133),(r4,A4,b 134),

(r5,A1,b1),(r5,A1,b2),(r5,A1,b3),(r5,A1,b4),(r5,A1,b5),(r5,A1,b6),(r5,A1,b7),(r5,A1,b8),(r5,A1,b9),( r5,A1,b10),(r5,A1,b11),(r5,A1,b12),(r5,A1,b13),(r5,A1,b14),(r5,A1,b15),(r5,A1,b16),(r5,A1,b17),(r5 ,A1,b18),(r5,A1,b19),(r5,A1,b20),(r5,A1,b21),(r5,A1,b22),(r5,A1,b23),(r5,A1,b24),(r5,A1,b25),(r5,A 1,b26),(r5,A1,b27),(r5,A1,b28),(r5,A1,b29),(r5,A1,b30),(r5,A1,b31),(r5,A1,b32),(r5,A1,b33),(r5,A1, b34),(r5,A1,b35),(r5,A1,b36),(r5,A1,b37),(r5,A1,b38),(r5,A1,b39),(r5,A1,b40),(r5,A1,b41),(r5,A1,b 42),(r5,A1,b43),(r5,A1,b44),(r5,A1,b45),(r5,A1,b46),(r5,A1,b47),(r5,A1,b48),(r5,A1,b49),(r5,A1,b5 0),(r5,A1,b43);(r5,A1,b52),(r5,A1,b53),(r5,A1,b54),(r5,A1,b55),(r5,A1,b56),(r5,A1,b57),(r5,A1,b58) ,(r5,A1,b59),(r5,A1,b60),(r5,A1,b61),(r5,A1,b62),(r5,A1,b63),(r5,A1,b64),(r5,A1,b65),(r5,A1,b66),( r5,A1,b67),(r5,A1,b68),(r5,A1,b69),(r5,A1,b70),(r5,A1,b71),(r5,A1,b72),(r5,A1,b73),(r5,A1,b74),(r5 ,A1,b75),(r5,A1,b76),(r5,A1,b77),(r5,A1,b78),(r5,A1,b79),(r5,A1,b80),(r5,A1,b81),(r5,A1,b82),(r5,A 1,b83),(r5,A1,b84),(r5,A1,b85),(r5,A1,b86),(r5,A1,b87),(r5,A1,b88),(r5,A1,b89),(r5,A1,b90),(r5,A1, b91),(r5,A1,b92),(r5,A1,b93),(r5,A1,b94),(r5,A1,b95),(r5,A1,b96),(r5,A1,b97),(r5,A1,b98),(r5,A1,b 99),(r5,A1,b100),(r5,A1,b101),(r5,A1,b102),(r5,A1,b103),(r5,A1,b104),(r5,A1,b105),(r5,A1,b106),(r 5,A1,b107),(r5,A1,b108),(r5,A1,b109),(r5,A1,b110),(r5,A1,b111),(r5,A1,b112),(r5,A1,b113),(r5,A1, b114),(r5,A1,b115),(r5,A1,b116),(r5,A1,b117),(r5,A1,b118),(r5,A1,b119),(r5,A1,b120),(r5,A1,b121), (r5,A1,b122),(r5,A1,b123),(r5,A1,b124),(r5,A1,b125),(r5,A1,b126),(r5,A1,b127),(r5,A1,b128),(r5,A 1,b129),(r5,A1,b130),(r5,A1,b131),(r5,A1,b132),(r5,A1,b133),(r5,A1,b134),(r5,A2,b1),(r5,A2,b2),(r 5,A2,b3),(r5,A2,b4),(r5,A2,b5),(r5,A2,b6),(r5,A2,b7),(r5,A2,b8),(r5,A2,b9),(r5,A2,b10),(r5,A2,b11), (r5,A2,b 12),(r5,A2,b 13),(r5,A2,b 14),(r5,A2,b 15),(r5,A2,b16),(r5,A2,b 17),(r5,A2,b 18),(r5,A2,b19),(r 5,A2,b20),(r5,A2,b21),(r5,A2,b22),(r5,A2,b23),(r5,A2,b24),(r5,A2,b25),(r5,A2,b26),(r5,A2,b27),(r5, A2,b28),(r5,A2,b29),(r5,A2,b30),(r5,A2,b31),(r5,A2,b32),(r5,A2,b33),(r5,A2,b34),(r5,A2,b35),(r5,A 2,b36),(r5,A2,b37),(r5,A2,b38),(r5,A2,b39),(r5,A2,b40),(r5,A2,b41),(r5,A2,b42),(r5,A2,b43),(r5,A2, b44),(r5,A2,b45),(r5,42,b46),(r5,A2,b47),(r5,A2,b48),(r5,A2,b49),(r5,A2,b50),(r5,A2,b51),(r5,A2,b 52),(r5,A2,b53),(r5,A2,b54),(r5,A2,b55),(r5,A2,b56),(r5,A2,b5'7),(r5,A2,b58),(r5,A2,b59),(r5,A2,b6 0),(r5,A2,b61),(r5,A2,b62),(r5,A2,b63),(r5,A2,b64),(r5,A2,b65),(r5,A2,b66),(r5,A2,b67),(r5,A2,b68) ,(r5,A2,b69),(r5,A2,b70),(r5,A2,b71),(r5,A2,b72),(r5,A2,b73),(r5,A2,b74),(r5,A2,b75),(r5,A2,b76),( r5,A2,b77),(r5,A2,b78),(r5,A2,b79),(r5,A2,b80),(r5,A2,b81),(r5,A2,b82),(r5,A2,b83),(r5,A2,b84),(r5 ,A2,b8,5),(r5,A2,b86),(r5,A2,b87),(r5,A2,b88),(r5,A2,b89),(r5,A2,b90),(r5,A2,b91),(r5,A2,b92),(r5,A 2,b93),(r5,A2,b94),(r5,A2,b95),(r5,A2,b96),(r5,A2,b97),(r5,A2,b98),(r5,A2,b99),(r5,A2,b100),(r5,A 2,b101),(r5,A2,b102),(r5,A2,b103),(r5,A2,b104),(r5,A2,b105),(r5,A2,b106),(r5,A2,b107),(r5,A2,b10 8),(r5,A2,b109),(r5,A2,b110),(r5,A2,b111),(r5,A2,b112),(r5,A2,b113),(r5,A2,b114),(r5,A2,b115),(r5, A2,b116),(r5,A2,b117),(r5,A2,b118),(r5,A2,b119),(r5,A2,b120),(r5,A2,b121),(r5,A2,b122),(r5,A2,b1 23),(r5,A2,b124),(r5,A2,b125),(r5,A2,b126),(r5,A2,b127),(r5,A2,b128),(r5,A2,b129),(r5,A2,b130),(r 5,A2,b131),(r5,A2,b132),(r5,A2,b133),(r5,A2,b134),(r5,A3,b1),(r5,A3,b2),(r5,A3,b3),(r5,A3,b4),(r5, A3,b5),(r5,A3,b6),(r5,A3,b7),(r5,A3,b8),(r5,A3,b9),(r5,A3,b10),(r5,A3,b11),(r5,A3,b12),(r5,A3,b13), (r5,A3,b14),(r5,A3,b15),(r5,A3,b16),(r5,A3,b17),(r5,A3,b18),(r5,A3,b19),(r5,A3,b20),(r5,A3,b21),(r 5,A3,b22),(r5,A3,b23),(r5,A3,b24),(r5,A3,b25),(r5,A3,b26),(r5,A3,b27),(r5,A3,b28),(r5,A3,b29),(r5, A3,b30),(r5,A3,b31),(r5,A3,b32),(r5,A3,b33),(r5,A3,b34),(r5,A3,b35),(r5,A,3,b36),(r5,A3,b37),(r5,A 3,b38),(r5,A3,b39),(r5,A3,b40),(r5,A3,b41),(r5,A3,b42),(r5,A3,b43),(r5,A3,b44),(r5,A3,b45),(r5,A3, b46),(r5,A3,b47),(r5,A3,b48),(r5,A3,b49),(r5,A3,b50),(r5,A3,b51),(r5,A3,b52),(r5,A3,b53),(r5,A3,b 54),(r5,A3,b55),(r5,A3,b56),(r5,A3,b57),(r5,A3,b58),(r5,A3,b59),(r5,A3,b60),(r5,A3,b61),(r5,A3,b6 2),(r5,A3,b63),(r5,A3,b64),(r5,A3,b65),(r5,A3,b66),(r5,A3,b68),(r5,A3,b68),(r5,A3,b69),(r5,A3,b70) ,(r5,A3,b71),(r5,A3,b72),(r5,A3,b73),(r5,A3,b74),(r5,A3,b75),(r5,A3,b76),(r5,A3,b77),(r5,A3,b78),( r5,A3,b79),(r5,A3,b80),(r5,A3,b81),(r5,A3,b82),(r5,A3,b83),(r5,A3,b84),(r5,A3,b85),(r5,A3,b86),(r5 ,A3,b87),(r5,A3,b88),(r5,A3,b89),(r5,A3,b90),(r5,A3,b91),(r5,A3,b92),(r5,A3,b93),(r5,A3,b94),(r5,A 3,b95),(r5,A3,b96),(r5,A3,b97),(r5,A3,b98),(r5,A3,b99),(r5,A3,b100),(r5,A3,b101),(r5,A3,b102),(r5, A3,b103),(r5,A3,b104),(r5,A3,b105),(r5,A3,b106),(r5,A3,b107),(r5,A3,b108),(r5,A3,b109),(r5,A3,b 110),(r5,A3,b111),(r5,A3,b112),(r5,A3,b113),(r5,A3,b114),(r5,A3,b115),(r5,A3,b116),(r5,A3,b117),( r5,A3,b118),(r5,A3,b119),(r5,A3,b120),(r5,A3,b121),(r5,A3,b122),(r5,A3,b123),(r5,A3,b124),(r5,A3 ,b125),(r5,A3,b126),(r5,A3,b127),(r5,A3,b128),(r5,A3,b129),(r5,A3,b130),(r5,A3,b131),(r5,A3,b132 ),(r5,A3,b133),(r5,A3,b134),(r5,A4,b1),(r5,A4,b2),(r5,A4,b3),(r5,A4,b4),(r5,A4,b5),(r5,A4,b6),(r5,A 4,b7),(r5,A4,b8),(r5,A4,b9),(r5,A4,b10),(r5,A4,b11),(r5,A4,b12),(r5,A4,b13),(r5,A4,b14),(r5,A4,b15 ),(r5,A4,b16),(r5,A4,b17),(r5,A4,b18),(r5,A4,b19),(r5,A4,b20),(r5,A4,b21),(r5,A4,b22),(r5,A4,b23),( r5,A4,b24),(r5,A4,b25),(r5,A4,b26),(r5,A4,b27),(r5,A4,b28),(r5,A4,b29),(r5,A4,b30),(r5,A4,b31),(r5 ,A4,b32),(r5,A4,b33),(r5,A4,b34),(r5,A4,b35),(r5,A4,b36),(r5,A4,b37),(r5,A4,b38),(r5,A4,b39),(r5,A 4,b40),(r5,A4,b41),(r5,A4,b42),(r5,A4,b43),(r5,A4,b44),(r5,A4,b45),(r5,A4,b46),(r5,A4,b47),(r5,A4, b48),(r5,A4,b49),(r5,A4,b50),(r5,A4,b51),(r5,A4,b52),(r5,A4,b53),(r5,A4,b54),(r5,A4,b55),(r5,A4,b 56),(r5,A4,b57),(r5,A4,b58),(r5,A4,b59),(r5,A4,b60),(r5,A4,b61),(r5,A4,b62),(r5,A4,b63),(r5,A4,b6 4),(r5,A4,b65),(r5,A4,b66),(r5,A4,b67),(r5,A4,b68),(r5,A4,b69),(r5,A4,b70),(r5,A4,b71),(r5,A4,b72) ,(r5,A4,b73),(r5,A4,b74),(r5,A4,b75),(r5,A4,b76),(r5,A4,b77),(r5,A4,b78),(r5,A4,b79),(r5,A4,b80),( r5,A4,b81),(r5,A4,b82),(r5,A4,b83),(r5,A4,b84),(r5,A4,b85),(r5,A4,b86),(r5,A4,b87),(r5,A4,b88),(r5 ,A4,b89),(r5,A4,b90),(r5,A4,b91),(r5,A4,b92),(r5,A4,b93),(r5,A4,b94),(r5,A4,b95),(r5,A4,b96),(r5,A 4,b97),(r5,A4,b98),(r5,A4,b99),(r5,A4,b100),(r5,A4,b101),(r5,A4,b102),(r5,A4,b103),(r5,A4,b104),( r5,A4,b105),(r5,A4,b106),(r5,A4,b107),(r5,A4,b108),(r5,A4,b109),(r5,A4,b110),(r5,A4,b111),(r5,A4 ,b112),(r5,A4,b113),(r5,A4,b114),(r5,A4,b115),(r5,A4,b116),(r5,A4,b117),(r5,A4,b118),(r5,A4,b119) ,(r5,A4,b120),(r5,A4,b121),(r5,A4,b122),(r5,A4,b123),(r5,A4,b124),(r5,A4,b125),(r5,A4,b126),(r5, A4,b127),(r5,A4,b128),(r5,A4,b129),(r5,A4,b130),(r5,A4,b131),(r5,A4,b132),(r5,A4,b133),(r5,A4,b 134),

(r6,A1,b1),(r6,A1,b2),(r6,A1,b3),(r6,A1,b4),(r6,A1,b5),(r6,A1,b6),(r6,A1,b7),(r6,A1,b8), (r6,A1,b9), ( r6,A1,b10),(r6,A1,b11),(r6,A1,b12),(r6,A1,b13),(r6,A1,b14),(r6,A1,b15),(r6,A1,b16),(r6,A1,b17),(r6 _{,}A1,b18),(r6,A1,b19),(r6,A1,b20),(r6,A1,b21),(r6,A1,b22),(r6,A1,b23),(r6,A1,b24),(r6,A1,b25),(r6,A 1,b26),(r6,A1,b27),(r6,A1,b28),(r6,A1,b29),(r6,A1,b30),(r6,A1,b31),(r6,A1,b32),(r6,A1,b33),(r6,A1, b34),(r6,A1,b35),(r6,A1,b36),(r6,A1,b37),(r6,A1,b38),(r6,A1,b39),(r6,A1,b40),(r6,A1,b41),(r6,A1,b 42),(r6,A1,b43),(r6,A1,b44),(r6,A1,b45),(r6,A1,b46),(r6,A1,b47),(r6,A1,b48),(r6,A1,b49),(r6,A1,b5 0),(r6,A1,b51),(r6,A1,b52),(r6,A1,b53),(r6,A1,b54),(r6,A1,b55),(r6,A1,b56),(r6,A1,b57),(r6,A1,b58) ,(r6,A1,b59),(r6,A1,b60),(r6,A1,b61),(r6,A1,b62),(r6,A1,b63),(r6,A1,b64),(r6,A1,b65),(r6,A1,b66),( r6,A1,b67),(r6,A1,b68),(r6,A1,b69),(r6,A1,b70),(r6,A1,b71),(r6,A1,b72),(r6,A1,b73),(r6,A1,b74),(r6 ,A1,b75),(r6,A1,b76),(r6,A1,b77),(r6,A1,b78),(r6,A1,b79),(r6,A1,b80),(r6,A1,b81),(r6,A1,b82),(r6,A 1,b83),(r6,A1,b84),(r6,A1,b85),(r6,A1,b86),(r6,A1,b87),(r6,A1,b88),(r6,A1,b89),(r6,A1,b90),(r6,A1, b91),(r6,A1,b92),(r6,A1,b93),(r6,A1,b94),(r6,A1,b95),(r6,A1,b96),(r6,A1,b97),(r6,A1,b98),(r6,A1,b 99),(r6,A1,b100),(r6,A1,b101),(r6,A1,b102),(r6,A1,b103),(r6,A1,b104),(r6,A1,b105),(r6,A1,b106),(r 6,A1,b107),(r6,A1,b108),(r6,A1,b109),(r6,A1,b110),(r6,A1,b111),(r6,A1,b112),(r6,A1,b113),(r6,A1, b114),(r6,A1,b115),(r6,A1,b116),(r6,A1,b117),(r6,A1,b118),(r6,A1,b119),(r6,A1,b120),(r6,A1,b121), (r6,A1,b122),(r6,A1,b123),(r6,A1,b124),(r6,A1,b125),(r6,A1,b126),(r6,A1,b127),(r6,A1,b128),(r6,A 1,b129),(r6,A1,b130),(r6,A1,b131),(r6,A1,b132),(r6,A1,b133),(r6,A1,b134),(r6,A2,b1),(r6,A2,b2),(r 6,A2,b3),(r6,A2,b4),(r6,A2,b5),(r6,A2,b6),(r6,A2,b7),(r6,A2,b8),(r6,A2,b9),(r6,A2,b10),(r6,A2,b11), (r6,A2,b12),(r6,A2,b13),(r6,A2,b14),(r6,A2,b15),(r6,A2,b16),(r6,A2,b17),(r6,A2,b18),(r6,A2,b19),(r 6,A2,b20),(r6,A2,b21),(r6,A2,b22),(r6,A2,b23),(r6,A2,b24),(r6,A2,b25),(r6,A2,b26),(r6,A2,b27),(r6, A2,b28),(r6,A2,b29),(r6,A2,b30),(r6,A2,b31),(r6,A2,b32),(r6,A2,b33),(r6,A2,b34),(r6,A2,b35),(r6,A 2,b36),(r6,A2,b37),(r6,A2,b38),(r6,A2,b39),(r6,A2,b40),(r6,A2,b41),(r6,A2,b42),(r6,A2,b43),(r6,A2, b44),(r6,A2,b45),(r6,A2,b46),(r6,A2,b47),(r6,A2,b48),(r6,A2,b49),(r6,A2,b50),(r6,A2,b51),(r6,A2,b 52),(r6,A2,b53),(r6,A2,b54),(r6,A2,b55),(r6,A2,b56),(r6,A2,b57),(r6,A2,b58),(r6,A2,b59),(r6,A2,b6 0),(r6,A2,b61),(r6,A2,b62),(r6,A2,b63),(r6,A2,b64),(r6,A2,b65),(r6,A2,b66),(r6,A2,b67),(r6,A2,b68) ,(r6,A2,b69),(r6,A2,b70),(r6,A2,b71),(r6,A2,b72),(r6,A2,b73),(r6,A2,b74),(r6,A2,b75),(r6,A2,b76),( r6,A2,b77),(r6,A2,b78),(r6,A2,b79),(r6,A2,b80),(r6,A2,b81),(r6,A2,b82),(r6,A2,b83),(r6,A2,b84),(r6 ,A2,b85),(r6,A2,b86),(r6,A2,b87),(r6,A2,b88),(r6,A2,b89),(r6,A2,b90),(r6,A2,b91),(r6,A2,b92),(r6,A 2,b93),(r6,A2,b94),(r6,A2,b95),(r6,A2,b96),(r6,A2,b97),(r6,A2,b98),(r6,A2,b99),(r6,A2,b100),(r6,A 2,b101),(r6,A2,b102),(r6,A2,b103),(r6,A2,b104),(r6,A2,b105),(r6,A2,b106),(r6,A2,b107),(r6,A2,b10 8),(r6,A2,b109),(r6,A2,b110),(r6,A2,b111),(r6,A2,b112),(r6,A2,b113),(r6,A2,b114),(r6,A2,b115),(r6, A2,b116),(r6,A2,b117),(r6,A2,b118),(r6,A2,b119),(r6,A2,b120),(r6,A2,b121),(r6,A2,b122),(r6,A2,b1 23),(r6,A2,b124),(r6,A2,b125),(r6,A2,b126),(r6,A2,b127),(r6,A2,b128),(r6,A2,b129),(r6,A2,b130),(r 6,A2,b131),(r6,A2,b132),(r6,A2,b133),(r6,A2,b134),(r6,A3,b1),(r6,A3,b2),(r6,A3,b3),(r6,A3,b4),(r6, A3,b5),(r6,A3,b6),(r6,A3,b7),(r6,A3,b8),(r6,A3,b9),(r6,A3,b10),(r6,A3,b11),(r6,A3,b12),(r6,A3,b13), (r6,A3,b14),(r6,A3,b15),(r6,A3,b16),(r6,A3,b17),(r6,A3,b18),(r6,A3,b19),(r6,A3,b20),(r6,A3,b21),(r 6,A3,b22),(r6,A3,b23),(r6,A3,b24),(r6,A3,b25),(r6,A3,b26),(r6,A3,b27),(r6,A3,b28),(r6,A3,b29),(r6, A3,b30),(r6,A3,b31),(r6,A3,b32),(r6,A3,b33),(r6,A3,b34),(r6,A3,b35),(r6,A3,b36),(r6,A3,b37),(r6,A 3,b38),(r6,A3,b39),(r6,A3,b40),(r6,A3,b41),(r6,A3,b42),(r6,A3,b43),(r6,A3,b44),(r6,A3,b45),(r6,A3, b46),(r6,A3,b47),(r6,A3,b48),(r6,A3,b49),(r6,A3,b50),(r6,A3,b51),(r6,A3,b52),(r6,A3,b53),(r6,A3,b 54),(r6,A3,b55),(r6,A3,b56),(r6,A3,b57),(r6,A3,b58),(r6,A3,b59),(r6,A3,b60),(r6,A3,b61),(r6,A3,b6 2),(r6,A3,b63),(r6,A3,b64),(r6,A3,b65),(r6,A3,b66),(r6,A3,b67),(r6,A3,b68),(r6,A3,b69),(r6,A3,b70) ,(r6,A3,b71),(r6,A3,b72),(r6,A3,b73),(r6,A3,b74),(r6,A3,b75),(r6,A3,b76),(r6,A3,b77),(r6,A3,b78),( r6,A3,b79),(r6,A3,b80),(r6,A3,b81),(r6,A3,b82),(r6,A3,b83),(r6,A3,b84),(r6,A3,b85),(r6,A3,b86),(r6 ,A3,b87),(r6,A3,b88),(r6,A3,b89),(r6,A3,b90),(r6,A3,b91),(r6,A3,b92),(r6,A3,b93),(r6,A3,b94),(r6,A 3,b95),(r6,A3,b96),(r6,A3,b97),(r6,A3,b98),(r6,A3,b99),(r6,A3,b100),(r6,A3,b101),(r6,A3,b102),(r6, A3,b103),(r6,A3,b104),(r6,A3,b105),(r6,A3,b106),(r6,A3,b107),(r6,A3,b108),(r6,A3,b109),(r6,A3,b 110),(r6,A3,b112),(r6,A3,b112),(r6,A3,b113),(r6,A3,b114),(r6,A3,b115),(r6,A3,b116),(r6,A3,b117),( r6,A3,b118),(r6,A3,b119),(r6,A3,b120),(r6,A3,b121),(r6,A3,b122),(r6,A3,b123),(r6,A3,b124),(r6,A3 ,b125),(r6,A3,b126),(r6,A3,b127),(r6,A3,b128),(r6,A3,b129),(r6,A3,b130),(r6,A3,b131),(r6,A3,b132 ),(r6,A3,b133),(r6,A3,b1.34),(r6,A4,b1),(r6,A4,b2),(r6,A4,b3),(r6,A4,b4),(r6,A4,b5),(r6,A4,b6),(r6,A 4,b7),(r6,A4,b8),(r6,A4,b9),(r6,A4,b10),(r6,A4,b11),(r6,A4,b12),(r6,A4,b13),(r6,A4,b14),(r6,A4,b15 ),(r6,A4,b16),(r6,A4,b17),(r6,A4,b18),(r6,A4,b29),(r6,A4,b20),(r6,A4,b21),(r6,A4,b22),(r6,A4,b23),( r6,A4,b24),(r6,A4,b25),(r6,A4,b26),(r6,A4,b27),(r6,A4,b28),(r6,A4,b29),(r6,A4,b30),(r6,A4,b31),(r6 ,A4,b32),(r6,A4,b33),(r6,A4,b34),(r6,A4,b35),(r6,A4,b36),(r6,A4,b37),(r6,A4,b38),(r6,A4,b39),(r6,A 4,b40),(r6,A4,b41),(r6,A4,b42),(r6,A4,b43),(r6,A4,b44),(r6,A4,b45),(r6,A4,b46),(r6,A4,b47),(r6,A4, b48),(r6,A4,b49),(r6,A4,b50),(r6,A4,b51),(r6,A4,b52),(r6,A4,b53),(r6,A4,b54),(r6,A4,b55),(r6,A4,b 56),(r6,A4,b57),(r6,A4,b58),(r6,A4,b59),(r6,A4,b60),(r6,A4,b61),(r6,A4,b62),(r6,A4,b63),(r6,A4,b6 4),(r6,A4,b65),(r6,A4,b66),(r6,A4,b67),(r6,A4,b68),(r6,A4,b69),(r6,A4,b70),(r6,A4,b71),(r6,A4,b72) ,(r6,A4,b73),(r6,A4,b74),(r6,A4,b75),(r6,A4,b76),(r6,A4,b77),(r6,A4,b78),(r6,A4,b79),(r6,A4,b80),( r6,A4,b81),(r6,A4,b82),(r6,A4,b83),(r6,A4,b84),(r6,A4,b85),(r6,A4,b86),(r6,A4,b87),(r6,A4,b88),(r6 ,A4,b89),(r6,A4,b90),(r6,A4,b91),(r6,A4,b92),(r6,A4,b93),(r6,A4,b94),(r6,A4,b95),(r6,A4,b96),(r6,A 4,b97),(r6,A4,b98),(r6,A4,b99),(r6,A4,b100),(r6,A4,b101),(r6,A4,b102),(r6,A4,b103),(r6,A4,b104),( r6,A4,b105),(r6,A4,b106),(r6,A4,b107),(r6,A4,b108),(r6,A4,b109),(r6,A4,b110),(r6,A4,b111),(r6,A4 ,b112),(r6,A4,b113),(r6,A4,b114),(r6,A4,b115),(r6,A4,b116),(r6,A4,b117),(r6,A4,b118),(r6,A4,b119) ,(r6,A4,b120),(r6,A4,b121),(r6,A4,b122),(r6,A4,b123),(r6,A4,b124),(r6,A4,b125),(r6,A4,b126),(r6, A4,b127),(r6,A4,b128),(r6,A4,b129),(r6,A4,b130),(r6,A4,b131),(r6,A4,b132),(r6,A4,b133),(r6,A4,b 134),

(r7,A1,b1),(r7,A1,b2),(r7,A1,b3),(r7,A1,b4),(r7,A1,b5),(r7,A1,b6),(r7,A1,b7),(r7,A1,b8),(r7,A1,b9),( r7,A1,b10),(r7,A1,b11),(r7,A1,b12),(r7,A1,b13),(r7,A1,b14),(r7,A1,b15),(r7,A1,b16),(r7,A1,b17),(r7 ,A1,b18),(r7,A1,b19),(r7,A1,b20),(r7,A1,b21),(r7,A1,b22),(r7,A1,b23),(r7,A1,b24),(r7,A1,b25),(r7,A 1,b26),(r7,A1,b27),(r7,A1,b28),(r7,A1,b29),(r7,A1,b30),(r7,A1,b31),(r7,A1,b32),(r7,A1,b33),(r7,A1, b34),(r7,A1,b35),(r7,A1,b36),(r7,A1,b37),(r7,A1,b38),(r7,A1,b39),(r7,A1,b40),(r7,A1,b41),(r7,A1,b 42),(r7,A1,b43),(r7,A1,b44),(r7,A1,b45),(r7,A1,b46),(r7,A1,b47),(r7,A1,b48),(r7,A1,b49),(r7,A1,b5 0),(r7,A1,b51),(r7,A1,b52),(r7,A1,b53),(r7,A1,b54),(r7,A1,b55),(r7,A1,b56),(r7,A1,b57),(r7,A1,b58) ,(r7,A1,b59),(r7,A1,b60),(r7,A1,b61),(r7,A1,b62),(r7,A1,b63),(r7,A1,b64),(r7,A1,b65),(r7,A1,b66),( r7,A1,b67),(r7,A1,b68),(r7,A1,b69),(r7,A1,b70),(r7,A1,b71),(r7,A1,b72),(r7,A1,b73),(r7,A1,b74),(r7 ,A1,b75),(r7,A1,b76),(r7,A1,b77),(r7,A1,b78),(r7,A1,b79),(r7,A1,b80),(r7,A1,b81),(r7,A1,b82),(r7,A 1,b83),(r7,A1,b84),(r7,A1,b85),(r7,A1,b86),(r7,A1,b87),(r7,A1,b88),(r7,A1,b89),(r7,A1,b90),(r7,A1, b91),(r7,A1,b92),(r7,A1,b93),(r7,A1,b94),(r7,A1,b95),(r7,A1,b96),(r7,A1,b97),(r7,A1,b98),(r7,A1,b 99),(r7,A1,b100),(r7,A1,b101),(r7,A1,b102),(r7,A1,b103),(r7,A1,b104),(r7,A1,b105),(r7,A1,b106),(r 7,A1,b107),(r7,A1,b108),(r7,A1,b109),(r7,A1,b110),(r7,A1,b111),(r7,A1,b112),(r7,A1,b113),(r7,A1, b114),(r7,A1,b115),(r7,A1,b116),(r7,A1,b117),(r7,A1,b118),(r7,A1,b119),(r7,A1,b120),(r7,A1,b121), (r7,A1,b122),(r7,A1,b123),(r7,A1,b124),(r7,A1,b125),(r7,A1,b126),(r7,A1,b127),(r7,A1,b128),(r7,A 1,b129),(r7,A1,b130),(r7,A1,b131),(r7,A1,b132),(r7,A1,b133),(r7,A1,b134),(r7,A2,b1),(r7,A2,b2),(r 7,A2,b3),(r7,A2,b4),(r7,A2,b5),(r7,A2,b6),(r7,A2,b7),(r7,A2,b8),(r7,A2,b9),(r7,A2,b10),(r7,A2,b11), (r7,A2,b12),(r7,A2,b13),(r7,A2,b14),(r7,A2,b15),(r7,A2,b16),(r7,A2,b17),(r7,A2,b18),(r7,A2,b19),(r 7,A2,b20),(r7,A2,b21),(r7,A2,b22),(r7,A2,b23),(r7,A2,b24),(r7,A2,b25),(r7,A2,b26),(r7,A2,b27),(r7, A2,b28),(r7,A2,b29),(r7,A2,b30),(r7,A2,b31),(r7,A2,b32),(r7,A2,b33),(r7,A2,b34),(r7,A2,b35),(r7,A 2,b36),(r7,A2,b37),(r7,A2,b38),(r7,A2,b39),(r7,A2,b40),(r7,A2,b41),(r7,A2,b42),(r7,A2,b43),(r7,A2, b44),(r7,A2,b45),(r7,A2,b46),(r7,A2,b47),(r7,A2,b48),(r7,A2,b49),(r7,A2,b50),(r7,A2,b51),(r7,A2,b 52),(r7,A2,b53),(r7,A2,b54),(r7,A2,b55),(r7,A2,b56),(r7,A2,b57),(r7,A2,b58),(r7,A2,b59),(r7,A2,b6 0),(r7,A2,b61),(r7,A2,b62),(r7,A2,b63),(r7,A2,b64),(r7,A2,b65),(r7,A2,b66),(r7,A2,b67),(r7,A2,b68) ,(r7,A2,b69),(r7,A2,b70),(r7,A2,b71),(r7,A2,b72),(r7,A2,b73),(r7,A2,b74),(r7,A2,b75),(r7,A2,b76),( r7,A2,b77),(r7,A2,b78),(r7,A2,b79),(r7,A2,b80),(r7,A2,b8.),(r7,A2,b82),(r7,A2,b83),(r7,A2,b84),(r7 ,A2,b85),(r7,A2,b86),(r7,A2,b87),(r7,A2,b88),(r7,A2,b89),(r7,A2,b90),(r7,A2,b91),(r7,A2,b92),(r7,A 2,b93),(r7,A2,b94),(r7,A2,b95),(r7,A2,b96),(r7,A2,b97),(r7,A2,b98),(r7,A2,b99),(r7,A2,b100),(r7,A 2,b101),(r7,A2,b102),(r7,A2,b103),(r7,A2,b104),(r7,A2,b105),(r7,A2,b106),(r7,A2,b107),(r7,A2,b10 8),(r7,A2,b109),(r7,A2,b110),(r7,A2,b111),(r7,A2,b112),(r7,A2,b113),(r7,A2,b114),(r7,A2,b115), (r7, A2,b116),(r7,A2,b117),(r7,A2,bl18),(r7,A2,b119),(r7,A2,b120),(r7,A2,b121),(r7,A2,b122),(r7,A2,b1 23),(r7,A2,b124),(r7,A2,b125),(r7,A2,b126),(r7,A2,b127),(r7,A2,b128),(r7,A2,b129),(r7,A2,b130),(r 7,A2,b131),(r7,A2,b132),(r7,A2,b133),(r7,A2,b134),(r7,A3,b1),(r7,A3,b2),(r7,A3,b3),(r7,A3,b4),(r7, A3,b5),(r7,A3,b6),(r7,A3,b7),(r7,A3,b8),(r7,A3,b9),(r7,A3,b10),(r7,A3,b11),(r7,A3,b12),(r7,A3,b13), (r7,A3,b14),(r7,A3,b15),(r7,A3,b16),(r7,A3,b17),(r7,A3,b18),(r7,A3,b19),(r7,A3,b20),(r7,A3,b21),(r 7,A3,b22),(r7,A3,b23),(r7,A3,b24),(r7,A3,b25),(r7,A3,b26),(r7,A3,b27),(r7,A3,b28),(r7,A3,b29),(r7, A3,b30),(r7,A3,b31),(r7,A3,b32),(r7,A3,b33),(r7,A3,b34),(r7,A3,b35),(r7,A3,b36),(r7,A3,b37),(r7,A 3,b38),(r7,A3,b39),(r7,A3,b40),(r7,A3,b41),(r7,A3,b42),(r7,A3,b43),(r7,A3,b44),(r7,A3,b45),(r7,A3, b46),(r7,A3,b47),(r7,A3,b48),(r7,A3,b49),(r7,A3,b50),(r7,A3,b51),(r7,A3,b52),(r7,A3,b53),(r7,A3,b 54),(r7,A3,b55),(r7,A3,b56),(r7,A3,b57),(r7,A3,b58),(r7,A3,b59),(r7,A3,b60),(r7,A3,b61),(r7,A3,b6 2),(r7,A3,b63),(r7,A3,b64),(r7,A3,b65),(r7,A3,b66),(r7,A3,b67),(r7,A3,b68),(r7,A3,b69),(r7,A3,b70) ,(r7,A3,b71),(r7,A3,b72),(r7,A3,b73),(r7,A3,b74),(r7,A3,b75),(r7,A3,b76),(r7,A3,b77),(r7,A3,b78),( r7,A3,b79),(r7,A3,b80),(r7,A3,b81),(r7,A3,b82),(r7,A3,b83),(r7,A3,b84),(r7,A3,b85),(r7,A3,b86),(r7 ,A3,b87),(r7,A3,b88),(r7,A3,b89),(r7,A3,b90),(r7,A3,b91),(r7,A3,b92),(r7,A3,b93),(r7,A3,b94),(r7,A 3,b95),(r7,A3,b96),(r7,A3,b97),(r7,A3,b98),(r7,A3,b99),(r7,A3-b100),(r7,A3,b101),(r7,A3,b102),(r7, A3,b103),(r7,A3,b104),(r7,A3,b105),(r7,A3,b106),(r7,A3,b107),(r7,A3,b108),(r7,A3,b109),(r7,A3,b 110),(r7,A3,b111),(r7,A3,b112),(r7,A3,b113),(r7,A3,b114),(r7,A3,b115),(r7,A3,b116),(r7,A3,b117),( r7,A3,b118),(r7,A3,b119),(r7,A3,b120),(r7,A3,b121),(r7,A3,b122),(r7,A3,b123),(r7,A3,b124),(r7,A3 ,b125),(r7,A3,b126),(r7,A3,b127),(r7,A3,b128),(r7,A3,b129),(r7,A3,b130),(r7,A3,b131),(r7,A3,b132 ),(r7,A3,b133),(r7,A3,b134),(r7,A4,b1),(r7,A4,b2),(r7,A4,b3),(r7,A4,b4),(r7,A4,b5),(r7,A4,b6),(r7,A 4,b7),(r7,A4,b8),(r7,A4,b9),(r7,A4,b10),(r7,A4,b11),(r7,A4,b12),(r7,A4,b13),(r7,A4,b14),(r7,A4,b15 ),(r7,A4,b16),(r7,A4,b17),(r7,A4,b18),(r7,A4,b19),(r7,A4,b20),(r7,A4,b21),(r7,A4,b22),(r7,A4,b23),( r7,A4,b24),(r7,A4,b25),(r7,A4,b26),(r7,A4,b27),(r7,A4,b28),(r7,A4,b29),(r7,A4,b30),(r7,A4,b31),(r7 ,A4,b32),(r7,A4,b33),(r7,A4,b34),(r7,A4,b35),(r7,A4,b36),(r7,A4,b37),(r7,A4,b38),(r7,A4,b39),(r7,A 4,b40),(r7,A4,b41),(r7,A4,b42),(r7,A4,b43),(r7,A4,b44),(r7,A4,b45),(r7,A4,b46),(r7,A4,b47),(r7,A4, b48),(r7,A4,b49),(r7,A4,b50),(r7,A4,b51),(r7,A4,b52),(r7,A4,b53),(r7,A4,b54),(r7,A4,b55),(r7,A4,b 56),(r7,A4,b57),(r7,A4,b58),(r7,A4,b59),(r7,A4,b60),(r7,A4,b61),(r7,A1,b62),(r7,A4,b63),(r7,A4,b6 4),(r7,A4,b65),(r7,A4,b66),(r7,A4,b67),(r7,A4,b68),(r7,A4,b69),(r7,A4,b70),(r7,A4,b71),(r7,A4,b72) ,(r7,A4,b73),(r7,A4,b74),(r7,A4,b75),(r7,A4,b76),(r7,A4,b77),(r7,A4,b78),(r7,A4,b79),(r7,A4,b80),( r7,A4,b81),(r7,A4,b82),(r7,A4,b83),(r7,A4,b84),(r7,A4,b85),(r7,A4,b86),(r7,A4,b87),(r7,A4,b88),(r7 ,A4,b89),(r7,A4,b90),(r7,A4,b91),(r7,A4,b92),(r7,A4,b93),(r7,A4,b94),(r7,A4,b95),(r7,A4,b96),(r7,A 4,b97),(r7,A4,b98),(r7,A4,b99),(r7,A4,b100),(r7,A4,b101),(r7,A4,b102),(r7,A4,b103),(r7,A4,b104),( r7,A4,b105),(r7,A4,b106),(r7,A4,b107),(r7,A4,b108),(r7,A4,b109),(r7,A4,b110),(r7,A4,b111),(r7,A4 ,b112),(r7,A4,b113),(r7,A4,b114),(r7,A4,b115),(r7,A4,b116),(r7,A4,b117),(r7,A4,b118),(r7,A4,b119) ,(r7,A4,b120),(r7,A4,b121),(r7,A4,b122),(r7,A4,b123),(r7,A4,b124),(r7,A4,b125),(r7,A4,b126),(r7, A4,b127),(r7,A4,b128),(r7,A4,b129),(r7,A4,b130),(r7,A4,b131),(r7,A4,b132),(r7,A4,b133),(r7,A4,b 134),

(r8,A1,b1),(r8,A1,b2),(r8,A1,b3),(r8,A1,b4),(r8,A1,b5),(r8,A1,b6),(r8,A1,b7),(r8,A1,b8),(r8,A1,b9),( r8,A1,b10),(r8,A1,b11),(r8,A1,b12),(r8,A1,b13),(r8,A1,b14),(r8,A1,b15),(r8,A1,b16),(r8,A1,b17),(r8 ,A1,b18),(r8,A1,b19),(r8,A1,b20),(r8,A1,b21),(r8,A1,b22),(r8,A1,b23),(r8,A1,b24),(r8,A1,b25),(r8,A 1,b26),(r8,A1,b27),(r8,A1,b28),(r8,A1,b29),(r8,A1,b30),(r8,A1,b31),(r8,A1,b32),(r8,A1,b33),(r8,A1, b34),(r8,A1,b35),(r8,A1,b36),(r8,A1,b37),(r8,A1,b38),(r8,A1,b39),(r8,A1,b40),(r8,A1,b41),(r8,A1,b 42),(r8,A1,b43),(r8,A1,b44),(r8,A1,b45),(r8,A1,b46),(r8,A1,b47),(r8,A1,b48),(r8,A1,b49),(r8,A1,b5 0),(r8,A1,b51),(r8,A1,b52),(r8,A1,b53),(r8,A1,b54),(r8,A1,b55),(r8,A1,b56),(r8,A1,b57),(r8,A1,b58) ,(r8,A1,b59),(r8,A1,b60),(r8,A1,b61),(r8,A1,b62),(r8,A1,b63),(r8,A1,b64),(r8,A1,b65),(r8,A1,b66),( r8,A1,b67),(r8,A1,b68),(r8,A1,b69),(r8,A1,b70),(r8,A1,b71),(r8,A1,b72),(r8,A1,b73),(r8,A1,b74),(r8 ,A1,b75),(r8,A1,b76),(r8,A1,b77),(r8,A1,b78),(r8,A1,b79),(r8,A1,b80),(r8,A1,b81),(r8,A1,b82),(r8,A 1,b83),(r8,A1,b84),(r8,A1,b85),(r8,A1,b86),(r8,A1,b87),(r8,A1,b88),(r8,A1,b89),(r8,A1,b90),(r8,A1, b91),(r8,A1,b92),(r8,A1,b93),(r8,A1,b94),(r8,A1,b95),(r8,A1,b96),(r8,A1,b97),(r8,A1,b98),(r8,A1,b 99),(r8,A1,b100),(r8,A1,b101),(r8,A1,b102),(r8,A1,b103),(r8,A1,b104),(r8,A1,b10j),(r8,Al,b106),(r 8,A1,b107),(r8,A1,b108),(r8,A1,b109),(r8,A1,b110),(r8,A1,b111),(r8,A1,b112),(r8,A1,b113),(r8,A1, b114),(r5,A1,b115),(r5,A1,b116),(r5,A1,b117),(r5,A1,b118),(r5,A1,b119),(r5,A1,b120),(r5,A1,b121), (r8,A1,b122),(r8,A1,b123),(r8,A1,b124),(r8,A1,b125),(r8,A1,b126),(r8,A1,b127),(r8,A1,b128),(r8,A 1,b129),(r8,A1,b130),(r8,A1,b131),(r8,A1,b132),(r8,A1,b133),(r8,A1,b134),(r8,A2,b1),(r8,A2,b2),(r 8,A2,b3),(r8,A2,b4),(r8,A2,b5),(r8,A2,b6),(r8,A2,b7),(r8,A2,b8),(r8,A2,b9),(r8,A2,b10),(r8,A2,b11), (r8,A2,b12),(r8,A2,b13),(r8,A2,b14),(r8,A2,b15),(r8,A2,b16),(r8,A2,b17),(r8,A2,b18),(r8,A2,b19),(r 8,A2,b20),(r8,A2,b21),(r8,A2,b22),(r8,A2,b23),(r8,A2,b24),(r8,A2,b25),(r8,A2,b26),(r8,A2,b27),(r8, A2,b28),(r8,A2,b29),(r8,A2,b30),(r8,A2,b31),(r8,A2,b32),(r8,A2,b33),(r8,A2,b34),(r8,A2,b35),(r8,A 2,b36),(r8,A2,b37),(r8,A2,b38),(r8,A2,b39),(r8,A2,b40),(r8,A2,b41),(r8,A2,b42),(r8,A2,b43),(r8,A2, b44),(r8,A2,b45),(r8,A2,b46),(r8,A2,b47),(r8,A2,b48),(r8,A2,b49),(r8,A2,b50),(r8,A2,b551),(r8,A2,b 52),(r8,A2,b53),(r8,A2,b54),(r8,A2,b55),(r8,A2,b56),(r8,A2,b57),(r8,A2,b58),(r8,A2,b59),(r8,A2,b6 0),(r8,A2,b61),(r8,A2,b62),(r8,A2,b63),(r8,A2,b64),(r8,A2,b65),(r8,A2,b66),(r8,A2,b67),(r8,A2,b68) ,(r8,A2,b69),(r8,A2,b70),(r8,A2,b71),(r8,A2,b72),(r8,A2,b73),(r8,A2,b74),(r8,A2,b75),(r8,A2,b76),( r8,A2,b77),(r8,A2,b78),(r8,A2,b79),(r8,A2,b80),(r8,A2,b81),(r8,A2,b82),(r8,A2,b83),(r8,A2,b84),(r8 ,A2,b85),(r8,A2,b86),(r8,A2,b87),(r8,A2,b88),(r8,A2,b89),(r8,A2,b90),(r8,A2,b91),(r8,A2,b92),(r8,A (r8,A 2,b101),(r8,A2,b102),(r8,A2,b103),(r8,A2,b104),(r8,A2,b105),(r8,A2,b106),(r8,A2,b107),(r8,A2,b10 8),(r8,A2,b109),(r8,A2,b110),(r8,A2,b111),(r8,A2,b112),(r8,A2,b113),(r8,A2,b114),(r8,A2,b115),(r8, A2,b116),(r8,A2,b117),(r8,A2,b118),(r8,A2,b119),(r8,A2,b120),(r8,A2,b121),(r8,A2,b122),(r8,A2,b 1 23),(r8,A2,b124),(r8,A2,b125),(r8,A2,b126),(r8,A2,b127),(r8,A2,b128),(r8,A2,b129),(r8,A2,b130),(r 8,A2,b131),(r8,A2,b132),(r8,A2,b133),(r8,A2,b134),(r8,A3,b1),(r8,A3.b2),(r8,A3,b3),(r8,A3,b4),(r8, A3,b5),(r8,A3,b6),(r8,A3,b7),(r8,A3,b8),(r8,A3,b9),(r8,A3,b10),(r8,A3,b11),(r8,A3,b12),(r8,A3,b13), (r8,A3,b14),(r8,A3,b15),(r8,A3,b16),(r8,A3,b17),(r8,A3,b18),(r8,A3,b19),(r8,A3,b20),(r8,A3,b21),(r 8,A3,b22),(r8,A3,b23),(r8,A3,b24),(r8,A3,b25),(r8,A3,b26),(r8,A3,b27),(r8,A3,b28),(r8,A3,b29),(r8, A3,b30),(r8,A3,b31),(r8,A3,b32),(r8,A3,b33),(r8,A3,b34),(r8,A3,b35),(r8,A3,b36),(r8,A3,b37),(r8,A 3,b38),(r8,A3,b39),(r8,A3,b40),(r8,A3,b41),(r8,A3,b42),(r8,A3,b43),(r8,A3,b44),(r8,A3,b45),(r8,A3, b46),(r8,A3,b47),(r8,A3,b48),(r8,A3,b49),(r8,A3,b50),(r8,A3,b51),(r8,A3,b52),(r8,A3,b53),(r8,A3,b 54),(r8,A3,b55),(r8,A3,b56),(r8,A3,b57),(r8,A3,b58),(r8,A3,b60),(r8,A3,b60),(r8,A3,b61),(r8,A3,b6 2),(r8,A3,b63),(r8,A3,b64),(r8,A3,b65),(r8,A3,b66),(r8,A3,b67),(r8,A3,b68),(r8,A3,b69),(r8,A3,b70) ,(r8,A3,b71),(r8,A3,b72),(r8,A3,b73),(r8,A3,b74),(r8,A3,b75),(r8,A3,b76),(r8,A3,b77),(r8,A3,b78),( r8,A3,b79),(r8,A3,b80),(r8,A3,b81),(r8,A3,b82),(r8,A3,b83),(r8,A3,b84),(r8,A3,b85),(r8,A3,b86),(r8 ,A3,b87),(r8,A3,b88),(r8,A3,b89),(r8,A3,b90),(r8,A3,b91),(r8,A3,b92),(r8,A3,b93),(r8,A3,b94),(r8,A 3,b95),(r8,A3,b96),(r8,A3,b97),(r8,A3,b98),(r8,A3,b99),(r8,A3,b100),(r8,A3,b101),(r8,A3,b102),(r8, A3,b103),(r8,A3,b104),(r8,A3,b105),(r8,A3,b106),(r8,A3,b107),(r8,A3,b108),(r8,A3,b109),(r8,A3,b 110),(r8,A3,b111),(r8,A3,b112),(r8,A3,b113),(r8,A3,b114),(r8,A3,b115),(r8,A3,b116),(r8,A3,b117),( r8,A3,b118),(r8,A3,b119),(r8.A3,b120),(r8,A3,b121),(r8,A3,b122),(r8,A3,b123),(r8,A3,b124),(r8,A3 ,b125),(r8,A3,b126),(r8,A3,b127),(r8,A3,b128),(r8,A3,b1.29),(r8,A3,b130),(r8,A3,b131),(r8,A3,b132 ),(r8,A3,b133),(r8,A3,b134),(r8,A4,b1),(r8,A4,b2),(r8,A4,b3),(r8,A4,b4),(r8,A4,b5),(r8,A4,b6),(r8,A 4,b7),(r8,A4,b8),(r8,A4,b9),(r8,A4,b10),(r8,A4,b11),(r8,A4,b12),(r8,A4,b13),(r8,A4,b14),(r8,A4,b15 ),(r8,A4,b16),(r8,A4,b17),(r8,A4,b18),(r8,A4,b19),(r8,A4,b20),(r8,A4,b21),(r8,A4,b22),(r8,A4,b23),( r8,A4,b24),(r8,A4,b25),(r8,A4,b26),(r8,A4,b27),(r8,A4,b28),(r8,A4,b29),(r8,A4,b30),(r8,A4,b31),(r8 ,A4,b32),(r8,A4,b33),(r8,A4,b34),(r8,A4,b35),(r8,A4,b36),(r8,A4,b37),(r8,A4,b38),(r8,A4,b39),(r8,A 4,b40),(r8,A4,b41),(r8,A4,b42),(r8,A4,b43),(r8,A4,b44),(r8,A4,b45),(r8,A4,b46),(r8,A4,b47),(r8,A4, b48),(r8,A4,b49),(r8,A4,b50),(r8,A4,b51),(r8,A4,b52),(r8,A4,b53),(r8,A4,b54),(r8,A4,b55),(r8,A4,b 56),(r8,A4,b57),(r8,A4,b58),(r8,A4,b59),(r8,A4,b60),(r8,A4,b61),(r8,A4,b62),(r8,A4,b63),(r8,A4,b6 4),(r8,A4,b65),(r8,A4,b66),(r8,A4,b67),(r8,A4,b68),(r8,A4,b69)-(r8,A4,b70),(r8,A4,b71),(r8,A4,b72) ,(r8,A4,b73),(r8,A4,b74),(r8,A4,b75),(r8,A4,b76),(r8,A4,b77),(r8,A4,b78),(r8,A4,b79),(r8,A4,b80),( r8,A4,b81),(r8,A4,b82),(r8,A4,b83),(r8,A4,b84),(r8,A4,b85),(r8,A4,b86),(r8,A4,b87),(r8,A4,b88),(r8 ,A4,b89),(r8,A4,b90),(r8,A4,b91),(r8,A4,b92),(r8,A4,b93),(r8,A4,b94),(r8,A1,b95),(r8,A4,b96),(r8,A 4,b97),(r8,A4,b98),(r8,A4,b99),(r8,A4,b100),(r8,A4,b101),(r8,A4,b102),(r8,A4,b103),(r8,A4,b104),( r8,A4,b105),(r8,A4,b106),(r8,A4,b107),(r8,A4,b108),(r8,A4,b109),(r8,A4,b110),(r8,A4,b111),(r8,A4 ,b112),(r8,A4,b113),(r8,A4,b114),(r8,A4,b115),(r8,A4,b116),(r8,A4,b117),(r8,A4,b118),(r8,A4,b119) ,(r8,A4,b120),(r8,A4,b121),(r8,A4,b122),(r8,A4,b123),(r8,A4,b124),(r8,A4,b125),(r8,A4,b126),(r8, A4,b127),(r8,A4,b128),(r8,A4,b129),(r8,A4,b130),(r8,A4,b131),(r8,A4,b132),(r8,A4,b133),(r8,A4,b 134),

(r9,A1,b1),(r9,A1,b2),(r9,A1,b3),(r9,A1,b4),(r9,A1,b5),(r9,A1,b6),(r9,A1,b7),(r9,A1,b8),(r9,A1,b9),( r9,A1,b10),(r9,A1,b11),(r9,A1,b12),(r9,A1,b13),(r9,A1,b14),(r9,A1,b15),(r9,A1,b16),(r9,A1,b17),(r9 ,A1,b18),(r9,A1,b19),(r9,A1,b20),(r9,A1,b21),(r9,A1,b22),(r9,A1,b23),(r9,A1,b24),(r9,A1,b25),(r9,A 1,b26),(r9,A1,b27),(r9,A1,b28),(r9,A1,b29),(r9,A1,b30),(r9,A1,b31),(r9,A1,b32),(r9,A1,b33),(r9,A1, b34),(r9,A1,b35),(r9,A1,b36),(r9,A1,b37),(r9,A1,b38),(r9,A1,b39),(r9,A1,b40),(r9,A1,b41),(r9,A1,b 42),(r9,A1,b43),(r9,A1,b44),(r9,A1,b45),(r9,A1,b46),(r9,A1,b47),(r9,A1,b48),(r9,A1,b49),(r9,A1,b5 0),(r9,A1,b51),(r9,A1,b52),(r9,A1,b53),(r9,A1,b54),(r9,A1,b55),(r9,A1,b56),(r9,A1,b57),(r9,A1,b58) ,(r9,A1,b59),(r9,A1,b60),(r9,A1,b61),(r9,A1,b62),(r9,A1,b63),(r9,A1,b64),(r9,A1,b65),(r9,A1,b66),( r9,A1,b67),(r9,A1,b68),(r9,A1,b69),(r9,A1,b70),(r9,A1,b71),(r9,A1,b72),(r9,A1,b73),(r9,A1,b74),(r9 ,A1,b75),(r9,A1,b76),(r9,A1,b77),(r9,A1,b78),(r9,A1,b79),(r9,A1,b80),(r9,A1,b81),(r9,A1,b82),(r9,A 1,b83),(r9,A1,b84),(r9,A1,b85),(r9,A1,b86),(r9,A1,b87),(r9,A1,b88),(r9,A1,b89),(r9,A1,b90),(r9,A1, b91),(r9,A1,b92),(r9,A1,b93),(r9,A1,b94),(r9,A1,b95),(r9,A1,b96),(r9,A1,b97),(r9,A1,b98),(r9,A1,b 99),(r9,A1,b100),(r9,A1,b101),(r9,A1,b102),(r9,A1,b103),(r9,A1,b104),(r9,A1,b105),(r9,A1,b106),(r 9,A1,b107),(r9,A1,b108),(r9,A1,b109),(r9,A1,b110),(r9,A1,b111),(r9,A1,b112),(r9,A1,b113),(r9,A1, b114),(r9,A1,b115),(r9,A1,b116),(r9,A1,b117),(r9,A1,b118),(r9,A1,b119),(r9,A1,b120),(r9,A1,b121), (r9,A1,b122),(r9,A1,b123),(r9,A1,b124),(r9,A1,b125),(r9,A1,b126),(r9,A1,b127),(r9,A1,b128),(r9,A 1,b129),(r9,A1,b130),(r9,A1,b131),(r9,A1,b132),(r9,A1,b133),(r9,A1,b134),(r9,A2,b1),(r9,A2,b2),(r 9,A2,b3),(r9,A2,b4),(r9,A2,b5),(r9,A2,b6),(r9,A2,b7),(r9,A2,b8),(r9,A2,b9),(r9,A2,b10),(r9,A2,b11), (r9,A2,b12),(r9,A2,b13),(r9,A2,b14),(r9,A2,b15),(r9,A2,b16),(r9,A2,b17),(r9,A2,b18),(r9,A2,b19),(r 9,A2,b20),(r9,A2,b21),(r9,A2,b22),(r9,A2,b23),(r9,A2,b24),(r9,A2,b25),(r9,A2,b26),(r9,A2,b27),(r9, A2,b28),(r9,A2,b29),(r9,A2,b30),(r9,A2,b31),(r9,A2,b32),(r9,A2,b33),(r9,A2,b34),(r9,A2,b35),(r9,A 2,b36),(r9,A2,b37),(r9,A2,b38),(r9,A2,b39),(r9,A2,b40),(r9,A2,b411),(r9,A2,b42),(r9,A2,b43),(r9,A2, b44),(r9,A2,b45),(r9,A2,b46),(r9,A2,b47),(r9,A2,b48),(r9,A2,b49),(r9,A2,b50),(r9,A2,b51),(r9,A2,b 52),(r9,A2,b53),(r9,A2,b54),(r9,A2,b55),(r9,A2,b56),(r9,A2,b57),(r9,A2,b58),(r9,A2,b59),(r9,A2,b6 0),(r9,A2,b61),(r9,A2,b62),(r9,A2,b63),(r9,A2,b64),(r9,A2,b65),(r9,A2,b66),(r9,A2,b67),(r9,A2,b68) ,r9,A2,b69),(r9,A2,b70),(r9,A2,b71),(r9,A2,b72),(r9,A2,b73),(r9,A2,b74),(r9,A2,b75),(r9,A2,b76),( r9,A2,b77),(r9,A2,b78),(r9,A2,b79),(r9,A2,b80),(r9,A2,b81),(r9,A2,b82),(r9,A2,b83),(r9,A2,b84),(r9 ,A2,b85),(r9,A2,b86),(r9,A2,b87),(r9,A2,b88),(r9,A2,b89),(r9,A2,b90),(r9,A2,b91),(r9,A2,b92),(r9,A 2,b93),(r9,A2,b94),(r9,A2,b95),(r9,A2,b96),(r9,A2,b97),(r9,A2,b98),(r9,A2,b99),(r9,A2,b100),(r9,A 2,b101),(r9,A2,b102),(r9,A2,b103),(r9,A2,b104),(r9,A2,b105),(r9,A2,b106),(r9,A2,b107),(r9,A2,b10 8),(r9,A2,b109),(r9,A2,b110),(r9,A2,b111),(r9,A2,b112),(r9,A2,b113),(r9,A2,b114),(r9,A2,b115),(r9, A2,b116),(r9,A2,b117),(r9,A2,b118),(r9,A2,b119),(r9,A2,b120),(r9,A2,b121),(r9,A2,b122),(r9,A2,b1 23),(r9,A2,b124),(r9,A2,b125),(r9,A2,b126),(r9,A2,b127),(r9,A2,b128),(r9,A2,b129),(r9,A2,b130),(r 9,A2,b131),(r9,A2,b132),(r9,A2,b133),(r9,A2,b134),(r9,A3,b1),(r9,A3,b2),(r9,A3,b3),(r9,A3,b4),(r9, A3,b5),(r9,A3,b6),(r9,A3,b7),(r9,A3,b8),(r9,A3,b9),(r9,A3,b10),(r9,A3,b11),(r9,A3,b12),(r9,A3,b13), (r9,A3,b14),(r9,A3,b15),(r9,A3,b16),(r9,A3,b17),(r9,A3,b18),(r9,A3,b19),(r9,A3,b20),(r9,A3,b21),(r 9,A3,b22),(r9,A3,b23),(r9,A3,b24),(r9,A3,b25),(r9,A3,b26),(r9,A3,b27),(r9,A3,b28),(r9,A3,b29),(r9, A3,b30),(r9,A3,b31),(r9,A3,b32),(r9,A3,b33),(r9,A3,b34),(r9,A3,b35),(r9,A3,b36),(r9,A3,b37),(r9,A 3,b38),(r9,A3,b39),(r9,A3,b40),(r9,A3,b41),(r9,A3,b42),(r9,A3,b43),(r9,A3,b44),(r9,A3,b45),(r9,A3, b46),(r9,A3,b47),(r9,A3,b48),(r9,A3,b49),(r9,A3,b50),(r9,A3,b51),(r9,A3,b52),(r9,A3,b53),(r9,A3,b 54),(r9,A3,b55),(r9,A3,b56),(r9,A3,b57),(r9,A3,b58),(r9,A3,b59),(r9,A3,b60),(r9,A3,b61),(r9,A3,b6 2),(r9,A3,b63),(r9,A3,b64),(r9,A3,b65),(r9,A3,b66),(r9,A3,b67),(r9,A3,b68),(r9,A3,b69),(r9,A3,b70) ,(r9,A3,b71),(r9,A3,b72),(r9,A3,b73),(r9,A3,b74),(r9,A3,b75),(r9,A3,b76),(r9,A3,b77),(r9,A3,b78),( r9,A3,b79),(r9,A3,b80),(r9,A3,b81),(r9,A3,b82),(r9,A3,b83),(r9,A3,b84),(r9,A3,b85),(r9,A3,b86),(r9 ,A3,b87),(r9,A3,b88),(r9,A3,b89),(r9,A3,b90),(r9,A3,b91),(r9,A3,b92),(r9,A3,b93),(r9,A3,b94),(r9,A 3,b95),(r9,A3,b96),(r9,A3,b97),(r9,A3,b98),(r9,A3,b99),(r9,A3,b100),(r9,A3,b101),(r9,A3,b102),(r9, A3,b103),(r9,A3,b104),(r9,A3,b105),(r9,A3,b106),(r9,A3,b107),(r9,A3,b108),(r9,A3,b109),(r9,A3,b 110),(r9,A3,b111),(r9,A3,b112),(r9,A3,b113),(r9,A.3,b114),(r9,A3,b115),(r9,A3,b116),(r9,A3,b117),( r9,A3,b118),(r9,A3,b119),(r9,A3,b120),(r9,A3,b121),(r9,A3,b122),(r9,A3,b123),(r9,A3,b124),(r9,A3 ,b125),(r9,A3,b126),(r9,A3,b127),(r9,A3,b128),(r9,A3,b129),(r9,A3,b130),(r9,A3,b131),(r9,A3,b132 ),(r9,A3,b133),(r9,A3,b134),(r9,A4,b1),(r9,A4,b2),(r9,A4,b3),(r9,A4,b4),(r9,A4,b5),(r9,A4,b6),(r9,A 4,b7),(r9,A4,b8),(r9,A4,b9),(r9,A4,b10),(r9,A4,b11),(r9,A4,b12),(r9,A4,b13),(r9,A4,b14),(r9,A4,b15 ),(r9,A4,b16),(r9,A4,b17),(r9,A4,b18),(r9,A4,b19),(r9,A4,b20),(r9,A4,b21),(r9,A4,b22),(r9,A4,b23), ( r9,A4,b24),(r9,A4,b25),(r9,A4,b26),(r9,A4,b27),(r9,A4,b28),(r9,A4,b29),(r9,A4,b30),(r9,A4,b31),(r9 ,A4,b32),(r9,A4,b33),(r9,A4,b34),(r9,A4,b35),(r9,A4,b36),(r9,A4,b37),(r9,A4,b38),(r9,A4,b39),(r9,A 4,b40),(r9,A4,b41),(r9,A4,b42),(r9,A4,b43),(r9,A4,b44),(r9,A4,b45),(r9,A4,b46),(r9,A4,b47),(r9,A4, b48),(r9,A4,b49),(r9,A4,b50),(r9,A4,b51),(r9,A4,b52),(r9,A4,b53),(r9,A4,b54),(r9,A4,b55),(r9,A4,b 56),(r9,A4,b57),(r9,A4,b58),(r9,A4,b59),(r9,A4,b60),(r9,A4,b61),(r9,A4,b62),(r9,A4,b63),(r9,A4,b6 4),(r9,A4,b65),(r9,A4,b66),(r9,A4,b67),(r9,A4,b68),(r9,A4,b69),(r9,A4,b70),(r9,A4,b71),(r9,A4,b72) ,(r9,A4,b73),(r9,A4,b74),(r9,A4,b75),(r9,A4,b76),(r9,A4,b77),(r9,A4,b78),(r9,A4,b79),(r9,A4,b80),( r9,A4,b81),(r9,A4,b82),(r9,A4,b83),(r9,A4,b84),(r9,A4,b85),(r9,A4,b86),(r9,A4,b87),(r9,A4,b88),(r9 ,A4,b89),(r9,A4,b90),(r9,A4,b91),(r9,A4,b92),(r9,A4,b93),(r9,A4,b94),(r9,A4,b95),(r9,A4,b96),(r9,A 4,b97),(r9,A4,b98),(r9,A4,b99),(r9,A4,b100),(r9,A4,b101),(r9,A4,b102),(r9,A4,b103),(r9,A4,b104),( r9,A4,b105),(r9,A4,b106),(r9,A4,b107),(r9,A4,b108),(r9,A4,b109),(r9,A4,b110),(r9,A4,b111),(r9,A4 ,b112),(r9,A4,b113),(r9,A4,b114),(r9,A4,b115),(r9,A4,b116),(r9,A4,b117),(r9,A4,b118),(r9,A4,b119) ,(r9,A4,b120),(r9,A4,b121),(r9,A4,b122),(r9,A4,b123),(r9,A4,b124),(r9,A4,b125),(r9,A4,b126),(r9, A4,b127),(r9,A4,b128),(r9,A4,b129),(r9,A4,b130),(r9,A4,b131),(r9,A4,b132),(r9,A4,b133),(r9,A4,b 134),

(r10,A1,b1),(r10,A1,b2),(r10,A1,b3),(r10,A1,b4),(r10,A1,b5),(r10,A1,b6),(r10,A1,b7),(r10,A1,b8),(r 10,A1,b9),(r10,A1,b10),(r10,A1,b11),(r10,A1,b12),(r10,A1,b13),(r10,A1,b14),(r10,A1,b15),(r10,A1, b16),(r10,A1,b17),(r10,A1,b18),(r10,A1,b19),(r10,A1,b20),(r10,A1,b21),(r10,A1,b22),(r10,A1,b23), (r10,A1,b24),(r10,A1,b25),(r10,A1,b26),(r10,A1,b27),(r10,A1,b28),(r10,A1,b29),(r10,A1,b30),(r10, A1,b31),(r10,A1,b32),(r10,A1,b33),(r10,A1,b34),(r10,A1,b35),(r10,A1,b36),(r10,A1,b37),(r10,A1,b 38),(r10,A1,b39),(r10,A1,b40),(r10,A1,b41),(r10,A1,b42),(r10,A1,b43),(r10,A1,b44),(r10,A1,b45),(r 10,A1,b46),(r10,A1,b47),(r10,A1,b48),(r10,A1,b49),(r10,A1,b50),(r10,A1,b51),(r10,A1,b52),(r10,A 1,b53),(r10,A1,b54),(r10,A1,b55),(r10,A1,b56),(r10,A1,b57),(r10,A1,b58),(r10,A1,b59),(r10,A1,b60 ),(r10,A1,b61),(r10,A1,b62),(r10,A1,b63),(r10,A1,b64),(r10,A1,b65),(r10,A1,b66),(r10,A1,b67),(r10 ,A1,b68),(r10,A1,b69),(r10,A1,b70),(r10,A1,b71),(r10,A1,b72),(r10,A1,b73),(r10,A1,b74),(r10,A1,b 75),(r10,A1,b76),(r10,A1,b77),(r10,A1,b78),(r10,A1,b79),(r10,A1,b80),(r10,A1,b81),(r10,A1,b82),(r 10,A1,b83),(r10,A1,b84),(r10,A1,b85),(r10,A1,b86),(r10,A1,b87),(r10,A1,b88),(r10,A1,b89),(r10,A 1,b90),(r10,A1,b91),(r10,A1,b92),(r10,A1,b93),(r10,A1,b94),(r10,A1,b95),(r10,A1,b96),(r10,A1,b97 ),(r10,A1,b98),(r10,A1,b99),(r10,A1,b100),(r10,A1,b101),(r10,A1,b102),(r10,A1,b103),(r10,A1,b10 4),(r10,A1,b105),(r10,A1,b106),(r10,A1,b107),(r10,A1,b108),(r10,A1,b109),(r10,A1,b110),(r10,A1, b111),(r10,A1,b112),(r10,A1,b113),(r10,A1,b114),(r10,A1,b115),(r10,A1,b116),(r10,A1,b117),(r10, Al,b118),(r10,A1,b119),(r10,A1,b120),(r10,A1,b121),(r10,A1,b122),(r10,Al,b123),(r10,A1,b124),(r 10,A1,b125),(r10,A1,b126),(r10,A1,b127),(r10,A1,b128),(r10,A1,b129),(r10,A1,b130),(r10,A1,b131 ),(r10,A1,b132),(r10,A1,b133),(r10,A1,b134),(r10,A2,b1),(r10,A2,b2),(r10,A2,b3),(r10,A2,b4),(r10, A2,b5),(r10,A2,b6),(r10,A2,b7),(r10,A2,b8),(r10,A2,b9),(r10,A2,b10),(r10,A2,b11),(r10,A2,b12),(r1 0,A2,b13),(r10,A2,b14),(r10,A2,b15),(r10,A2,b16),(r10,A2,b17),(r10,A2,b18),(r10,A2,b19),(r10,A2, b20),(r10,A2,b21),(r10,A2,b22),(r10,A2,b23),(r10,A2,b24),(r10,A2,b25),(r10,A2,b26),(r10,A2,b27), (r10,A2,b28),(r10,A2,b29),(r10,A2,b30),(r10,A2,b31),(r10,A2,b32),(r10,A2,b33),(r10,A2,b34),(r10, A2,b35),(r10,A2,b36),(r10,A2,b37),(r10,A2,b38),(r10,A2,b39),(r10,A2,b40),(r10,A2,b41),(r10,A2,b 42),(r10,A2,b43),(r10,A2,b44),(r10,A2,b45),(r10,A2,b46),(r10,A2,b47),(r10,A2,b48),(r10,A2,b49),(r 10,A2,b,50),(r10,A2,b51),(r10,A2,b,52),(r10,A2,b53),(r10,A2,b54),(r10,A2,b55),(r10,A2,b56),(r10,A 2,b57),(r10,A2,b58),(r10,A2,b59),(r10,A2,b60),(r10,A2,b61),(r10,A2,b62),(r10,A2,b63),(r10,A2,b64 ),(r10,A2,b65),(r10,A2,b66),(r10,A2,b67),(r10,A2,b68),(r10,A2,b69),(r10,A2,b70),(r10,A2,b71),(r10 ,A2,b72),(r10,A2,b73),(r10,A2,b74),(r10,A2,b75),(r10,A2,b76),(r10,A2,b77),(r10,A2,b78),(r10,A2,b 79),(r10,A2,b80),(r10,A2,b81),(r10,A2,b82),(r10,A2,b83),(r10,A2,b84),(r10,A2,b85),(r10,A2,b86),(r 10,A2,b87),(r10,A2,b88),(r10,A2,b89),(r10,A2,b90),(r10,A2,b91),(r10,A2,b92),(r10,A2,b93),(r10,A 2,b94),(r10,A2,b95),(r10,A2,b96),(r10,A2,b97),(r10,A2,b98),(r10,A2,b99),(r10,A2,b100),(r10,A2,b1 01),(r10,A2,b102),(r10,A2,b103),(r10,A2,b104),(r10,A2,b105),(r10,A2,b106),(r10,A2,b107),(r10,A2 ,b108),(r10,A2,b109),(r10,A2,b110),(r10,A2,b111),(r10,A2,b112),(r10,A2,b113),(r10,A2,b114),(r10, A2,b115),(r10,A2,b116),(r10,A2,b117),(r10,A2,b118),(r10,A2,b119),(r10,A2,b120),(r10,A2,b121),(r 10,A2,b122),(r10,A2,b123),(r10,A2,b124),(r10,A2,b125),(r10,A2,b126),(r10,A2,b127),(r10,A2,b128 ),(r10,A2,b129),(r10,A2,b130),(r10,A2,b131),(r10,A2,b132),(r10,A2,b133),(r10,A2,b134),(r10,A3,b 1),(r10,A3,b2),(r10,A3,b3),(r10,A13,b4),(r10,A3,b5),(r10,A3,b6),(r10,A3,b7),(r10,A3,b8),(r10,A3,b9) ,(r10,A3,b10),(r10,A3,b11),(r10,A3,b12),(r10,A3,b13),(r10,A3,b14),(r10,A3,b15),(r10,A3,b16),(r10, A3,b17),(r10,A3,b18),(r10,A3,b19),(r10,A3,b20),(r10,A3,b21),(r10,A3,b22),(r10,A3,b23),(r10,A3,b 24),(r10,A3,b25),(r10,A3,b26),(r10,A3,b27),(r10,A3,b28),(r10,A3,b29),(r10,A3,b30),(r10,A3,b31),(r 10,A3,b32),(r10,A3,b33),(r10,A3,b34),(r10,A3,b35),(r10,A3,b36),(r10,A3,b37),(r10,A3,b38),(r10,A 3,b39),(r10,A3,b40),(r10,A3,b41),(r10,A3,b42),(r10,A3,b43),(r10,A3,b44),(r10,A3,b45),(r10,A3,b46 ),(r10,A3,b47),(r10,A3,b48),(r10,A3,b49),(r10,A3,b50),(r10,A3,b51),(r10,A3,b52),(r10,A3,b53),(r10 ,A3,b54),(r10,A3,b55),(r10,A3,b56),(r10,A3,b57),(r10,A3,b58),(r10,A3,b59),(r10,A3,b60),(r10,A3,b 61),(r10,A3,b62),(r10,A3,b63),(r10,A3,b64),(r10,A3,b65),(r10,A3,b66),(r10,A3,b67),(r10,A3,b68),(r 10,A3,b69),(r10,A3,b70),(r10,A3,b71),(r10,A3,b72),(r10,A3,b73),(r10,A3,b74),(r10,A3,b75),(r10,A 3,b76),(r10,A3,b77),(r10,A3,b78),(r10,A3,b79),(r10,A3,b80),(r10,A3,b81),(r10,A3,b82),(r10,A3,b83 ),(r10,A3,b84),(r10,A3,b85),(r10,A3,b86),(r10,A3,b87),(r10,A3,b88),(r10,A3,b89),(r10,A3,b90),(r10 ,A3,b91),(r10,A3,b92),(r10,A3,b93),(r10,A3,b94),(r10,A3,b95),(r10,A*3,b96),(r10,A3,b97),(r10,A3,b 98),(r10,A3,b99),(r10,A3,b100),(r10,A3,b101),(r10,A3,b102),(r10,A3,b103),(r10,A3,b104),(r10,A3, b105),(r10,A3,b106),(r10,A3,b107),(r10,A3,b208),(r10,A3,b109),(r10,A3,b110),(r10,A3,b111),(r10, A3,b112),(r10,A3,b113),(r10,A3,b114),(r10,A3,b115),(r10,A3,b116),(r10,A3,b117),(r10,A3,b118),(r 10,A3,b119),(r10,A3,b120),(r10,A3,b121),(r10,A3,b122),(r10,A3,b123),(r10,A3,b124),(r10,A3,b125 ),(r10,A3,b126),(r10,A3,b127),(r10,A3,b128),(r10,A3,b129),(r10,A3,b130),(r10,A3,b131),(r10,A3,b 132),(r10,A3,b133),(r10,A3,b134),(r10,A4,b1),(r10,A4,b2),(r10,A4,b3),(r10,A4,b4),(r10,A4,b5),(r10 ,A4,b6),(r10,A4,b7),(r10,A4,b8),(r10,A4,b9),(r10,A4,b10),(r10,A4,b11),(r10,A4,b12),(r10,A4,b13),( r10,A4,b14),(r10,A4,b15),(r10,A4,b16),(r10,A4,b17),(r10,A4,b18),(r10,A4,b19),(r10,A4,b20),(r10, A4,b21),(r10,A4,b22),(r10,A4,b23),(r10,A4,b24),(r10,A4,b25),(r10,A4,b26),(r10,A4,b27),(r10,A4,b 28),(r10,A4,b29),(r10,A4,b30),(r10,A4,b31),(r10,A4,b32),(r10,A4,b33),(r10,A4,b34),(r10,A4,b35),(r 10,A4,b36),(r10,A4,b37),(r10,A4,b38),(r10,A4,b39),(r10,A4,b40),(r10,A4,b41),(r10,A4,b42),(r10,A 4,b43),(r10,A4,b44),(r10,A4,b45),(r10,A4,b46),(r10,A4,b47),(r10,A4,b48),(r10,A4,b49),(r10,A4,b50 ),(r10,A4,b51),(r10,A4,b52),(r10,A4,b53),(r10,A4,b54),(r10,A4,b55),(r10,A4,b56),(r10,A4,b57),(r10 ,A4,b58),(r10,A4,b59),(r10,A4,b60),(r10,A4,b61),(r10,A4,b62),(r10,A4,b63),(r10,A4,b64),(r10,A4,b 65),(r10,A4,b66),(r10,A4,b67),(r10,A4,b68),(r10,A4,b69),(r10,A4,b70),(r10,A4,b71),(r10,A4,b72),(r 10,A4,b73),(r10,A4,b74),(r10,A4,b75),(r10,A4,b76),(r10,A4,b77),(r10,A4,b78),(r10,A4,b79),(r10,A 4,b80),(r10,A4,b81),(r10,A4,b82),(r10,A4,b83),(r10,A4,b84),(r10,A4,b85),(r10,A4,b86),(r10,A4,b87 ),(r10,A4,b88),(r10,A4,b89),(r10,A4,b90),(r10,A4,b91),(r10,A4,b92),(r10,A4,b93),(r10,A4,b94),(r10 ,A4,b95),(r10,A4,b96),(r10,A4,b97),(r10,A4,b98),(r10,A4,b99),(r10,A4,b100),(r10,A4,b101),(r10,A 4,b102),(r10,A4,b103),(r10,A4,b104),(r10,A4,b105),(r10,A4,b106),(r10,A4,b107),(r10,A4,b108),(r1 0,A4,b109),(r10,A4,b110),(r10,A4,b111),(r10,A4,b112),(r10,A4,b113),(r10,A4,b114),(r10,A4,b115),( r10,A4,b116),(r10,A4,b117),(r10,A4,b118),(r10,A4,b119),(r10,A4,b120),(r10,A4,b121),(r10,A4,b12 2),(r10,A4,b123),(r10,A4,b124),(r10,A4,b125),(r10,A4,b126),(r10,A4,b127),(r10,A4,b128),(r10,A4, b129),(r10,A4,b130),(r10,A4,b131),(r10,A4,b132),(r10,A4,b133),(r10,A4,b134),

(r11,A1,b1),(r11,A1,b2),(r11,A1,b3),(r11,A1,b4),(r11,A1,b5),(r11,A1,b6),(r11,A1,b7),(r11,A1,b8),(r1 1,A1,b9),(r11,A1,b10),(r11,A1,b11),(r11,A1,b12),(r11,A1,b13),(r11,A1,b14),(r11,A1,b15),(r11,A1,b1 6),(r11,A1,b17),(r11,A1,b18),(r11,A1,b19),(r11,A1,b20),(r11,A1,b21),(r11,A1,b22),(r11,A1,b23),(r1 1,A1,b24),(r11,A1,b25),(r11,A1,b26),(r11,A1,b27),(r11,A1,b28),(r11,A1,b29),(r11,A1,b30),(r11,A1, b31),(r11,A1,b32),(r11,A1,b33),(r11,A1,b34),(r11,A1,b35),(r11,A1,b36),(r11,A1,b37),(r11,A1,b38),( r11,A1,b39),(r11,A1,b40),(r11,A1,b41),(r11,A1,b42),(r11,A1,b43),(r11,A1,b44),(r11,A1,b45),(r11,A 1,b46),(r11,A1,b47),(r11,A1,b48),(r11,A1,b49),(r11,A1,b50),(r11,A1,b51),(r11,A1,b52),(r11,A1,b53) ,(r11,A1l,b54),(r11,A1,b55),(r11,A1,b56),(r11,A1,b57),(r11,A1,b58),(r11,A1,b59),(r11,A1,b60),(r11, A1,b61),(r11,A1,b62),(r11,A1,b63),(r11,A1,b64),(r11,A1,b65),(r11,Al,b66),(r11,A1,b67),(r11,A1,b6 8),(r11,A1,b69),(r11,A1,b70),(r11,A1,b71),(r11,A1,b72),(r11,A1,b73),(r11,A1,b74),(r11,A1,b75),(r 1,A1,b76),(r11,A1,b77),(r11,A1,b78),(r11,A1,b79),(r11,A1,b80),(r11,A1,b81),(r11,A1,b82),(r11,A1, b83),(r11,A1,b84),(r11,A1,b85),(r11,A1,b86),(r11,A1,b87),(r11,A1,b88),(r11,A1,b89),(r11,A1,b90),( r11,A1,b91),(r11,A1,b92),(r11,A1,b93),(r11,A1,b94),(r11,A1,b95),(r11,A1,b96),(r11,A1,b97),(r11,A 1,b98),(r11,A1,b99),(r11,A1,b100),(r11,A1,b101),(r11,A1,b102),(r11,A1,b103),(r11,A1,b104),(r11,A 1,b105),(r11,A1,b106),(r11,A1,b107),(r11,A1,b108),(r11,A1,b109),(r11,A1,b110),(r11,A1,b111),(r11 ,A1,b112),(r11,A1,b113),(r11,A1,b114),(r11,A1,b115),(r11,A1,b116),(r11,A1,b117),(r11,A1,b118),(r 11,A1,b119),(r11,A1,b120),(r11,A1,b121),(r11,A1,b122),(r11,A1,b123),(r11,A1,b124),(r11,A1,b125) ,(r11,A1,b126),(r11,A1,b127),(r11,A1,b128),(r11,A1,b129),(r11,A1,b130),(r11,A1,b131),(r11,A1,b1 32),(r11,A1,b133),(r11,A1,b134),(r11,A2,b1),(r11,A2,b2),(r11,A2,b3),(r11,A2,b4),(r11,A2,b5),(r11,A 2,b6),(r11,A2,b7),(r11,A2,b8),(r11,A2,b9),(r11,A2,b10),(r11,A2,b11),(r11,A2,b12),(r11,A2,b13),(r11 ,A2,b14),(r11,A2,b15),(r11,A2,b16),(r11,A2,b17),(r11,A2,b18),(r11,A2,b19),(r11,A2,b20),(r11,A2,b 21),(r11,A2,b22),(r11,A2,b23),(r11,A2,b24),(r11,A2,b25),(r11,A2,b26),(r11,A2,b27),(r11,A2,b28),(r 11,A2,b29),(r11,A2,b30),(r11,A2,b31),(r11,A2,b32),(r11,A2,b33),(r11,A2,b34),(r11,A2,b35),(r11,A2, b36),(r11,A2,b37),(r11,A2,b38),(r11,A2,b39),(r11,A2,b40),(r11,A2,b41),(r11,A2,b42),(r11,A2,b43),( r11,A2,b44),(r11,A2,b45),(r11,A2,b46),(r11,A2,b47),(r11,A2,b48),(r11,A2,b49),(r11,A2,b50),(r11,A 2,b51),(r11,A2,b52),(r11,A2,b53),(r11,A2,b54),(r11,A2,b55),(r11,A2,b56),(r11,A2,b57),(r11,A2,b58) ,(r11,A2,b,59),(r11,A2,b60),(r11,A2,b61),(r11,A2,b62),(r11,A2,b611),(r11,A2,b64),(r11,A2,b65),(r11, A2,b66),(r11,A2,b67),(r11,A2,b68),(r11,A2,b69),(r11,A2,b70),(r11,A2,b71),(r11,A2,b72),(r11,A2,b7 3),(r11,A2,b74),(r11,A2,b75),(r11,A2,b76),(r11,A2,b77),(r11,A2,b78),(r11,A2,b79),(r11,A2,b80),(r1 1,A2,b81),(r11,A2,b82),(r11,A2,b83),(r11,A2,b84),(r11,A2,b85),(r11,A2,b86),(r11,A2,b87),(r11,A2, b88),(r11,A2,b89),(r11,A2,b90),(r11,A2,b91),(r11,A2,b92),(r11,A2,b93),(r11,A2,b94),(r11,A2,b95),( r11,A2,b96),(r11,A2,b97),(r11,A2,b98),(r11,A2,b99),(r11,A2,b100),(r11,A2,b101),(r11,A2,b102),(r1 1,A2,b103),(r11,A2,b104),(rl1,A2,b105),(r11,A2,b106),(r11,A2,b107),(r11,A2,b108),(r11,A2,b109), (r11,A2,b110),(r11,A2,b111),(r11,A2,b112),(r11,A2,b113),(r11,A2,b114),(r11,A2,b115),(r11,A2,bl16 ),(r11,A2,b117),(r11,A2,b118),(r11,A2,b11.9),(r11,A2,b120),(r11,A2,b121),(r11,A2,b122),(r11,A2,b1 23),(r11,A2,b124),(r11,A2,b125),(r11,A2,b126),(r11,A2,b127),(r11,A2,b128),(r11,A2,b129),(r11,A2, b130),(r11,A2,b131),(r11,A2,b132),(r11,A2,b133),(r11,A2,b134),(r11,A3,b1),(r11,A3,b2),(r11,A3,b3 ),(r11,A3,b4),(r11,A3,b5),(r11,A3,b6),(r11,A3,b7),(r11,A3,b8),(r11,A3,b9),(r11,A3,b10),(r11,A3,b11) ,(r11,A3,b12),(r11,A3,b13),(r11,A3,b14),(r11,A3,b15),(r11,A3,b16),(r11,A3,b17),(r11,A3,b18),(r11, A3,b19),(r11,A3,b20),(r11,A3,b21),(r11,A3,b22),(r11,A3,b23),(r11,A3,b24),(r11,A3,b25),(r11,A3,b2 6),(r11,A3,b27),(r11,A3,b28),(r11,A3,b29),(r11,A3,b30),(r11,A3,b31),(r11,A3,b32),(r11,A3,b33),(r1 1,A3,b34),(r11,A3,b35),(r11,A3,b36),(r11,A3,b37),(r11,A3,b38),(r11,A3,b39),(r11,A3,b40),(r11,A3, b41),(r11,A3,b42),(r11,A3,b43),(r11,A3,b44),(r11,A3,b45),(r11,A3,b46),(r11,A3,b47),(r11,A3,b48),( r11,A3,b49),(r11,A3,b50),(r11,A3,b51),(r11,A3,b52),(r11,A3,b53),(r11,A3,b54),(r11,A3,b55),(r11,A 3,b56),(r11,A3,b57),(r11,A3,b58),(r11,A3,b59),(r11,A3,b60),(r11,A3,b61),(r11,A3,b62),(r11,A3,b63) ,(r11,A3,b64),(r11,A3,b65),(r11,A3,b66),(r11,A3,b67),(r11,A3,b68),(r11,A3,b69),(r11,A3,b70),(r11, A3,b71),(r11,A3,b72),(r11,A3,b73),(r11,A3,b74),(r11,A3,b75),(r11,A3,b76),(r11,A3,b77),(r11,A3,b7 8),(r11,A3,b79),(r11,A3,b80),(r11,A3,b81),(r11,A3,b82),(r11,A3,b83),(r11,A3,b84),(r11,A3,b85),(r1 1,A3,b86),(r11,A3,b87),(r11,A3,b88),(r11,A3,b89),(r11,A3,b90),(r11,A3,b91),(r11,A3,b92),(r11,A3, b93),(r11,A3,b94),(r11,A3,b95),(r11,A3,b96),(r11,A3,b97),(r11,A3,b98),(r11,A3,b99),(r11,A3,b100), (r11,A3,b101),(r11,A3,b102),(r11,A3,b103),(r11,A3,b104),(r11,A3,b105),(r11,A3,b106),(r11,A3,b10 7),(r11,A3,b108),(r11,A3,b109),(r11,A3,b110),(r11,A3,b111),(r11,A3,b112),(r11,A3,b113),(r11,A3,b 114),(r11,A3,b115),(r11,A3,b116),(r11,A3,b117),(r11,A3,b118),(r11,A3,b119),(r11,A3,b120),(r11,A3 ,b121),(r11,A11,b122),(r11,A3,b123),(r11,A3,b124),(r11,A3,b125),(r11,A3,b126),(r11,A3,b127),(r11, A3,b128),(r11,A3,b129),(r11,A3,b130),(r11,A3,b131),(r11,A3,b132),(r11,A3,b133),(r11,A3,b134),(r 11,A4,b1),(r11,A4,b2),(r11,A4,b3),(r11,A4,b4),(r11,A4,b5),(r11,A4,b6),(r11,A4,b7),(r11,A4,b8),(r11, A4,b9),(r11,A4,b10),(r11,A4,b11),(r11,A4,b12),(r11,A4,b13),(r11,A4,b1_4),(r11,A4,b15),(r11,A4,b16 ),(r11,A4,b17),(r11,A4,b18),(r11,A4,b19),(r11,A4,b20),(r11,A4,b21),(r11,A4,b22),(r11,A4,b23),(r11, A4,b24),(r11,A4,b25),(r11,A4,b26),(r11,A4,b27),(r11,A4,b28),(r11,A4,b29),(r11,A4,b30),(r11,A4,b3 1),(r11,A4,b32),(r11,A4,b33),(r11,A4,b34),(r11,A4,b35),(r11,A4,b36),(r11,A4,b37),(r11,A4,b38),(r1 1,A4,b39),(r11,A4,b40),(r11,A4,b41),(r11,A4,b42),(r11,A4,b43),(r11,A4,b44),(r11,A4,b45),(r11,A4, b46),(r11,A4,b47),(r11,A4,b48),(r11,A4,b49),(r11,A4,b50),(r11,A4,b51),(r11,A4,b52),(r11,A4,b53),( r11,A4,b54),(r11,A4,b55),(r11,A4,b56),(r11,A4,b57),(r11,A4,b58),(r11,A4,b59),(r11,A4,b60),(r11,A 4,b61),(r11,A4,b62),(r11,A4,b63),(r11,A4,b64),(r11,A4,b65),(r11,A4,b66),(r11,A4,b67),(r11,A4,b68) ,(r11,A4,b69),(r11,A4,b70),(r11,A4,b71),(r11,A4,b72),(r11,A4,b73),(r11,A4,b74),(r11,A4,b75),(r11, A4,b76),(r11,A4,b77),(r11,A4,b78),(r11,A4,b79),(r11,A4,b80),(r11,A4,b81),(r11,A4,b82),(r11,A4,b8 3),(r11,A4,b84),(r11,A4,b85),(r11,A4,b86),(r11,A4,b87),(r11,A4,b88),(r11,A4,b89),(r11,A4,b90),(r1 1,A4,b91),(r11,A4,b92),(r11,A4,b93),(r11,A4,b94),(r11,A4,b95),(r11,A4,b96),(r11,A4,b97),(r11,A4, b98),(r11,A4,b99),(r11,A4,b100),(r11,A4,b101),(r11,A4,b 02),(r11,A4,b103),(r11,A4,b104),(r11,A4, b105),(r11,A4,b106),(r11,A4,b107),(r11,A4,b108),(r11,A4,b109),(r11,A4,b110),(r11,A4,b111),(r11,A 4,b112),(r11,A4,b113),(r11,A4,b114),(r11,A4,b115),(r11,A4,b116),(r11,A4,b117),(r11,A4,b118),(r11, A4,b119),(r11,A4,b120),(r11,A4,b121),(r11,A4,b122),(r11,A4,b123),(r11,A4,b124),(r11,A4,b125),(r 11,A4,b126),(r11,A4,b127),(r11,A4,b128),(r11,A4,b129),(r11,A4,b130),(r11,A4,b131),(r11,A4,b132) ,(r11,A4,b133),(r11,A4,b134),

(r12,A1,b1),(r12,A1,b2),(r12,A1,b3),(r12,A1,b4),(r12,A1,b5),(r12,A1,b6),(r12,A1,b7),(r12,A1,b8),(r 12,A1,b9),(r12,A1,b10),(r12,A1,b11),(r12,A1,b12),(r12,A1,b13),(r12,A1,b14),(r12,A1,b15),(r12,A1, b16),(r12,A1,b17),(r12,A1,b18),(r12,A1,b19),(r1,A1,b20),(r12,A1,b21),(r12,A1,b22),(r12,A1,b23), (r12,A1,b24),(r12,A1,b25),(r12,A1,b26),(r12,A1,b27),(r12,A1,b28),(r12,A1,b29),(r12,A1,b30),(r12, A1,b31),(r12,A1,b32),(r12,A1,b33),(r12,A1,b34),(r12,A1,b35),(r12,A1,b36),(r12,A1,b37),(r12,A1,b 38),(r12,A1,b39),(r12,A1,b40),(r12,A1,b41),(r12,A1,b42),(r12,A1,b43),(r12,A1,b44),(r12,A1,b45),(r 12,A1,b46),(r12,A1,b47),(r12,A1,b48),(r12,A1,b49),(r12,A1,b50),(r12,A1,b51),(r12,A1,b52),(r12,A 1,b53),(r12,A1,b54),(r12,A1,b55),(r12,A1,b56),(r12,A1,b57),(r12,A1,b58),(r12,A1,b59),(r12,A1,b60 ),(r12,A1,b61),(r12,A1,b62),(r12,A1,b63),(r12,A1,b64),(r12,A1,b65),(r12,A1,b66),(r12,A1,b67),(r12 ,A1,b68),(r12,A1,b69),(r12,A1,b70),(r12,A1,b71),(r12,A1,b72),(r12,A1,b73),(r12,A1,b74),(r12,A1,b 75),(r12,A1,b76),(r12,A1,b77),(r12,A1,b78),(r12,A1,b79),(r12,A1,b80),(r12,A1,b81),(r12,A1,b82),(r 12,A1,b83),(r12,A1,b84),(r12,A1,b85),(r12,A1,b86),(r12,A1,b87),(r12,A1,b88),(r12,A1,b89),(r12,A 1,b90),(r12,A1,b91),(r12,A1,b92),(r12,A1,b93),(r12,A1,b94),(r12,A1,b95),(r12,A1,b96),(r12,A1,b97 ),(r12,A1,b98),(r12,A1,b99),(r12,A1,b100)(r12,A1,b101),(r12,A1,b102),(r12,A1,b103),(r12,A1,b10 4),(r12,A1,b105),(r12,A1,b106),(r12,A1,b107),(r12,A1,b108),(r12,A1,b109),(r12,A1,b110),(r12,A1, b111),(r12,A1,b112),(r12,A1,b113),(r12,A1,b114),(r12,A1,b115),(r12,A1,b116),(r12,A1,b117),(r12, A1,b118),(r12,A1,b119),(r12,A1,b120),(r12,A1,b121),(r12,A1,b122),(r12,A1,b123),(r12,A1,b124),(r 12,A1,b125),(r12,A1,b126),(r12,A1,b127),(r12,A1,b128),(r12,A1,b129),(r12,A1,b130),(r12,A1,b131 ),(r12,A1,b132),(r12,A1,b133),(r12,A1,b134),(r12,A2,b1),(r12,A2,b2),(r12,A2,b3),(r12,A2,b4),(r12, A2,b5),(r12,A2,b6),(r12,A2,b7),(r12,A2,b8),(r12,A2,b9),(r12,A2,b10),(r12,A2,b11),(r12,A2,b12),(r1 2,A2,b13),(r12,A2,b14),(r12,A2,b15),(r12,A2,b16),(r12,A2,b17),(r12,A2,b18),(r12,A2,b19),(r12,A2, b20),(r12,A2,b21),(r12,A2,b22),(r12,A2,b23),(r12,A2,b24),(r12,A2,b25),(r12,A2,b26),(r12,A2,b27), (r12,A2,b28),(r12,A2,b29),(r12,A2,b30),(r12,A2,b31),(r12,A2,b32),(r12,A2,b33),(r12,A2,b34),(r12, A2,b35),(r12,A2,b36),(r12,A2,b37),(r12,A2,b38),(r12,A2,b39),(r12,A2,b40),(r12,A2,b41),(r12,A2,b 42),(r12,A2,b43),(r12,A2,b44),(r12,A2,b45),(r12,A2,b46),(r12,A2,b47),(r12,A2,b48),(r12,A2,b49),(r 12,A2,b50),(r12,A2,b51),(r12,A2,b52),(r12,A2,b53),(r12,A2,b54),(r12,A2,b55),(r12,A2,b56),(r12,A 2,b57),(r12,A2,b58),(r12,A2,b59),(r12,A2,b60),(r12,A2,b61),(r12,A2,b62),(r12,A2,b63),(r12,A2,b64 ),(r12,A2,b65),(r12,A2,b66),(r12,A2,b67),(r12,A2,b68),(r12,A2,b69),(r12,A2,b70),(r12,A2,b71),(r12 ,A2,b72),(r12,A2,b73),(r12,A2,b74),(r12,A2,b75),(r12,A2,b76),(r12,A2,b77),(r12,A2,b78),(r12,A2,b 79),(r12,A2,b80),(r12,A2,b81),(r12,A2,b82),(r12,A2,b83),(r12,A2,b84),(r12,A2,b85),(r12,A2,b86),(r 12,A2,b87),(r12,A2,b88),(r12,A2,b89),(r12,A2,b90),(r12,A2,b91),(r12,A2,b92),(r12,A2,b93),(r12,A 2,b94),(r12,A2,b95),(r12,A2,b96),(r12,A2,b97),(r12,A2,b98),(r12,A2,b99),(r12,A2,b100),(r12,A2,b1 01),(r12,A2,b102),(r12,A2,b103),(r12,A2,b104),(r12,A2,b105),(r12,A2,b106),(r12,A2,b107),(r12,A2 ,b108),(r12,A2,b109),(r12,A2,b110),(r12,A2,b111),(r12,A2,b112),(r12,A2,b113),(r12,A2,b114),(r12, A2,b115),(r12,A2,b116),(r12,A2,b117),(r12,A2,b118),(r12,A2,b119),(r12,A2,b120),(r12,A2,b121),(r 12,A2,b122),(r12,A2,b123),(r12,A2,b124),(r12,A2,b125),(r12,A2,b126),(r12,A2,b127),(r12,A2,b128 ),(r12,A2,b129),(r12,A2,b130),(r12,A2,b131),(r12,A2,b132),(r12,A2,b133),(r12,A2,b134),(r12,A3,b 1),(r12,A3,b2),(r12,A3,b3),(r12,A3,b4),(r12,A3,b5),(r12,A3,b6),(r12,A3,b7),(r12,A3,b8),(r12,A3,b9) ,(r12,A3,b10),(r12,A3,b11),(r12,A3,b12),(r12,A3,b13),(r12,A3,b14),(r12,A3,b15),(r12,A3,b16),(r12, A3,b17),(r12,A3,b18),(r12,A3,b19),(r12,A3,b20),(r12,A3,b21),(r12,A3,b22),(r12,A3,b23),(r12,A3,b 24),(r12,A3,b25),(r12,A3,b26),(r12,A3,b27),(r12,A3,b28),(r12,A3,b29),(r12,A3,b30),(r12,A3,b31),(r 12,A3,b32),(r12,A3,b33),(r12,A3,b34),(r12,A3,b35),(r12,A3,b36),(r12,A3,b37),(r12,A3,b38),(r12,A 3,b39),(r12,A3,b40),(r12,A3,b41),(r12,A3,b42),(r12,A3,b43),(r12,A3,b44),(r12,A3,b45),(r12,A3,b46 ),(r12,A3,b47),(r12,A3,b48),(r12,A3,b49),(r12,A3,b50),(r12,A3,b51),(r12,A3,b52),(r12,A3,b53),(r12 ,A3,b54),(r12,A3,b55),(r12,A3,b56),(r12,A3,b57),(r12,A3,b58),(r12,A3,b59),(r12,A3,b60),(r12,A3,b 61),(r12,A3,b62),(r12,A3,b63),(r12,A3,b64),(r12,A3,b65),(r12,A3,b66),(r12,A3,b67),(r12,A3,b68),(r 12,A3,b69),(r12,A3,b70),(r12,A11,b71),(r12,A3,b72),(r12,A3,b73),(r12,A3,b74),(r12,A3,b75),(r12,A 3,b76),(r12,A3,b77),(r12,A3,b78),(r12,A3,b79),(r12,A3,b80),(r12,A3,b81),(r12,A3,b82),(r12,A3,b83 ),(r12,A3,b84),(r12,A3,b85),(r12,A3,b86),(r12,A3,b87),(r12,A3,b88),(r12,A3,b89),(r12,A3,b90),(r12 ,A3,b91),(r12,A3,b92),(r12,A3,b93),(r12,A3,b94),(r12,A3,b95),(r12,A3,b96),(r12,A3,b97),(r12,A3,b 98),(r12,A3,b99),(r12,A3,b100),(r12,A3,b101),(r12,A3,b102),(r12,A3,b103),(r12,A3,b104),(r12,A3, b105),(r12,A3,b106),(r12,A3,b107),(r12,A3,b108),(r12,A3,b109),(r12,A3,b110),(r12,A3,b111),(r12, A3,b112),(r12,A3,b113),(r12,A3,b114),(r12,A3,b115),(r12,A3,b116),(r12,A3,b117),(r12,A3,b118),(r 12,A3,b119),(r12,A3,b120),(r12,A3,b121),(r12,A3,b122),(r12,A3,b123),(r12,A3,b124),(r12,A3,b125 ),(r12,A3,b126),(r12,A3,b127),(r12,A3,b128),(r12,A3,b129),(r12,A3,b130),(r12,A3,b131),(r12,A3,b 132),(r12,A3,b133),(r12,A3,b134),(r12,A4,b1),(r12,A4,b2),(r12,A4,b3),(r12,A4,b4),(r12,A4,b5),(r12 ,A4,b6),(r12,A4,b7),(r12,A4,b8),(r12,A4,b9),(r12,A4,b10),(r12,A4,b11),(r12,A4,b12),(r12,A4,b13), ( r12,A4,b14),(r12,A4,b15),(r12,A4,b16),(r12,A4,b17),(r12,A4,b18),(r12,A4,b19),(r12,A4,b20),(r12, A4,b21),(r12,A4,b22),(r12,A4,b23),(r12,A4,b24),(r12,A4,b25),(r12,A4,b26),(r12,A4,b27),(r12,A4,b 28),(r12,A4,b29),(r12,A4,b30),(r12,A4,b31),(r12,A4,b32),(r12,A4,b33),(r12,A4,b34),(r12,A4,b35),(r 12,A4,b36),(r12,A4,b37),(r12,A4,b38),(r12,A4,b39),(r12,A4,b40),(r12,A4,b41),(r12,A4,b42),(rl2,A 4,b43),(r12,A4,b44),(r12,A4,b45),(r12,A4,b46),(r12,A4,b47),(r12,A4,b48),(r12,A4,b49),(r12,A4,b50 ),(r12,A4,b51),(r12,A4,b52),(r12,A4,b53),(r12,A4,b54),(r12,A4,b55),(r12,A4,b56),(r12,A4,b57),(r12 ,A4,b58),(r12,A4,b59),(r12,A4,b60),(r12,A4,b61),(r12,A4,b62),(r12,A4,b63),(r12,A4,b64),(r12,A4,b 65),(r12,A4,b66),(r12,A4,b67),(r12,A4,b68),(r12,A4,b69),(r12,A4,b70),(r12,A4,b71),(r12,A4,b72),(r 12,A4,b73),(r12,A4,b74),(r12,A4,b75),(r12,A4,b76),(r12,A4,b77),(r12,A4,b78),(r12,A4,b79),(r12,A 4,b80),(r12,A4,b81),(r12,A4,b82),(r12,A4,b83),(r12,A4,b84),(r12,A4,b85),(r12,A4,b86),(r12,A4,b87 ),(r12,A4,b88),(r12,A4,b89),(r12,A4,b90),(r12,A4,b91),(r12,A4,b92),(r12,A4,b93),(r12,A4,b94),(r12 ,A4,b95),(r12,A4,b96),(r12,A4,b97),(r12,A4,b98),(r12,A4,b99),(r12,A4,b100),(r12,A4,b101),(r12,A 4,b102),(r12,A4,b103),(r12,A4,b104),(r12,A4,b105),(r12,A4,b106),(r12,A4,b107),(r12,A4,b108),(r1 2,A4,b109),(r12,A4,b110),(r12,A4,b111),(r12,A4,b112),(r12,A4,b113),(r12,A4,b114),(r12,A4,b115),( r12,A4,b116),(r12,A4,b117),(r12,A4,b118),(r12,A4,b119),(r12,A4,b120),(r12,A4,b121),(r12,A4,b12 2),(r12,A4,b123),(r12,A4,b124),(r12,A4,b125),(r12,A4,b126),(r12,A4,b127),(r12,A4,b128),(r12,A4, b129),(r12,A4,b130),(r12,A4,b131),(r12,A4,b132),(r12,A4,b133),(r12,A4,b134),

(r13,A1,b1),(r13,A1,b2),(r13,A1,b3),(r13,A1,b4),(r13,A1,b5),(r13,A1,b6),(r13,A1,b7),(r13,A1,b8),(r 13,A1,b9),(r13,A1,b10),(r13,A1,b11),(r13,A1,b12),(r13,A1,b13),(r13,A1,b14),(r13,A1,b15),(r13,A1, b16),(r13,A1,b17),(r13,A1,b18),(r13,A1,b19),(r13,A1,b20),(r13,A1,b21),(r13,A1,b22),(r13,A1,b23), (r13,A1,b24),(r13,A1,b25),(r13,A1,b26),(r13,A1,b27),(r13,A1,b28),(r13,A1,b29),(r13,A1,b30),(r13, A1,b31),(r13,A1,b32),(r13,A1,b33),(r13,A1,b34),(r13,A1,b35),(r13,A1,b36),(r13,A1,b37),(r13,A1,b 38),(r13,A1,b39),(r13,A1,b40),(r3,A1,b41),(r13,A1,b42),(r13,A1,b43),(r13,A1,b44),(r13,A1,b45),(r 13,A1,b46),(r13,A1,b47),(r13,A1,b48),(r13,A1,b49),(r13,A1,b50),(r13,A1,b51),(r13,A1,b52),(r13,A 1,b53),(r13,A1,b54),(r13,A1,b55),(r13,A1,b56),(r13,A1,b57),(r13,A1,b58),(r13,A1,b59),(r13,A1,b60 ),(r13,A1,b61),(r13,A1,b62),(r13,A1,b63),(r13,A1,b64),(r13,A1,b65),(r13,A1,b66),(r13,A1,b67),(r13 ,A1,b68),(r13,A1,b69),(r13,A1,b70),(r13,A1,b71),(r13,A1,b72),(r13,A1,b73),(r13,A1,b74),(r13,A1,b 75),(r13,A1,b76),(r13,A1,b77),(r13,A1,b78),(r13,A1,b79),(r13,A1,b80),(r13,A1,b81),(r13,A1,b82),(r 13,A1,b83),(r13,A1,b84),(r13,A1,b85),(r13,A1,b86),(r13,A1,b87),(r13,A1,b88),(r13,A1,b89),(r13,A 1,b90),(r13,A1,b91),(r13,A1,b92),(r13,A1,b93),(r13,A1,b94),(r13,A1,b95),(r13,A1,b96),(r13,A1,b97 ),(r13,A1,b98),(r13,A1,b99),(r13,A1,b100),(r13,A1,b101),(r13,A1,b102),(r13,A1,b103),(r13,A1,b10 4),(r13,A1,b105),(r13,A1,b106),(r13,A1,b107),(r13,A1,b108),(r13,A1,b109),(r13,A1,bl10),(r13,A1, b111),(r13,A1,b112),(r13,A1,b113),(r13,A1,b114),(r13,A1,b115),(r13,A1,b116),(r13,A1,b117),(r13, A1,b118),(r13,A1,b119),(r13,A1,b120),(r13,A1,b121),(r13,A1,b122),(r13,A1,b123),(r13,A1,b124),(r 13,A1,b125),(r13,A1,b126),(r13,A1,b127),(r13,A1,b128),(r13,A1,b129),(r13,A1,b130),(r13,A1,b131 ),(r13,A1,b132),(r13,A1,b133),(r13,A1,b134),(r13,A2,b 1),(r13,A2,b2),(r13,A2,b3),(r13,A2,b4),(r13, A2,b5),(r13,A2,b6),(r13,A2,b7),(r13,A2,b8),(r13,A2,b9),(r13,A2,b10),(r13,A2,b11),(r13,A2,b12),(r1 3,A2,b13),(r13,A2,b14),(r13,A2,b15),(r13,A2,b16),(r13,A2,b17),(r13,A2,b18),(r13,A2,b19),(r13,A2, b20),(r13,A2,b21),(r13,A2,b22),(r13,A2,b23),(r13,A2,b24),(r13,A2,b25),(r13,A2,b26),(r13,A2,b27), (r13,A2,b28),(r13,A2,b29),(r13,A2,b30),(r13,A2,b31),(r13,A2,b32),(r13,A2,b33),(r13,A2,b34),(r13, A2,b35),(r13,A2,b36),(r13,A2,b37),(r13,A2,b38),(r13,A2,b39),(r13,A2,b40),(r13,A2,b41),(r13,A2,b 42),(r13,A2,b43),(r13,A2,b44),(r13,A2,b45),(r13,A2,b46),(r13,A2,b47),(r13,A2,b48),(r13,A2,b49),(r 13,A2,b50),(r13,A2,b51),(r13,A2,b52),(r13,A2,b53),(r13,A2,b54),(r13,A2,b55),(r13,A2,b55),(r13,A 2,b57),(r13,A2,b58),(r13,A2,b59),(r13,A2,b60),(r13,A2,b61),(r13,A2,b62),(r13,A2,b63),(r13,A2,b64 ), (r 13,A2, b65), (r 13,A2,b66), (r 13,A2, b67), (r 13,A2, b68), (r 13,A2, b69), (r 13,A2,b70), (r 13,A2, b7 1), (r 13 ,A2,b72),(r13,A2,b73),(r13,A2,b74),(r13,A2,b75),(r13,A2,b76),(r13,A2,b77),(r13,A2,b78),(r13,A2,b 79),(r13,A2,b80),(r13,A2,b81),(r13,A2,b82),(r13,A2,b83),(r13,A2,b84),(r13,A2,b85),(r13,A2,b86),(r 13,A2,b87),(r13,A2,b88),(r13,A2,b89),(r13,A2,b90),(r13,A2,b91),(r13,A2,b92),(r13,A2,b93),(rl3,A 2,b94),(r13,A2,b95),(r13,A2,b96),(r13,A2,b97),(r13,A2,b98),(r13,A2,b99),(r13,A2,b100),(r13,A2,bl 1 01),(r13,A2,b102),(r13,A2,b103),(r13,A2,b104),(r13,A2,b105),(r13,A2,b106),(r13,A2,b107),(r13,A2 ,b108),(r13,A2,b109),(r13,A2,b110),(r13,A2,b11),(r13,A2,b112),(r13,A2,b113),(r13,A2,b114),(r13, A2,b115),(r13,A2,b116),(r13,A2,b117),(r13,A2,b118),(r13,A2,b119),(r13,A2,b120),(r13,A2,b121),(r 13,A2,b122),(r13,A2,b123),(r13,A2,b124),(r13,A2,b125),(r13,A2,b126),(r13,A2,b12'l),(r13,A2,b128 ),(r13,A2,b129),(r13,A2,b130),(r13,A2,b131),(r13,A2,b132),(r13,A2,b133),(r13,A2,b134),(r13,A3,b 1),(r13,A3,b2),(r13,A3,b3),(r13,A3,b4),(r13,A3,b5),(r13,A3,b6),(r13,A3,b7),(r13,A3,b8),(r13,A3,b9) ,(r13,A3,b10),(r13,A3,b11),(r13,A3,b12),(r13,A3,b13),(r13,A3,b14),(r13,A3,b15),(r13,A3,b16),(r13, A3,b17),(r13,A3,b18),(r13,A3,b19),(r13,A3,b20),(r13,A3,b21),(r13,A3,b22),(r13,A3,b23),(r13,A3,b 24),(r13,A3,b25),(r13,A3,b26),(r13,A3,b27),(r13,A3,b28),(r13,A3,b29),(r13,A3,b30),(r13,A3,b31),(r 13,A3,b32),(r13,A3,b33),(r13,A3,b34),(r13,A3,b35),(r13,A3,b36),(r13,A3,b37),(r13,A3,b38),(r13,A 3,b39),(r13,A3,b40),(r13,A3,b41),(r13,A3,b42),(r13,A3,b43),(r13,A3,b44),(r13,A3,b41),(r13,A3,b46 ),(r13,A3,b47),(r13,A3,b48),(r13,A3,b49),(r13,A3,b50),(r13,A3,b5l),(r13,A3,b52),(r13,A3,b53),(r13 ,A3,b54),(r13,A3,b55),(r13,A3,b56),(r13,A3,b57),(r13,A3,b58),(r13,A3,b59),(r13,A3,b60),(r13,A3,b 61),(r13,A3,b62),(r13,A3,b63),(r13,A3,b64),(r13,A3,b65),(r13,A3,b66),(r13,A3,b67),(r13,A3,b68),(r 13,A3,b69),(r13,A3,b70),(r13,A3,b71),(r13,A3,b72),(r13,A3,b73),(r13,A3,b74),(r13,A3,b75),(r13,A 3,b76),(r13,A3,b177),(r13,A3,b78),(r13,A3,b79),(r13,A3,b80),(r13,A3,b81),(r13,A3,b82),(r13,A3,b83 ),(r13,A3,b84),(r13,A3,b85),(r13,A3,b86),(r13,A3,b87),(r13,A3,b88),(r13,A3,b89),(r13,A3,b90),(r13 ,A3,b91),(r13,A3,b92),(r13,A3,b93),(r1,3,A3,b94),(r13,A3,b95),(r13,A3,b96),(r13,A3,b97),(r13,A3,b 98),(r13,A3,b99),(r13,A3,b100),(r13,A3,b101),(r13,A3,b102),(r13,A3,b103),(r13,A3,b104),(r13,A3, b105),(r13,A3,b106),(r13,A3,b107),(r13,A3,b108),(r13,A3,b109),(r13,A3,b110),(r13,A3,b111),(r13, A3,b112),(r13,A3,b113),(r13,A3,b114),(r13,A3,b115),(r13,A3,b116),(r13,A3,b117),(r13,A3,b118),(r 13,A3,b119),(r13,A3,b120),(r13,A3,b121),(r13,A3,b122),(r13,A3,b123),(r13,A3,b124),(r13,A3,b125 ),(r13,A3,b126),(r13,A3,b127),(r13,A3,b128),(r13,A3,b129),(r13,A3,b130),(r13,A3,b131),(r13,A3,b 132),(r13,A3,b133),(r13,A3,b134),(r13,A4,b1),(r13,A4,b2),(r13,A4,b3),(r13,A4,b4),(r13,A4,b5),(r13 ,A4,b6),(r13,A4,b7),(r13,A4,b8),(r13,A4,b9),(r13,A4,b10),(r13,A4,b11),(r13,A4,b12),(r13,A4,b13),( r13,A4,b14),(r13,A4,b15),(r13,A4,b16),(r13,A4,b17),(r13,A4,b18),(r13,A4,b19),(r13,A4,b20),(r13, A4,b21),(r13,A4,b22),(r13,A4,b23),(r13,A4,b24),(r13,A4,b25),(r13,A4,b26),(r13,A4,b27),(r13,A4,b 28),(r13,A4,b29),(r13.A4,b30),(r13,A4,b31),(r13,A4,b32),(r13,A4,b33),(r13,A4,b34),(r13,A4,b35),(r 13,A4,b36),(r13,A4,b37),(r13,A4,b38),(r13,A4,b39),(r13,A4,b40),(r13,A4,b41),(r13,A4,b42),(r13,A 4,b43),(r13,A4,b44),(r13,A4,b45),(r13,A4,b46),(r13,A4,b47),(r3,A4,b48),(r13,A4,b49),(r13,A4,b50 ),(r13,A4,b51),(r13,A4,b52),(r13,A4,b53),(r13,A4,b54),(r13,A4,b55),(r13,A4,b56),(r13,A4,b57),(r13 ,A4,b58),(r13,A4,b59),(r13,A4,b60),(r13,A4,b61),(r13,A4,b62),(r13,A4,b63),(r13,A4,b64),(r13,A4,b 65),(r13,A4,b66),(r13,A4,b67),(r13,A4,b68),(r13,A4,b69),(r13,A4,b70),(r13,A4,b71),(r13,A4,b72),(r 13,A4,b73),(r13,A4,b74),(r13,A4,b75),(r13,A4,b76),(r13,A4,b77),(r13,A4,b78),(r13,A4,b79),(rl3,A 4,b80),(r13,A4,b81),(r13,A4,b82),(r13,A4,b83),(r13,A4,b84),(r13,A4,b85),(r13,A4,b86),(r13,A4,b87 ),(r13,A4,b88),(r13,A4,b89),(r13,A4,b90),(r13,A4,b91),(r13,A4,b92),(r13,A4,b93),(r13,A4,b94),(r13 ,A4,b95),(r13,A4,b96),(r13,A4,b97),(r13,A4,b98),(r13,A4,b99),(r13,A4,b100),(r13,A4,b101),(r13,A 4,b102),(r13,A4,b103),(r13,A4,b104),(r13,A4,b105),(r13,A4,b106),(r13,A4,b107),(r13,A4,b108),(r1 3,A4,b109),(r13,A4,b110),(r13,A4,b111),(r13,A4,b112),(r13,A4,b113),(r13,A4,b114),(r13,A4,b115),( r13,A4,b116),(r13,A4,b117),(r13,A4,b118),(r13,A4,b119),(r13,A4,b120),(r13,A4,b121),(r13,A4,b12 2),(r13,A4,b123),(r13,A4,b124),(r13,A4,b125),(r13,A4,b126),(r13,A4,b127),(r13,A4,b128),(r13,A4, b129),(r13,A4,b130),(r13,A4,b131),(r13,A4,b132),(r13,A4,b133),(r13,A4,b134),

(r14,A1,b1),(r14,A1,b2),(r14,A1,b3),(r14,A1,b4),(r14,A1,b5),(r14,A1,b6),(r14,A1,b7),(r14,A1,b8),(r 14,A1,b9),(r14,A1,b10),(r14,A1,b11),(r14,A1b12),(r4,A1,b13),(r14,A1,b14),(r14,A1,b15),(r14,A1, b16),(r14,A1,b17),(r14,A1,b18),(r14,A1,b19),(r14,A1,b20),(r14,A1,b21),(r14,A1,b22),(r14,A1,b23), (r14,A1,b24),(r14,A1,b25),(r14,A1,b26),(r14,A1,b27),(r14,A1,b28),(r14,A1,b29),(r14,A1,b30),(r14, A1,b31),(r14,A1,b32),(r14,A1,b33),(r14,A1,b34),(r14,A1,b35),(r14,A1,b36),(r14,A1,b37),(r14,A1,b 38),(r14,A1,b39),(r14,A1,b40),(r14,A1,b41),(r14,A1,b42),(r14,A1,b43),(r14,A1,b44),(r14,A1,b45),(r 14,A1,b46),(r14,A1,b47),(r14,A1,b48),(r14,A1,b49),(r14,A1,b50),(r14,A1,b51),(r14,A1,b52),(r14,A 1,b53),(r14,A1,b54),(r14,A1,b55),(r14,A1,b56),(r14,A1,b57),(r14,A1,b58),(r14,A1,b59),(r14,A1,b60 ),(r14,A1,b61),(r14,A1,b62),(r14,A1,b63),(r14,A1,b64),(r14,A1,b65),(r14,A1,b66),(r14,A1,b67),(r14 ,A1,b68),(r14,A1,b69),(r14,A1,b70),(r14,A1,b71),(r14,A1,b72),(r14,A1,b73),(r14,A1,b74),(r14,A1,b 75),(r14,A1,b76),(r14,A1,b77),(r14,A1,b78),(r14,A1,b79),(r14,A1,b80),(r14,A1,b81),(r14,A1,b82),(r 14,A1,b83),(r14,A1,b84),(r14,A1,b85),(r14,A1,b86),(r14,A1,b87),(r14,A1,b88),(r14,A1,b89),(r14,A 1,b90),(r14,A1,b91),(r14,A1,b92),(r14,A1,b93),(r14,A1,b94),(r14,A1,b95),(r14,A1,b96),(r14,A1,b97 ),(r14,A1,b98),(r14,A1,b99),(r14,A1,b100),(r14,A1,b101),(r14,A1,b102),(r14,A1,b103),(r14,A1,b10 4),(r14,A1,b105),(r14,A1,b106),(r14,A1,b107),(r14,A1,b108),(r14,A1,b109),(r14,A1,b110),(r14,A1, b111),(r14,A1,b112),(r14,A1,b113),(r14,A1,b114),(r14,A1,b115),(r14,A1,b116),(r14,A1,b117),(r14, A1,b118),(r14,A1,b119),(r14,A1,b120),(r14,A1,b121),(r14,A1,b122),(r14,A1,b123),(r14,A1,b124),(r 14,A1,b125),(r14,A1,b126),(r14,A1,b127),(r14,A1,b128),(r14,A1,b129),(r14,A1,b130),(r14,A1,b131 ),(r14,A1,b132),(r14,A1,b133),(r14,A1,b134),(r14,A2,b1),(r14,A2,b2),(r14,A2,b3),(r14,A2,b4),(r14, A2,b5),(r14,A2,b6),(r14,A2,b7),(r14,A2,b8),(r14,A2,b9),(r14,A2,b10),(r14,A2,b11),(r14,A2,b12),(r1 4,A2,b13),(r14,A2,b14),(r14,A2,b15),(r14,A2,b16),(r14,A2,b17),(r14,A2,b18),(r14,A2,b19),(r14,A2, b20),(r14,A2,b21),(r14,A2,b22),(r14,A2,b23),(r14,A2,b24),(r14,A2,b25),(r14,A2,b26),(r14,A2,b27), (r14,A2,b28),(r14,A2,b29),(r14,A2,b30),(r14,A2,b31),(r14,A2,b32),(r14,A2,b33),(r14,A2,b34),(r14, A2,b35),(r14,A2,b36),(r14,A2,b37),(r14,A2,b38),(r14,A2,b39),(r14,A2,b40),(r14,A2,b41),(r14,A2,b 42),(r14,A2,b43),(r14,A2,b44),(r14,A2,b45),(r4,A2,b46),(r14,A2,b47),(r14,A2,b48),(r14,A2,b49),(r 14,A2,b50),(r14,A2,b51),(r14,A2,b52),(r14,A2,b53),(r14,A2,b54),(r14,A2,b55),(r14,A2,b56),(r14,A 2,b57),(r14,A2,b58),(r14,A2,b59),(r14,A2,b60),(r14,A2,b61),(r14,A2,b62),(r14,A2,b63),(r14,A2,b64 ),(r14,A2,b65),(r14,A2,b66),(r14,A2,b67),(r14,A2,b68),(r14,A2,b69),(r14,A2,b70),(r14,A2,b71),(rl4 ,A2,b72),(r14,A2,b73),(r14,A2,b74),(r14,A2,b75),(r14,A2,b76),(r14,A2,b77),(r14,A2,b78),(r14,A2,b 79),(r14,A2,b80),(r14,A2,b81),(r14,A2,b82),(r14,A2,b83),(r14,A2,b84),(r14,A2,b85),(r14,A2,b86),(r 14,A2,b87),(r14,A2,b88),(r14,A2,b89),(r14,A2,b90),(r14,A2,b91),(r14,A2,b92),(r14,A2,b93),(r14,A 2,b94),(r14,A2,b95),(r14,A2,b96),(r14,A2,b97),(r14,A2,b98),(r14,A2,b99),(r14,A2,b100),(r14,A2,b1 01),(r14,A2,b102),(r14,A2,b103),(r14,A2,b104),(r14,A2,b105),(r14,A2,b106),(r14,A2,b107),(r14,A2 ,b108),(r14,A2,b109),(r14,A2,b110),(r14,42,b111),(r14,A2,b112),(r14,A2,b113),(r14,A2,b114),(r14, A2,b115),(r14,A2,b116),(r14,A2,b117),(r14,A2,b118),(r14,A2,b119),(r14,A2,b120),(r14,A2,b121),(r 14,A2,b122),(r14,A2,b123),(r14,A2,b124),(r14,A2,b125),(r14,A2,b126),(r14,A2,b127),(r14,A2,b128 ),(r14,A2,b129),(r14,A2,b130),(r14,A2,b131),(r14,A2,b132),(r14,A2,b133),(r14,A2,b134),(r14,A3,b 1),(r14,A3,b2),(r14,A3,b3),(r14,A13,b4),(r14,A3,b5),(r14,A3,b6),(r14,A3,b7),(r14,A3,b8),(r14,A3,b9) ,(r14,A3,b10),(r14,A3,b11),(r14,A3,b12),(r14,A3,b13),(r14,A3,b14),(r14,A3,b15),(r14,A3,b16),(r14, A3,b17),(r14,A3,b18),(r14,A3,b19),(r14,A3,b20),(r14,A3,b21),(r14,A3,b22),(r14,A3,b23),(r14,A3,b 24),(r14,A3,b25),(r14,A3,b26),(r14,A3,b27),(r14,A3,b28),(r14,A3,b29),(r14,A3,b30),(r14,A3,b31),(r 14,A3,b32),(r14,A3,b33),(r14,A3,b34),(r14,A3,b35),(r14,A3,b36),(r14,A3,b37),(r14,A3,b38),(r14,A 3,b39),(r14,A3,b40),(r14,A3,b41),(r14,A3,b42),(r14,A3,b43),(r14,A3,b44),(r14,A3,b45),(r14,A3,b46 ),(r14,A3,b47),(r14,A3,b48),(r14,A3,b49),(r14,A3,b50),(r14,A3,b51),(r14,A3,b52),(r14,A3,b53),(r14 ,A3,b54),(r14,A3,b55),(r14,A3,b56),(r14,A3,b57),(r14,A3,b58),(r14,A3,b59),(r14,A3,b60),(r14,A3,b 61),(r14,A3,b62),(r14,A3,b63),(r14,A3,b64),(r14,A3,b65),(r14,A3,b66),(r14,A3,b67),(r14,A3,b68),(r 14,A3,b69),(r14,A3,b70),(r14,A3,b71),(r14,A3,b72),(r14,A3,b73),(r14,A3,b74),(r14,A3,b75),(r14,A 3,b76),(r14,A3,b77),(r14,A3,b78),(r14,A3,b79),(r14,A3,b80),(r14,A3,b81),(r14,A3,b82),(r14,A3,b83 ),(r14,A3,b84),(r14,A3,b85),(r14,A3,b86),(r14,A3,b87),(r14,A3,b88),(r14,A3,b89),(r14,A3,b90),(r14 ,A3,b91),(r14,A3,b92),(r14,A3,b93),(r14,A3,b94),(r14,A3,b95),(r14,A3,b96),(r14,A3,b97),(r14,A3,b 98),(r14,A3,b99),(r14,A3,b100),(r14,A3,b101),(r14,A3,b102),(r14,A3,b103),(r14,A3,b104),(r14,A3, b105),(r14,A3,b106),(r14,A3,b107),(r14,A3,b108),(r14,A3,b109),(r14,A3,b110),(r14,A3,b111),(r14, A3,b112),(r14,A3,b113),(r14,A3,b114),(r14,A3,b115),(r14,A3,b116),(r14,A3,b117),(r14,A3,b118),(r 14,A3,b119),(r14,A3,b120),(r14,A3,b121),(r14,A3,b122),(r14,A3,b123),(r14,A3,b124),(r14,A3,b125 ),(r14,A3,b126),(r14,A3,b127),(r14,A3,b128),(r14,A3,b129),(r14,A3,b130),(r14,A3,b131),(r14,A3,b 132),(r14,A3,b133),(r14,A3,b134),(r14,A4,b1),(r14,A4,b2),(r14,A4,b3),(r14,A4,b4),(r14,A4,b5),(r14 ,A4,b6),(r14,A4,b7),(r14,A4,b8),(r14,A4,b9),(r14,A4,b10),(r14,A4,b11),(r14,A4,b12),(r14,A4,b13),( r14,A4,b14),(r14,A4,b15),(r14,A4,b16),(r14,A4,b17),(r14,A4,b18),(r14,A4,b19),(r14,A4,b20),(r14, A4,b21),(r14,A4,b22),(r14,A4,b23),(r14,A4,b24),(r14,A4,b25),(r14,A4,b26),(r14,A4,b27),(r14,A4,b 28),(r14,A4,b29),(r14,A4,b30),(r14,A4,b31),(r14,A4,b32),(r14,A4,b33),(r14,A4,b34),(r14,A4,b35),(r 14,A4,b36),(r14,A4,b37),(r14,A4,b38),(r14,A4,b39),(r14,A4,b40),(r14,A4,b41),(r14,A4,b42),(r14,A 4,b43),(r14,A4,b44),(r14,A4,b45),(r14,A4,b46),(r14,A4,b47),(r14,A4,b48),(r14,A4,b49),(r14,A4,b50 ),(r14,A4,b51),(r14,A4,b52),(r14,A4,b53),(r14,A4,b54),(r14,A4,b55),(r14,A4,b56),(r14,A4,b57),(r14 ,A4,b58),(r14,A4,b59),(r14,A4,b60),(r14,A4,b61),(r14,A4,b62),(r14,A4,b63),(r14,A4,b64),(r14,A4,b 65),(r14,A4,b66),(r14,A4,b67),(r14,A4,b68),(r14,A4,b69),(rL4,A4,b70),(r14,A4,b'71),(r14,A4,b72),(r 14,A4,b73),(r14,A4,b74),(r14,A4,b75),(r14,A4,b76),(r14,A4,b77),(r14,A4,b78),(r14,A4,b79),(r14,A 4,b80),(r14,A4,b81),(r14,A4,b82),(r14,A4,b83),(r14,A4,b84),(r14,A4,b85),(r4,A4,b86),(r14,A4,b87 ),(r14,A4,b88),(r14,A4,b89),(r14,A4,b90),(r14,A4,b91),(r14,A4,b92),(r1.4,A4,b93),(r14,A4,b94),(r14 ,A4,b95),(r14,A4,b96),(r14,A4,b97),(r14,A4,b98),(r14,A4,b99),(r14,A4,b100),(r14,A4,b101),(r14,A 4,b102),(r14,A4,b103),(r14,A4,b104),(r14,A4,b105),(r14,A4,b106),(r14,A4,b107),(r14,A4,b108),(r1 4,A4,b109),(r14,A4,b110),(r14,A4,b111),(r14,A4,b112),(r14,A4,b113),(r14,A4,b114),(r14,A4,b115),( r14,A4,b116),(r14,A4,b117),(r14,A4,b118),(r14,A4,b119),(r14,A4,b120),(r14,A4,b121),(r14,A4,b12 2),(r14,A4,b123),(r14,A4,b124),(r14,A4,b125),(r14,A4,b126),(r14,A4,b127),(r14,A4,b128),(r14,A4, b129),(r14,A4,b130),(r14,A4,b131),(r14,A4,b132),(r14,A4,b133),(r14,A4,b134),

(r15,A1,b1),(r15,A1,b2),(r15,A1,b3),(r15,A1,b4),(r15,A1,b5),(r15,A1,b6),(r15,A1,b7),(r15,A1,b8),(r 15,A1,b9),(r15,A1,b10),(r15,A1,b11),(r15,A1,b12),(r15,A1,b13),(r15,A1,b14),(r1,A1,b15),(r15,A1, b16),(r15,A1,b17),(r15,A1,b18),(r15,A1,b19),(r15,A1,b20),(r15,A1,b21),(r15,A1,b22),(r15,A1,b23), (r15,A1,b24),(r15,A1,b25),(r15,A1,b26),(r15,A1,b27),(r15,A1,b28),(r15,A1,b29),(r15,A1,b30),(r15, A1,b31),(r15,A1,b32),(r15,A1,b33),(r15,A1,b34),(r15,A1,b35),(r15,A1,b36),(r15,A1,b37),(r15,A1,b 38),(r15,A1,b39),(r15,A1,b40),(r15,A1,b41),(r15,A1,b42),(r15,A1,b43),(r15,A1,b44),(r15,A1l,b45),(r 15,A1,b46),(r15,A1,b47),(r15,A1,b48),(r15,A1,b49),(r15,A1,b50),(r15,A1,b51),(r15,A1,b52),(r15,A 1,b53),(r15,A1,b54),(r15,A1,b55),(r15,A1,b56),(r15,A1,b57),(r15,A1,b58),(r15,A1,b59),(r15,A1,b60 ),(r15,A1,b61),(r15,A1,b62),(r15,A1,b63),(r15,A1,b64),(r15,A1,b65),(r15,A1,b66),(r15,A1,b67),(r15 ,A1,b68),(r15,A1,b69),(r15,A1,b70),(r15,A1,b71),(r15,A1,b72),(r15,A1,b73),(r15,A1,b74),(r15,A1,b 75),(r15,A1,b76),(r15,A1,b77),(r15,A1,b78),(r15,A1,b79),(r15,A1,b80),(r15,A1,b81),(r15,A1,b82),(r 15,A1,b83),(r15,A1,b84),(r15,A1,b85),(r15,A1,b86),(r15,A1,b87),(r15,A1,b88),(r15,A1,b89),(r15,A 1,b90),(r15,A1,b91),(r15,A1,b92),(r15,A1,b93),(r15,A1,b94),(r15,A1,b95),(r15,A1,b96),(r15,A1,b97 ),(r15,A1,b98),(r15,A1,b99),(r15,A1,b100),(r15,A1,b101),(r15,A1,b102),(r125,A1,b103),(r15,A1,b10 4),(r15,A1,b105),(r15,A1,b106),(r15,A1,b107),(r15,A1,b108),(r15,A1,b109),(r15,Al,b110),(r15,A1, b111),(r15,A1,b112),(r15,A1,b113),(r15,A1,b114),(r15,A1,b115),(r15,A1,b116),(r15,A1,b117),(r15, A1,b118),(r15,A1,b119),(r15,A1,b120),(r15,A1,b121),(r15,A1,b122),(r15,A1,b123),(r15,A1,b124),(r 15,A1,b125),(r15,A1,b126),(r15,A1,b127),(r15,A1,b128),(r15,A1,b129),(r15,A1,b130),(r15,A1,b131 ),(r15,A1,b132),(r15,A1,b133),(r15,A1,b134),(r15,A2,b1),(r15,A2,b2),(r15,A2,b3),(r15,A2,b4),(r15, A2,b5),(r15,A2,b6),(r15,A2,b7),(r15,A2,b8),(r15,A2,b9),(r15,A2,b10),(r15,A2,b11),(r15,A2,b12),(r1 5,A2,b13),(r15,A2,b14),(r15,A2,b15),(r15,A2,b16),(r15,A2,b17),(r15,A2,b18),(r15,A2,b19),(r15,A2, b20),(r15,A2,b21),(r15,A2,b22),(r15,A2,b23),(r15,A2,b24),(r15,A2,b25),(r15,A2,b26),(r15,A2,b27), (r15,A2,b28),(r15,A2,b29),(r15,A2,b30),(r15,A2,b31),(r15,A2,b32),(r15,A2,b33),(r15,A2,b34),(r15, A2,b35),(r15,A2,b36),(r15,A2,b37),(r15,A2,b38),(r15,A2,b39),(r15,A2,b40),(r15,A2,b41),(r15,A2,b 42),(r15,A2,b43),(r15,A2,b44),(r15,A2,b45),(r15,A2,b46),(r15,A2,b47),(r15,A2,b48),(r15,A2,b49),(r 15,A2,b50),(r15,A2,b51),(r15,A2,b52),(r15,A2,b53),(r15,A2,b54),(r15,A2,b55),(r15,A2,b56),(rlS,A 2,b57),(r15,A2,b58),(r15,A2,b59),(r15,A2,b60),(r15,A2,b61),(r15,A2,b62),(r15,A2,b63),(r15,A2,b64 ),(r15,A2,b65),(r15,A2,b66),(r15,A2,b67),(r15,A2,b68),(r15,A2,b69),(r15,A2,b70),(r15,A2,b71),(r15 ,A2,b72),(r15,A2,b73),(r15,A2,b74),(r15,A2,b75),(r15,A2,b76),(r15,A2,b77),(r15,A2,b78),(r15,A2,b 79),(r15,A2,b80),(r15,A2,b81),(r15,A2,b82),(r15,A2,b83),(r15,A2,b84),(r15,A2,b85),(r15,A2,b86),(r 15,A2,b87),(r15,A2,b88),(r15,A2,b89),(r15,A2,b90),(r15,A2,b91),(r15,A2,b92),(r15,A2,b93),(r15,A 2,b94),(r15,A2,b95),(r15,A2,b96),(r15,A2,b97),(r15,A2,b98),(r15,A2,b99),(r15,A2,b100),(r15,A2,b1 01),(r15,A2,b102),(r15,A2,b103),(r15,A2,b104),(r15,A2,b105),(r15,A2,b106),(r15,A2,b107),(r15,A2 ,b108),(r15,A2,b109),(r15,A2,b110),(r15,A2,b111),(r15,A2,blI2),(r15,A2,b113),(r15,A2,b114),(r15, A2,b115),(r15,A2,b116),(r15,A2,b117),(r15,A2,b118),(r15,A2,b119),(r15,A2,b120),(r15,A2,b121),(r 15,A2,b122),(r15,A2,b123),(r15,A2,b124),(r15,A2,b125),(r15,A2,b126),(r15,A2,b127),(r15,A2,b128 ),(r15,A2,bl29),(r15,A2,b130),(r15,A2,b131),(r15,A2,b132),(r15,A2,b133),(r15,A2,b134),(r15,A3,b 1),(r15,A3,b2),(r15,A3,b3),(r15,A3,b4),(r15,A3,b5),(r15,A3,b6),(r15,A3,b7),(r15,A3,b8),(r15,A3,b9) ,(r15,A3,b10),(r15,A3,b11),(r15,A3,b12),(r15,A3,b13),(r15,A3,b14),(r15,A3,b15),(r15,A3,b16),(r15, A3,b17),(r15,A3,b18),(r15,A3,b19),(r15,A3,b20),(r15,A3,b21),(r15,A3,b22),(r15,A3,b23),(r15,A3,b 24),(r15,A3,b25),(r15,A3,b26),(r15,A3,b27),(r15,A3,b28),(r15,A3,b29),(r15,A3,b30),(r15,A3,b31),(r 15,A3,b32),(r15,A3,b33),(r15,A3,b34),(r15,A3,b35),(r15,A3,b36),(r15,A3,b37),(r15,A3,b38),(r15,A 3,b39),(r15,A3,b40),(r15,A3,b41),(r15,A3,b42),(r15,A3,b43),(r15,A3,b44),(r15,A3,b45),(r15,A11,b46 ),(r15,A3,b47),(r15,A3,b48),(r15,A3,b49),(r15,A3,b50),(r15,A3,b51),(r15,A3,b52),(r15,A3,b53),(rl5 ,A3,b54),(r15,A3,b55),(r15,A3,b56),(r15,A43,b57),(r15,A3,b58),(r15,A3,b59),(r15,A3,b60),(r15,A3,b 61),(r15,A3,b62),(r15,A3,b63),(r15,A3,b64),(r15,A3,b65),(r15,A3,b66),(r15,A3,b67),(r15,A3,b68),(r 15,A3,b69),(r15,A3,b70),(r15,A3,b71),(r15,A3,b72),(r15,A3,b73),(r15,A3,b74),(r15,A3,b75),(r15,A 3,b76),(r15;A3,b77),(r15,A3,b78),(r15,A3,b79),(r15,A3,b80),(r15,A3,b81),(r15,A3,b82),(r15,A3,b83 ),(r15,A3,b84),(r15,A3,b85),(r15,A3,b86),(r15,A3,b87),(r15,A3,b88),(r15,A3,b89),(r15,A3,b90),(r15 ,A3,b91),(r15,A3,b92),(r15,A3,b93),(r15,A3,b94),(r15,A3,b95),(r15,A3,b96),(r15,A3,b97),(r15,A3,b 98),(r15,A3,b99),(r15,A3,b100),(r15,A3,b101),(r15,A3,b102),(r15,A3,b103),(r15,A3,b104),(r15,A3, b105),(r15,A3,b106),(r15,A3,b107),(r15,A3,b108),(r15,A3,b109),(r15,A3,b110),(r15,A3,b111),(r15, A3,b112),(r15,A3,b113),(r15,A3,b114),(r15,A3,b115),(r15,A3,b116),(r15,A3,b117),(r15,A3,b118),(r 15,A3,b119),(r15,A3,b120),(r15,A3,b121),(r15,A3,b122),(r15,A3,b123),(r15,A3,b124),(r15,A3,b125 ),(r15,A3,b126),(r15,A3,b127),(r15,A3,b128),(r15,A3,b129),(r15,A3,b130),(r15,A3,b131),(r15,A3,b 132),(r15,A3,b133),(r15,A3,b134),(r15,A4,b1),(r15,A4,b2),(r15,A4,b3),(r15,A4,b4),(r15,A4,b5),(r15 ,A4,b6),(r15,A4,b7),(r15,A4,b8),(r15,A4,b9),(r15,A4,b10),(r15,A4,b11),(r15,A4,b12),(r15,A4,b13), ( r15,A4,b14),(r15,A4,b15),(r15,A4,b16),(r15,A4,b17),(r15,A4,b18),(r15,A4,b19),(r15,A4,b20),(r15, A4,b21),(r15,A4,b22),(r15,A4,b23),(r15,A4,b24),(r15,A4,b25),(r15,A4,b26),(r15,A4,b27),(r15,A4,b 28),(r15,A4,b29),(r15,A4,b30),(r15,A4,b31),(r15,A4,b32),(r15,A4,b33),(r15,A4,b34),(r15,A4,b35),(r 15,A4,b36),(r15,A4,b37),(r15,A4,b38),(r15,A4,b39),(r15,A4,b40),(r15,A4,b41),(r15,A4,b42),(r15,A 4,b43),(r15,A4,b44),(r15,A4,b45),(r15,A4,b46),(r15,A4,b47),(r15,A4,b48),(r15,A4,b49),(r15,A4,b50 ),(r15,A4,b51),(r15,A4,b52),(r15,A4,b53),(r15,A4,b54),(r15,A4,b55),(r15,A4,b56),(r15,A4,b57),(r15 ,A4,b58),(r15,A4,b59),(r15,A4,b60),(r15,A4,b61),(r15,A4,b62),(r15,A4,b63),(r15,A4,b64),(r15,A4,b 65),(r15,A4,b66),(r15,A4,b67),(r15,44,b68),(r15,A4,b69),(r15,A4,b70),(r15,A4,b71),(r15,A4,b72),(r 15,A4,b73),(r15,A4,b74),(r15,A4,b75),(r15,A4,b76),(r15,A4,b77),(r15,A4,b78),(r15,A4,b79),(r15,A 4,b80),(r15,A4,b81),(r15,A4,b82),(r15,A4,b83),(r15,A4,b84),(r15,A4,b85),(r15,A4,b86),(r15,A4,b87 ),(r15,A4,b88),(r15,A4,b89),(r15,A4,b90),(r15,A4,b91),(r15,A4,b92),(r15,A4,b93),(r15,A4,b94),(r15 ,A4,b95),(r15,A4,b96),(r15,A4,b97),(r15,A4,b98),(r15,A4,b99),(r15,A4,b100),(r15,A4,b101),(r15,A 4,b102),(r15,A4,b103),(r15,A4,b104),(r15,A4,b105),(r15,A4,b106),(r15,A4,b107),(r15,A4,b108),(r1 5,A4,b109),(r15,A4,b110),(r15,A4,b111),(r15,A4,b112),(r15,A4,b113),(r15,A4,b114),(r15,A4,b115),( r15,A4,b116),(r15,A4,b117),(r15,A4,b118),(r15,A4,b119),(r15,A4,b120),(r15,A4,b121),(r5,A4,b12 2),(r15,A4,b123),(r15,A4,b124),(r15,A4,b125),(r15,A4,b126),(r15,A4,b127),(r15,A4,b128),(r15,A4, b129),(r15,A4,b130),(r15,A4,b131),(r15,A4,b132),(r15,A4,b133), or (r15,A4,b134).

The present compounds are useful in disease induced by the production, secretion or deposition of-amyloid β protein, and are effective in treatment and/or prevention, and symptom improvement of such as dementia of the Alzheimer's type (Alzheimer's disease, senile dementia of Alzheimer type), Down's syndrome, memory impairment, prion disease (Creutzfeldt-Jakob disease), mild cognitive impairment (MCI), Dutch type of hereditary cerebral hemorrhage with amyloidosis, cerebral amyloid angiopathy, other type of degenerative dementia, mixed dementia with Alzheimer's and vascular type, dementia with Parkinson's Disease, dementia with progressive supranuclear palsy, dementia with Cortico-basal degeneration, Alzheimer's disease with diffuse Lewy body disease, age-related macular degeneration, Parkinson's Disease, amyloid angiopathy and so on.
In the present invention, "treating Alzheimer's disease" includes prevention of advancing in severity of MCI and prevention of onset of familial Alzheimer's disease. In the present invention, "a pharmaceutical composition for treating Alzheimer's disease" includes a pharmaceutical composition for prevention of advancing in severity of MCI and prevention of onset of familial Alzheimer's disease.

Since the present compound has high inhibitory activity on BACE1, and/or has high selectivity on other enzymes, it can be a medicament with reduced side effect. Further, since the compound has high effect of reducing amyloid β production in a cell system, particularly, has high effect of reducing amyloid β production in brain, it can be an excellent medicament. In addition, by converting the compound into an optically active compound having suitable stereochemistry, the compound can be a medicament having a larger safety margin on the side effect. In addition, the present compound also has advantages that metabolism stability is high, solubility is high, oral absorbability is high, good bioavailability is exhibited, clearance is good, brain transference is high, a half life is high, non-protein binding rate is high, hERG channel inhibition is low, CYP inhibition is low, CYP MBI (irreversible inhibition (mechanism-based inhibition)) is low and/or an Ames test is negative.

The present compounds can be administrated in combination with other pharmaceutical agents such as other therapeutic drugs for Alzheimer's disease, e.g., acetylcholinesterase and the like. The present compounds can be treated with concomitantly with the anti-dementia agents such as Donepezil Hydrochloride, Tacrine, Galantamine, Rivastigmine, Zanapezil, Memantine, and Vinpocetine.

When the present compound is administered to a human, it can be administered orally as powders, granules, tablets, capsules, pills, solutions, or the like, or parenterally as injectables, suppositories, transdermal absorbable agents, inhalations, or the like. In addition, the present compound can be formulated into pharmaceutical preparations by adding pharmaceutical additives such as excipients, binders, wetting agents, disintegrating agents, lubricants and the like, which are suitable for formulations and an effective amount of the present compound.

A dose is different depending on state of disease, an administration route, and an age and a weight of a patient, and is usually 0.1 µg to 1 g/day, preferably 0.01 to 200 mg/day when orally administered to an adult, and is usually 1 µg to 10 g/day, preferably 0.1 to 2 g/day when parenterally administered.

### [Examples]

Following examples and test examples illustrate the present invention in more detail, but the present invention is not limited by these examples.

¹H-NMR was measured in deuterium chloroform (CDCl₃) using tetramethylsilane as an internal standard. δ values were shown as ppm. Binding constants (J) were shown as Hz. In the data, s, d, t, m, br, and brs means singlet, doublet, triplet, multiplet, broad-line and broad-singlet, respectively.

Dashed bond shows relative configuration and all compounds are racemates.

In example, the meaning of each abbreviation is as follows:
- Me: methyl
- Et: ethyl
- Bz: benzoyl
- Boc: t-butoxycarbonyl
- Fmoc: 9-fluorenylmethoxycarbonyl
- Ns: nitrobenzenesulfonyl
- THF: tetrahydrofuran
- DMF: dimethylformamide
- DMSO: dimethyl sulfoxide
- TFA: trifluoroacetyl
- DBU: 1,8-diazabicyclo[5.4.0]undeca-7-ene

LC/MS data of the present compounds were measured under the following conditions, and a retention time and [M+H]⁺ are shown.

### Method A

Column: Xbridge (registered trade name) C18 (5 µm, i.d. 4.6 x 50 mm)(Waters)
Flow rate: 3 mL/min.
UV detection wavelength: 254 nm
Mobile phase: [A] 0.1% formic acid-containing aqueous solution; [B] 0.1% formic acid-containing acetonitrile solution
Gradient: performing linear gradient of 10% to 100% solvent [B] for 3 minutes, and keeping 100% solvent [B] for 1 minute

### Method B

Column: Gemini-NX (5 µm, i.d.4.6 x 50 mm) (Phenomenex)
Flow rate: 3 mL/min.
UV detection wavelength: 254 nm
Mobile phase: [A]0.1% formic acid-containing aqueous solution; [B]0.1% formic acid containing methanol solution
Gradient: performing linear gradient of 5% to 100% solvent [B] for 3.5 minutes, and keeping 100% solvent [B] for 5 minutes.

### Method C

Column: ACQUITY UPLC (registered trade name) BEH C18 (1.7 µm i.d.2.1 x 50 mm) (Waters) Flow rate: 1.0 mL/min.
UV detection wavelength: 254 nm
Mobile phase:[A] 0.1% formic acid-containing aqueous solution, [B] 0.1% formic acid-containing acetonitrile solution
Gradient: performing linear gradient of 10% to 100% solvent [B] for 3.5 minutes, and keeping 100% solvent [B] for 0.5 minutes.

### Method D

Column: ACQUITY UPLC (registered trade name) BEH C18 (1.7 µm i.d.2.1 x 50 mm) (Waters) Flow rate: 0.9 mL/min
UV detection wavelength: 254 nm
Mobile phase: [A] 0.1% formic acid-containing aqueous solution, [B] 0.1% formic acid-containing acetonitrile solution
Gradient: performing linear gradient 10% to 100% solvent [B] for 3.5 minutes, and keeping 100% solvent [B] for 0.5 minute.

### Example 1 Synthesis of Compound (I-1)

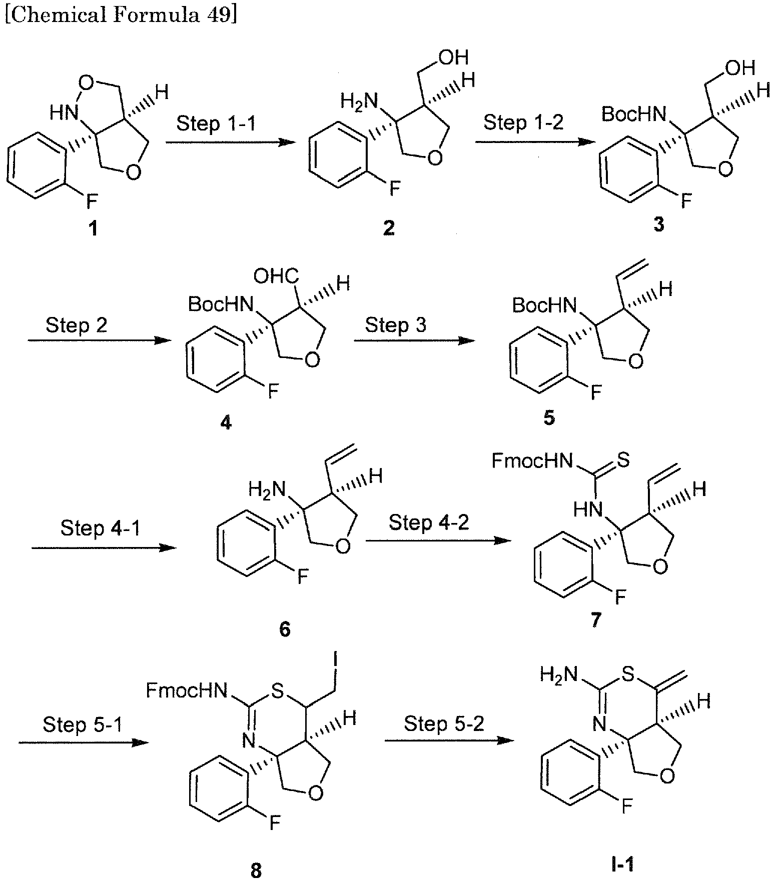

### Steps 1-1 and 1-2

A mixed solution of a known compound (1) (3.13 g) which can be prepared according to the method described in Patent Literature 4 and 5% Rh/C (924 mg) in ethanol (40 ml) was stirred under hydrogen atmosphere at room temperature for 22 hours. The insoluble material was removed by filtration and the filtrate was concentrated. The residue was dissolved in THF (50 mL) and di-tert-butyldicarbonate (3.92 g) was added. The mixed solution was stirred at 55 °C for 15 hours. The reaction solution was evaporated under reduced pressure. The crude product was purified by silica gel chromatography to give Compound (3)(4.08 g).
¹H-NMR(CDCl₃) δ:1.37(s,9H),3.27(m,1H),3.55-3.70(m,2H),3.70-3.85(m,2H),3.95-4.15(m,2H),4.20 -4.30(m,2H),5.79(brs,1H),7.02(dd,J=12.2,8.1Hz,1H),7.14(t,J=7.5Hz,1H),7.22.7.31(m,1H),7.80(m, 1H)

### Step 2

To a solution of Compound (3) (4.06 g) in ethyl acetate (50 mL) was added 2-iodoxybenzoic acid at room temperature. The reaction mixture was stirred at 80 °C for 4.5 hours. The insoluble material was removed by filtration and the filtrate was evaporated under the reduced pressure. The crude product was purified by silica gel chromatography to give Compound (4) (3.36 g).
¹H-NMR(CDCl₃)δ:1.30(s,9H),3.78(m,1H),4.10(t,J=8.6Hz,1H),4.29(dd,J=9.6,2.1Hz,1H),4.39(dd,J =9.0,7.0Hz,1H),5.49(brs,1H),7.08(ddd,J=12.2,8.1,1.3Hz,1H),7.17(td,J=7.6,1.3Hz,1H),7.27-7.35(m ,1H),7.50(td,J=8.0,1.7,1H),9.86(d,J=2.6,1H)

### Step 3

To a solution of methyltriphenylphosphonium iodide (3.02 g) in THF (15 mL) was dropwisely added 1.57 M n-butyllithium hexane solution (4.76 mL) under nitrogen atmosphere under ice-cooling. The mixture solution was stirred at the same temperature for 30 minutes. To the mixture solution was dropwisely added a solution of Compound 4 (771 mg) in THF (10 mL) under nitrogen atmosphere under ice-cooling. The reaction mixture was stirred at the same temperature for 30 minutes. To the reaction mixture was added an aqueous solution of ammonium chloride and the mixture was allowed to warm to room temperature. To the reaction mixture was added ethyl acetate and the organic layer was partitioned. The organic layer was washed with brine. The organic layer was dried over anhydrous magnesium sulfate and the insoluble material was removed by filtration. The filtrate was evaporated under reduced pressure. The residue was purified by silica gel chromatography to give Compound (5) (62 mg).
¹H-NMR(CDCl₃)δ:1.37(s,9H),3.21(m,1H),3.82(dd,J=8.5,6.7Hz,1H),4.06(t,J=7.8Hz,1H),4.21(m, H),4.78(m,1H),5.20-5.35(m,3H),5.75-5.87(m,1H),7.01-7.14(m,2H),7,11(td,J=7.7,1.4Hz,1H),7.26(m ,1H),7.40(td,J=8.1,1.7Hz,1H)

### Steps 4-1 and 4-2

To a solution of Compound 5 (61 mg) in methylene chloride (1 mL) was added trifluoroacetic acid (0.5 mL) under ice-cooling. The reaction mixture was warmed to room temperature and stirred for 40 minutes. To the reaction mixture was added a saturated aqueous sodium bicarbonate solution (10 mL) under ice-cooling and the mixture was extracted with ethyl acetate. The organic layer was washed with brine. The organic layer was dried over anhydrous magnesium sulfate and the insoluble material was removed by filtration. The filtrate was evaporated under reduced pressure.

The residue was dissolved in acetone (1 mL) and Fmoc-isothiocyanate (61 mg) was added under ice-cooling. The reaction mixture was warmed to room temperature and stirred at room temperature for 1.5 hours. The solvent was evaporated under reduced pressure. The residue was purified by silica gel chromatography to give Compound (7) (95 mg).
¹H-NMR(CDCl₃)δ:3.26(m,1H),3.87(dd,J=9.0,5.6Hz,1H),4.09(dd,J=8.9,6.5Hz,1H),4.28(t,J=6.7,1H ),4.38(d,J=10.7,1H),4.55(d,J=6.9,2H),5.31(d,J=10.1,1H),5.39(d,J=17.1,1H),5.48(d,J=10.4,1H),5.9 6(m,1H),7.09(ddd,J=12.3,8.1,1.3Hz,1H),7.18(td,J=7.6,1.3Hz,1H),7.32(m,1H),7.38(td,J=7.4,1.2Hz, 1H),7.48-7.52(m,3H),7.60(d,J=7.6,2H),7.83(d,J=7.6,2H),7.95(brs,1H),10.46(brs;1H)

### Steps 5-1 and 5-2

To a solution of Compound (7) (21 mg) in methylene chloride (1 mL) was added iodine (22 mg) under ice-cooling. The reaction solution was warmed to room temperature and stirred at room temperature for 30 minutes. To the reaction mixture were added 10% Na₂S₂O₃ 5H₂O (1 mL), and a saturated aqueous NaHCO₃ solution (2 mL), and the mixture was extracted with ethyl acetate. The organic layer was washed with brine. The organic layer was dried over anhydrous magnesium sulfate and the insoluble material was removed by filtration. The filtrate was evaporated under reduced pressure.

The residue was dissolved in THF (1 mL) and DBU (20 mg) was added under ice-cooling. After the mixture was stirred at the same temperature for 15 minutes, it was warmed to room temperature and stirred at room temperature for 1.5 hours. Water and ethyl avetate were added to the reaction mixture and the organic layer was partitioned. The organic layer was washed with brine and dried over anhydrous magnesium sulfate. The insoluble material was removed by filtration and the filtrate was evaporated under reduced pressure. The residue was purified by silica gel chromatography to give Compound (I-1) (10 mg).

### Example 2 Synthesis of Compounds (I-2) and (I-3)

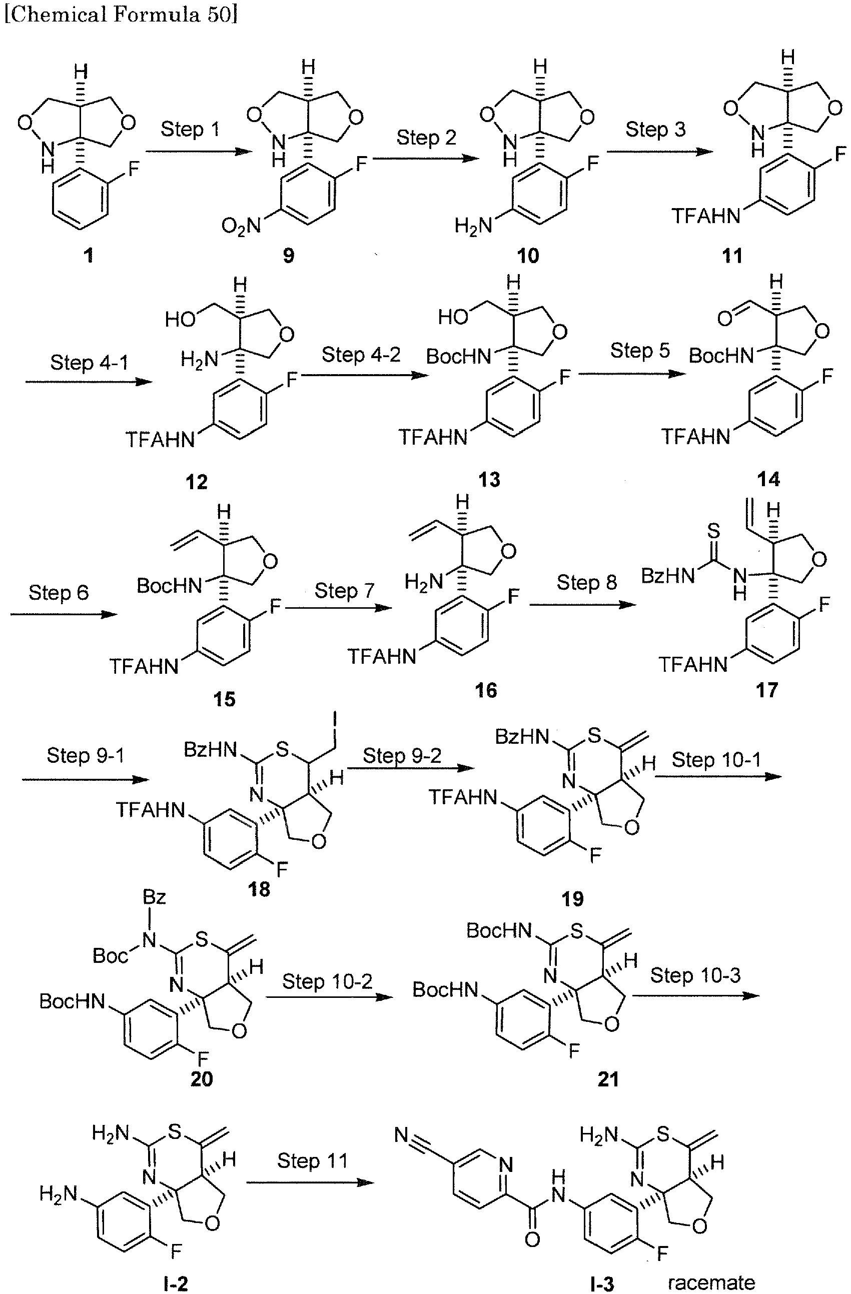

### Step 1

To a solution of a known compound (1) (550 mg) which can be prepared according to the method described in Patent Literature 4 in concentrated sulfuric acid (3.3 mL) was dropwisely added fuming nitric acid (0.12 mL) under ice-cooling and the mixture was stirred at the same temperature for 1 hour. After the reaction mixture was poured into ice water and a saturated aqueous sodium bicarbonate solution, the mixture was extracted with chloroform. The organic layer was dried over anhydrous magnesium sulfate. The insoluble material was removed by filtration and the filtrate was evaporated under reduced pressure. The residue was purified by silica gel chromatography to give Compound (9) (610 mg).
¹H-NMR(CDCl₃)δ:3.41(1H,q,J=7.4Hz),3.55-3.61(1H,m),3.87(2H,ddd,J=21.0,10.4,3.2Hz),4.03(1H ,d,J=9.6Hz),4.13-4.19(1H,m),4.57(1H,t,J=8.4Hz),5.17(1H,s),7.21(1H,dd,J=10.3,8.9Hz),8.19(1H,d dd,J=8.9,4.3,3.0Hz),8.88(1H,dd,J=6.7,3.0Hz)

### Step 2

To a solution of Compound (9) (30 mg) in ethanol (0.75 mL) were added reduced iron (53 mg) and a saturated aqueous ammonium chloride solution (0.19 mL). The mixed solution was stirred at 50 °C for 1 hour and cooled to room temperature. The solution was filtered and the filtrate was extracted with ethyl acetate. The organic layer was washed with brine and dried over anhydrous magnesium sulfate. The insoluble material was removed by filtration and the filtrate was evaporated under reduced pressure. The residue was purified by silica gel chromatography to give Compound (10) (610 mg).
¹H-NMR(CDCl₃)δ:3.39(1H,dd,J=14.2,7.5Hz),3.43-3.76(3H,m),3.79-3.88(2H,m),3.95(1H,d,J=9.5H z),4.10-4.17(1H,m),4.51(1H,t,J=7.9Hz),5.06(1H,s),6.53(1H,ddd,J=8.6,3.9,3.1Hz),6.84(1H,dd,J=11. 2,8.6Hz),7.22(1H,brs)

### Step 3

To a solution of Compound (10) (490 mg) in methanol (5 mL) were added ethyl trifluoroacetate (652 mg) and triethylamine (0.30 mL) at room temperature. The mixture was stirred at the same temperature for 4 hours. After being diluted with water, the aqueous layer was extracted with ethyl acetate and the organic layer was washed with brine. The organic layer was dried over anhydrous magnesium sulfate. The insoluble material removed by filtration and the filtrate was evaporated under reduced pressure. The residue was purified by silica gel chromatography to give Compound (11) (610 mg).
¹H-NMR(CDCl₃)δ:3.40(1H,dd,J=14.4,7.7Hz),3.57(1H,t,J=8.1Hz),3.86(2H,d,J=2.7Hz),4.00(1H,d, J=9.3Hz),4.16(1H,ddd,J=9.3,5.8,2.3Hz),4.55(1H,t,J=8.5Hz),5.14(1H,s),7.11(1H,dd,J=10.8,8.8Hz), 7.78(1H,dd,J=6.6,2.9Hz),7.89(1H,ddd,J=8.8,4.3,2.9Hz),7.95(1H,s)

### Steps 4-1 and 4-2

To a solution of Compound (11) (627 mg) in ethanol (12 mL) was added 5% Rh/C (121 mg) at room temperature. The mixed solution was stirred at the same temperature for 29 hours under hydrogen atmosphere. The insoluble material was removed by filtration and the filtrate was evaporated under reduced pressure.

The residue containing Compound (12) was dissolved in THF (3 mL). To the solution was added di-tert-butyldicarbonate (855 mg) at room temperature and the mixture was refluxed for 1 hour. The reaction mixture was cooled to room temperature and evaporated under reduced pressure. The residue was purified by silica gel chromatography to give Compound (13) (695 mg).
¹H-NMR(CDCl₃)δ:1.37(9H,s),3.16-3.31(1H,m),3.55(1H,t,J=8.5Hz),3.77(2H,t,J=6.4Hz),3.87-4.11( 3H,m),4.23(1H,d,J=8.4Hz),5.76(1H,s),7.07(1H,dd,J=11.3,8.8Hz),7.68-7.75(1H,m),7.85-7.92(1H,m ),8.57(1H,s)

### Step 5

To a solution of Compound (13) (690 mg) in ethyl acetate (7 mL) was added 2-iodoxybenzoic acid (549 mg) at room temperature. The reaction mixture was stirred at 80 °C for 5 hours. The insoluble material was removed by filtration and the filtrate was evaporated under reduced pressure. The crude product was purified by silica gel chromatography to give Compound (14) (386 mg).
¹H-NMR(CDCl₃)δ:1.33(s,9H),3.76(m,1H),4.07-4.17(m,2H),4.29(dd,J=9.5,2.1Hz,1H),4.40(dd,J=9. 2,6.9Hz, 1H),5.60(brs, 1H),7.12(m, 1H),7.63-7.70(m,2H),8.17(brs,1H),9.85(d,J=2.9,1H)

### Step 6

To a solution of methyltriphenylphosphonium iodide (2.74g) in THF (10 mL) was added n-butyl lithium hexane solution (2.61 mL, 2.6 M) was dropwisely added under nitrogen atmosphere under ice-cooling. The reaction mixture was stirred at the same temperature for 30 minutes. After the mixture was stirred at room temperature for 1 hour, a solution of Compound (14) (285 mg) in THF (1 mL) was dropwisely added to the reaction mixture under nitrogen atmosphere. After the mixture was stirred at the same temperature for 30 minutes, it was poured into an ice water containing ammonium chloride and extracted with ethyl acetate. The organic layer was washed with brine and dried over anhydrous magnesium sulfate. The insoluble material was removed by filtration and the filtrate was evaporated under reduced pressure. The residue was purified by silica gel chromatography to give Compound (15) (38 mg).
¹H-NMR(CDCl₃)δ:1.36(9H,s),3.15-3.22(1H,m),3.81(1H,dd,J=8.8,6.1Hz),4.04-4.15(2H,m),4.76-4.8 0(1H,m),5.24-5.37(3H,m),5.81(1H,ddd,J=17.9,9.5,7.8Hz),7.09(1H,dd,J=11.4,8.9Hz),7.45-7.46(1H, m),7.64-7.69(1H,m),7.90(1H,s)

### Step 7

To a solution of Compound (15) (52 mg) in methylene chloride (2 mL) was added trifluoroacetic acid (0.2 mL) under ice-cooling and the mixture was stirred at room temperature for 3 hours. To the reaction mixture was added a saturated aqueous sodium bicarbonate solution under ice cooling and the aqueous layer was extracted with methylene chloride. The organic layer was washed with brine and dried over anhydrous magnesium sulfate. The insoluble material was removed by filtration and the filtrate was evaporated under reduced pressure. The residue was purified by silica gel chromatography to give Compound (16) (37 mg).
¹H-NMR(CDCl₃)δ:3.45(1H,dd,J=17.1,8.2Hz),3.93-3.99(2H,m),4.13-4.20(2H,m),5.17-5.29(2H,m), 5.76-5.87(1H,m),7.09(1H,dd,J=11.4,8.8Hz),7.53(1H,ddd,J=8.8,4.0,2.8Hz),7.80(1H,dd,J=6.9,2.7Hz ),7.84(1H,s)

### Step 8

To a solution of Compound (16) (37 mg) in acetone (2 mL) was added benzoyl isothiocyanate (22 mg) under ice-cooling and the mixture was stirred at room temperature for 150 minutes. The solvent was evaporated under reduced pressure. The residue was purified by silica gel chromatography to give Compound (17) (56 mg).
¹H-NMR(CDCl₃)δ:3.26(1H,dd,J=14.7,6.3Hz),3.89(1H,dd,J=9.0,5.8Hz),4.08-4.15(1H,m),4.36(1H, d,J=10.2Hz),5.26(1H,d,J=10.2Hz),5.43(1H,d,J=16.9Hz),5.54(1H,d,J=10.2Hz),5.91-6.03(1H,m),7.1 1(1H,dd,J=11.2,8.9Hz),7.40(1H,dd,J=6.9,2.7Hz),7.48-7.53(2H,m),7.59-7.65(1H,m),7.75-7.85(3H, m),7.96(1H,s),8.86(1H,s),11.48(1H,s)

### Steps 9-1 and 9-2

To a solution of Compound (17) (56 mg) in methylene chloride (2 mL) was added iodine (62 mg) under ice-cooling. The reaction mixture was warmed to room temperature and stirred at room temperature for 50 minutes. An aqueous sodium thiosulfate solution and a saturated aqueous sodium bicarbonate solution were added to the reaction mixture and the aqueous layer was extracted with methylene chloride. The organic layer was washed with brine and dried over anhydrous magnesium sulfate. The insoluble material was removed by filtration and the filtrate was evaporated under reduced pressure.

The residue containing Compound (18) was dissolved in THF (2 mL) and was added DBU (44 mg) under ice-cooling. The mixture was stirred at room temperature for 50 minutes. Water and ethyl acetate were added to the mixture and the mixture was partitioned. The organic layer was washed with brine and dried over anhydrous magnesium sulfate. The insoluble material was removed by filtration and the filtrate was evaporated under reduced pressure. The residue was purified by silica gel chromatography to give Compound (19) (36 mg).
¹H-NMR(CDCl₃)δ:3.97(1H,t,J=9.0Hz),4.09(1H,t,J=9.0Hz),4.25(1H,t,J=9.0Hz),4.31-4.32(2H,m),5 .38(1H,d,J=0.8Hz),5.46(1H,s),7.15(1H,dd,J=11. 1,8.8Hz),7.40-7.46(2H,m),7.5-0.7.68(3H,m),8.05-8. 08(3H,m)

### Steps 10-1, 10-2 and 10-3

To a solution of Compound (19) (36 mg) in THF (1.5 mL) were added di-tert-butyldicarbonate (45 mg) and DMAP (11 mg) at room temperature. The mixture was stirred at room temperature for 1 hour and the evaporated at 40 °C under reduced pressure. The residue was roughly purified by silica gel chromatography.

After the obtained crude purified product (43 mg) containing Compound (20) was dissolved in THF (0.75 mL), methanol (0.75 mL) and water (0.45 mL) were added thereto. Potassium carbonate (61 mg) was addedto the mixture at room temperature, and the mixture was stirred at the same temperature for 2 hours. After the reaction mixture was warmed to 45 °C and stirred for 150 minutes, it was cooled to room temperature. To the reaction mixture was added potassium carbonate (61 mg), and the mixture was stirred at the same temperature for 1 hour. The reaction mixture was diluted with water and extracted with ethyl acetate. The organic layer was washed with brine and dried over anhydrous magnesium sulfate. The insoluble material was removed by filtration and the filtrate was evaporated under reduced pressure.

The obtained residue containing Compound (21) (35 mg) was dissolved in methylene chloride (1.5 mL). To the mixture was added trifluoroacetic acid (0.15 mL) under ice-cooling and the mixture was stirred at room temperature for 150 minutes. 2N Aqueous solution of sodium hydroxide (1 mL) and water were added to the reaction mixture. The aqueous layer was extracted with chloroform-methanol (9:1). The organic layer was washed with brine and dried over anhydrous magnesium sulfate. The insoluble material was removed by filtration and the filtrate was evaporated under reduced pressure. The residue was purified by silica gel chromatography to give Compound (1-2) (18 mg).
¹H-NMR(CDCl₃)δ:3.52(2H,s),3.74-3.89(2H,m),4.08(1H,d,J=8.1Hz),4.14(1H,dd,J=8.1,3.3Hz),4.37 (1H,dd,J=8.1,1.1Hz),5.16(1H,s),5.28(1H,s),6.51(1H,ddd,J=8.7,3.6,3.0Hz),6.61(1H,dd,J=6.6,3.0Hz ),6.83(1H,dd,J=11.7,8.7Hz)

### Step 11

To a solution of Compound (I-2) (18 mg) in methanol (0.75 mL) was added 2M hydrochloric acid (0.032 mL) and the mixture was stirred at room temperature for 10 minutes. To the mixture were added 5-cyanopicolinic acid hydrate (11 mg) and 1-ethyl -3-(3-dimethylaminopropyl)carbodiimide hydrochloride (13 mg) under ice-cooling. The mixture was stirred at the same temperature for 10 minutes, warmed to room temperature and stirred for 50 minutes. To the reaction mixture were added 5-cyanopicolinic acid hydrate (11 mg) and 1-ethyl -3-(3-dimethylaminopropyl)carbodlimide hydrochloride (13 mg) under ice-cooling. The mixture was warmed to room temperature and stirred for 50 minutes. 2N Aqueous solution of sodium hydroxide (0.032 mL) and 50% aqueous solution of potassium carbonate were added to the reaction mixture under ice-cooling. To the mixture were added water and ethyl acetate and the organic layer was partitioned. The organic layer was washed with brine and dried over anhydrous magnesium sulfate. The insoluble material was removed by filtration and the filtrate was evaporated under reduced pressure. Diethyl ether was added to the residue and the precipitated solid was collected by filtration. The obtained solid was washed with diethyl ether and ethyl acetate to give Compound (I-3) (13 mg).

### Example 3 Synthesis of Compound (I-17)

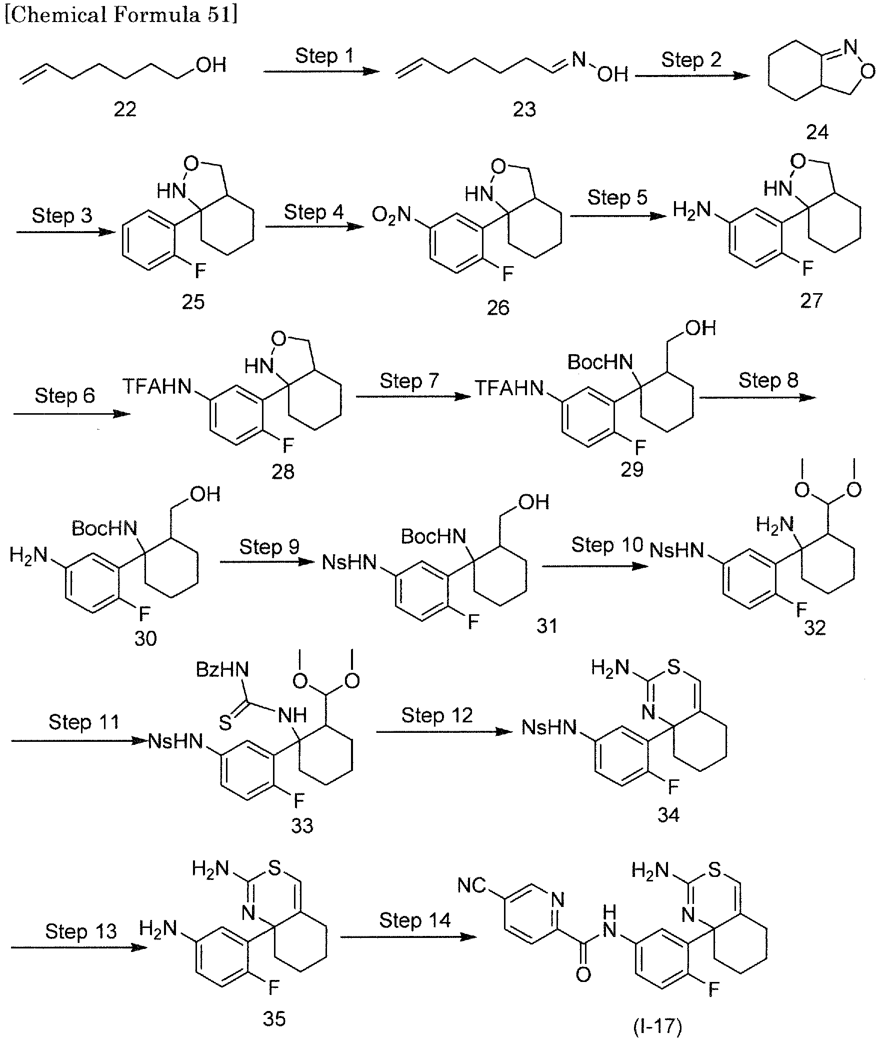

### Step 1

To a solution of Compound (22) (1.50 g) in ethyl acetate (15 ml) was added 2-iodoxy benzoic acid (4.78 g) at room temperature. The mixture was heated under reflux for 3 hours. After the reaction mixture was cooled to room temperature and filtered, the filtrate was evaporated under reduced pressure at room temperature. To a solution of the residue in a 50% aqueous ethanol solution (40 ml) were added hydroxylamine hydrochloride (1.37 g) and sodium acetate (1.94 g) at room temperature and the mixture was stirred for 90 minutes. The solvent was evaporated under reduced pressure and ethyl acetate was added to the residue. The organic layer was successively washed with water and brine, and dried over anhydrous magnesium sulfate. The extract was filtered and the filtrate was evaporated under reduced pressure to give Compound (23) (1.27 g).
MS ; m/z 128 [M⁺+H]
¹H-NMR (CDCl₃) δ: 1.40-1.55 (4H, m), 2.04-2.12 (2H, m), 2.17-2.24 (1H, m), 2.35-2.42 (1H, m), 4.92-5.04 (2H, m), 5.72-5.86 (1H, m), 6.71 and 7.41 (1H, each t, J = 5.5 Hz and J = 6.1 Hz), 7.19 and 7.52 (1H, each br s).

### Step 2

To a solution of Compound (23) (1.26 g) in methylene chloride (44 ml) was added a 5% aqueous solution of sodium hypochlorite (14.7 ml) at room temperature. The mixture was stirred at the same temperature for 30 minutes and the partitioned organic layer was dried over magnesium sulfate. The extract was filtered and the filtrate was evaporated under reduced pressure. The obtained residue was purified by chromatography to give Compound (24) (1.07 g).
MS; m/z 126 [M⁺+H]
¹H-NMR (CDCl₃) δ: 1.26-1.50 (3H, m), 1.81-1.92 (1H, m), 1.94-2.19 (3H, m), 2.76-2.82 (1H, m), 3.11-3.18 (1H, m), 3.77 (1H, dd, J = 10.2, 8.0 Hz), 4.49 (1H, dd, J = 10.2, 8.0 Hz).

### Step 3

To a solution of 2-bromofluorobenzene (2.98 g) in toluene/tetrahydrofuran (36 ml/9 ml) was dropwisely added n-butyllithium hexane solution (1.65 mol/L, 10.3 ml) at -70 °C under nitrogen atmosphere. The mixture was stirred at the same temperature for 30 minutes. Boron trifluoride-diethyl ether complex (1.08 ml) was dropwisely added the mixture and the mixture stirred for 25 minutes. A solution of Compound (24) (1.07 g) in toluene (54 ml) was dropwisely added to the mixture at the same temperature and the mixture was stirred for 75 minutes. To the mixture was added an aqueous solution of ammonium chloride and the aqueous layer was extracted with ethyl acetate. The obtained organic layer was washed with brine, dried over magnesium sulfate and filtered. The filtrate was evaporated under reduced pressure. The obtained residue was roughly purified by column chromatography and a solid was precipitated from diethyl ether-hexane solution to give Compound (25) (1.17 g).
MS ; m/z 222 [M⁻+H]
¹H-NMR (CDCl₃) δ: 1.31-1.60 (3H, m), 1.63-1.91 (4H, m), 2.19-2.29 (1H, m), 2.87-2.94 (1H, m), 3.55 (1H, br s), 3.66 (1H, d, J = 6.7 Hz), 5.96 (1H, s), 7.01 (1H, ddd, J = 12.3, 8.0, 1.3 Hz), 7.12 (1H, td, J = 7.5, 1.3 Hz), 7.19-7.26 (1H, m), 7.83 (1H, t, J = 7.8 Hz).

### Step 4

To a solution of Compound (25) (1.17 g) in concentrated sulfuric acid (7 ml) was dropwisely added fuming nitric acid (0.24 ml) under ice-cooling. The mixture was stirred at the same temperature for 1 hour. After an aqueous solution of sodium carbonate was added to the mixture and quenched, the mixture was extracted with chloroform. The obtained organic layer was dried over magnesium sulfate and filtered. The filtrate was evaporated under reduced pressure and ethyl acetate-diethyl ether-hexane solution was added to the residue. The precipitated solid was collected by filtration to give Compound (26) (1.29 g).
MS ; m/z 267 [M⁺ +H]
¹H-NMR (CDCl₃) δ: 1.31-1.59 (3H, m), 1.70-1.96 (4H, m), 2.11-2.22 (1H, m), 2.85-2.92 (1H, m), 3.51 (1H, dd, J = 7.0, 5.6 Hz), 3.68 (1H, d, J = 7.0 Hz), 6.00 (1H, s), 7.16 (1H, dd, J = 10.8, 8.9 Hz), 8.14 (1H, ddd, J = 8.9, 4.1, 3.0 Hz), 8.81 (1H, dd, J = 6.9, 3.0 Hz).

### Step 5

To a solution of Compound (26) (1.29 g) in ethanol (32 ml) were added iron (2.15 g) and an aqueous solution of ammonium chloride (8 mol/L, 7,84 ml) at room temperature. The mixture was stirred at 45 °C for 2 hours. The reaction mixture was filtered and the filtrate was washed with water and brine. The organic layer was dried over magnesium sulfate and the extract was filtered. The filtrate was evaporated under reduced pressure and diethyl ether-hexane solution was added to the residue. The precipitated solid was collected by filtration to give Compound (27) (989 mg).
MS ; m/z 237 [M⁺ +H]
¹H-NMR (CDCl₃) δ: 1.24-1.53 (3H, m), 1.67-1.90 (4H, m), 2.21 (1H, td, J = 13.8, 4.9 Hz), 2.84-2.91 (1H, m), 3.56-3.66 (4H, m), 5.93 (1H, br s), 6.50 (1H, ddd, J = 8.4, 3.4, 3.0 Hz), 6.80 (1H, dd, J = 11.7, 8.4 Hz), 7.16 (1H, dd, J = 6.8, 3.0 Hz).

### Step 6

To a solution of Compound (27) (1.08 g) in methanol (10 ml) were added ethyl trifluoroacetate (1.14 ml) and triethylamine (0.63 ml) at room temperature. The mixture was stirred at the same temperature for 4 hours and the solvent was evaporated under reduced pressure. The residue was dissolved in ethyl acetate, washed with water and brine, and dried over magnesium sulfate. The extract was filtered and the filtrate was evaporated under reduced pressure. The obtained residue was purified by column chromatography to give Compound (28) (1.41 g).
MS; m/z 333 [M⁺ +H]
¹ H-NMR (CDCl₃) δ: 1.30-1.56 (3H, m), 1.73-1.94 (4H, m), 2.21 (1H, td, J = 13.9, 4.8 Hz), 2.85-2.93 (1H, m), 3.52 (1H, t, J = 6.1 Hz), 3.67 (1H, d, J = 7.0 Hz), 5.99 (1H, s), 7.07 (1H, dd, J = 11.3. 8.8 Hz), 7.64 (1H, dd, J = 6.9, 2.9 Hz), 7.90-7.95 (2H, m).

### Step 7

To a solution of Compound (28) (1.41 g) in ethanol (14 ml) was added 5% Rh/C at room temperature. The mixture was stirred at the same temperature for 23 hours under hydrogen atmosphere. The reaction mixture was filtered through Celite and the obtained filtrate was evaporated under reduced pressure. The obtained residue was dissolved in tetrahydrofuran (10 ml) and was added di-tert-butyl dicarbonate at room temperature. The mixture was stirred at 70 °C for 14 hours and the solvent was evaporated under reduced pressure. The obtained residue was purified by column chromatography to give Compound (29) (1.23 g).
MS ; m/z 435 [M⁺ +H]
¹H-NMR (CDCl₃) δ: 1.35-1.64 (13H, m), 1.83-2.02 (3H, m), 2.14-2.19 (1H, m), 2.79-2.89 (1H, m), 3.42-3.54 (2H, m), 6.34 (1H, br s), 7.03 (1H, dd, J = 12.0, 8.8 Hz), 7.44-7.47 (1H, m), 7.54-7.60 (1H, m), 8.04 (1H, br s).

### Step 8

To a solution of Compound (29) (1.23 g) in tetrahydrofuran/methanol/water (6 ml/8 ml/8 ml) was added potassium carbonate (938 mg) at room temperature. The mixture was stirred at 50 °C for 5.5 hours and ethyl acetate and water were added to the mixture. The organic layer was partitioned, washed with brine and dried over magnesium sulfate. The extract was filtered and the filtrate was evaporated under reduced pressure. The obtained residue was purified by column chromatography to give Compound (30) (791 mg).
MS; m/z 339 [M⁺ +H]
¹H-NMR (CDCl₃) δ: 1.36-2.13 (19H, m), 2.90 (1H, d, J = 13.4 Hz), 3.43-3.53 (2H, m), 6.35 (1H, br s), 6.50 (1H, dt, J = 8.5, 3.0 Hz), 6.68 (1H, dd, J = 6.8, 3.0 Hz), 6.78 (1H, dd, J = 12.4, 8.5 Hz).

### Step 9

To a solution of Compound (30) (789 mg) in tetrahydrofuran/water (12 ml/2.4 ml) were added 2-nitrobenzenesulfonyl chloride (1.03 g) and triethyl amine (0.97 ml) under ice-cooling. The mixture was stirred at room temperature for 4.5 hours. To the reaction mixture were added a saturated aqueous sodium bicarbonate solution and ethyl acetate. The organic layer was partitioned, washed with brine and dried over magnesium sulfate. The extract was filtered and the filtrate was evaporated under reduced pressure. To the residue was added methylene chloride and the precipitated solid was collected by filtration to give Compound (31) (1.02 g). MS ; m/z 524 [M⁺ +H]
¹H-NMR (CDCl₃) δ: 1.23-1.55 (13H, m), 1.84-2.06 (4H, m), 2.80 (1H, d, J = 12.7 Hz), 3.18-3.24 (1H, m), 3.39 (1H, td, J = 7.4, 3.7 Hz), 6.27 (1H, br s), 6.89 (1H, dd, J = 11.9, 8.5 Hz), 7.05 (1H, dt, J = 8.5, 3.0 Hz), 7.17 (1H, s), 7.28 (1H, dd, J = 7.0, 2.7 Hz), 7.58 (1H, td, J = 7.7, 1.2 Hz), 7.67 (1H, td, J = 7.7, 1.2 Hz), 7.83 (2H, t, J = 7.8 Hz).

### Step 10

To a solution of Compound (31) (1.02 g) in ethyl acetate (10 ml) was added 2-iodoxybenzoic acid (761 mg) at room temperature. The mixture was heated under reflux for 6 hours. After the reaction mixture was cooled to room temperature and filtered, the filtrate was evaporated under reduced pressure at room temperature. To the residue was added 2 mol/L hydrochloric acid-methanol solution at room temperature and the mixture was stirred at the same temperature for 2 hours. After standing overnight, an aqueous solution of sodium carbonate and ethyl acetate were added to the reaction solution. The organic layer was partitioned, washed with brine and dried over magnesium sulfate. The extract was filtered and the filtrate was evaporated under reduced pressure. The obtained residue was purified by column chromatography to give Compound (32) (875 mg).
MS m/z 468 [M⁺ +H]
¹H-NMR (CDCl₃) δ: 1.27-1.41 (2H, m), 1.49-1.64 (3H, m), 1.70-1.85 (2H, m), 2.03-2.13 (1H, m), 2.47 (1H, dt, J = 12.0, 3.2 Hz), 3.05 (3H, s), 3.14 (3H, s), 3.62 (1H, d, J = 2.7 Hz), 6.91 (1H, dd, J = 11.9, 8.6 Hz), 7.12 (1H, ddd, J = 8.6, 3.9, 2.9 Hz), 7.57 (1H, td, J = 7.6, 1.2 Hz), 7.65-7.72 (2H, m), 7.84 (2H, ddd, J = 11.7, 7.9, 1.2 Hz).

### Step 11

To a solution of Compound (32) (872 g) in acetone (8.7 ml) was added benzoyl isothiocyanate (342 mg) under ice-cooling. The mixture was stirred at room temperature for 50 minutes. The solvent was evaporated under reduced pressure and the obtained residue was roughly purified by column chromatography. Diethyl ether-hexane solution was added to the oil and the precipitated solid was collected by filtration to give Compound (33) (1.06 g).
MS ; m/z 629 [M⁺ -H]

### Step 12

Compound (33) (500 mg) was dissolved in concentrated sulfuric acid (6.34 ml) at room temperature and the solution was stirred at the same temperature for 15.5 hours. To the mixture was added an aqueous solution of sodium carbonate under ice-cooling and extracted with ethyl acetate. The organic layer was partitioned, washed with brine and dried over magnesium sulfate. The extract was filtered and the filtrate was evaporated under reduced pressure. The obtained residue was purified by column chromatography to give Compound (34) (170 mg).
MS; m/z 463 [M⁺ +H]
¹H-NMR (CDCl₃) δ: 1.19-1.48 (2H, m), 1.61-1.69 (2H, m), 1.81-1.90 (1H, m), 2.06-2.18 (1H, m), 2.32-2.39 (1H, m), 2.56-2.64 (1H, m), 6.04 (1H, d, J = 1.7 Hz), 6.89-6.98 (1H, m), 7.01-7.08 (2H, m), 7.55 (1H, td, J = 7.7, 1.3 Hz), 7.65-7.75 (2H, m), 7.83 (1H, dd, J = 7.9, 1.3 Hz).

### Step 13

To a solution of Compound (34) (365 mg) in N,N-dimethylacetamide were added triethylamine (1.09 ml) and 4-chlorobenzenethiol (571 mg) at room temperature. The mixture was stirred at 60 °C for 2 hours. After the mixture was acidified with 1 mol/L aqueous hydrochloric acid, it was washed with ethyl acetate. The aqueous layer was alkalinized with an aqueous solution of sodium carbonate and extracted with chloroform-methanol (9:1) solution. The partitioned organic layer was dried over magnesium sulfate. The extract was filtered and the filtrate was evaporated under reduced pressure. The obtained residue was purified by column chromatography to give Compound (35) (146 mg).
MS ; m/z 278 [M⁺ +H]
¹H-NMR (CDCl₃) δ: 1.37-1.61 (2H, m), 1.69-1.76 (2H, m), 1.87-1.96 (1H, m), 2.27-2.42 (2H, m), 2.71-2.78 (1H, m), 3.52 (2H, br s), 6.00 (1H, d, J = 1.2 Hz), 6.52 (1H, ddd, J = 8.5, 3.3, 2.9 Hz), 6.61 (1H, dd, J = 6.6, 2.9 Hz), 6.83 (1H, dd, J = 11.9, 8.5 Hz).

### Step 14

To a solution of Compound (35) (42 mg) in methanol (1 ml) was added an aqueous hydrochloric acid (2 mol/1.76 µl) at room temperature. The mixture was stirred at the same temperature for 10 minutes. To the mixture were added 5-methoxypyrazine-2-carboxylic acid (27.7 mg), 1-ethyl-3-(3-dimet.hylaminopropyl)carbodiimide hydrochloride (31.9 mg) under ice-cooling and the mixture was stirred at room temperature for 50 minutes. The mixture was alkalinized with an aqueous solution of sodium hydroxide (2 mol/L, 76 µL) and 50% aqueous solution of potassium carbonate, and extracted with ethyl acetate. The organic layer was washed with water and brine and dried over magnesium sulfate. The extract was filtered and the filtrate was evaporated under reduced pressure. The obtained residue was roughly purified by chromatography and diethyl ether was added to the oil. The precipitated solid was collected by filtration to give Compound (I-17) (34 mg).

### Example 4 Synthesis of Compound (I-40)

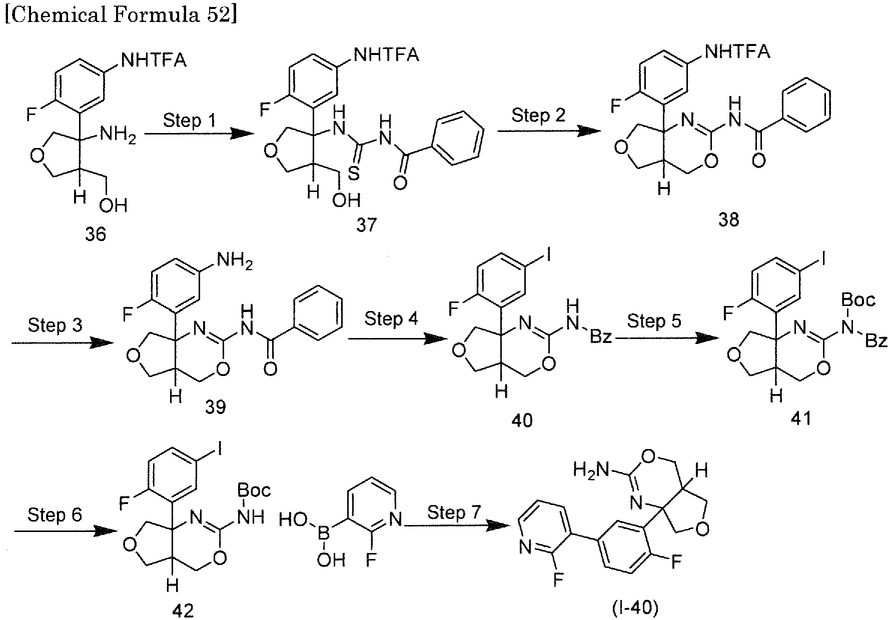

### Step 1

To a solution of Compound (36) (4.03 g) in acetone (30 ml) was added benzoyl isothiocyanate (1.85 ml) with stirring in an ice bath. The mixture was warmed to room temperature and stirred for 3 hours. The solvent was evaporated under reduced pressure and the obtained residue was roughly purified by chromatography to give Compound (37) (6.07 g). MS ; m/z 486 [M⁺ +H]

### Step 2

To a solution of Compound (37) (6.07 g) in dichloromethane (60 ml) was gradually added meta-chloroperoxybenzoic acid (7.09 g) with stirring in an ice bath. The mixture was warmed to room temperature and stirred for 3 hours. To the reaction mixture was added a saturated aqueous sodium bicarbonate solution and the mixture was extracted with ethyl acetate. The organic layer was washed with a saturated aqueous sodium bicarbonate solution and brine, and dried over sodium sulfate. The solvent was evaporated under reduced pressure and the obtained residue was purified by chromatography to give Compound (38) (3.61 g).
My ; m/z 452 [M⁺ +H]
¹H-NMR (CDCl₃) δ: 3.91-4.27 (7H, m), 7.12 (1H, t, J = 10.0 Hz), 7.33 (2H, t, J = 7.2 Hz), 7.44 (1H, t, J = 6.4 Hz), 7.72 (1H, d, J = 5.2 Hz), 7.95 (2H, s), 8.06 (3H, d, J = 7.2 Hz), 10.04 (1H, s).

### Step 3

To a solution of Comopund (38) (2.6 g) in tetrahydrofuran (10 ml)-methanol (10 ml)-water (10 ml) was added potassium carbonate (2.37 g) at room temperature. The mixture was stirred at 40 °C for 23 hours. Water was added to the mixture and extracted with ethyl acetate. The organic layer was washed with water and brine, and dried over sodium sulfate. The solvent was evaporated under reduced pressure to give Compound (39) (1.77 g).
MS ; m/z 356 [M⁺ +H]
¹H-NMR (DMSO-d₆) δ: 3.86-4.17 (5H, m), 4.32 (1H, d, J = 11.1 Hz), 4.46 (1H, d, J = 11.1 Hz), 5.16 (2H, s), 6.58 (1H, s), 6.75 (1H, s), 6.98 (1H, t, J = 10.4 Hz), 7.45 (2H, t, J = 7.2 Hz), 7.53 (1H, t, J = 7.2 Hz), 8.09 (2H, d, J = 8.0 Hz), 11.46 (1H, s).

### Step 4

To a solution of Compound (39) (1.77 g) in concentrated hydrochloric acid (17 ml) was dropwisely added an aqueous solution (7.5 ml) of sodium nitrite (687 mg) with stirring in an ice bath. The mixture was warmed to room temperature and stirred at the same temperature for 1 hour. The reaction mixture was gradually added to a solution of potassium iodide (4.1 g) in water (10 ml) in an ice bath. The mixture was warmed to room temperature and stirred for 2 hours. Water was added to the mixture and extracted with ethyl acetate. The organic layer was washed with 10% sodium thiosulfate and brine, and dried over sodium sulfate. The solvent was evaporated under reduced pressure and the residue was purified by chromatography to give Compound (40) (1.28 g).
MS ; m/z 465 [M⁺ +H]
¹H-NMR (CDCl₃) δ: 3.25 (1H, s), 4.08-4.14 (3H, m), 4.23-4.32 (3H, m), 6.92 (1H, t, J = 10.0 Hz), 7.39-7.50 (3H, m), 7.69 (1H, s), 7.80 (1H, d, J = 6.4 Hz), 8.21 (2H, d, J = 6.8 Hz), 11.81 (1H, s).

### Step 5

To a solution of Compound (40) (1.28 g) in tetrahydrofuran (15 ml) were added di-tert-butyl dicarbonate (0.89 ml) and dimethylamino pyridine (402 mg). The mixture was stirred at room temperature for 5 hours. Water was added to the mixture and extracted with ethyl acetate. The organic layer was washed with water and brine and dried over sodium sulfate. The solvent was evaporated under reduced pressure to give Comopund (41) (1.74 g) as a crude product.
MS ; m/z 567 [M⁺+H]

### Step 6

To a solution of Compound (41) (1.55 g) in tetrahydrofuran (5 ml), methanol (5 ml) and water (1 ml) was added potassium carbonate (1.14 g). The mixture was stirred at room temperature for 17 hours. To the mixture was added a saturated aqueous sodium bicarbonate solution and the mixture was extracted with ethyl acetate. The organic layer was washed with water and brine and dried over sodium sulfate. The solvent was evaporated under reduced pressure and ethyl acetate was added to the residue. The precipitated solid was collected to give Compound (42) (972 mg).
MS ; m/z 463 [M⁺ +H]

### Step 7

Compound (42) (80 mg), aryl boronic acid (1.5 equivalent) and dichlorobis(triphenylphosphine)palladium (14 mg) were dissolved in dimethylformamide (0.5 ml). To a solution was added a 2 mol/L aqueous solution of sodium carbonate (260 ml) and stirred at 100 °C for 6 hours under nitrogen atmosphere. Water was added to the mixture and extracted with ethyl acetate. The organic layer was washed with water and brine and dried over sodium sulfate. The solvent was evaporated under reduced pressure and the obtained residue was purified by reversed phase chromatography to give Compound (I-40) (27 mg).

### Example 5 Synthesis of Compound (I-35)

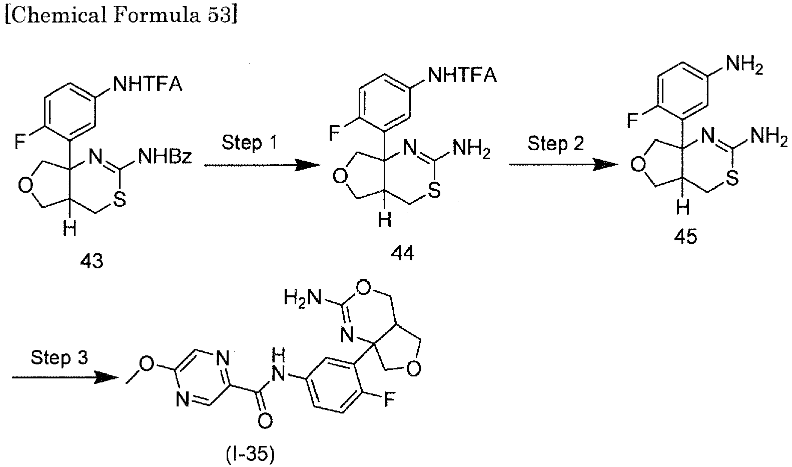

### Step 1

A solution of Compound (43) (1.80 g) in concentrated sulfuric acid (1 ml) was stirred at 60 °C for 1.5 hours. The reaction mixture was poured into ice water and neutralized with a 2 mol/L aqueous solution of sodium hydroxide. The aqueous layer was extracted with ethyl acetate. The organic layer was washed with a saturated aqueous sodium bicarbonate solution and brine, and dried over sodium sulfate. The solvent was evaporated under reduced pressure to give Compound (44) (1.39 g).
MS ; m/z 364 [M⁺ +H]
¹H-NMR (CDCl₃) δ: 1.26 (1H, t, J = 7.2 Hz), 3.01-3.07 (2H, m), 3.79 (1H, dd, J = 2.3, 8.2 Hz), 4.10-4.15 (2H, m), 4.41 (1H, dd, J = 1.8, 8.7 Hz), 7.10 (1H, dd, J = 11.7, 9.0 Hz), 7.44-7.48 (1H, m), 7.74-7.77 (1H, m).

### Step 2

To a solution of Compound (44) (1.39 g) in methanol (15 ml), tetrahydrofuran (5 ml) and water (5 ml) was added potassium carbonate (1.57 g) and the mixture was stirred at 40 °C for 17 hours. To the mixture was added a saturated aqueous sodium bicarbonate solution and the mixture was extracted with chloroform-methanol (9:1) solution. The organic layer was washed with brine and dried over sodium sulfate. The solvent was evaporated under reduced pressure to give Compound (45) (923 g).
MS ; m/z 268 [M⁺ +H]
¹H-NMR (CDCl₃) δ: 2.79 (1H, dd, J = 14.2, 6.0 Hz), 3.04-3.11 (2H, m), 3.59 (2H, s), 3.77 (1H, dd, J = 8.5, 2.2 Hz), 4.10 (2H, dt, J = 17.0, 6.8 Hz), 4.44 (1H, dd, J = 8.4, 1.4 Hz), 6.50-6.55 (1H, m), 6.75-6.87 (2H, m).

### Step 3

To a solution of Compound (45) (50 mg) in methanol (1 ml) was added 2 mol/L hydrochloric acid (0.099 ml) and the mixture was stirred at room temperature for 15 minutes. Carboxylic acid (34 mg) and WSCD hydrochloride were added to the reaction mixture and the mixture was stirred at the same temperature for 1.5 hours. A 10% aqueous sodium bicarbonate solution was added to the mixture and the precipitated solid was collected by filtration. The solid was washed with water and heat-dried to give Compound (I-35) (34 mg).

The following compounds are prepared according to the above examples. In the tables, RT represents a retention time (minutes).

**[Table 1-1]**

| Compound No. | Structure | NMR (solvent: shift value ascending order) | MS [M+1] | LC/MS RT | LC/MS Method |
|---|---|---|---|---|---|
| I-1 | 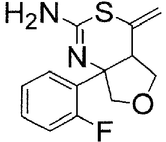 | ¹H-NMR (CDCl₃) δ: 3.76 (m, 1H), 3.86 (dd, *J* = 9.6, 7.8 Hz, 1H), 4.11 (t, *J* = 8.2 Hz, 1H), 4.25 (dd, *J* = 8.2, 3.6 1H), 4.38 (dd, *J* = 8.2, 1.1, 1H), 5.18 (s, 1H), 5.30 (m, 1H), 7.00-7.11 (m, 2H), 7.25 (m, 1H), 7.33 (td, *J* = 7.9. 1.8, 1H). | 265 | 1.13 | B |
| I-2 | 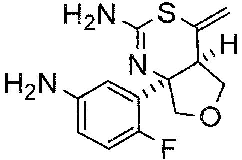 | ¹H-NMR (CDCl₃) δ: 3.52 (2H, s), 3.74-3.89 (2H, m), 4.08 (1H, d, *J* = 8.1 Hz), 4.14 (1H, dd, *J* = 8.1, 3.3 Hz), 4.37 (1H, dd, *J* = 8 1, 1.1 Hz), 5.16 (1H, s), 5.28 (1H, s), 6.51 (1H, ddd, *J* = 8.7, 3.6, 3.0 Hz), 6.61 (1H, dd, *J* = 6.6, 3 0 Hz), 6.83 (1H, dd, *J* = 11.7, 8.7 Hz). | | | |
| I-3 | 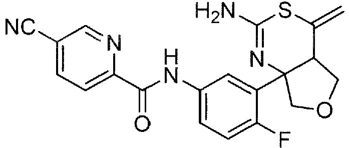 | 1H-NMR (CDCl3) δ: 3.86 (2H, td, J = 17 0, 8 9 Hz), 4.12 (1H, d, J = 8.2 Hz), 4.18 (1H, dd, J = 8.0, 3.3 Hz), 4.40 (1H, dd, J = 8.1, 1 2 Hz), 4 56 (2H, br s), 5 20 (1H, s), 5.33 (1H, s), 7.09 (1H, dd, J = 11.5, 8.8 Hz), 7.55 (1H, dd, J = 6.9, 2 7 Hz), 7.83 (1H, ddd, J = 8.8, 4.0, 2.8 Hz), 8.19 (1H, dd, J = 8.2, 2.0 Hz), 8.41 (1H, dd, J = 8.2, 0.8 Hz), 8.88 (1H, dd, J = 2.0, 0.9 Hz), 9.79 (1H, s). | 410 | 1 06 | A |

**[Table 1-2]**

| Compound No | Structure | Compound No | Structure |
|---|---|---|---|
| I-4 | 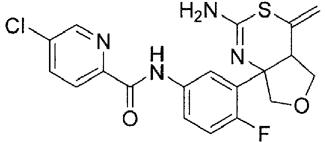 | I-10 | 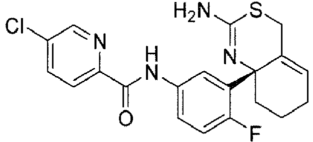 |
| I-5 | 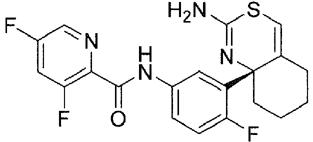 | I-11 | 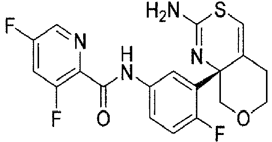 |
| I-6 | 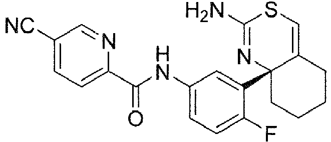 | I-12 | 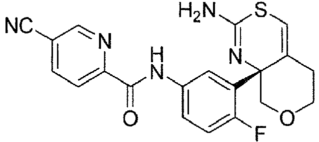 |
| I-7 | 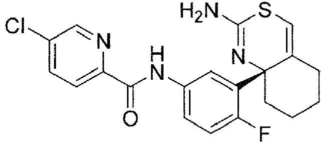 | I-13 | 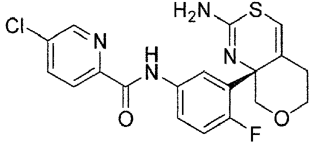 |
| I-8 | 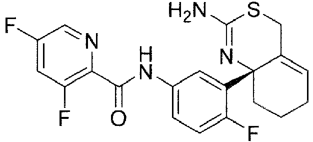 | I-14 | 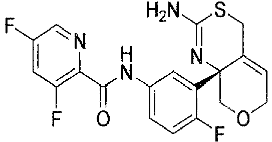 |
| I-9 | 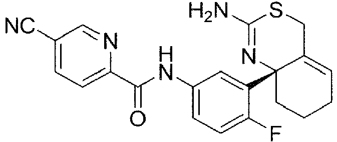 | I-15 | 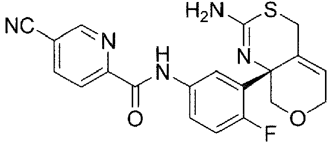 |

**[Table 1-3]**

| No | Structure | No. | Structure |
|---|---|---|---|
| I-16 | 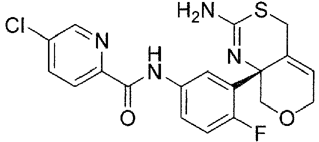 | I-22 | 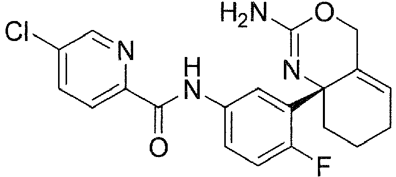 |
| I-17 | 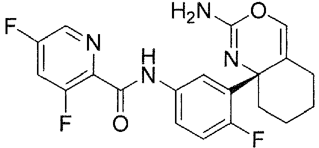 | I-23 | 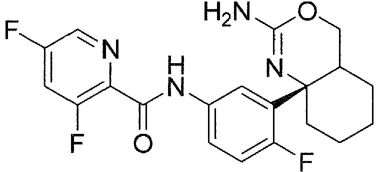 |
| I-18 | 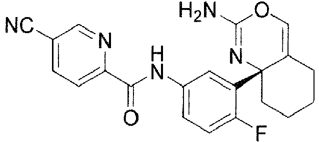 | I-24 | 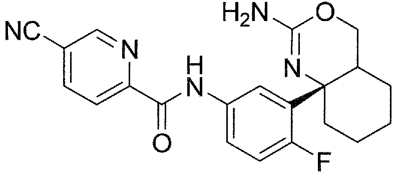 |
| 1-19 | 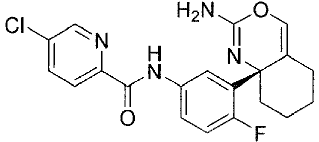 | I-25 | 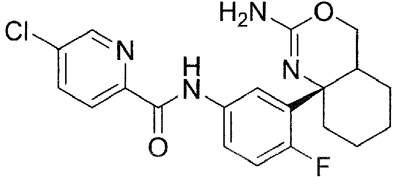 |
| I-20 | 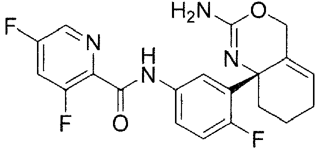 | I-26 | 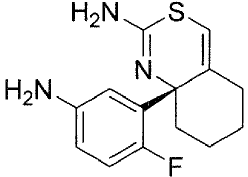 |
| I-21 | 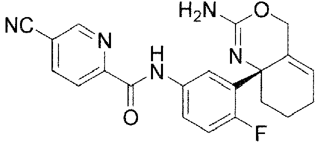 | I-27 | 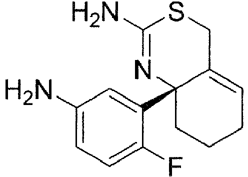 |

**[Table 1-4]**

| No | Structure | No. | Structure |
|---|---|---|---|
| I-28 | 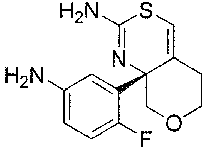 | I-31 | 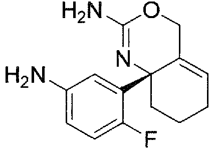 |
| I-29 | 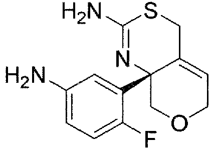 | I-32 | 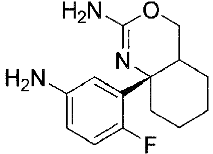 |
| I-30 | 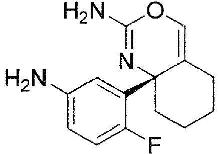 | | |

**[Table 1-5]**

| Compound No | Structure | NMR(solvent shift value ascending order) | MS [M+1] | LC/MS RT | LC/MS Method |
|---|---|---|---|---|---|
| I-33 | 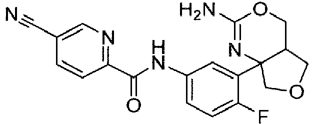 | 1H-NMR (DMSO-d6) δ 3 21 (1H, dd, J = 5 2, 20 Hz), 3 88-4 25 (5H, m), 4.35-4 60 (2H, m), 7 38 (1H, t, J = 103Hz), 8.08-820 (2H, m), 8 35 (1H, d, J = 8.2 Hz), 8 65 (1H, dt, J = 8.2, 2 1 Hz), 9.26 (1H, s), 11 04 (1H, s). | 382 | 136 | B |
| I-34 | 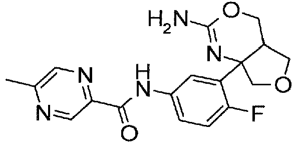 | 1H-NMR (CDCl3) δ 0.85 (2H, s), 2 69 (3H, s), 3 07 (1H, s), 3.78 (1H, d, J = 7.2 Hz), 3.95-4 05 (4H, m), 4.22-4 32 (3H, m), 7 09 (1H, t, J = 9 6 Hz), 7.75 (1H, d, J = 6.4 Hz), 8 01 (1H, s), 8 43 (1H, s), 9 35 (1H, s), 9 68 (1H, s) | 372 | 084 | C |
| I-35 | 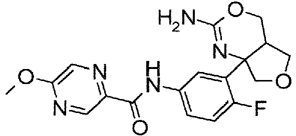 | 1H-NMR (DMSO-d6) δ: 2.88 (1H, s). 3.66 (1H, d, J = 6 4 Hz), 3.79-3 87 (2H, m), 4 02-4.06 (6H, m), 553 (2H, s), 7.15 (1H, t, J=104Hz), 779 (1H, s), 806 (1H, d, J = 6.0 Hz), 840 (1H, s), 8.88 (1H, s), 10 52 (1H, s) | 388 | 0.91 | A |
| I-36 | 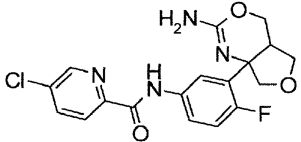 | 1H-NMR (DMSO-d6) δ 2.88 (1H, s), 3 66 (1H, d, J = 60 Hz), 3.79-3 88 (2H, m), 4 00-4 06 (3H, m), 5 56 (2H, s), 7 15 (1H, t, J = 10.4 Hz), 7 82 (1H, s), 8.06 (1H, d, J = 4 4 Hz). 8 17 (2H, dd, J = 8 4, 14 0 Hz), 8.77 (1H, s), 10 68 (1H, s) | 391 | 1.05 | A |
| I-37 | 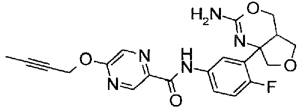 | 1H-NMR (DMSO-d6) δ 1 85 (3H, s), 2 88 (1H, s), 3 66 (1H, d, J = 5 8 Hz), 3 79-3 88 (2H, m), 3 99-4 08 (3H, m), 5 08 (2H, s), 5 53 (2H, s), 7 15 (1H, t, J = 10 2 Hz), 7 78 (1H, s), 807 (1H, d, J = 54Hz), 843 (1H, s), 8 88 (1H, s), 10 54 (1H, s) | 426 | 1 16 | A |
| I-38 | 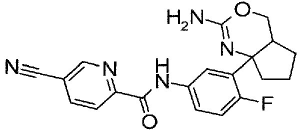 | 1H-NMR (DMSO-d6) δ 170 (5H, s), 1 94-199 (1H, m), 2 31 (1H, s), 3 69 (1H, d, J = 10 4 Hz), 3 89 (1H, d, J = 8 8 Hz), 5.40 (2H, s), 7 13 (1H, s), 7 77 (1H, s), 7 97 (1H, s), 8 28 (1H, d, J = 72 Hz), 8 59 (1H, d, J = 72 Hz), 9 20 (1H, s) 10 79 (1H, s) | 380 | 11 | A |

**[Table 1-6]**

| Compound No | Structure | NMR(solvent shift value ascending order) | MS [M+1] | LC/MS RT | LC/MS Method |
|---|---|---|---|---|---|
| I-39 | 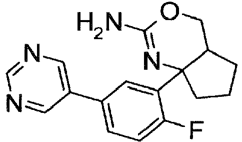 | 1H-NMR (DMSO-d6) δ 1 72-1 88 (5H, m), 2 05 (1H, br s), 2 32-2 39 (1H, m), 2.73 (1H, s), 391 (1H, d, J=104Hz), 406 (1H, d, J= 10 4 Hz). 7 36 (1H, t, J = 10.4 Hz), 7 75 (1H, s), 7.87 (1H, d, J = 6 8 Hz), 9.07 (2H, s), 9 19 (1H,s) | 313 | 0.84 | D |
| I-40 | 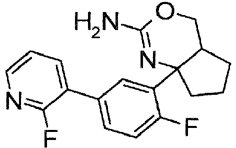 | 1H-NMR (DMSO-d6) δ:1 69-1.79 (2H, m), 1 91 (1H, s), 2.14 (2H, br s), 2 99 (1H, s), 4 27 (1H, d, J = 12 8 Hz), 4.41 (1H, d, J = 120 Hz) 7.41-7 51 (2H, m). 7 68 (2H, s), 8.28-8 20 (2H, m) | 330 | 1 12 | D |
| I-41 | 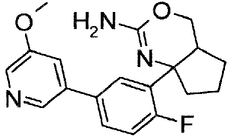 | 1H-NMR (DMSO-d6) δ: 1.72 (4H, s), 1 98 (1H, d, J = 7 6 Hz), 2 33 (1H d, J = 7.6 Hz), 2 59 (1H, s), 3 70 (1H, d, J = 10.8 Hz), 3 90 (4H, s), 5 58 (2H, s), 7 26 (1H, t, J = 10 0 Hz), 7 50 (1H, s), 7 63 (1H, br s), 7 87 (1H, d, J = 8.0 Hz), 8.28 (1H, s), 8 38 (1H, s). | 342 | 0.9 | D |
| I-42 | 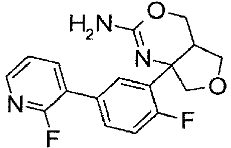 | 1H-NMR (DMSO-d6) δ 2 96 (1H, s), 3.67 (1H, d, J = 7 2 Hz), 3.84 (2H, d, J = 9 2 Hz), 4 01 (1H, d, J = 11 2 Hz), 4 11 (2H, s), 5 70 (2H, s), 7 33 (1H, t, J = 10.2 Hz), 7 48 (1H, t, J = 5 2 Hz), 7 57 (1H, s), 7.89 (1H, d, J = 7 2 Hz), 8 07 (1H, t, J = 8 4 Hz), 8 26 (1H, d, J = 4 4 Hz) | 332 | 0.82 | A |
| I-43 | 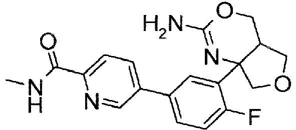 | 1H-NMR (DMSO-d6) δ 2 85 (3H, d, J = 3 6 Hz), 2 97 (1H, s), 3 66 (1H, d, J = 6.8 Hz), 3 86 (2H, d, J = 10 4 Hz), 4 00 (1H, d, J = 10 4 Hz), 4 10 (2H, s), 5 67 (2H, s), 7 34 (1H, t, J = 9 6 Hz) 7 74 (1H, s), 7 99 (1H, d, J = 5 6 Hz), 8 10 (1H, d, J = 7 8 Hz), 8 18 (1H, d, J = 8 .4 Hz), 8 79 (1H, s), 8 85 (1H, s) | 371 | 0.76 | A |
| I-44 | 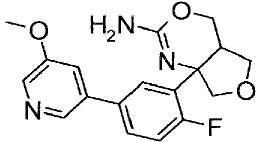 | 1H-NMR (DMSO-d6) δ 2 97 (1H, s), 3 67 (1H, d. J = 7 6 Hz), 3 82-3 91 (5H, m), 4 00 (1H, d J = 11 2 Hz), 4 10 (2H, s), 5 69 (2H, s), 7 31 (1H, t, J = 10 0 Hz), 7 53 (1H, s), 7 69 (1H, s), 7 94 (1H, d, J = 6 8 Hz), 8 30 (1H, s), 8 41 (1H, s) | 344 | 0 59 | A |

**[Table 1-7]**

| Compound No | Structure | NMR(solvent shift value ascending order) | MS [M+1] | LC/MS RT | LC/MS Method |
|---|---|---|---|---|---|
| I-45 | 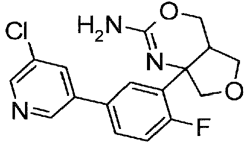 | 1H-NMR (DMSO-d6) δ 2 97 (1H s), 3 67 (1H, d, J = 6 4 Hz), 3 82-3.90 (2H, m), 4 00 (1H, d, J = 10 0 Hz), 4 09 (2H, d, J = 6.4 Hz), 5.66 (2H, s), 7 33 (1H, t, J = 10.0 Hz), 7 71 (1H, s), 7 95 (1H, d, J = 6 8 Hz), 8.16 (1H, s), 8 62 (1H, s), 8 79 (1H, s) | 348 | 0 96 | A |
| I-46 | 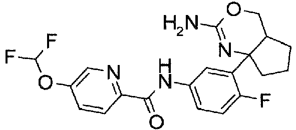 | 1H-NMR (DMSO-d6) δ 1 70 (4H, s), 1 96 (1H, s), 2 30 (1H, s), 3 70 (1H, d, J = 10 0 Hz), 3 89 (1H, d, J = 10 8 Hz), 5 45 (2H, s), 7.11 (1H, t, J = 10.0 Hz), 7 48 (1H, t, J = 73.0 Hz) 7.76 (1H, s), 7.92 (2H, dd, J = 22 2, 7 8 Hz), 8 22 (1H, d, J = 8 6 Hz), 8 61 (1H, s), 10 57 (1H, s) | 441 | 1 93 | D |
| I-47 | 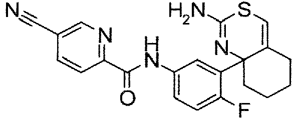 | 1H-NMR (CDCl3) δ 1 40-1 61 (2H, m), 1 71-1 80 (2H, br m), 1 91-2 00 (1H, m), 2 33-2.51 (2H, m), 2 75-2 83 (1H, m), 6 14 (1H, d, *J* = 12 Hz), 7.07 (1H, dd, *J* = 11 4, 9.6 Hz), 7 65 (2H, dd, *J* = 6 3, 2.9 Hz), 8 19 (1H, dd, *J* = 8.1, 2 1 Hz), 8 42 (1H, d, *J* = 8 1 Hz), 8 89 (1H, d, *J* = 1 4 Hz), 9.78 (1H, s) | 408 | 1.3 | D |

The effect of the present compound is confirmed by the following test Examples.

### (Test Example 1: Assay of BACE 1 inhibiting activity)

48.5 µL of substrate peptide solution (Biotin-XSEVNLDAEFRHDSGC-Eu: X=ε-amino-n-capronic acid, Eu=Europium cryptate) was added to each well of 96-hole half-area plate (a black plate: Costar), and after addition of 0.5 µl of the test compound (dissolved in N,N'-dimethylformaldehyde) and 1 µl of Recombinant human BACE1(R&D Systems), the reaction mixture was incubated at 30°C for 3 hours. The substrate peptide was synthesized by reacting Cryptate TBPCOOH mono SMP (CIS bio international) with Biotin-XSEVNLDAEFRHDSGC (Peptide Institute, Inc.). The final concentrations of the substrate peptide and Recombinant human BACE1 were adjusted to 18 nmol/ L and 7.4 nmol/ L, respectively, and the reaction was performed in sodium acetate buffer (50 mmol/L sodium acetate, pH 5.0, 0.008% Triton X-100).
After the incubation for reaction, 50 µl of 8.0 µg/ml Streptavidin-XL665 (CIS bio international) dissolved in phosphate buffer (150 mmol/L K₂ HPO₄-KH₂ PO₄, pH 7.0, 0.008 % Triton X-100, 0.8 mol/L KF) was added to each well and left stand at 30°C for an hour. After then, fluorescence intensity was measured (excitation wavelength: 320 nm, measuring wavelength: 620 nm and 665 nm) using Wallac 1420 multilabel counter (Perkin Elmer life sciences). Enzymatic activity was determined from counting ratio of each wavelength (10,000 x Count 665/ Count 620) and 50% inhibitory concentration against the enzymatic activity was calculated.

### Result

Compound I-3: 0.029 µM
Compound I-37: 0.027 µM
In addition, Compounds I-33, 35, 36 and 38 showed its IC₅₀ value of 1 µM or less.

### (Test Example 2: Measurement of β-amyloid (Aβ) production inhibitory effect in cell)

Neuroblastoma SH-SY5Y cells (SH/APPwt) with human wild-type β-APP excessively expressed therein were prepared at 8 X 105 cells/mL, and 150 µl portions thereof were inoculated into each well of a 96-well culture plate (Falcon). The cells were cultured for 2 hours at 37 °C in a 5% gaseous carbon dioxide incubator. Then, a solution which had been preliminarily prepared by adding and suspending the test compound (DMSO (dimethyl sulfoxide) solution) so as to be 2 µl/50 µl medium was added to the cell sap. Namely, the final DMSO concentration was 1%, and the amount of the cell culture was 200 µl. After the incubation was performed for 24 hours from the addition of the test compound, 100 µl of the culture supernatant was collected from each fraction. The amount of the Aβ in each fraction was measured.

The Aβ amount was measured as follows. 10 µl of a homogeneous time resolved fluorescence (HTRF) measurement reagent (Amyloid β 1-40 peptide; IBA Molecular Holding, S.A.) and 10 µl of the culture supernatant were put into a 384-well half area microplate (black microplate, Costar) and mixed with each other, and then left standing overnight at 4 °C while the light was shielded. Then, the fluorescence intensity (excitation wavelength: 337 nm, measurement wavelength: 620 nm and 665 nm) was measured with a Wallac 1420 multilabel counter (Perkin Elmer life sciences). The Aβ amount was determined from the count rate at each measurement wavelength (10000 X Count 665/Count 620), and the amount needed to inhibit Aβ production by 50 % (IC₅₀) was calculated from at least six different dosages.

### Result

Compound I-37 : 0.001 µM
In addition, Compounds I-3, 33 to 36, and 38 to 46 showed its IC₅₀ value of 1 µM or less.

### (Test Example 3: Lowering effect on brain β amyloid in rats)

A test compound is suspended in 0.5% methylcellulose, the final concentration is adjusted to 2 mg/mL, and this is orally administered to male Crj:SD rat (7 to 9 weeks old) at 10 mg/kg. In a vehicle control group, only 0.5% methylcellulose is administered, and an administration test is performed at 3 to 8 animals per group. A brain is isolated 3 hours after administration, a cerebral hemisphere is isolated, a weight thereof is measured, the hemisphere is rapidly frozen in liquid nitrogen, and stored at -80°C until extraction date. The frozen cerebral hemisphere is transferred to a homogenizer manufactured by Teflon (registered trade name) under ice cooling, a 5-fold volume of a weight of an extraction buffer (containing 1% CHAPS ({3-[(3-chloroamidopropyl) dimethylammonio]-1-propanesulfonate}), 20 mmol/L M Tris-HCl (pH 8.0), 150 mmol/L NaCl, Complete (Roche) protease inhibitor) is added, up and down movement is repeated, and this is homogenized to solubilize for 2 minutes. The suspension is transferred to a centrifugation tube, allowed to stand on an ice for 3 hours or more and, thereafter centrifuged at 100,000 x g, 4°C for 20 minutes. After centrifugation, the supernatant is transferred to an ELISA plate (product No. 294-62501, Wako Junyaku Kogyo) for measuring β amyloid 40. ELISA measurement is performed according to the attached instruction. The lowering effect is calculated as a ratio compared to the brain β amyloid 40 level of vehicle control group of each test.

### (Test Example 4: CYP3A4 fluorescent MBI test)

The CYP3A4 fluorescent MBI test is a test of investigating enhancement of CYP3A4 inhibition of a compound by a metabolism reaction, and the test was performed using, as CYP3A4 enzyme expressed in *Escherichia coli* and employing, as an index, a reaction in which 7-benzyloxytrifluoromethylchmarin (7-BFC) is debenzylated by the CYP3A4 enzyme to produce a metabolite, 7-hydroxytrifluoromethylchmarin (HFC) emitting fluorescent light.

The reaction conditions were as follows: substrate, 5.6 µmol/L 7-BFC; pre-reaction time, 0 or 30 minutes; reaction time, 15 minutes; reaction temperature, 25°C (room temperature); CYP3A4 content (expressed in *Escherichia coli*), at pre-reaction 62.5 pmol/mL, at reaction 6.25 pmol/mL (at 10-fold dilution test drug concentration, 0.625, 1.25, 2.5, 5, 10, 20 µmol/L (six points).

An enzyme in a K-Pi buffer (pH 7.4) and a test drug solution as a pre-reaction solution were added to a 96-well plate at the composition of the pre-reaction, a part of it was transferred to another 96-well plate so that it was 1/10 diluted by a substrate in a K-Pi buffer, NADPH as a co-factor was added to initiate a reaction as an index (without preincubation) and, after a predetermined time of a reaction, acetonitrile/0.5 mol/L Tris (trishydroxyaminomethane) = 4/1 was added to stop the reaction. In addition, NADPH was added to a remaining preincubation solution to initiate a preincubation (with preincubation) and, after a predetermined time of a preincubation, a part was transferred to another plate so that it was 1/10 diluted with a substrate and a K-Pi buffer to initiate a reaction as an index. After a predetermined time of a reaction, acetonitrile/0.5 mol/L Tris (trishydroxyaminomethane) = 4/1 was added to stop the reaction. For the plate on which each index reaction had been performed, a fluorescent value of 7-HFC which is a metabolite was measured with a fluorescent plate reader. (Ex = 420 nm, Em = 535 nm).

Addition of only DMSO which is a solvent dissolving a drug to a reaction system was adopted as a control (100%), remaining activity (%) was calculated at each concentration of a test drug added as the solution, and IC₅₀ was calculated by reverse-presumption by a logistic model using a concentration and an inhibition rate. When a difference of IC₅₀ value was 5 µM or more, the result was defined as (+). When the difference was 3 µM or less, the result was defined as (-).

### Result

Compound I-44 : (-)

### (Test Example 5: CYP inhibition test)

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenadine hydroxylation (CYP3A4) as typical substrate metabolism reactions of human main five CYP enzyme forms (CYP1A2, 2C9, 2C19, 2D6, 3A4), an inhibitory degree of each metabolite production amount by a test compound was assessed.

The reaction conditions were as follows: substrate, 0.5 µmol/L ethoxyresorufin (CYP1A2), 100 µmol/L tolbutamide (CYP2C9), 50 µmol/L S-mephenytoin (CYP2C19), 5 µmol/L dextromethorphan (CYP2D6), 1 µmol/L terfenadine (CYP3A4); reaction time, 15 minutes; reaction temperature, 37°C; enzyme, pooled human hepatic microsome 0.2 mg protein/mL; test drug concentration, 1, 5, 10, 20 µmol/L (four points).

Each five kinds of substrates, human hepatic microsome, and a test drug in 50 mmol/L Hepes buffer as a reaction solution was added to a 96-well plate at the composition as described above, NADPH, as a cofactor was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (GYP1A2 metabolite) in the supernatant was quantified by a fluorescent multilabel counter and tolbutamide hydroxide (CYP2C9 metabolite), mephenytoin 4' hydroxide (CYP2C19 metabolite), dextrorphan (CYP2D6 metabolite), and terfenadine alcohol (CYP3A4 metabolite) were quantified by LC/MS/MS.

Addition of only DMSO being a solvent dissolving a drug to a reaction system was adopted as a control (100%), remaining activity (%) was calculated at each concentration of a test drug added as the solution and IC₅₀ was calculated by reverse presumption by a logistic model using a concentration and an inhibition rate.

### Result

Compound I-33: IC₅₀ >20 (five kinds)

### (Test Example 6: FAT Test)

Each 20 µL of freeze-stored *Salmonella typhimurium* (TA98 and TA100 strain) is inoculated in 10 mL of liquid nutrient medium (2.5% Oxoid nutrient broth No.2), and the cultures are incubated at 37°C under shaking for 10 hours. 9 mL of TA98 culture is centrifuged (2000 x g, 10 minutes) to remove medium, and the bacteria is suspended in 9 mL of Micro F buffer (K₂HPO₄: 3.5 g/L, KH₂PO₄: 1 g/L, (NH₄)₂SO₄: 1 g/L, trisodium citrate dihydrate: 0.25 g/L, MgSO₄·7H₂O: 0.1 g/L), and the suspension is added to 110 mL of Exposure medium (Micro F buffer containing Biotin: 8 µg/mL, histidine: 0.2 µg/mL, glucose: 8 mg/mL). 3.16 mL of TA100 culture is added to 120 mL of Exposure medium to prepare the test bacterial solution. 588 µL of the test bacterial solution (or mixed solution of 498 µl of the test bacterial solution and 90 µL of the S9 mix in the case with metabolic activation system) are mixed with each 12 µL of the following solution: DMSO solution of the test substance (eight dose levels from maximum dose 50 mg/mL at 2-fold ratio); DMSO as negative control; 50 µg/mL of 4-nitroquinoline-1-oxide DMSO solution as positive control for TA98 without metabolic activation system; 0.25 µg/mL of 2-(2-furyl)-3-(5-nitro-2-furyl)acrylamide DMSO solution as positive control for TA100 without metabolic activation system; 40 µg/mL of 2-aminoanthracene DMSO solution as positive control for TA98 with metabolic activation system; or 20 µg/mL of 2-aminoanthracene DMSO solution as positive control for TA100 with metabolic activation system. 12 µL of the solution and 588 µL of the test bacterial solution (a mixed solution of 498 µl of the test bacterial solution and 90 µL of S9 mix with metabolic activation condition) were mixed and incubated at 37°C under shaking for 90 minutes. 460 µL of the bacterial solution exposed to the test substance is mixed with 2300 µL of Indicator medium (Micro F buffer containing biotin: 8 µg/mL, histidine: 0.2 µg/mL, glucose: 8 mg/mL, Bromo Cresol Purple: 37.5 µg/mL), each 50 µL is dispensed into 48 wells per dose in the microwell plates, and is subjected to stationary cultivation at 37°C for 3 days. A well containing the bacteria, which has obtained the ability of proliferation by mutation in the gene coding amino acid (histidine) synthetase, turns the color from purple to yellow due to pH change. The mutagenicity is evaluated by counting the number of the yellow wells among the 48 total wells per dose and comparing with the negative control group. (-) means that mutagenicity is negative and (+) means positive.

### (Test Example 7: Solubility test)

A 2-fold dilution series (12 points) of a 10 mM solution of a test compound in DMSO was added to a medium (JP-I, JP-II) (2%), and solubility was assessed by 3 stages (High; > 40 µM, Medium; 3-40 µM, Low; < 3 µM) from a turbidity after 4 hours (crystallization information).

### (Test Example 8: Metabolism Stability Test)

Using commercially available pooled human hepatic microsomes, a test compound is reacted for a constant time, a remaining rate is calculated by comparing a reacted sample and an unreacted sample, thereby, a degree of metabolism in liver is assessed.

A reaction is performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 50 µL of the reaction solution is added to 100 µL of a methanol/acetonitrile = 1/1 (v/v), mixed and centrifuged at 3000 rpm for 15 minutes. The test compound in the supernatant is quantified by LC/MS/MS, and a remaining amount of the test compound after the reaction is calculated, letting a compound amount at 0 minute reaction time to be 100%.

### Result

Compound I-42: 94.7%

### (Test Example 9: hERG Test)

For the purpose of assessing risk of an electrocardiogram QT interval prolongation, effects on delayed rectifier K+ current (I_{Kr}), which plays an important role in the ventricular repolarization process of the compound of the present invention, is studied using HEK293 cells expressing human ether-a-go-go related gene (hERG) channel.

After a cell was retained at a membrane potential of -80 mV by whole cell patch clamp method using an automated patch clamp system (PatchXpress 7000A, Axon Instruments Inc.), I_{Kr} induced by depolarization pulse stimulation at +40 mV for 2 seconds and, further, repolarization pulse stimulation at -50 mV for 2 seconds was recorded. After the generated current was stabilized, extracellular solution (NaCl: 135 mmol/L, KCl: 5.4 mmol/L, NaH₂PO₄: 0.3 mmol/L, CaCl₂·2H₂O: 1.8 mmol/L, MgCl₂·6H₂O: 1 mmol/L, glucose: 10 mmol/L, HERPES (4-(2-hydroxyethyl)-1-piperazine ethanesulfonic acid): 10 mmol/L, pH=7.4) in which the test compound had been dissolved at an objective concentration was applied to the cell under the room temperature condition for 10 minutes. From the recording I_{Kr}, an absolute value of the tail peak current was measured based on the current value at the resting membrane potential using analysis software (DataXpress ver.1, Molecular Devices Corporation). Further, the % inhibition relative to the tail peak current before application of the test compound was calculated, and compared with the vehicle-applied group (0.1% dimethyl sulfoxide solution) to assess influence of the test compound on I_{Kr}.

### Result

Compound I-39: 19.3%

### (Test Example 10: Powder solubility test)

Appropriate amounts of the test substances are put into appropriate containers. To the respective containers are added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and water to reach 100 mL). In the case that the test compound is dissolved after the addition of the test fluid, the bulk powder is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. The dilutions are observed for bubbles and precipitates, and then the containers are sealed and shaken. Quantification is performed by HPLC with an absolute calibration method.

### (Test Example 11: BA tests)

Materials and methods for studies on oral absorption
(1) Animal: mice or rats
(2) Breeding conditions: mice or rats are allowed to freely take solid feed and sterilized tap water
(3) Dose and grouping: orally or intravenously administered at a predetermined dose; grouping is as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood is collected over time, and the plasma concentration of drug is measured by LC/MS/MS
(7) Statistical analysis: regarding the transition of the plasma concentration, the area under the plasma concentration-time curve (AUC) is calculated by non-linear least squares program WinNonlin (Registered trade name), and the bioavailability (BA) is calculated from the AUCs of the oral administration group and intravenous administration group

### (Test Example 12: Brain distribution studies)

Intravenous administration is carried out to a rat by 0.5 mg/mL/kg dosage of the compound. 30 minutes later, all blood is drawn from vena cava inferior under isoflurane anesthesia for death from exsanguination. Then, the brain is extracted and 20-25% of homogenate thereof is prepared with distilled water. On the other hand, the obtained blood is used as plasma after centrifuging. Then, to the brain sample is added the control plasma at 1:1. To the plasma samples is added the control brains at 1:1. Each sample is measured using LC/MS/MS. The obtained area ratio (a brain/plasma) is used for the brain Kp value.

### Formulation Example 1

A granule containing the following ingredients is produced.

| | | |
|---|---|---|
| Ingredient | Compound of the formula (I) | 10 mg |
| | Lactose | 700 mg |
| | Corn starch | 274 mg |
| | HPC-L | 16 mg |
| | | 1000 mg |

The compound of the formula (I), and lactose are passed through a 60 mesh sieve. Corn starch is passed through a 120 mesh sieve. These are mixed with a V-type mixer. To the mixed powder is added a HPC-L (low viscosity hydroxypropyl cellulose) aqueous solution, this is kneaded, granulated (extrusion granulation, pore diameter 0.5 to 1 mm), and dried. The resulting dry granule is passed through a vibration sieve (12/60 mesh) to give a granule.

### Formulation Example 2

A granule for filling a capsule containing the following ingredients is produced.

| | | |
|---|---|---|
| Ingredient | Compound of the formula (I) | 15 mg |
| | Lactose | 90 mg |
| | Corn starch | 42 mg |
| | HPC-L | 3 mg |
| | | 150 mg |

The compound of the formula (I), and lactose are passed through a 60 mesh sieve. Corn starch is passed through a 120 mesh sieve. These are mixed, a HPC-L solution is added to the mixed powder, this is kneaded, granulated, and dried. The resulting dry granule is adjusted in a size, and 150 mg of it is filled into a No.4 hard gelatin capsule.

### Formulation Example 3

A tablet containing the following ingredients is produced.

| | | |
|---|---|---|
| Ingredient | Compound of the formula (I) | 10 mg |
| | Lactose | 90 mg |
| | Microcrystalline cellulose | 30 mg |
| | CMC-Na | 15 mg |
| | Magnesium stearate | 5 mg |
| | | 150 mg |

The compound of the formula (I), lactose, microcrystalline cellulose, and CMC-Na (carboxymethyl cellulose sodium salt) are passed through a 60 mesh sieve, and mixed. Magnesium stearate is mixed into the mixed powder to give a mixed powder for tabletting. The present mixed powder is directly compressed to give a 150 mg of a tablet.

### Formulation Example 4

The following ingredients are warmed, mixed, and sterilized to give an injectable.

| | | |
|---|---|---|
| Ingredient | Compound of the formula (I) | 3 mg |
| | Nonionic surfactant | 15 mg |
| | Purified water for injection | 1 ml |

### [Industrial applicability]

The present compound can be a medicament useful as an agent for treating or preventing diseases induced by production, secretion and/or deposition of amyloid β protein.

## Claims

1. A compound of formula (I): 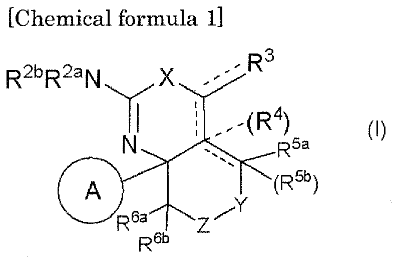
wherein -X- is -O-, -S- or -N(R¹)-,
R¹ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
ring A is a substituted or unsubstituted carbocycle or a substituted or unsubstituted heterocycle, 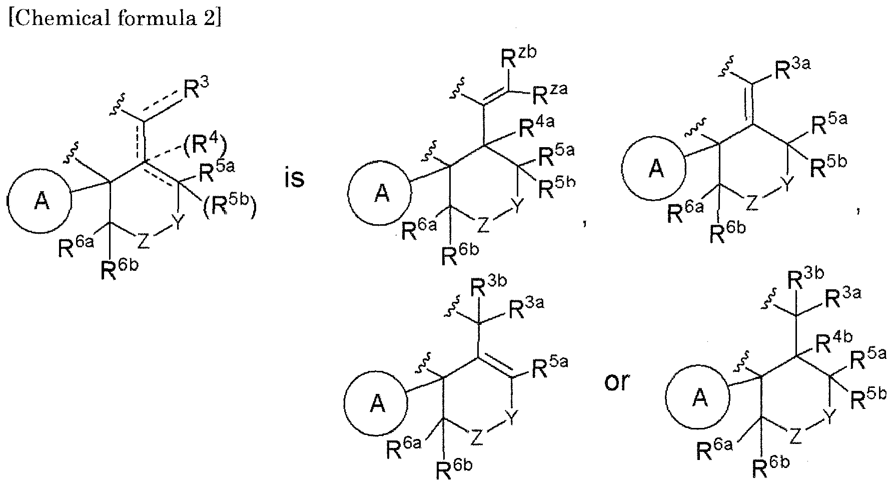
wherein -Y- is a bond, substituted or unsubstituted C1-C3 alkylene, substituted or unsubstituted C2-C3 alkenylene, N(R⁷)- -O-, -S-, -SO- or -SO₂-,
-Z- is a bond, substituted or unsubstituted C1-C3 alkylene, substituted or unsubstituted C2-C3 alkenylene, -N(R⁷)Ak-, -OAk-, -SAk-, -SOAk- or -SO₂Ak-,
Ak is a bond, substituted or unsubstituted C1-C3 alkylene or substituted or unsubstituted C2-C3 alkenylene,
provided that when -Y- is -N(R⁷)-, -O-, -S-, -SO-or -SO₂-, then Ak is not a bond,
R⁷ is hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted acyl, substituted or unsubstituted acyloxy, cyano, carboxy, substituted or unsubstituted alkoxycarbonyl, substituted or unsubstituted alkenyloxycarbonyl, substituted or unsubstituted alkynyloxycarbonyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenyl sulfonyl, substituted or unsubstituted alkynylsulfonyl, substituted or unsubstituted carbamoyl, substituted or unsubstituted thiocarbamoyl, substituted or unsubstituted sulfamoyl, substituted or unsubstituted amino, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R^{z a} and R^{z b} are each independently hydrogen, halogen, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl, or R^{z a} and R^{z b} together with the carbon atom to which they are attached may form a substituted or unsubstituted non-aromatic carbocycle or a substituted or unsubstituted non-aromatic heterocycle,
R^{3a} and R^{3b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl, or R^{3a} and R^{3b} together with the carbon atom to which they are attached may form a substituted or unsubstituted non-aromatic carbocycle or a substituted or unsubstituted non-aromatic heterocycle,
R^{4a} and R^{1b} are each independently hydrogen, halogen, hydroxy, cyano, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl,
provided that when -X- is -S-, then R^{4b} is not hydrogen, and
R^{5a}, R^{5b} , R^{6a} and R^{6b} are each independently hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl, or
R^{5a} and R^{5b} together with the carbon atom to which they are attached may form a substituted or unsubstituted carbocycle or a substituted or unsubstituted heterocycle,
R^{6a} and R^{6b} together with the carbon atom to which they are attached may form a substituted or
unsubstituted carbocycle or a substituted or unsubstituted heterocycle, its pharmaceutically acceptable salt or a solvate thereof.

2. The compound according to claim 1 wherein -X- is -O- or -S-, its pharmaceutically acceptable salt or a solvate thereof.

3. The compound according to claim 1 or 2 wherein 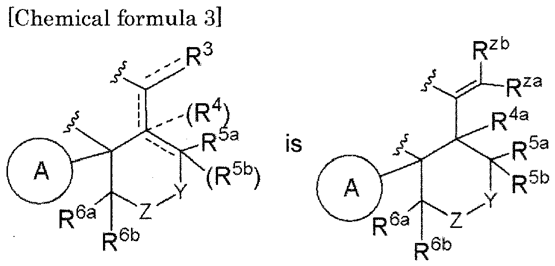
R^{za} and R^{zb} are each independently hydrogen, halogen or substituted or unsubstituted alkyl, R^{4a},
R^{5a} , R^{5b} , R^{6a} and R^{6b} are each independently hydrogen or substituted or unsubstituted alkyl, its pharmaceutically acceptable salt or a solvate thereof.

4. The compound according to any one of claims 1 to 3 wherein -Y- is substituted or unsubstituted C1-C3 alkylene or -O-, and -Z- is a bond,
its pharmaceutically acceptable salt or a solvate thereof.

5. The compound according to any one of claims 1 to 4 wherein ring A is 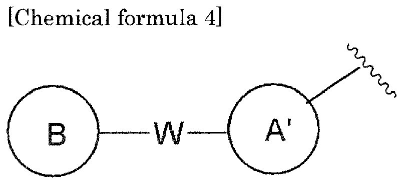
wherein ring A' and ring B are each independently a substituted or unsubstituted carbocycle or a substituted or unsubstituted heterocycle,
-W- is a bond, -L¹ -C(=O)N(R⁸)-L² -, -L¹ -N(R⁸)C(=O)-L² -or -L¹-N(R⁸)-L²-,
L¹ and L² are each independently a bond, substituted or unsubstituted alkylene, substituted or unsubstituted alkenylene or substituted or unsubstituted alkynylene,
R⁸ is hydrogen, substituted or unsubstituted alkyl, substituted or unsubstituted alkenyl, substituted or unsubstituted alkynyl or substituted or unsubstituted acyl,
its pharmaceutically acceptable salt or a solvate thereof.

6. The compound according to claim 5 wherein -W- is -C(=O)N(R⁸)-, its pharmaceutically acceptable salt or a solvate thereof.

7. The compound according to claim 5 or 6 wherein ring A' is substituted or unsubstituted benzene, ring B is substituted or unsubstituted pyridine, substituted or unsubstituted pyrimidine or substituted or unsubstituted pyrazine, its pharmaceutically acceptable salt or a solvate thereof.

8. A pharmaceutical composition comprising the compound according to any one of claims 1 to 7, its pharmaceutically acceptable salt or a solvate thereof as an active ingredient.

9. A pharmaceutical composition having BACE 1 inhibitory activity comprising the compound according to any one of claims 1 to 7, its pharmaceutically acceptable salt or a solvate thereof as an active ingredient.

10. The pharmaceutical composition according to claim 8 or 9, which is a medicament for treating or preventing the diseases induced by production, secretion or deposition of amyloid-β proteins.

11. The pharmaceutical composition according to claim 8 or 9, which is a medicament for treating or preventing Alzheimer's disease.

12. A method for inhibiting BACE1 activity comprising administering the compound according to any one of claims 1 to 7, its pharmaceutically acceptable salt or a solvate thereof.

13. The compound according to any one of claims 1 to 7, its pharmaceutically acceptable salt or a solvate thereof for use in a method for inhibiting BACE1 activity.

14. A method for treating or preventing diseases induced by production, secretion or deposition of amyloid-β proteins comprising administering the compound according to any one of claims 1 to 7, its pharmaceutically acceptable salt or a solvate thereof.

15. The compound according to any one of claims 1 to 7, its pharmaceutically acceptable salt or a solvate thereof for use in a method for treating or preventing diseases induced by production, secretion or deposition of amyloid-β proteins.

16. A method for treating or preventing Alzheimer's disease comprising administering the compound according to any one of claims 1 to 7, its pharmaceutically acceptable salt or a solvate thereof.

17. The compound according to any one of claims 1 to 7, its pharmaceutically acceptable salt or a solvate thereof for use in a method for treating or preventing Alzheimer's disease.
